# EUROPEAN PATENT APPLICATION

(11) **EP 4 696 711 A1**
(43) Date of publication of application: **18.02.2026**
(21) Application number: 24788203.8
(22) Date of filing: 12.04.2024
(51) Int. Cl.: C07K 16/28, C07K 16/30, A61P 35/00, A61K 47/68

(54) **ANTIGEN BINDING MOLECULE SPECIFICALLY BINDING TO EGFR AND MUC1, DRUG CONJUGATE THEREOF, AND MEDICAL USE THEREOF**

(30) Priority: 13.04.2023 CN 202310394160; 21.12.2023 CN 202311772280
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: LIU, Ziheng, Shanghai 200245 (CN); HU, Bing, Shanghai 200245 (CN); SUN, Le, Shanghai 200245 (CN); LIU, Shimeng, Shanghai 200245 (CN); GAO, Xinyu, Shanghai 200245 (CN); LI, Xun, Shanghai 200245 (CN); CAO, Liqun, Shanghai 200245 (CN); YE, Xin, Shanghai 200245 (CN); HE, Feng, Shanghai 200245 (CN)
(74) Representative: Regimbeau
(86) International application number: PCT/CN2024/087447
(87) International publication number: WO 2024/213106

(57) **Abstract**

The present invention relates to an antigen binding molecule specifically binding to EGFR and MUC1, a drug conjugate thereof, and a medical use thereof. Specifically, the present invention relates to an anti-MUC1 antibody or an antigen binding fragment thereof, an anti-EGFR antibody or an antigen binding fragment thereof, an antigen binding molecule specifically binding to EGFR and MUC1, a drug conjugate thereof, and a medical use thereof.

## Description

The present disclosure claims priority to CN202310394160.8, which was filed on Apr. 13, 2023, and CN202311772280.3, which was filed on Dec. 21, 2023.

### TECHNICAL FIELD

The present disclosure pertains to the technical field of biology and relates to an anti-MUC1 antibody or an antigen-binding fragment thereof, an anti-EGFR antibody or an antigen-binding fragment thereof, and an antigen-binding molecule that specifically binds to EGFR and MUC1, as well as drug conjugates thereof and pharmaceutical use thereof.

### BACKGROUND

The statements herein merely provide background information related to the present disclosure and may not necessarily constitute the prior art.

MUC1 is a transmembrane glycoprotein with abundant glycosylation, and its extracellular region is a dimer formed from two chains through hydrogen bonding, which are MUC1-N and MUC1-C. MUC1-N has abundant O-glycosylation and a small amount of N-glycosylation, and its amino acid backbone consists of multiple repeats of VNTR. MUC1-C contains an extracellular domain, a transmembrane domain, and an intracellular domain.

In normal tissues, MUC1 is present in full-length form at the apical end of epithelial cells, while EGFR is present at the basal end of epithelial cells. As tumor cells lack apical-basal polarity, EGFR and MUC1 are uniformly distributed on the cell surface, such that they are spatially close to each other. Moreover, the O-glycosylation of MUC1-N on tumor cells will become significantly sparse. As tumors progress, MUC 1-N is shed under the catalytic effects of inflammatory factor-related enzymes in the tumor microenvironment, such that MUC1-C is exposed.

MUC1-N can be shed into the blood, causing MUC1-N-targeting antibodies to show off-target binding. This was the main reason for the poor clinical efficacy of MUC1-N-targeting antibodies. As for MUC1-C-targeting antibodies, there are very limited data. The present disclosure provides an MUC1-C-targeting antibody, which has no off-target effect and can be used as a beneficial complement to MUC1-C antibodies. As EGFR is also expressed at certain levels in normal tissues, EGFR monoclonal antibodies cause significant safety concerns in clinical use, which limits their efficacy. The EGFR-MUC1 bispecific antibody of the present disclosure preferentially targets tumor cells with double expression, showing less binding to normal tissues. In addition, the conjugation to a small-molecule toxin can reduce the side effects and improve its efficacy. Moreover, the EGFR-MUC1 bispecific antibody demonstrates a wider range of indications and covers more patients than EGFR monoclonal antibodies or MUC1 monoclonal antibodies.

### SUMMARY

The present disclosure provides an anti-MUC1 antibody or an antigen-binding fragment thereof that specifically binds to MUC1-C of human MUC1 and does not bind to MUC1-N.

The present disclosure provides an anti-MUC1 antibody or an antigen-binding fragment thereof that comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. the HCDR1, HCDR2, and HCDR3 of the heavy chain variable region comprise the amino acid sequences of a HCDR1, a HCDR2, and a HCDR3 in SEQ ID NO: 4, respectively, and the LCDR1, LCDR2, and LCDR3 of the light chain variable region comprise the amino acid sequences of a LCDR1, a LCDR2, and a LCDR3 in SEQ ID NO: 5, respectively; or
b. the HCDR1, HCDR2, and HCDR3 of the heavy chain variable region comprise the amino acid sequences of a HCDR1, a HCDR2, and a HCDR3 of any one of SEQ ID NO: 6 or 47, respectively, and the LCDR1, LCDR2, and LCDR3 of the light chain variable region comprise the amino acid sequences of a LCDR1, a LCDR2, and a LCDR3 in SEQ ID NO: 7, respectively; or
c. the HCDR1, HCDR2, and HCDR3 of the heavy chain variable region comprise the amino acid sequences of a HCDR1, a HCDR2, and a HCDR3 in SEQ ID NO: 8, respectively, and the LCDR1, LCDR2, and LCDR3 of the light chain variable region comprise the amino acid sequences of a LCDR1, a LCDR2, and a LCDR3 in SEQ ID NO: 9, respectively; or
d. the HCDR1, HCDR2, and HCDR3 of the heavy chain variable region comprise the amino acid sequences of a HCDR1, a HCDR2, and a HCDR3 of any one of SEQ ID NO: 63, 10, or 64, respectively, and the LCDR1, LCDR2, and LCDR3 of the light chain variable region comprise the amino acid sequences of a LCDR1, a LCDR2, and a LCDR3 in SEQ ID NO: 11, respectively.

In some embodiments, provided is the anti-MUC1 antibody or the antigen-binding fragment thereof according to the foregoing, wherein:
a. the HCDR1, HCDR2, and HCDR3 of the heavy chain variable region comprise the amino acid sequences of a HCDR1, a HCDR2, and a HCDR3 in SEQ ID NO: 4, respectively, and the LCDR1, LCDR2, and LCDR3 of the light chain variable region comprise the amino acid sequences of a LCDR1, a LCDR2, and a LCDR3 in SEQ ID NO: 5, respectively; or
b. the HCDR1, HCDR2, and HCDR3 of the heavy chain variable region comprise the amino acid sequences of a HCDR1, a HCDR2, and a HCDR3 in SEQ ID NO: 6, respectively, and the LCDR1, LCDR2, and LCDR3 of the light chain variable region comprise the amino acid sequences of a LCDR1, a LCDR2, and a LCDR3 in SEQ ID NO: 7, respectively; or
c. the HCDR1, HCDR2, and HCDR3 of the heavy chain variable region comprise the amino acid sequences of a HCDR1, a HCDR2, and a HCDR3 in SEQ ID NO: 8, respectively, and the LCDR1, LCDR2, and LCDR3 of the light chain variable region comprise the amino acid sequences of a LCDR1, a LCDR2, and a LCDR3 in SEQ ID NO: 9, respectively; or
d. the HCDR1, HCDR2, and HCDR3 of the heavy chain variable region comprise the amino acid sequences of a HCDR1, a HCDR2, and a HCDR3 in SEQ ID NO: 63, respectively, and the LCDR1, LCDR2, and LCDR3 of the light chain variable region comprise the amino acid sequences of a LCDR1, a LCDR2, and a LCDR3 in SEQ ID NO: 11, respectively.

In some embodiments, provided is the anti-MUC1 antibody or the antigen-binding fragment thereof according to any one of the foregoing, wherein:
the HCDR1, HCDR2, and HCDR3 of the heavy chain variable region comprise the amino acid sequences of a HCDR1, a HCDR2, and a HCDR3 in SEQ ID NO: 47, respectively, and the LCDR1, LCDR2, and LCDR3 of the light chain variable region comprise the amino acid sequences of a LCDR1, a LCDR2, and a LCDR3 in SEQ ID NO: 7, respectively.

In some embodiments, provided is the anti-MUC1 antibody or the antigen-binding fragment thereof according to any one of the foregoing, wherein:
the HCDR1, HCDR2, and HCDR3 of the heavy chain variable region comprise the amino acid sequences of a HCDR1, a HCDR2, and a HCDR3 in SEQ ID NO: 10, respectively, and the LCDR1, LCDR2, and LCDR3 of the light chain variable region comprise the amino acid sequences of a LCDR1, a LCDR2, and a LCDR3 in SEQ ID NO: 11, respectively.

In some embodiments, provided is the anti-MUC1 antibody or the antigen-binding fragment thereof according to any one of the foregoing, wherein:
the HCDR1, HCDR2, and HCDR3 of the heavy chain variable region comprise the amino acid sequences of a HCDR1, a HCDR2, and a HCDR3 in SEQ ID NO: 64, respectively, and the LCDR1, LCDR2, and LCDR3 of the light chain variable region comprise the amino acid sequences of a LCDR1, a LCDR2, and a LCDR3 in SEQ ID NO: 11, respectively.

In some embodiments, provided is the anti-MUC1 antibody or the antigen-binding fragment thereof according to any one of the foregoing, wherein the HCDR1, HCDR2, and HCDR3 of the heavy chain variable region and the LCDR1, LCDR2, and LCDR3 of the light chain variable region are defined according to the same numbering scheme selected from the group consisting of Kabat, IMGT, Chothia, AbM, and Contact.

In some embodiments, the HCDR1, HCDR2, and HCDR3 of the heavy chain variable region and the LCDR1, LCDR2, and LCDR3 of the light chain variable region are defined according to the Kabat numbering scheme. In some embodiments, the HCDR1, HCDR2, and HCDR3 of the heavy chain variable region and the LCDR1, LCDR2, and LCDR3 of the light chain variable region are defined according to the IMGT numbering scheme. In some embodiments, the HCDR1, HCDR2, and HCDR3 of the heavy chain variable region and the LCDR1, LCDR2, and LCDR3 of the light chain variable region are defined according to the Chothia numbering scheme. In some embodiments, the HCDR1, HCDR2, and HCDR3 of the heavy chain variable region and the LCDR1, LCDR2, and LCDR3 of the light chain variable region are defined according to the AbM numbering scheme. In some embodiments, the HCDR1, HCDR2, and HCDR3 of the heavy chain variable region and the LCDR1, LCDR2, and LCDR3 of the light chain variable region are defined according to the Contact numbering scheme.

In some embodiments, provided is the anti-MUC1 antibody or the antigen-binding fragment thereof according to any one of the foregoing, wherein:
a. in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17; or
b. in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of any one of SEQ ID NO: 20 or 113; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23; or
c. in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29; or
d. in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of any one of SEQ ID NO: 114, 32, or 115; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35.

In some embodiments, provided is the anti-MUC1 antibody or the antigen-binding fragment thereof according to any one of the foregoing, wherein:
a. in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17;
b. in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of SEQ ID NO: 20; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23; or
c. in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29; or
d. in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 114; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35.

In some embodiments, provided is the anti-MUC1 antibody or the antigen-binding fragment thereof according to any one of the foregoing, wherein in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of SEQ ID NO: 113; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23.

In some embodiments, provided is the anti-MUC1 antibody or the antigen-binding fragment thereof according to any one of the foregoing, wherein in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 115; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35.

In some embodiments, provided is the anti-MUC1 antibody or the antigen-binding fragment thereof according to any one of the foregoing, wherein in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 32; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35.

In some embodiments, provided is the anti-MUC1 antibody or the antigen-binding fragment thereof according to any one of the foregoing, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17.

In some embodiments, the anti-MUC1 antibody or the antigen-binding fragment thereof according to any one of the foregoing is a murine antibody, a chimeric antibody, a humanized antibody, or a fully human-derived antibody. In some embodiments, the anti-MUC 1 antibody or the antigen-binding fragment thereof is a chimeric antibody or a humanized antibody. In some embodiments, the anti-MUC1 antibody or the antigen-binding fragment thereof is a humanized antibody.

In some embodiments, the anti-MUC1 antibody or the antigen-binding fragment thereof according to any one of the foregoing comprises human antibody framework regions (FRs).

In some embodiments, provided is the anti-MUC1 antibody or the antigen-binding fragment thereof according to any one of the foregoing, wherein:
a. the heavy chain variable region comprises an amino acid sequence having at least 70% (e.g., at least 70%, 80%, 85%, 90%, 95%, 98%, or 99%) sequence identity to SEQ ID NO: 36, 37, or 38, and the light chain variable region comprises an amino acid sequence having at least 70% (e.g., at least 70%, 80%, 85%, 90%, 95%, 98%, or 99%) sequence identity to SEQ ID NO: 39, 40, 41, or 42; or
   the heavy chain variable region comprises an amino acid sequence having at least 70% (e.g., at least 70%, 80%, 85%, 90%, 95%, 98%, or 99%) sequence identity to SEQ ID NO: 4, and the light chain variable region comprises an amino acid sequence having at least 70% (e.g., at least 70%, 80%, 85%, 90%, 95%, 98%, or 99%) sequence identity to SEQ ID NO: 5; or
b. the heavy chain variable region comprises an amino acid sequence having at least 70% (e.g., at least 70%, 80%, 85%, 90%, 95%, 98%, or 99%) sequence identity to SEQ ID NO: 43, 44, 45, 46, 47, 48, or 49, and the light chain variable region comprises an amino acid sequence having at least 70% (e.g., at least 70%, 80%, 85%, 90%, 95%, 98%, or 99%) sequence identity to SEQ ID NO: 50, 51, or 52; or
   the heavy chain variable region comprises an amino acid sequence having at least 70% (e.g., at least 70%, 80%, 85%, 90%, 95%, 98%, or 99%) sequence identity to SEQ ID NO: 6, and the light chain variable region comprises an amino acid sequence having at least 70% (e.g., at least 70%, 80%, 85%, 90%, 95%, 98%, or 99%) sequence identity to SEQ ID NO: 7; or
c. the heavy chain variable region comprises an amino acid sequence having at least 70% (e.g., at least 70%, 80%, 85%, 90%, 95%, 98%, or 99%) sequence identity to SEQ ID NO: 53, 54, or 55, and the light chain variable region comprises an amino acid sequence having at least 70% (e.g., at least 70%, 80%, 85%, 90%, 95%, 98%, or 99%) sequence identity to SEQ ID NO: 56, 57, 58, or 59; or
   the heavy chain variable region comprises an amino acid sequence having at least 70% (e.g., at least 70%, 80%, 85%, 90%, 95%, 98%, or 99%) sequence identity to SEQ ID NO: 8, and the light chain variable region comprises an amino acid sequence having at least 70% (e.g., at least 70%, 80%, 85%, 90%, 95%, 98%, or 99%) sequence identity to SEQ ID NO: 9; or
d. the heavy chain variable region comprises an amino acid sequence having at least 70% (e.g., at least 70%, 80%, 85%, 90%, 95%, 98%, or 99%) sequence identity to SEQ ID NO: 63, 60, 61, 62, or 64, and the light chain variable region comprises an amino acid sequence having at least 70% (e.g., at least 70%, 80%, 85%, 90%, 95%, 98%, or 99%) sequence identity to SEQ ID NO: 67, 68, 65, or 66; or
   the heavy chain variable region comprises an amino acid sequence having at least 70% (e.g., at least 70%, 80%, 85%, 90%, 95%, 98%, or 99%) sequence identity to SEQ ID NO: 10, and the light chain variable region comprises an amino acid sequence having at least 70% (e.g., at least 70%, 80%, 85%, 90%, 95%, 98%, or 99%) sequence identity to SEQ ID NO: 11.

In some embodiments, provided is the anti-MUC1 antibody or the antigen-binding fragment thereof according to any one of the foregoing, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 28S, 38K, 40R, 48I, 71A, 73K, 76D, and 82aR; and/or the light chain variable region comprises a FR1, a FR2, and a FR3 that are derived from IGKV1-39*01, 1GKV6-21*02, or IGKV3-11*01 and a FR4 derived from IGKJ4*01, and the FRs are unsubstituted or comprise one or more amino acid substitutions selected from the group consisting of 3V, 43S, 47W, 49Y, and 60G. In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise one or more amino acid substitutions selected from the group consisting of 1E, 28S, 38K, 40R, 48I, 71A, 73K, 76D, and 82aR; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise one or more amino acid substitutions selected from the group consisting of 3V, 43S, 47W, 49Y, and 60G. In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise one or more amino acid substitutions selected from the group consisting of 1E, 28S, 38K, 40R, 48I, 71A, 73K, 76D, and 82aR; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise one or more amino acid substitutions selected from the group consisting of 3V, 43S, and 47W. In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise amino acid substitutions of 1E, 71A, 73K, and 76D; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise amino acid substitutions of 43S and 47W. In some embodiments, the variable regions and CDRs described above are defined according to the Kabat numbering scheme.

In some embodiments, provided is the anti-MUC1 antibody or the antigen-binding fragment thereof according to any one of the foregoing, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 28R, 30I, 39E, 40R, 43H, 69F, 71A, 76N, 82bQ, 83T, and 84N; and/or the light chain variable region comprises a FR1, a FR2, and a FR3 that are derived from IGKV4-1*01 or IGKV3-11*01 and a FR4 derived from IGKJ4*01, and the FRs are unsubstituted or comprise one or more amino acid substitutions selected from the group consisting of 1D, 4M, 45K, 68R, and 83V. In some embodiments, provided is the anti-MUC1 antibody or the antigen-binding fragment thereof, wherein in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of SEQ ID NO: 20 or 113, and the FRs of the heavy chain variable region comprise one or more amino acid substitutions selected from the group consisting of 1E, 28R, 30I, 39E, 40R, 43H, 69F, 71A, 76N, 82bQ, 83T, and 84N; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23, and the FRs of the light chain variable region comprise one or more amino acid substitutions selected from the group consisting of 1D, 4M, 45K, 68R, and 83V. In some embodiments, the variable regions and CDRs described above are defined according to the Kabat numbering scheme.

In some embodiments, provided is the anti-MUC1 antibody or the antigen-binding fragment thereof according to any one of the foregoing, wherein the heavy chain variable region comprises a FR1, a FR2, and a FR3 that are derived from IGHV1-3*01 and a FR4 derived from IGHJ6*01, and the FRs are unsubstituted or comprise one or more amino acid substitutions selected from the group consisting of 1E, 2I, 12V, 40R, 44G, 47Y, 48I, 69L, 71V, and 76R; and/or the light chain variable region comprises a FR1, a FR2, and a FR3 that are derived from IGKV1-39*01 and a FR4 derived from IGKJ4*01, and the FRs are unsubstituted or comprise one or more amino acid substitutions selected from the group consisting of 4L, 36F, 42T, 43S, 47W, 60P, 70S, and 75V. In some embodiments, provided is the anti-MUC1 antibody or the antigen-binding fragment thereof, wherein in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26, and the FRs of the heavy chain variable region comprise one or more amino acid substitutions selected from the group consisting of 1E, 2I, 12V, 40R, 44G, 47Y, 48I, 69L, 71V, and 76R; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29, and the FRs of the light chain variable region comprise one or more amino acid substitutions selected from the group consisting of 4L, 36F, 42T, 43S, 47W, 60P, 70S, and 75V. In some embodiments, the variable regions and CDRs described above are defined according to the Kabat numbering scheme.

In some embodiments, provided is the anti-MUC1 antibody or the antigen-binding fragment thereof according to any one of the foregoing, wherein the heavy chain variable region comprises a FR1, a FR2, and a FR3 that are derived from IGHV1-3*01 and a FR4 derived from IGHJ1*01, and the FRs are unsubstituted or comprise one or more amino acid substitutions selected from the group consisting of 12V, 20M, 24T, 40R, 44G, 48I, 69L, and 71S; and/or the light chain variable region comprises a FR1, a FR2, and a FR3 that are derived from IGKV1-39*01 and a FR4 derived from IGKJ4*01, and the FRs are unsubstituted or comprise one or more amino acid substitutions selected from the group consisting of 4L, 36L, 39E, 42G, 44I, 46R, 60K, 66R, 69S, and 71Y. In some embodiments, provided is the anti-MUC1 antibody or the antigen-binding fragment thereof, wherein in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 114, 32, or 115, and the FRs of the heavy chain variable region comprise one or more amino acid substitutions selected from the group consisting of 12V, 20M, 24T, 40R, 44G, 48I, 69L, and 71S; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35, and the FRs of the light chain variable region comprise one or more amino acid substitutions selected from the group consisting of 4L, 36L, 39E, 42G, 44I, 46R, 60K, 66R, 69S, and 71Y. In some embodiments, provided is the anti-MUC1 antibody or the antigen-binding fragment thereof, wherein in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 114, and the FRs of the heavy chain variable region comprise one or more amino acid substitutions selected from the group consisting of 12V, 20M, 24T, 40R, 44G, 48I, 69L, and 71S; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35, and the FRs of the light chain variable region comprise one or more amino acid substitutions selected from the group consisting of 4L, 36L, 39E, 42G, 44I, 46R, 60K, 66R, 69S, and 71Y. In some embodiments, provided is the anti-MUC1 antibody or the antigen-binding fragment thereof, wherein in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 115, and the FRs of the heavy chain variable region comprise one or more amino acid substitutions selected from the group consisting of 12V, 20M, 24T, 40R, 44G, 48I, 69L, and 71S; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35, and the FRs of the light chain variable region comprise one or more amino acid substitutions selected from the group consisting of 4L, 36L, 39E, 42G, 44I, 46R, 60K, 66R, 69S, and 71Y. In some embodiments, the variable regions and CDRs described above are defined according to the Kabat numbering scheme.

In some embodiments, provided is the anti-MUC1 antibody or the antigen-binding fragment thereof according to any one of the foregoing, wherein:
a. the heavy chain variable region comprises the amino acid sequence of SEQ ID NO: 36, and the light chain variable region comprises the amino acid sequence of SEQ ID NO: 39 or 40; or
   the heavy chain variable region comprises the amino acid sequence of SEQ ID NO: 37, and the light chain variable region comprises the amino acid sequence of SEQ ID NO: 39 or 40; or
   the heavy chain variable region comprises the amino acid sequence of SEQ ID NO: 4, and the light chain variable region comprises the amino acid sequence of SEQ ID NO: 5; or
b. the heavy chain variable region comprises the amino acid sequence of SEQ ID NO: 43, and the light chain variable region comprises the amino acid sequence of SEQ ID NO: 50; or
   the heavy chain variable region comprises the amino acid sequence of SEQ ID NO: 44, and the light chain variable region comprises the amino acid sequence of SEQ ID NO: 51; or
   the heavy chain variable region comprises the amino acid sequence of SEQ ID NO: 45, and the light chain variable region comprises the amino acid sequence of SEQ ID NO: 50 or 51; or
   the heavy chain variable region comprises the amino acid sequence of SEQ ID NO: 46, and the light chain variable region comprises the amino acid sequence of SEQ ID NO: 51; or
   the heavy chain variable region comprises the amino acid sequence of SEQ ID NO: 6, and the light chain variable region comprises the amino acid sequence of SEQ ID NO: 7; or
c. the heavy chain variable region comprises the amino acid sequence of SEQ ID NO: 53, and the light chain variable region comprises the amino acid sequence of SEQ ID NO: 56, 57, or 59; or
   the heavy chain variable region comprises the amino acid sequence of SEQ ID NO: 54, and the light chain variable region comprises the amino acid sequence of SEQ ID NO: 57; or
   the heavy chain variable region comprises the amino acid sequence of SEQ ID NO: 8, and the light chain variable region comprises the amino acid sequence of SEQ ID NO: 9; or
d. the heavy chain variable region comprises the amino acid sequence of SEQ ID NO: 60, and the light chain variable region comprises the amino acid sequence of SEQ ID NO: 65 or 68; or
   the heavy chain variable region comprises the amino acid sequence of SEQ ID NO: 61, and the light chain variable region comprises the amino acid sequence of SEQ ID NO: 65, 66, 67, or 68; or
   the heavy chain variable region comprises the amino acid sequence of SEQ ID NO: 63, and the light chain variable region comprises the amino acid sequence of SEQ ID NO: 67 or 68; or
   the heavy chain variable region comprises the amino acid sequence of SEQ ID NO: 10, and the light chain variable region comprises the amino acid sequence of SEQ ID NO: 11.

In some embodiments, provided is the anti-MUC1 antibody or the antigen-binding fragment thereof according to any one of the foregoing, wherein:
the heavy chain variable region comprises the amino acid sequence of SEQ ID NO: 36, and the light chain variable region comprises the amino acid sequence of SEQ ID NO: 39; or
the heavy chain variable region comprises the amino acid sequence of SEQ ID NO: 36, and the light chain variable region comprises the amino acid sequence of SEQ ID NO: 40; or
the heavy chain variable region comprises the amino acid sequence of SEQ ID NO: 37, and the light chain variable region comprises the amino acid sequence of SEQ ID NO: 39; or
the heavy chain variable region comprises the amino acid sequence of SEQ ID NO: 37, and the light chain variable region comprises the amino acid sequence of SEQ ID NO: 40; or
the heavy chain variable region comprises the amino acid sequence of SEQ ID NO: 4, and the light chain variable region comprises the amino acid sequence of SEQ ID NO: 5.

In some embodiments, provided is the anti-MUC1 antibody or the antigen-binding fragment thereof according to any one of the foregoing, wherein the heavy chain variable region comprises the amino acid sequence of SEQ ID NO: 36, and the light chain variable region comprises the amino acid sequence of SEQ ID NO: 39.

In some embodiments, provided is the anti-MUC1 antibody or the antigen-binding fragment thereof according to any one of the foregoing, wherein:
a. the amino acid sequence of the heavy chain variable region is set forth in SEQ ID NO: 36, and the amino acid sequence of the light chain variable region is set forth in SEQ ID NO: 39 or 40; or
   the amino acid sequence of the heavy chain variable region is set forth in SEQ ID NO: 37, and the amino acid sequence of the light chain variable region is set forth in SEQ ID NO: 39 or 40; or
   the amino acid sequence of the heavy chain variable region is set forth in SEQ ID NO: 4, and the amino acid sequence of the light chain variable region is set forth in SEQ ID NO: 5; or
b. the amino acid sequence of the heavy chain variable region is set forth in SEQ ID NO: 43, and the amino acid sequence of the light chain variable region is set forth in SEQ ID NO: 50; or
   the amino acid sequence of the heavy chain variable region is set forth in SEQ ID NO: 44, and the amino acid sequence of the light chain variable region is set forth in SEQ ID NO: 51; or
   the amino acid sequence of the heavy chain variable region is set forth in SEQ ID NO: 45, and the amino acid sequence of the light chain variable region is set forth in SEQ ID NO: 50 or 51; or
   the amino acid sequence of the heavy chain variable region is set forth in SEQ ID NO: 46, and the amino acid sequence of the light chain variable region is set forth in SEQ ID NO: 51; or
   the amino acid sequence of the heavy chain variable region is set forth in SEQ ID NO: 6, and the amino acid sequence of the light chain variable region is set forth in SEQ ID NO: 7; or
c. the amino acid sequence of the heavy chain variable region is set forth in SEQ ID NO: 53, and the amino acid sequence of the light chain variable region is set forth in SEQ ID NO: 56, 57, or 59; or
   the amino acid sequence of the heavy chain variable region is set forth in SEQ ID NO: 54, and the amino acid sequence of the light chain variable region is set forth in SEQ ID NO: 57; or
   the amino acid sequence of the heavy chain variable region is set forth in SEQ ID NO: 8, and the amino acid sequence of the light chain variable region is set forth in SEQ ID NO: 9; or
d. the amino acid sequence of the heavy chain variable region is set forth in SEQ ID NO: 60, and the amino acid sequence of the light chain variable region is set forth in SEQ ID NO: 65 or 68; or
   the amino acid sequence of the heavy chain variable region is set forth in SEQ ID NO: 61, and the amino acid sequence of the light chain variable region is set forth in SEQ ID NO: 65, 66, 67, or 68; or
   the amino acid sequence of the heavy chain variable region is set forth in SEQ ID NO: 63, and the amino acid sequence of the light chain variable region is set forth in SEQ ID NO: 67 or 68; or
   the amino acid sequence of the heavy chain variable region is set forth in SEQ ID NO: 10, and the amino acid sequence of the light chain variable region is set forth in SEQ ID NO: 11.

In some embodiments, provided is the anti-MUC1 antibody or the antigen-binding fragment thereof according to any one of the foregoing, wherein:
the amino acid sequence of the heavy chain variable region is set forth in SEQ ID NO: 36, and the amino acid sequence of the light chain variable region is set forth in SEQ ID NO: 39; or
the amino acid sequence of the heavy chain variable region is set forth in SEQ ID NO: 36, and the amino acid sequence of the light chain variable region is set forth in SEQ ID NO: 40; or
the amino acid sequence of the heavy chain variable region is set forth in SEQ ID NO: 37, and the amino acid sequence of the light chain variable region is set forth in SEQ ID NO: 39; or
the amino acid sequence of the heavy chain variable region is set forth in SEQ ID NO: 37, and the amino acid sequence of the light chain variable region is set forth in SEQ ID NO: 40; or
the amino acid sequence of the heavy chain variable region is set forth in SEQ ID NO: 4, and the amino acid sequence of the light chain variable region is set forth in SEQ ID NO: 5.

In some embodiments, provided is the anti-MUC1 antibody or the antigen-binding fragment thereof according to any one of the foregoing, wherein the amino acid sequence of the heavy chain variable region is set forth in SEQ ID NO: 36, and the amino acid sequence of the light chain variable region is set forth in SEQ ID NO: 39.

In some embodiments, the anti-MUC1 antibody or the antigen-binding fragment thereof according to any one of the foregoing is an antibody fragment; in some embodiments, the antibody fragment is selected from the group consisting of Fab, Fab', F(ab')2, Fd, Fv, scFv, dsFv, and dAb.

In some embodiments, the anti-MUC1 antibody or the antigen-binding fragment thereof according to any one of the foregoing comprises a heavy chain constant region and a light chain constant region. In some embodiments, the heavy chain constant region is a human IgG1, IgG2, IgG3, or IgG4 heavy chain constant region. In some embodiments, the light chain constant region is a human κ or λ light chain constant region.

In some embodiments, the heavy chain constant region comprises the amino acid sequence of SEQ ID NO: 69 or 186, and the light chain constant region comprises the amino acid sequence of SEQ ID NO: 70.

In some embodiments, the heavy chain constant region comprises the amino acid sequence of SEQ ID NO: 69, and the light chain constant region comprises the amino acid sequence of SEQ ID NO: 70.

In some embodiments, the anti-MUC1 antibody or the antigen-binding fragment thereof according to any one of the foregoing comprises a heavy chain and a light chain, wherein:
a. the heavy chain comprises an amino acid sequence having at least 70% (e.g., at least 70%, 80%, 85%, 90%, 95%, 98%, or 99%) sequence identity to SEQ ID NO: 71, 73, 75, or 77, and the light chain comprises an amino acid sequence having at least 70% (e.g., at least 70%, 80%, 85%, 90%, 95%, 98%, or 99%) sequence identity to SEQ ID NO: 72, 74, 76, or 78; or
b. the heavy chain comprises an amino acid sequence having at least 70% (e.g., at least 70%, 80%, 85%, 90%, 95%, 98%, or 99%) sequence identity to SEQ ID NO: 79, 81, 83, 85, or 87, and the light chain comprises an amino acid sequence having at least 70% (e.g., at least 70%, 80%, 85%, 90%, 95%, 98%, or 99%) sequence identity to SEQ ID NO: 80, 82, 84, 86, or 88; or
c. the heavy chain comprises an amino acid sequence having at least 70% (e.g., at least 70%, 80%, 85%, 90%, 95%, 98%, or 99%) sequence identity to SEQ ID NO: 89, 91, 93, or 95, and the light chain comprises an amino acid sequence having at least 70% (e.g., at least 70%, 80%, 85%, 90%, 95%, 98%, or 99%) sequence identity to SEQ ID NO: 90, 92, 94, or 96; or
d. the heavy chain comprises an amino acid sequence having at least 70% (e.g., at least 70%, 80%, 85%, 90%, 95%, 98%, or 99%) sequence identity to SEQ ID NO: 97, 99, 101, 103, 105, 107, 109, or 111, and the light chain comprises an amino acid sequence having at least 70% (e.g., at least 70%, 80%, 85%, 90%, 95%, 98%, or 99%) sequence identity to SEQ ID NO: 98, 100, 102, 104, 106, 108, 110, or 112.

In some embodiments, the anti-MUC1 antibody or the antigen-binding fragment thereof according to any one of the foregoing comprises a heavy chain and a light chain, wherein:
a. the amino acid sequence of the heavy chain is set forth in SEQ ID NO: 71, and the amino acid sequence of the light chain is set forth in SEQ ID NO: 72; or
   the amino acid sequence of the heavy chain is set forth in SEQ ID NO: 73, and the amino acid sequence of the light chain is set forth in SEQ ID NO: 74; or
   the amino acid sequence of the heavy chain is set forth in SEQ ID NO: 75, and the amino acid sequence of the light chain is set forth in SEQ ID NO: 76; or
   the amino acid sequence of the heavy chain is set forth in SEQ ID NO: 77, and the amino acid sequence of the light chain is set forth in SEQ ID NO: 78;
b. the amino acid sequence of the heavy chain is set forth in SEQ ID NO: 79, and the amino acid sequence of the light chain is set forth in SEQ ID NO: 80; or
   the amino acid sequence of the heavy chain is set forth in SEQ ID NO: 81, and the amino acid sequence of the light chain is set forth in SEQ ID NO: 82; or
   the amino acid sequence of the heavy chain is set forth in SEQ ID NO: 83, and the amino acid sequence of the light chain is set forth in SEQ ID NO: 84; or
   the amino acid sequence of the heavy chain is set forth in SEQ ID NO: 85, and the amino acid sequence of the light chain is set forth in SEQ ID NO: 86; or
   the amino acid sequence of the heavy chain is set forth in SEQ ID NO: 87, and the amino acid sequence of the light chain is set forth in SEQ ID NO: 88;
c. the amino acid sequence of the heavy chain is set forth in SEQ ID NO: 89, and the amino acid sequence of the light chain is set forth in SEQ ID NO: 90; or
   the amino acid sequence of the heavy chain is set forth in SEQ ID NO: 91, and the amino acid sequence of the light chain is set forth in SEQ ID NO: 92; or
   the amino acid sequence of the heavy chain is set forth in SEQ ID NO: 93, and the amino acid sequence of the light chain is set forth in SEQ ID NO: 94; or
   the amino acid sequence of the heavy chain is set forth in SEQ ID NO: 95, and the amino acid sequence of the light chain is set forth in SEQ ID NO: 96;
d. the amino acid sequence of the heavy chain is set forth in SEQ ID NO: 97, and the amino acid sequence of the light chain is set forth in SEQ ID NO: 98; or
   the amino acid sequence of the heavy chain is set forth in SEQ ID NO: 99, and the amino acid sequence of the light chain is set forth in SEQ ID NO: 100; or
   the amino acid sequence of the heavy chain is set forth in SEQ ID NO: 101, and the amino acid sequence of the light chain is set forth in SEQ ID NO: 102; or
   the amino acid sequence of the heavy chain is set forth in SEQ ID NO: 103, and the amino acid sequence of the light chain is set forth in SEQ ID NO: 104; or
   the amino acid sequence of the heavy chain is set forth in SEQ ID NO: 105, and the amino acid sequence of the light chain is set forth in SEQ ID NO: 106; or
   the amino acid sequence of the heavy chain is set forth in SEQ ID NO: 107, and the amino acid sequence of the light chain is set forth in SEQ ID NO: 108; or
   the amino acid sequence of the heavy chain is set forth in SEQ ID NO: 109, and the amino acid sequence of the light chain is set forth in SEQ ID NO: 110; or
   the amino acid sequence of the heavy chain is set forth in SEQ ID NO: 111, and the amino acid sequence of the light chain is set forth in SEQ ID NO: 112.

In some embodiments, the anti-MUC1 antibody or the antigen-binding fragment thereof according to any one of the foregoing comprises a heavy chain and a light chain, wherein the amino acid sequence of the heavy chain is set forth in SEQ ID NO: 71, and the amino acid sequence of the light chain is set forth in SEQ ID NO: 72.

In some embodiments, the present disclosure further provides an isolated anti-MUC1 antibody or an antigen-binding fragment thereof that competes for binding to human MUC1 with the anti-MUC1 antibody according to any one of the foregoing.

In some embodiments, the isolated anti-MUC1 antibody or the antigen-binding fragment thereof of the present disclosure binds to human MUC1 with a KD of less than 5 × 10⁻⁸ M (e.g., less than 4 × 10⁻⁸ M, less than 3 × 10⁻⁸ M, less than 2.5 × 10⁻⁸ M, less than 2 × 10⁻⁸ M, less than 1.5 × 10⁻⁸ M, less than 1 × 10⁻⁸ M, less than 9 × 10⁻⁹ M, less than 8 × 10⁻⁹ M, less than 7 × 10⁻⁹ M, less than 6 × 10⁻⁹ M, less than 5 × 10⁻⁹ M, less than 4 × 10⁻⁹ M, less than 3 × 10⁻⁹ M, or less than 2 × 10⁻⁹ M), as measured by Biacore. In another aspect, the present disclosure provides an anti-EGFR antibody or an antigen-binding fragment thereof that comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129, 128, 130, or 131; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
b. in the heavy chain variable region, the HCDR1 comprises the amino acid sequence of SEQ ID NO: 126 or 133, the HCDR2 comprises the amino acid sequence of SEQ ID NO: 117, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
c. in the heavy chain variable region, the HCDR1 comprises the amino acid sequence of SEQ ID NO: 116, the HCDR2 comprises the amino acid sequence of SEQ ID NO: 127, 132, or 134, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
d. in the heavy chain variable region, the HCDR1 comprises the amino acid sequence of SEQ ID NO: 126, the HCDR2 comprises the amino acid sequence of SEQ ID NO: 127, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121;
e. in the heavy chain variable region, the HCDR1 comprises the amino acid sequence of SEQ ID NO: 126, the HCDR2 comprises the amino acid sequence of SEQ ID NO: 117, and the HCDR3 comprises the amino acid sequence of SEQ ID NO: 128, 129, 130, or 131; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121.

In some embodiments, the anti-EGFR antibody or the antigen-binding fragment thereof according to the foregoing comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
b. in the heavy chain variable region, the HCDR1 comprises the amino acid sequence of SEQ ID NO: 126, the HCDR2 comprises the amino acid sequence of SEQ ID NO: 117, and the HCDR3 comprises the amino acid sequence of SEQ ID NO: 128 or 130; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
c. in the heavy chain variable region, the HCDR1 comprises the amino acid sequence of SEQ ID NO: 133, the HCDR2 comprises the amino acid sequence of SEQ ID NO: 117, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121.

In some embodiments, the anti-EGFR antibody or the antigen-binding fragment thereof according to any one of the foregoing comprises a heavy chain variable region and a light chain variable region, wherein the heavy chain variable region comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the light chain variable region comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the anti-EGFR antibody or the antigen-binding fragment thereof according to any one of the foregoing, wherein the HCDR1, HCDR2, and HCDR3 of the heavy chain variable region and the LCDR1, LCDR2, and LCDR3 of the light chain variable region are defined according to the same numbering scheme selected from the group consisting of Kabat, IMGT, Chothia, AbM, and Contact. In some embodiments, the CDRs are defined according to the Kabat numbering scheme. In some embodiments, the CDRs are defined according to the IMGT numbering scheme. In some embodiments, the CDRs are defined according to the Chothia numbering scheme. In some embodiments, the CDRs are defined according to the AbM numbering scheme. In some embodiments, the CDRs are defined according to the Contact numbering scheme.

In some embodiments, the anti-EGFR antibody or the antigen-binding fragment thereof according to any one of the foregoing is a murine antibody, a chimeric antibody, a humanized antibody, or a fully human-derived antibody. In some embodiments, the anti-EGFR antibody or the antigen-binding fragment thereof is a chimeric antibody or a humanized antibody. In some embodiments, the anti-EGFR antibody or the antigen-binding fragment thereof is a humanized antibody.

In some embodiments, the anti-EGFR antibody or the antigen-binding fragment thereof according to any one of the foregoing comprises human antibody framework regions (FRs).

In some embodiments, the anti-EGFR antibody or the antigen-binding fragment thereof according to any one of the foregoing comprises a heavy chain variable region and a light chain variable region, wherein the heavy chain variable region comprises the amino acid sequence of SEQ ID NO: 138, 135, 136, 137, 139, 140, 141, 142, 143, 144, 145, 146, 147, or 148, and the light chain variable region comprises the amino acid sequence of SEQ ID NO: 149;
preferably, the heavy chain variable region comprises the amino acid sequence of SEQ ID NO: 138, 142, 144, or 147, and the light chain variable region comprises the amino acid sequence of SEQ ID NO: 149;
more preferably, the heavy chain variable region comprises the amino acid sequence of SEQ ID NO: 138, and the light chain variable region comprises the amino acid sequence of SEQ ID NO: 149.

In some embodiments, the anti-EGFR antibody or the antigen-binding fragment thereof according to any one of the foregoing is an antibody fragment; in some embodiments, the antibody fragment is selected from the group consisting of Fab, Fab', F(ab')2, Fd, Fv, scFv, dsFv, and dAb.

In some embodiments, the anti-EGFR antibody or the antigen-binding fragment thereof according to any one of the foregoing comprises a heavy chain constant region and a light chain constant region. In some embodiments, the heavy chain constant region is a human IgG1, IgG2, IgG3, or IgG4 heavy chain constant region. In some embodiments, the light chain constant region is a human κ or λ light chain constant region. In some embodiments, the heavy chain constant region comprises the amino acid sequence of SEQ ID NO: 69 or 186, and the light chain constant region comprises the amino acid sequence of SEQ ID NO: 70. In some embodiments, the heavy chain constant region comprises the amino acid sequence of SEQ ID NO: 69, and the light chain constant region comprises the amino acid sequence of SEQ ID NO: 70.

In some embodiments, the anti-EGFR antibody or the antigen-binding fragment thereof according to any one of the foregoing comprises a heavy chain and a light chain, wherein the heavy chain comprises the amino acid sequence of SEQ ID NO: 153, 150, 151, 152, 154, 155, 156, 157, 158, 159, 160, 161, 162, or 163, and the light chain comprises the amino acid sequence of SEQ ID NO: 164;
preferably, the heavy chain comprises the amino acid sequence of SEQ ID NO: 153, 157, 159, or 162, and the light chain comprises the amino acid sequence of SEQ ID NO: 164;
more preferably, the heavy chain comprises the amino acid sequence of SEQ ID NO: 153, and the light chain comprises the amino acid sequence of SEQ ID NO: 164.

In some embodiments, the present disclosure further provides an isolated anti-EGFR antibody or an antigen-binding fragment thereof that competes for binding to human EGFR with the anti-EGFR antibody according to any one of the foregoing.

In another aspect, the present disclosure provides an antigen-binding molecule that specifically binds to EGFR and MUC1, wherein the antigen-binding molecule comprises at least one antigen-binding moiety that specifically binds to EGFR and at least one antigen-binding moiety that specifically binds to MUC1; the antigen-binding moiety that specifically binds to EGFR comprises a heavy chain variable region EGFR-VH and a light chain variable region EGFR-VL, and the antigen-binding moiety that specifically binds to MUC1 comprises a heavy chain variable region MUC1-VH and a light chain variable region MUC1-VL, wherein:
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, 128, 130, or 131, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126 or 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 116, a HCDR2 comprising the amino acid sequence of SEQ ID NO: 127, 132, or 134, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
d. the EGFR-VH comprises a HCDR1 comprising the amino acid sequence of SEQ ID NO: 126, a HCDR2 comprising the amino acid sequence of SEQ ID NO: 127, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
e. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128, 129, 130, or 131, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the antigen-binding molecule that specifically binds to EGFR and MUC1 according to the foregoing, wherein:
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128 or 130, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the antigen-binding molecule that specifically binds to EGFR and MUC1 according to any one of the foregoing, wherein the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the antigen-binding molecule that specifically binds to EGFR and MUC1 according to any one of the foregoing, wherein the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17.

In some embodiments, provided is the antigen-binding molecule that specifically binds to EGFR and MUC1 according to any one of the foregoing, wherein:
the EGFR-VH comprises the amino acid sequence of SEQ ID NO: 138, 135, 136, 137, 139, 140, 141, 142, 143, 144, 145, 146, 147, or 148, and the EGFR-VL comprises the amino acid sequence of SEQ ID NO: 149;
preferably, the EGFR-VH comprises the amino acid sequence of SEQ ID NO: 138, 142, 144, or 147, and the EGFR-VL comprises the amino acid sequence of SEQ ID NO: 149; more preferably, the EGFR-VH comprises the amino acid sequence of SEQ ID NO: 138, and the EGFR-VL comprises the amino acid sequence of SEQ ID NO: 149.

In some embodiments, provided is the antigen-binding molecule that specifically binds to EGFR and MUC1 according to any one of the foregoing, wherein the MUC1-VH comprises the amino acid sequence of SEQ ID NO: 36, and the MUC1-VL comprises the amino acid sequence of SEQ ID NO: 39.

In some embodiments, provided is the antigen-binding molecule that specifically binds to EGFR and MUC1 according to any one of the foregoing, wherein the antigen-binding moiety that specifically binds to EGFR or the antigen-binding moiety that specifically binds to MUC1 independently comprises a Titin chain and an Obscurin chain that are capable of forming a dimer (derived from WO2022237882A1).

In some embodiments, provided is the antigen-binding molecule that specifically binds to EGFR and MUC1 according to any one of the foregoing, wherein the Titin chain comprises the amino acid sequence of SEQ ID NO: 165, and the Obscurin chain comprises the amino acid sequence of SEQ ID NO: 166.

In some embodiments, the antigen-binding molecule that specifically binds to EGFR and MUC1 according to any one of the foregoing further comprises an Fc region, wherein the Fc region is preferably an IgG Fc region, and further preferably an IgG1 Fc region; more preferably, the Fc region comprises one or more amino acid substitutions capable of reducing the binding of the Fc region to an Fcγ receptor.

In some embodiments, the antigen-binding molecule that specifically binds to EGFR and MUC1 according to any one of the foregoing further comprises an Fc region, wherein the Fc region comprises a first subunit Fc1 and a second subunit Fc2 that are capable of associating with each other, and the Fc1 and Fc2 each independently comprise one or more amino acid substitutions that reduce the homodimerization of the Fc region. It should be understood that, in the context of the present application, Fc1 and Fc2, when referred to, function to form a dimer; therefore, Fc1 and Fc2 are interchangeable.

In some embodiments, provided is the antigen-binding molecule that specifically binds to EGFR and MUC1 according to any one of the foregoing, wherein the Fc1 has a knob structure according to the knob-into-hole technique, and the Fc2 has a hole structure according to the knob-into-hole technique; or vice versa: the Fc2 has a knob structure according to the knob-into-hole technique, and the Fc1 has a hole structure according to the knob-into-hole technique.

In some embodiments, provided is the antigen-binding molecule that specifically binds to EGFR and MUC1 according to any one of the foregoing, wherein the Fc1 has a knob structure according to the knob-into-hole technique, and the Fc2 has a hole structure according to the knob-into-hole technique.

In some embodiments, provided is the antigen-binding molecule that specifically binds to EGFR and MUC1 according to any one of the foregoing, wherein in the Fc1, the amino acid at position 358 is C, and the amino acid at position 370 is W; and in the Fc2, the amino acid at position 357 is C, the amino acid at position 374 is S, the amino acid at position 376 is A, and the amino acid at position 415 is V, as numbered according to the EU index; or vice versa: in the Fc2, the amino acid at position 358 is C, and the amino acid at position 370 is W; and in the Fc1, the amino acid at position 357 is C, the amino acid at position 374 is S, the amino acid at position 376 is A, and the amino acid at position 415 is V, as numbered according to the EU index.

In some embodiments, provided is the antigen-binding molecule that specifically binds to EGFR and MUC1 according to any one of the foregoing, wherein in the Fc1, the amino acid at position 358 is C, and the amino acid at position 370 is W; and in the Fc2, the amino acid at position 357 is C, the amino acid at position 374 is S, the amino acid at position 376 is A, and the amino acid at position 415 is V, as numbered according to the EU index.

In some embodiments, provided is the antigen-binding molecule that specifically binds to EGFR and MUC1 according to any one of the foregoing, wherein the Fc1 comprises the amino acid sequence of SEQ ID NO: 169, and the Fc2 comprises the amino acid sequence of SEQ ID NO: 170; or vice versa: the Fc2 comprises the amino acid sequence of SEQ ID NO: 169, and the Fc1 comprises the amino acid sequence of SEQ ID NO: 170.

In some embodiments, provided is the antigen-binding molecule that specifically binds to EGFR and MUC1 according to any one of the foregoing, wherein the Fc1 comprises the amino acid sequence of SEQ ID NO: 169, and the Fc2 comprises the amino acid sequence of SEQ ID NO: 170;

In some embodiments, provided is the antigen-binding molecule that specifically binds to EGFR and MUC1 according to any one of the foregoing, wherein the Fc1 comprises the amino acid sequence of SEQ ID NO: 182, and the Fc2 comprises the amino acid sequence of SEQ ID NO: 183; or vice versa: the Fc2 comprises the amino acid sequence of SEQ ID NO: 182, and the Fc1 comprises the amino acid sequence of SEQ ID NO: 183.

In some embodiments, provided is the antigen-binding molecule that specifically binds to EGFR and MUC1 according to any one of the foregoing, wherein the Fc1 comprises the amino acid sequence of SEQ ID NO: 182, and the Fc2 comprises the amino acid sequence of SEQ ID NO: 183.

In some embodiments, provided is the antigen-binding molecule that specifically binds to EGFR and MUC1 according to any one of the foregoing, wherein the antigen-binding molecule comprises one antigen-binding moiety that specifically binds to EGFR and one antigen-binding moiety that specifically binds to MUC1.

In some embodiments, provided is the antigen-binding molecule that specifically binds to EGFR and MUC1 according to any one of the foregoing, wherein the antigen-binding molecule comprises one antigen-binding moiety that specifically binds to EGFR and one antigen-binding moiety that specifically binds to MUC1; the antigen-binding moiety that specifically binds to MUC1 is a Fab, and the antigen-binding moiety that specifically binds to EGFR is a replaced Fab comprising a Titin chain and an Obscurin chain that are capable of forming a dimer; or the antigen-binding moiety that specifically binds to EGFR is a Fab, and the antigen-binding moiety that specifically binds to MUC1 is a replaced Fab comprising a Titin chain and an Obscurin chain that are capable of forming a dimer.

In some embodiments, provided is the antigen-binding molecule that specifically binds to EGFR and MUC1 according to any one of the foregoing, wherein the antigen-binding molecule comprises one antigen-binding moiety that specifically binds to EGFR and one antigen-binding moiety that specifically binds to MUC1; the antigen-binding moiety that specifically binds to MUC1 is a Fab, and the antigen-binding moiety that specifically binds to EGFR is a replaced Fab comprising a Titin chain and an Obscurin chain that are capable of forming a dimer.

In some embodiments, provided is the antigen-binding molecule that specifically binds to EGFR and MUC1 according to any one of the foregoing, wherein the antigen-binding molecule comprises one first chain having a structure represented by formula (a), one second chain having a structure represented by formula (b), one third chain having a structure represented by formula (c), and one fourth chain having a structure represented by formula (d):

formula (a) [MUC1-VH]-[CH1]-[Fc],

formula (b) [MUC1-VL]-[CL],

formula (c) [EGFR-VH]-[linker 1]-[Titin]-[Fc2], and

formula (d) [EGFR-VL]-[linker 2]-[Obscurin],

wherein:
linker 1 and linker 2 are identical or different and are peptide linkers; or linker 1 or linker 2 is absent;
the structures represented by formulas (a), (b), (c), and (d) are arranged from the N-terminus to the C-terminus.

In some embodiments, provided is the antigen-binding molecule that specifically binds to EGFR and MUC1 according to any one of the foregoing, wherein the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128 or 130, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the antigen-binding molecule that specifically binds to EGFR and MUC1 according to any one of the foregoing, wherein the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, provided is the antigen-binding molecule that specifically binds to EGFR and MUC1 according to any one of the foregoing, wherein the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, 142, 144, or 147, and the EGFR-VL comprises the amino acid sequence of SEQ ID NO: 149.

In some embodiments, provided is the antigen-binding molecule that specifically binds to EGFR and MUC1 according to any one of the foregoing, wherein the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149.

In some embodiments, provided is the antigen-binding molecule that specifically binds to EGFR and MUC1 according to any one of the foregoing, wherein linker 1 is a peptide linker known in the art, as long as the antigen-binding molecule is capable of exhibiting the desired antigen binding activity. For example, the peptide linker may be a flexible peptide having 1-50 or 3-20 amino acid residues. In some embodiments, the peptide linkers each independently have a structure of L₁-(GGGGS)t-L₂, wherein L₁ is a bond, A, G, GS, GGG, GGS, or GGGGS (SEQ ID NO: 168), t is 0, 1, 2, 3, 4, 5, 6, 7, 8, 9, or 10, and L2 is a bond, G, GG, GGG, or GGGG (SEQ ID NO: 181); and the peptide linkers are not bonds. In some embodiments, linker 1 and linker 2 are identical, and the amino acid sequence of the linkers is set forth in SEQ ID NO: 168.

In some embodiments, provided is the antigen-binding molecule that specifically binds to EGFR and MUC1 according to any one of the foregoing, wherein the CH1 is the CH1 sequence of an IgG. In some embodiments, the CH1 is the CH1 of IgG1. In some embodiments, the CH1 comprises the amino acid sequence of SEQ ID NO: 167.

In some embodiments, provided is the antigen-binding molecule that specifically binds to EGFR and MUC1 according to any one of the foregoing, wherein the CL is the light chain constant region of an antibody. In some embodiments, provided is the antigen-binding molecule according to any one of the foregoing, wherein the CL is a kappa or lamada light chain constant region. In some embodiments, the CL comprises the amino acid sequence of SEQ ID NO: 70.

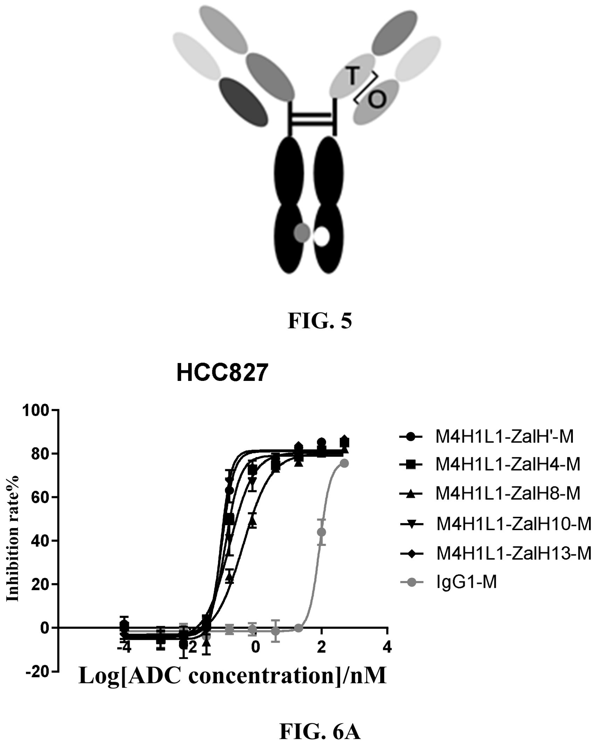

In some embodiments, the format of the antigen-binding molecule that specifically binds to EGFR and MUC1 according to any one of the foregoing is an asymmetrically structured molecule comprising four chains, as shown in FIG. 5.

In some embodiments, the format of the antigen-binding molecule that specifically binds to EGFR and MUC1 according to any one of the foregoing is an asymmetrically structured molecule comprising four chains, as shown in FIG. 5, wherein the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

In some embodiments, the format of the antigen-binding molecule that specifically binds to EGFR and MUC1 according to any one of the foregoing is an asymmetrically structured molecule comprising four chains, as shown in FIG. 5, wherein the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 174, 172, 175, 176, or 177, and one fourth chain comprising the amino acid sequence of SEQ ID NO: 173, or the antigen-binding molecule comprises one first chain comprising the amino acid sequence of SEQ ID NO: 178, one second chain comprising the amino acid sequence of SEQ ID NO: 74, one third chain comprising the amino acid sequence of SEQ ID NO: 179, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 174, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 172, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 175, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 176, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 171, one second chain comprising the amino acid sequence of SEQ ID NO: 74, one third chain comprising the amino acid sequence of SEQ ID NO: 177, and one fourth chain comprising the amino acid sequence of SEQ ID NO: 173.

In some embodiments, the antigen-binding molecule that specifically binds to EGFR and MUC1 according to any one of the foregoing comprises one first chain comprising the amino acid sequence of SEQ ID NO: 178, one second chain comprising the amino acid sequence of SEQ ID NO: 74, one third chain comprising the amino acid sequence of SEQ ID NO: 179, and one fourth chain comprising the amino acid sequence of SEQ ID NO: 173.

In some embodiments, the antigen-binding molecule that specifically binds to EGFR and MUC1 according to any one of the foregoing comprises one first chain set forth in SEQ ID NO: 171, one second chain set forth in SEQ ID NO: 74, one third chain set forth in SEQ ID NO: 174, and one fourth chain set forth in SEQ ID NO: 173.

In some embodiments, the antigen-binding molecule that specifically binds to EGFR and MUC1 according to any one of the foregoing comprises one first chain set forth in SEQ ID NO: 171, one second chain set forth in SEQ ID NO: 74, one third chain set forth in SEQ ID NO: 172, and one fourth chain set forth in SEQ ID NO: 173.

In some embodiments, the antigen-binding molecule that specifically binds to EGFR and MUC1 according to any one of the foregoing comprises one first chain set forth in SEQ ID NO: 171, one second chain set forth in SEQ ID NO: 74, one third chain set forth in SEQ ID NO: 175, and one fourth chain set forth in SEQ ID NO: 173.

In some embodiments, the antigen-binding molecule that specifically binds to EGFR and MUC1 according to any one of the foregoing comprises one first chain set forth in SEQ ID NO: 171, one second chain set forth in SEQ ID NO: 74, one third chain set forth in SEQ ID NO: 176, and one fourth chain set forth in SEQ ID NO: 173.

In some embodiments, the antigen-binding molecule that specifically binds to EGFR and MUC1 according to any one of the foregoing comprises one first chain set forth in SEQ ID NO: 171, one second chain set forth in SEQ ID NO: 74, one third chain set forth in SEQ ID NO: 177, and one fourth chain set forth in SEQ ID NO: 173.

In some embodiments, the antigen-binding molecule that specifically binds to EGFR and MUC1 according to any one of the foregoing comprises one first chain set forth in SEQ ID NO: 178, one second chain set forth in SEQ ID NO: 74, one third chain set forth in SEQ ID NO: 179, and one fourth chain set forth in SEQ ID NO: 173.

In another aspect, the present disclosure provides an immunoconjugate comprising the anti-MUC1 antibody or the antigen-binding fragment thereof according to any one of the foregoing and an effector, or the anti-EGFR antibody or the antigen-binding fragment thereof according to any one of the foregoing and an effector, or the antigen-binding molecule that specifically binds to EGFR and MUC1 according to any one of the foregoing and an effector, wherein the effector is conjugated to the anti-MUC1 antibody or the antigen-binding fragment thereof, the anti-EGFR antibody or the antigen-binding fragment thereof, or the antigen-binding molecule that specifically binds to EGFR and MUC1; preferably, the effector is selected from the group consisting of an anti-tumor agent, an immunomodulator, a biological response modifier, a lectin, a cytotoxic drug, a chromophore, a fluorophore, a chemiluminescent compound, an enzyme, and a metal ion, and any combination thereof.

In some embodiments, the effector is a cytotoxic drug; preferably, the cytotoxic drug is an exatecan-based drug or an MMAE/MMAF-based drug.

In another aspect, the present disclosure provides an antibody-drug conjugate represented by general formula (Pc-L-Y-D) or a pharmaceutically acceptable salt thereof, wherein: 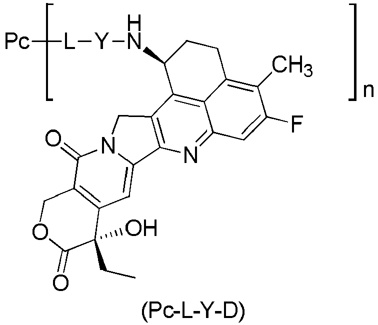
Y is selected from the group consisting of -O-(CR^{a}R^{b})ₘ-CR¹R²-C(O)-, -O-CR¹R²-(CR^{a}R^{b})ₘ-, -O-CR¹R²-, -NH-(CR^{a}R^{b})ₘ-CR¹R²-C(O)-, and -S-(CR^{a}R^{b})ₘ-CR¹R²-C(O)-;
R^{a} and R^{b} are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, heterocyclyl, aryl, and heteroaryl;
or, R^{a} and R^{b}, together with the carbon atom to which they are attached, form cycloalkyl, heterocyclyl, aryl, or heteroaryl;
R¹ is selected from the group consisting of halogen, alkyl, haloalkyl, deuterated alkyl, hydroxy, alkoxy, cyano, amino, cycloalkyl, cycloalkylalkyl, alkoxyalkyl, heterocyclyl, aryl, and heteroaryl;
R² is selected from the group consisting of hydrogen, deuterium, halogen, alkyl, haloalkyl, deuterated alkyl, hydroxy, alkoxy, cyano, amino, cycloalkyl, cycloalkylalkyl, alkoxyalkyl, heterocyclyl, aryl, and heteroaryl;
or, R¹ and R², together with the carbon atom to which they are attached, form cycloalkyl, heterocyclyl, aryl, or heteroaryl;
or, R^{a} and R², together with the carbon atoms to which they are attached, form cycloalkyl, heterocyclyl, aryl, or heteroaryl;
m is 0, 1, 2, 3, or 4;
n is 1 to 10;
L is a linker unit;
Pc is the anti-MUC1 antibody or the antigen-binding fragment thereof according to any one of the foregoing, or the anti-EGFR antibody or the antigen-binding fragment thereof according to any one of the foregoing, or the antigen-binding molecule that specifically binds to EGFR and MUC1 according to any one of the foregoing; preferably, Pc is the antigen-binding molecule that specifically binds to EGFR and MUC1 according to any one of the foregoing.

In some embodiments, provided is the antibody-drug conjugate represented by general formula (Pc-L-Y-D) or the pharmaceutically acceptable salt thereof, wherein:
Y is

   -O-(CR^{a}R^{b})ₘ-CR¹R²-C(O)-,
wherein R^{a} and R^{b} are identical or different and are each independently selected from the group consisting of hydrogen, deuterium, halogen, and alkyl;
R¹ is cycloalkyl-alkyl or cycloalkyl;
R² is selected from the group consisting of hydrogen, haloalkyl, and cycloalkyl;
or, R¹ and R², together with the carbon atom to which they are attached, form cycloalkyl;
m is 0, 1, 2, 3, or 4;
n is 1 to 10;
L is a linker unit;
Pc is the anti-MUC1 antibody or the antigen-binding fragment thereof according to any one of the foregoing, or the anti-EGFR antibody or the antigen-binding fragment thereof according to any one of the foregoing, or the antigen-binding molecule that specifically binds to EGFR and MUC1 according to any one of the foregoing; preferably, Pc is the antigen-binding molecule that specifically binds to EGFR and MUC1 according to any one of the foregoing.

In some embodiments, provided is the antibody-drug conjugate represented by general formula (Pc-L-Y-D) or the pharmaceutically acceptable salt thereof, wherein:
Y is

   -O-(CR^{a}R^{b})ₘ-CR¹R²-C(O)-,
wherein R^{a} and R^{b} are identical or different and are each independently selected from the group consisting of hydrogen, deuterium, halogen, and C₁₋₆ alkyl;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
m is 0, 1, 2, 3, or 4;
n is 1 to 10;
L is a linker unit;
Pc is an antigen-binding molecule that specifically binds to EGFR and MUC1, wherein the antigen-binding molecule comprises one antigen-binding moiety that specifically binds to EGFR and one antigen-binding moiety that specifically binds to MUC1; the antigen-binding moiety that specifically binds to EGFR comprises a heavy chain variable region EGFR-VH and a light chain variable region EGFR-VL, and the antigen-binding moiety that specifically binds to MUC1 comprises a heavy chain variable region MUC1-VH and a light chain variable region MUC1-VL, wherein:
   the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
   preferably, the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149;
   more preferably, the antigen-binding molecule comprises one first chain comprising the amino acid sequence of SEQ ID NO: 171, one second chain comprising the amino acid sequence of SEQ ID NO: 74, one third chain comprising the amino acid sequence of SEQ ID NO: 174, and one fourth chain comprising the amino acid sequence of SEQ ID NO: 173; or one first chain comprising the amino acid sequence of SEQ ID NO: 178, one second chain comprising the amino acid sequence of SEQ ID NO: 74, one third chain comprising the amino acid sequence of SEQ ID NO: 179, and one fourth chain comprising the amino acid sequence of SEQ ID NO: 173.

In some embodiments, provided is the antibody-drug conjugate represented by general formula (Pc-L-Y-D) or the pharmaceutically acceptable salt thereof, wherein:
Y is

   -O-(CR^{a}R^{b})ₘ-CR¹R²-C(O)-,
wherein R^{a} and R^{b} are identical or different and are each independently selected from the group consisting of hydrogen, deuterium, halogen, and C₁₋₆ alkyl;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
m is 0, 1, 2, 3, or 4;
n is 1 to 10;
L is a linker unit;
Pc is an anti-MUC1 antibody or an antigen-binding fragment thereof that comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
   in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17;
   preferably, the heavy chain variable region comprises the amino acid sequence of SEQ ID NO: 36, and the light chain variable region comprises the amino acid sequence of SEQ ID NO: 39;
   more preferably, Pc is an anti-MUC1 antibody comprising a heavy chain and a light chain, wherein the heavy chain comprises the amino acid sequence of SEQ ID NO: 71, and the light chain comprises the amino acid sequence of SEQ ID NO: 72.

In some embodiments, provided is the antibody-drug conjugate represented by general formula (Pc-L-Y-D) or the pharmaceutically acceptable salt thereof, wherein:
Y is

   -O-(CR^{a}R^{b})ₘ-CR¹R²-C(O)-,
wherein R^{a} and R^{b} are identical or different and are each independently selected from the group consisting of hydrogen, deuterium, halogen, and C₁₋₆ alkyl;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
m is 0, 1, 2, 3, or 4;
n is 1 to 10;
L is a linker unit;
Pc is an anti-EGFR antibody or an antigen-binding fragment thereof that comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
   in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121;
   preferably, the heavy chain variable region comprises the amino acid sequence of SEQ ID NO: 138, and the light chain variable region comprises the amino acid sequence of SEQ ID NO: 149;
   more preferably, Pc is an anti-EGFR antibody comprising a heavy chain and a light chain, wherein the heavy chain comprises the amino acid sequence of SEQ ID NO: 153, and the light chain comprises the amino acid sequence of SEQ ID NO: 164. In some embodiments, provided is the antibody-drug conjugate represented by general formula (Pc-L-Y-D) or the pharmaceutically acceptable salt thereof, wherein the linker unit -L-is -L¹-L²-L³-L⁴-,
   wherein L¹ is selected from the group consisting of -(succinimid-3-yl-*N*)-W-C(O)-, -CH₂-C(O)-NR³-W-C(O)-, and -C(O)-W-C(O)-, and W is selected from the group consisting of alkylene and alkylene-cycloalkyl, wherein the alkylene or alkylene-cycloalkyl is independently and optionally substituted with one or more substituents selected from the group consisting of halogen, hydroxy, cyano, amino, alkyl, haloalkyl, deuterated alkyl, alkoxy, and cycloalkyl;
   L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
   L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, haloalkyl, deuterated alkyl, alkoxy, and cycloalkyl;
   L⁴ is selected from the group consisting of -NR⁵(CR⁶R⁷)ₜ-, -C(O)NR⁵-, -C(O)NR⁵(CH₂)ₜ-, and a chemical bond, wherein t is 1, 2, 3, 4, 5, or 6;
   R³, R⁴, and R⁵ are identical or different and are each independently selected from the group consisting of hydrogen, alkyl, haloalkyl, deuterated alkyl, and hydroxyalkyl;
   R⁶ and R⁷ are identical or different and are each independently selected from the group consisting of hydrogen, halogen, alkyl, haloalkyl, deuterated alkyl, and hydroxyalkyl; preferably, the linker unit -L¹-L²-L³-L⁴- is as follows:
      L¹ is 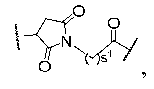 wherein s¹ is 2, 3, 4, 5, 6, 7, or 8;
      L² is a chemical bond;
      L³ is a tetrapeptide residue; preferably, L³ is a tetrapeptide residue represented by GGFG (SEQ ID NO: 180);
      L⁴ is -NR⁵(CR⁶R⁷)t-, wherein R⁵*,* R⁶, or R⁷ is identical or different and is independently hydrogen or C₁₋₆ alkyl, and t is 1 or 2;
      the L¹ terminus of -L- is attached to Pc, and the L⁴ terminus is attached to Y.

In some embodiments, the antibody-drug conjugate represented by general formula (Pc-L-Y-D) or the pharmaceutically acceptable salt thereof is an antibody-drug conjugate represented by general formula (Pc-Lₐ-Y-D) or a pharmaceutically acceptable salt thereof: 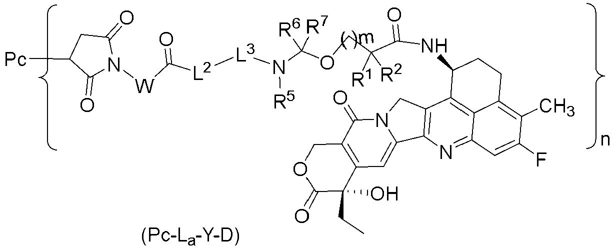 wherein:
Pc is the anti-MUC1 antibody or the antigen-binding fragment thereof according to any one of the foregoing, or the anti-EGFR antibody or the antigen-binding fragment thereof according to any one of the foregoing, or the antigen-binding molecule that specifically binds to EGFR and MUC1 according to any one of the foregoing; preferably, Pc is the antigen-binding molecule that specifically binds to EGFR and MUC1 according to any one of the foregoing;
m is 0, 1, 2, 3, or 4;
n is 1 to 10;
R¹ is cycloalkyl-alkyl or cycloalkyl;
R² is selected from the group consisting of hydrogen, haloalkyl, and cycloalkyl;
or, R¹ and R², together with the carbon atom to which they are attached, form cycloalkyl;
W is selected from the group consisting of alkylene and alkylene-cycloalkyl, wherein the alkylene and alkylene-cycloalkyl are each independently and optionally substituted with one or more substituents selected from the group consisting of halogen, hydroxy, cyano, amino, alkyl, haloalkyl, deuterated alkyl, alkoxy, and cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, haloalkyl, deuterated alkyl, alkoxy, and cycloalkyl;
R⁴ and R⁵ are selected from the group consisting of hydrogen, alkyl, haloalkyl, deuterated alkyl, and hydroxyalkyl;
R⁶ and R⁷ are identical or different and are each independently selected from the group consisting of hydrogen, halogen, alkyl, haloalkyl, deuterated alkyl, and hydroxyalkyl.

In some embodiments, the antibody-drug conjugate represented by general formula (Pc-L-Y-D) or the pharmaceutically acceptable salt thereof is an antibody-drug conjugate represented by general formula (Pc-Lₐ-Y-D) or a pharmaceutically acceptable salt thereof: 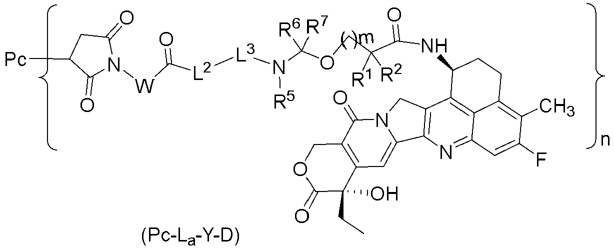 wherein:
Pc is the antigen-binding molecule that specifically binds to EGFR and MUC1 according to any one of the foregoing;
m is 0, 1, 2, 3, or 4;
n is 1 to 10;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
W is selected from the group consisting of C₁₋₆ alkylene and C₁₋₆ alkylene-3- to 6-membered cycloalkyl, wherein the C₁₋₆ alkylene and C₁₋₆ alkylene-3- to 6-membered cycloalkyl are each independently and optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
R⁴ and R⁵ are selected from the group consisting of hydrogen, C₁₋₆ alkyl, C₁₋₆ haloalkyl, C₁₋₆ deuterated alkyl, and C₁₋₆ hydroxyalkyl;
R⁶ and Rare identical or different and are each independently selected from the group consisting of hydrogen, halogen, C₁₋₆ alkyl, C₁₋₆ haloalkyl, C₁₋₆ deuterated alkyl, and C₁₋₆ hydroxyalkyl.

In some embodiments, the antibody-drug conjugate represented by general formula (Pc-L-Y-D) or the pharmaceutically acceptable salt thereof is an antibody-drug conjugate represented by general formula (Pc-Lₐ-Y-D) or a pharmaceutically acceptable salt thereof: 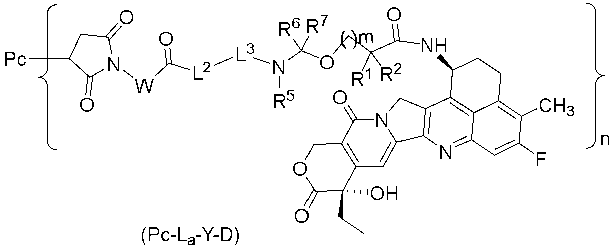 wherein:
Pc is the anti-MUC1 antibody or the antigen-binding fragment thereof according to any one of the foregoing;
m is 0, 1, 2, 3, or 4;
n is 1 to 10;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
W is selected from the group consisting of C₁₋₆ alkylene and C₁₋₆ alkylene-3- to 6-membered cycloalkyl, wherein the C₁₋₆ alkylene and C₁₋₆ alkylene-3- to 6-membered cycloalkyl are each independently and optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
R⁴ and R⁵ are selected from the group consisting of hydrogen, C₁₋₆ alkyl, C₁₋₆ haloalkyl, C₁₋₆ deuterated alkyl, and C₁₋₆ hydroxyalkyl;
R⁶ and Rare identical or different and are each independently selected from the group consisting of hydrogen, halogen, C₁₋₆ alkyl, C₁₋₆ haloalkyl, C₁₋₆ deuterated alkyl, and C₁₋₆ hydroxyalkyl.

In some embodiments, the antibody-drug conjugate represented by general formula (Pc-L-Y-D) or the pharmaceutically acceptable salt thereof is an antibody-drug conjugate represented by general formula (Pc-Lₐ-Y-D) or a pharmaceutically acceptable salt thereof: 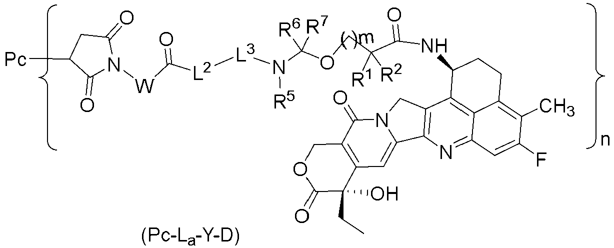 wherein:
Pc is the anti-EGFR antibody or the antigen-binding fragment thereof according to any one of the foregoing;
m is 0, 1, 2, 3, or 4;
n is 1 to 10;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
W is selected from the group consisting of C₁₋₆ alkylene and C₁₋₆ alkylene-3- to 6-membered cycloalkyl, wherein the C₁₋₆ alkylene and C₁₋₆ alkylene-3- to 6-membered cycloalkyl are each independently and optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
R⁴ and R⁵ are selected from the group consisting of hydrogen, C₁₋₆ alkyl, C₁₋₆ haloalkyl, C₁₋₆ deuterated alkyl, and C₁₋₆ hydroxyalkyl;
R⁶ and Rare identical or different and are each independently selected from the group consisting of hydrogen, halogen, C₁₋₆ alkyl, C₁₋₆ haloalkyl, C₁₋₆ deuterated alkyl, and C₁₋₆ hydroxyalkyl.

In some embodiments, provided is the antibody-drug conjugate represented by general formula (Pc-L-Y-D) or general formula (Pc-Lₐ-Y-D) or the pharmaceutically acceptable salt thereof, wherein m is 0.

In some embodiments, provided is the antibody-drug conjugate represented by general formula (Pc-L-Y-D) or general formula (Pc-Lₐ-Y-D) or the pharmaceutically acceptable salt thereof, wherein R¹ is 3- to 6-membered cycloalkyl; preferably, R¹ is cyclopropyl.

In some embodiments, provided is the antibody-drug conjugate represented by general formula (Pc-L-Y-D) or general formula (Pc-Lₐ-Y-D) or the pharmaceutically acceptable salt thereof, wherein R² is hydrogen.

In some embodiments, provided is the antibody-drug conjugate represented by general formula (Pc-L-Y-D) or general formula (Pc-Lₐ-Y-D) or the pharmaceutically acceptable salt thereof, wherein R¹ and R², together with the carbon atom to which they are attached, form 3- to 6-membered cycloalkyl.

In some embodiments, provided is the antibody-drug conjugate represented by general formula (Pc-L-Y-D) or general formula (Pc-Lₐ-Y-D) or the pharmaceutically acceptable salt thereof, wherein W is selected from the group consisting of C₁₋₆ alkylene.

In some embodiments, provided is the antibody-drug conjugate represented by general formula (Pc-L-Y-D) or general formula (Pc-Lₐ-Y-D) or the pharmaceutically acceptable salt thereof, wherein L² is a chemical bond.

In some embodiments, provided is the antibody-drug conjugate represented by general formula (Pc-L-Y-D) or general formula (Pc-Lₐ-Y-D) or the pharmaceutically acceptable salt thereof, wherein L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl; preferably, L³ is a tetrapeptide residue; more preferably, L³ is a tetrapeptide residue represented by GGFG (SEQ ID NO: 180).

In some embodiments, provided is the antibody-drug conjugate represented by general formula (Pc-L-Y-D) or general formula (Pc-Lₐ-Y-D) or the pharmaceutically acceptable salt thereof, wherein R⁵ is hydrogen.

In some embodiments, provided is the antibody-drug conjugate represented by general formula (Pc-L-Y-D) or general formula (Pc-Lₐ-Y-D) or the pharmaceutically acceptable salt thereof, wherein R⁶ and R⁷ are both hydrogen.

In some embodiments, the antibody-drug conjugate represented by general formula (Pc-L-Y-D) or general formula (Pc-Lₐ-Y-D) or the pharmaceutically acceptable salt thereof is an antibody-drug conjugate represented by general formula (Pc-9-A) or a pharmaceutically acceptable salt thereof: 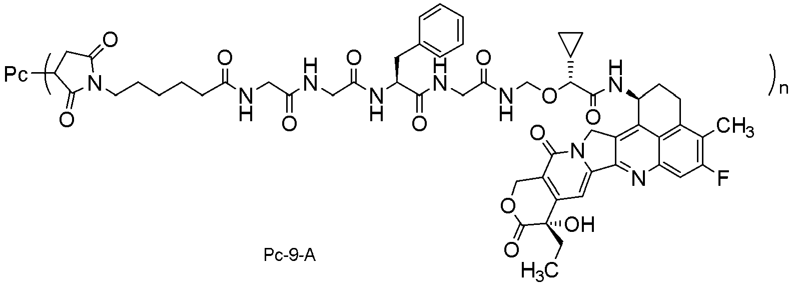 wherein:
n is 1 to 8;
Pc is the antigen-binding molecule that specifically binds to EGFR and MUC1 according to any one of the foregoing.

In some embodiments, the antibody-drug conjugate represented by general formula (Pc-L-Y-D) or general formula (Pc-Lₐ-Y-D) or the pharmaceutically acceptable salt thereof is an antibody-drug conjugate represented by general formula (Pc-9-A) or a pharmaceutically acceptable salt thereof: 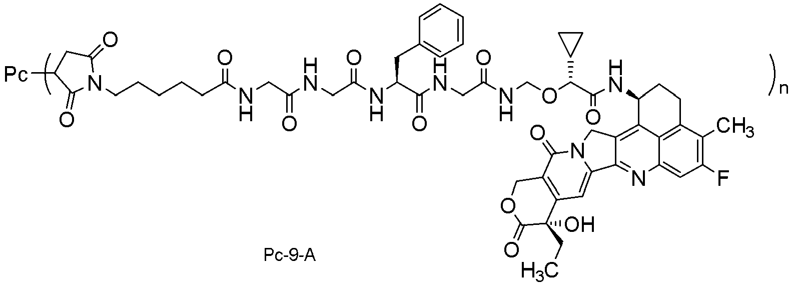 wherein:
n is 1 to 8;
Pc is the anti-MUC1 antibody or the antigen-binding fragment thereof according to any one of the foregoing.

In some embodiments, the antibody-drug conjugate represented by general formula (Pc-L-Y-D) or general formula (Pc-Lₐ-Y-D) or the pharmaceutically acceptable salt thereof is an antibody-drug conjugate represented by general formula (Pc-9-A) or a pharmaceutically acceptable salt thereof: 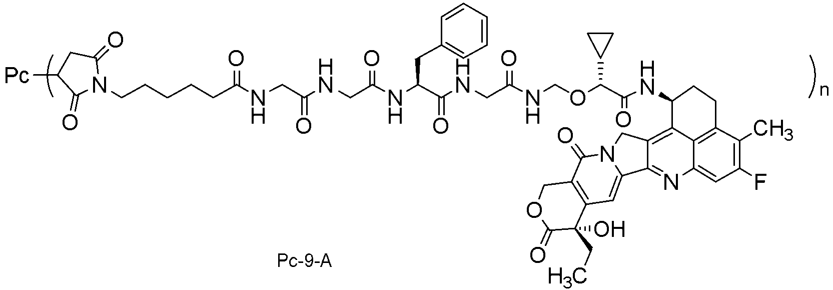 wherein:
n is 1 to 8;
Pc is the anti-EGFR antibody or the antigen-binding fragment thereof according to any one of the foregoing.

In another aspect, the present disclosure provides a method for preparing an antibody-drug conjugate represented by general formula (Pc-Lₐ-Y-D) or a pharmaceutically acceptable salt thereof, wherein the method comprises the following step: 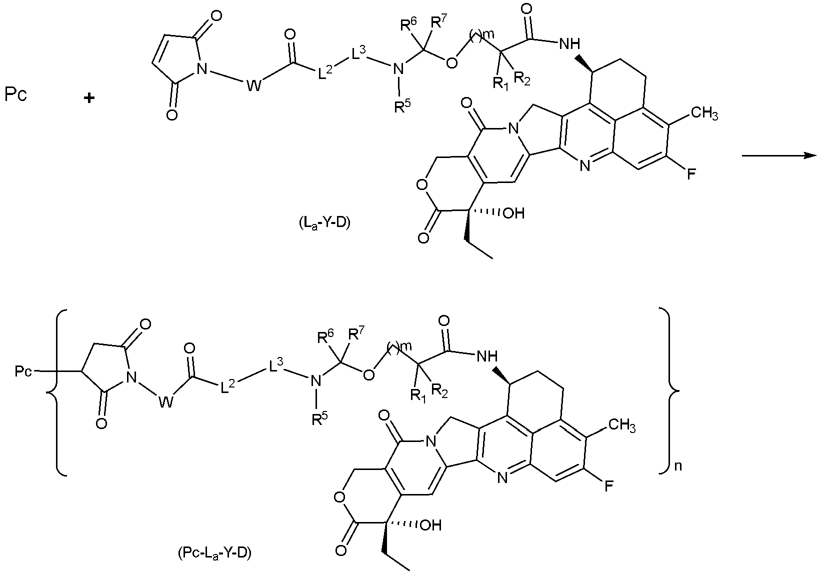 subjecting reduced Pc to a coupling reaction with a compound represented by general formula (Lₐ-Y-D) or a salt thereof to give the antibody-drug conjugate represented by general formula (Pc-Lₐ-Y-D) or the pharmaceutically acceptable salt thereof, wherein:
Pc is the anti-MUC1 antibody or the antigen-binding fragment thereof according to any one of the foregoing, or the anti-EGFR antibody or the antigen-binding fragment thereof according to any one of the foregoing, or the antigen-binding molecule that specifically binds to EGFR and MUC1 according to any one of the foregoing; preferably, Pc is the antigen-binding molecule that specifically binds to EGFR and MUC1 according to any one of the foregoing; W, L², L³, R¹, R², R⁵ to R⁷, m, and n are as defined in any one of the foregoing.

In some embodiments, n is 0 to 10 (including decimals or integers; the same applies hereinafter).

In some embodiments, n is 1 to 10; for example, n averages 1, 2, 3, 4, 5, 6, 7, 8, 9, or 10.

In some embodiments, n is 1 to 8; preferably, n is 2 to 8; more preferably, n is 4 to 8; most preferably, n is 4 to 6.

In another aspect, the present disclosure provides a compound represented by general formula (Pc-M') or a pharmaceutically acceptable salt thereof: 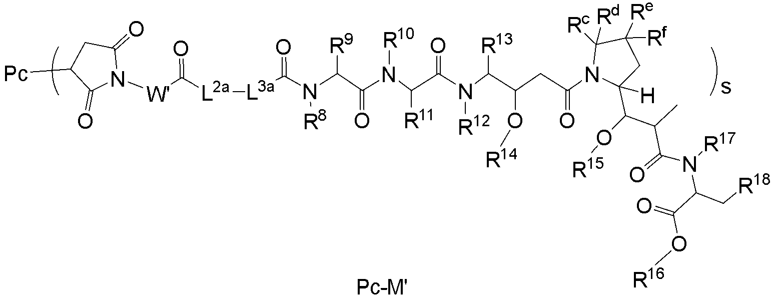 wherein:
Pc is an antigen-binding molecule that specifically binds to EGFR and MUC1, wherein the antigen-binding molecule comprises one antigen-binding moiety that specifically binds to EGFR and one antigen-binding moiety that specifically binds to MUC1; the antigen-binding moiety that specifically binds to EGFR comprises a heavy chain variable region EGFR-VH and a light chain variable region EGFR-VL, and the antigen-binding moiety that specifically binds to MUC1 comprises a heavy chain variable region MUC1-VH and a light chain variable region MUC1-VL, wherein:
   the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
   preferably, the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149;
   more preferably, the antigen-binding molecule comprises one first chain comprising the amino acid sequence of SEQ ID NO: 171, one second chain comprising the amino acid sequence of SEQ ID NO: 74, one third chain comprising the amino acid sequence of SEQ ID NO: 174, and one fourth chain comprising the amino acid sequence of SEQ ID NO: 173; or one first chain comprising the amino acid sequence of SEQ ID NO: 178, one second chain comprising the amino acid sequence of SEQ ID NO: 74, one third chain comprising the amino acid sequence of SEQ ID NO: 179, and one fourth chain comprising the amino acid sequence of SEQ ID NO: 173;
   W' is selected from the group consisting of alkylene and alkylene-cycloalkyl, wherein the alkylene and alkylene-cycloalkyl are each independently and optionally substituted with one or more substituents selected from the group consisting of halogen, hydroxy, cyano, amino, alkyl, haloalkyl, deuterated alkyl, alkoxy, and cycloalkyl;
   L^{2a} is selected from the group consisting of -NR^{4a}(CH₂CH₂O)p²CH₂CH₂C(O)-, -NR^{4a}(CH₂CH₂O)p²CH₂C(O)-, -S(CH₂)p²C(O)-, and a chemical bond, wherein p² is an integer from 1 to 20;
   L^{3a} is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, haloalkyl, deuterated alkyl, alkoxy, and cycloalkyl;
   R^{4a} is selected from the group consisting of hydrogen, alkyl, haloalkyl, deuterated alkyl, and hydroxyalkyl;
   R^{c}, R^{d}, R^{e}, and R^{f} are identical or different, and at least one of them is selected from the group consisting of halogen, alkenyl, alkyl, and cycloalkyl, and the rest are hydrogen; or any two of R^{c}, R^{d}, R^{e}, and R^{f}, together with the carbon atom(s) to which they are attached, form cycloalkyl, and the rest are optionally selected from the group consisting of hydrogen, alkyl, and cycloalkyl;
   R⁸, R¹⁰, R¹², and R¹⁴ to R¹⁷ are identical or different and are each independently selected from the group consisting of hydrogen, alkyl, haloalkyl, hydroxyalkyl, cycloalkyl, heterocyclyl, aryl, and heteroaryl;
   R⁹, R¹¹, and R¹³ are identical or different and are each independently selected from the group consisting of hydrogen, halogen, hydroxy, cyano, amino, alkyl, haloalkyl, hydroxyalkyl, alkoxy, cycloalkyl, heterocyclyl, aryl, and heteroaryl;
   R¹⁸ is aryl or heteroaryl, and the aryl or heteroaryl is optionally further substituted with a substituent selected from the group consisting of hydrogen, halogen, hydroxy, alkyl, haloalkyl, hydroxyalkyl, alkoxy, cycloalkyl, heterocyclyl, aryl, and heteroaryl;
   s is 1 to 10.

In some embodiments, provided is the compound represented by general formula (PC-M') or the pharmaceutically acceptable salt thereof according to the foregoing, wherein W' is selected from the group consisting of C₁₋₆ alkylene.

In some embodiments, provided is the compound represented by general formula (PC-M') or the pharmaceutically acceptable salt thereof according to the foregoing, wherein L^{2a} is a chemical bond.

In some embodiments, provided is the compound represented by general formula (PC-M') or the pharmaceutically acceptable salt thereof according to the foregoing, wherein L^{3a} is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl; preferably, L³ is a tetrapeptide residue; more preferably, L³ is a tetrapeptide residue represented by GGFG (SEQ ID NO: 180).

In some embodiments, provided is the compound represented by general formula (PC-M') or the pharmaceutically acceptable salt thereof according to the foregoing, wherein one of R^{c} and R^{d} and one of R^{e} and R^{f}, together with the carbon atoms to which they are attached, form 3- to 6-membered cycloalkyl, and the rest are hydrogen; preferably, one of R^{c} and R^{d} and one of R^{e} and R^{f}, together with the carbon atoms to which they are attached, form cyclopropyl, and the rest are hydrogen.

In some embodiments, provided is the compound represented by general formula (PC-M') or the pharmaceutically acceptable salt thereof according to the foregoing, wherein R⁸, R¹⁰, R¹², and R¹⁴ to R¹⁷ are identical or different and are each independently hydrogen or C₁₋₆ alkyl.

In some embodiments, provided is the compound represented by general formula (PC-M') or the pharmaceutically acceptable salt thereof according to the foregoing, wherein R⁸, R¹², R¹⁴, and R¹⁵ are identical or different and are each independently hydrogen or C₁₋₆ alkyl.

In some embodiments, provided is the compound represented by general formula (PC-M') or the pharmaceutically acceptable salt thereof according to the foregoing, wherein R¹⁰ and R¹⁶ are both hydrogen.

In some embodiments, provided is the compound represented by general formula (PC-M') or the pharmaceutically acceptable salt thereof according to the foregoing, wherein R¹⁷ is hydrogen.

In some embodiments, provided is the compound represented by general formula (PC-M') or the pharmaceutically acceptable salt thereof according to the foregoing, wherein R⁹, R¹¹, and R¹³ are identical or different and are each independently hydrogen or C₁₋₆ alkyl; preferably, R⁹, R¹¹, and R¹³ are identical or different and are each independently C₁₋₆ alkyl.

In some embodiments, provided is the compound represented by general formula (PC-M') or the pharmaceutically acceptable salt thereof according to the foregoing, wherein R¹⁸ is 6- to 10-membered aryl, and the 6- to 10-membered aryl is optionally further substituted with a substituent selected from the group consisting of hydrogen, halogen, hydroxy, C₁₋₆ alkyl, C₁₋₆ haloalkyl, C₁₋₆ hydroxyalkyl, and C₁₋₆ alkoxy; preferably, R¹⁸ is phenyl, and the phenyl is optionally further substituted with a substituent selected from the group consisting of hydrogen, halogen, C₁₋₆ alkyl, and C₁₋₆ haloalkyl; more preferably, R¹⁸ is phenyl, and the phenyl is optionally further substituted with halogen.

In some embodiments, provided is the compound represented by general formula (Pc-M') or the pharmaceutically acceptable salt thereof according to the foregoing, wherein:
Pc is the antigen-binding molecule that specifically binds to EGFR and MUC1 according to any one of the foregoing;
W' is selected from the group consisting of C₁₋₆ alkylene;
L^{2a} is a chemical bond;
L^{3a} is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
one of R^{c} and R^{d} and one of R^{e} and R^{f}, together with the carbon atoms to which they are attached, form 3-6 membered cycloalkyl, and the rest are optionally selected from the group consisting of hydrogen, C₁₋₆ alkyl, and 3-6 membered cycloalkyl;
R⁸, R¹⁰, R¹², and R¹⁴ to R¹⁷ are identical or different and are each independently hydrogen or C₁₋₆ alkyl;
R⁹, R¹¹, and R¹³ are identical or different and are each independently hydrogen or C₁₋₆ alkyl;
R¹⁸ is 6- to 10-membered aryl, and the 6- to 10-membered aryl is optionally further substituted with a substituent selected from the group consisting of hydrogen, halogen, hydroxy, C₁₋₆ alkyl, C₁₋₆ haloalkyl, C₁₋₆ hydroxyalkyl, and C₁₋₆ alkoxy;
s is 1 to 10.

In some embodiments, provided is the compound represented by general formula (PC-M') or the pharmaceutically acceptable salt thereof according to the foregoing, wherein:
Pc is the antigen-binding molecule that specifically binds to EGFR and MUC1 according to any one of the foregoing;
W' is selected from the group consisting of C₁₋₆ alkylene;
L^{2a} is a chemical bond;
L^{3a} is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
one of R^{c} and R^{d} and one of R^{e} and R^{f}, together with the carbon atoms to which they are attached, form cyclopropyl, and the rest are hydrogen;
R⁸, R¹⁰, R¹², and R¹⁴ to R¹⁷ are identical or different and are each independently hydrogen or C₁₋₆ alkyl;
R⁹, R¹¹, and R¹³ are identical or different and are each independently hydrogen or C₁₋₆ alkyl;
R¹⁸ is 6- to 10-membered aryl, and the 6- to 10-membered aryl is optionally further substituted with a substituent selected from the group consisting of hydrogen, halogen, hydroxy, C₁₋₆ alkyl, C₁₋₆ haloalkyl, C₁₋₆ hydroxyalkyl, and C₁₋₆ alkoxy;
s is 4 to 8.

In some embodiments, the compound represented by general formula (Pc-M') or the pharmaceutically acceptable salt thereof according to the foregoing is a compound represented by general formula (Pc-M) or a pharmaceutically acceptable salt thereof, wherein:
Pc is the antigen-binding molecule that specifically binds to EGFR and MUC1 according to any one of the foregoing; 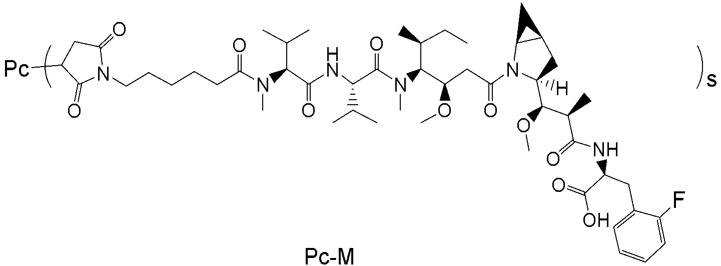
s is 4 to 6.

In another aspect, the present disclosure provides a method for preparing an antibody-drug conjugate represented by general formula (Pc-M') or a pharmaceutically acceptable salt thereof, wherein the method comprises the following step: 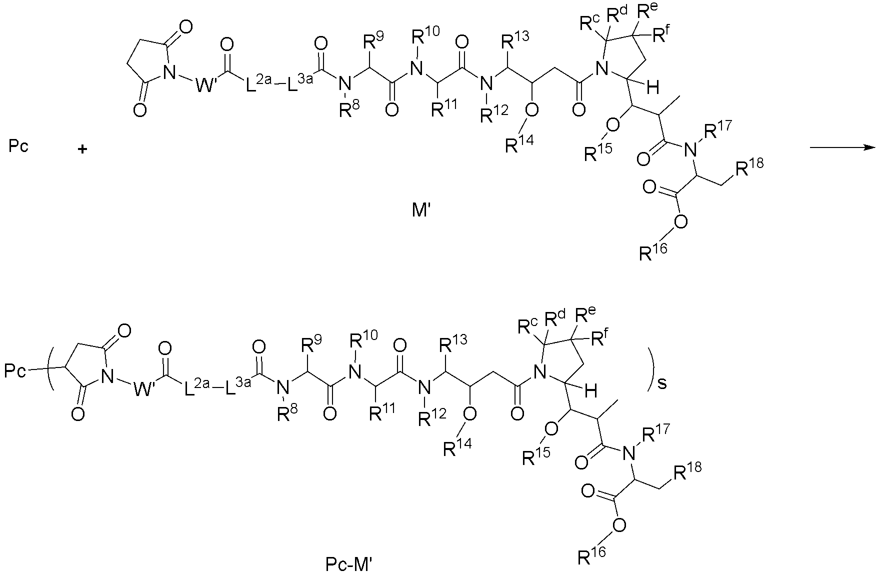 subjecting reduced Pc to a coupling reaction with compound M' or a salt thereof to give the antibody-drug conjugate represented by general formula (Pc-M') or the pharmaceutically acceptable salt thereof, wherein:
Pc is the antigen-binding molecule that specifically binds to EGFR and MUC1 according to any one of the foregoing;
W', L^{2a}, L^{3a}, R⁸ to R¹⁸, R^{c}, R^{d}, R^{e}, R^{f}, and s are as defined in any one of the foregoing. In some embodiments, s is 0 to 10 (including decimals or integers; the same applies hereinafter).

In some embodiments, s is 1 to 10; for example, s averages 1, 2, 3, 4, 5, 6, 7, 8, 9, or 10. In some embodiments, s is 1 to 8; preferably, s is 2 to 8; more preferably, s is 4 to 8; most preferably, s is 4 to 6.

In another aspect, the present disclosure provides a pharmaceutical composition comprising the anti-MUC1 antibody or the antigen-binding fragment thereof according to any one of the foregoing, the anti-EGFR antibody or the antigen-binding fragment thereof according to any one of the foregoing, the antigen-binding molecule that specifically binds to EGFR and MUC1 according to any one of the foregoing, the antibody-drug conjugate or the pharmaceutically acceptable salt thereof according to any one of the foregoing, and one or more pharmaceutically acceptable carriers, diluents, or excipients.

In some embodiments, the present disclosure provides a pharmaceutical composition comprising the anti-MUC1 antibody or the antigen-binding fragment thereof according to any one of the foregoing, and one or more pharmaceutically acceptable carriers, diluents, or excipients.

In some embodiments, the present disclosure provides a pharmaceutical composition comprising the anti-EGFR antibody or the antigen-binding fragment thereof according to any one of the foregoing, and one or more pharmaceutically acceptable carriers, diluents, or excipients.

In some embodiments, the present disclosure provides a pharmaceutical composition comprising the antigen-binding molecule that specifically binds to EGFR and MUC1 according to any one of the foregoing, and one or more pharmaceutically acceptable carriers, diluents, or excipients.

In some embodiments, the present disclosure provides a pharmaceutical composition comprising a drug conjugate of the anti-MUC1 antibody or the antigen-binding fragment thereof according to any one of the foregoing or a pharmaceutically acceptable salt thereof, and one or more pharmaceutically acceptable carriers, diluents, or excipients.

In some embodiments, the present disclosure provides a pharmaceutical composition comprising a drug conjugate of the anti-EGFR antibody or the antigen-binding fragment thereof according to any one of the foregoing or a pharmaceutically acceptable salt thereof, and one or more pharmaceutically acceptable carriers, diluents, or excipients. In some embodiments, the present disclosure provides a pharmaceutical composition comprising a drug conjugate of the antigen-binding molecule that specifically binds to EGFR and MUC1 according to any one of the foregoing (i.e., an EGFR-MUC1 bispecific antibody ADC) or a pharmaceutically acceptable salt thereof, and one or more pharmaceutically acceptable carriers, diluents, or excipients.

In another aspect, the present disclosure provides an isolated nucleic acid encoding the anti-MUC1 antibody or the antigen-binding fragment thereof according to any one of the foregoing or the anti-EGFR antibody or the antigen-binding fragment thereof according to any one of the foregoing.

In some embodiments, the present disclosure provides an isolated nucleic acid encoding the anti-MUC1 antibody or the antigen-binding fragment thereof according to any one of the foregoing.

In some embodiments, the present disclosure provides an isolated nucleic acid encoding the anti-EGFR antibody or the antigen-binding fragment thereof according to any one of the foregoing.

In another aspect, the present disclosure provides a host cell comprising the isolated nucleic acid according to any one of the above.

In another aspect, the present disclosure provides a method for preventing or treating a disease, wherein the method comprises administering to a subject a prophylactically or therapeutically effective amount of the anti-MUC1 antibody or the antigen-binding fragment thereof according to any one of the foregoing, the anti-EGFR antibody or the antigen-binding fragment thereof according to any one of the foregoing, the antigen-binding molecule that specifically binds to EGFR and MUC1 according to any one of the foregoing, the antibody-drug conjugate or the pharmaceutically acceptable salt thereof according to any one of the foregoing, or a pharmaceutical composition thereof.

In some embodiments, the method for preventing or treating a disease according to the foregoing comprises administering to the subject a prophylactically or therapeutically effective amount of the anti-MUC1 antibody or the antigen-binding fragment thereof according to any one of the foregoing or a pharmaceutical composition thereof.

In some embodiments, the method for preventing or treating a disease according to the foregoing comprises administering to the subject a prophylactically or therapeutically effective amount of the anti-EGFR antibody or the antigen-binding fragment thereof according to any one of the foregoing or a pharmaceutical composition thereof.

In some embodiments, the method for preventing or treating a disease according to the foregoing comprises administering to the subject a prophylactically or therapeutically effective amount of the antigen-binding molecule that specifically binds to EGFR and MUC1 according to any one of the foregoing or a pharmaceutical composition thereof. In some embodiments, the method for preventing or treating a disease according to the foregoing comprises administering to the subject a prophylactically or therapeutically effective amount of a drug conjugate of the anti-MUC1 antibody or the antigen-binding fragment thereof according to any one of the foregoing or a pharmaceutically acceptable salt thereof or a pharmaceutical composition thereof.

In some embodiments, the method for preventing or treating a disease according to the foregoing comprises administering to the subject a prophylactically or therapeutically effective amount of a drug conjugate of the anti-EGFR antibody or the antigen-binding fragment thereof according to any one of the foregoing or a pharmaceutically acceptable salt thereof or a pharmaceutical composition thereof.

In some embodiments, the method for preventing or treating a disease according to the foregoing comprises administering to the subject a prophylactically or therapeutically effective amount of a drug conjugate of the antigen-binding molecule that specifically binds to EGFR and MUC1 according to any one of the foregoing (i.e., an EGFR-MUC1 bispecific antibody ADC) or a pharmaceutically acceptable salt thereof or a pharmaceutical composition thereof.

In another aspect, the present disclosure provides use in the manufacture of a medicament for preventing or treating a disease, wherein the use comprises administering to a subject a prophylactically or therapeutically effective amount of the anti-MUC1 antibody or the antigen-binding fragment thereof according to any one of the above, the anti-EGFR antibody or the antigen-binding fragment thereof according to any one of the foregoing, the antigen-binding molecule that specifically binds to EGFR and MUC1 according to any one of the foregoing, the antibody-drug conjugate or the pharmaceutically acceptable salt thereof according to any one of the foregoing, or a pharmaceutical composition thereof.

In some embodiments, the use in the manufacture of a medicament for preventing or treating a disease according to the foregoing comprises administering to the subject a prophylactically or therapeutically effective amount of the anti-MUC1 antibody or the antigen-binding fragment thereof according to any one of the above or a pharmaceutical composition thereof.

In some embodiments, the use in the manufacture of a medicament for preventing or treating a disease according to the foregoing comprises administering to the subject a prophylactically or therapeutically effective amount of the anti-EGFR antibody or the antigen-binding fragment thereof according to any one of the above or a pharmaceutical composition thereof.

In some embodiments, the use in the manufacture of a medicament for preventing or treating a disease according to the foregoing comprises administering to the subject a prophylactically or therapeutically effective amount of the antigen-binding molecule that specifically binds to EGFR and MUC1 according to any one of the above or a pharmaceutical composition thereof.

In some embodiments, the use in the manufacture of a medicament for preventing or treating a disease according to the foregoing comprises administering to the subject a prophylactically or therapeutically effective amount of a drug conjugate of the anti-MUC1 antibody or the antigen-binding fragment thereof according to any one of the above or a pharmaceutically acceptable salt thereof or a pharmaceutical composition thereof.

In some embodiments, the use in the manufacture of a medicament for preventing or treating a disease according to the foregoing comprises administering to the subject a prophylactically or therapeutically effective amount of a drug conjugate of the anti-EGFR antibody or the antigen-binding fragment thereof according to any one of the above or a pharmaceutically acceptable salt thereof or a pharmaceutical composition thereof.

In some embodiments, the use in the manufacture of a medicament for preventing or treating a disease according to the foregoing comprises administering to the subject a prophylactically or therapeutically effective amount of a drug conjugate of the antigen-binding molecule that specifically binds to EGFR and MUC1 according to any one of the above (i.e., an EGFR-MUC1 bispecific antibody ADC) or a pharmaceutically acceptable salt thereof.

In another aspect, the present disclosure provides the anti-MUC1 antibody or the antigen-binding fragment thereof according to any one of the above, the anti-EGFR antibody or the antigen-binding fragment thereof according to any one of the foregoing, the antigen-binding molecule that specifically binds to EGFR and MUC1 according to any one of the foregoing, the antibody-drug conjugate or the pharmaceutically acceptable salt thereof according to any one of the foregoing, or a pharmaceutical composition thereof, for use as a medicament. In some embodiments, the medicament is used for preventing or treating a disease.

In some embodiments, provided is the anti-MUC1 antibody or the antigen-binding fragment thereof according to any one of the above or a pharmaceutical composition thereof for use as a medicament. In some embodiments, the medicament is used for preventing or treating a disease.

In some embodiments, provided is the anti-EGFR antibody or the antigen-binding fragment thereof according to any one of the above or a pharmaceutical composition thereof for use as a medicament. In some embodiments, the medicament is used for preventing or treating a disease.

In some embodiments, provided is the antigen-binding molecule that specifically binds to EGFR and MUC1 according to any one of the above or a pharmaceutical composition thereof for use as a medicament. In some embodiments, the medicament is used for preventing or treating a disease.

In some embodiments, provided is a drug conjugate of the anti-MUC1 antibody or the antigen-binding fragment thereof according to any one of the above or a pharmaceutically acceptable salt thereof or a pharmaceutical composition thereof for use as a medicament. In some embodiments, the medicament is used for preventing or treating a disease.

In some embodiments, provided is a drug conjugate of the anti-EGFR antibody or the antigen-binding fragment thereof according to any one of the above or a pharmaceutically acceptable salt thereof or a pharmaceutical composition thereof for use as a medicament. In some embodiments, the medicament is used for preventing or treating a disease.

In some embodiments, provided is a drug conjugate of the antigen-binding molecule that specifically binds to EGFR and MUC1 according to any one of the above (i.e., an EGFR-MUC1 bispecific antibody ADC) or a pharmaceutically acceptable salt thereof or a pharmaceutical composition thereof for use as a medicament. In some embodiments, the medicament is used for preventing or treating a disease.

In some embodiments, the disease according to any one of the above is a tumor; in some embodiments, the disease is selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutation), cervical cancer, colorectal cancer (e.g., colon cancer and rectal cancer), fallopian tube cancer, gallbladder cancer, gastric cancer, head and neck cancer, idiopathic myelofibrosis, renal cancer (e.g., renal cell carcinoma, rhabdoid tumor of the kidney, and Wilms tumor), leukemia, liver cancer (e.g., hepatocellular carcinoma), esophageal cancer (e.g., esophageal squamous cell carcinoma), lung cancer (e.g., non-small cell lung cancer and small cell lung cancer), medulloblastoma, melanoma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, sarcoma (e.g., chondrosarcoma, Ewing sarcoma, osteosarcoma, rhabdomyosarcoma, synovial sarcoma, and soft tissue sarcoma), squamous cell carcinoma (e.g., cutaneous squamous cell carcinoma), thyroid cancer, endometrial cancer, vestibular schwannoma, blastoma, vulvar cancer, thymoma, testicular cancer, cholangiocarcinoma, pheochromocytoma, paraganglioma, and adenoid cystic carcinoma; in some embodiments, the cancer is selected from the group consisting of lung cancer, head and neck cancer, esophageal cancer, breast cancer, pancreatic cancer, prostate cancer, thyroid cancer, gastric cancer, ovarian cancer, colorectal cancer, liver cancer, gallbladder cancer, renal cancer, cervical cancer, and bladder cancer. In some embodiments, the cancer is lung cancer, preferably non-small cell lung cancer.

The ordinal numbers "first", "second", "third", "1", "2", "3", etc. (e.g., "first subunit", "Fc2", "third chain", and "linker 2") in the present disclosure are used only to distinguish between different features, elements, components, or steps and are not intended to limit the quantity, order, or level.

### BRIEF DESCRIPTION OF THE DRAWINGS

FIGs. 1 to 2 show the results of *in vitro* internalization experiments of chimeric antibodies on T47D cells.
FIGs. 3A to 3E show FACS-based experimental results demonstrating the affinity of chimeric antibodies and humanized antibodies for HCC827-human-MUC1-C cells.
FIGs. 3F to 3H show FACS-based experimental results demonstrating the affinity of chimeric antibodies and humanized antibodies for CHOK1-human-MUC1-C cells.
FIGs. 3I to 3P show FACS-based experimental results demonstrating the affinity of chimeric antibodies and humanized antibodies for T47D (with high MUC1 expression and low EGFR expression) cells.
FIGs. 4A to 4F show the results of *in vitro* internalization experiments of MUC1-C humanized antibodies on T47D cells.
FIG. 5 shows a schematic diagram of the molecular format of EGFR-MUC1 bispecific antibodies.
FIGs. 6A to 6B show experimental results demonstrating the killing activity of EGFR-MUC1 bispecific antibody ADCs with different mutations (conjugated to toxin M) against tumor cell lines HCC827 (with high EGFR expression and low MUC1 expression) and HCC70 (with moderate EGFR expression and moderate MUC1 expression).
FIG. 7A shows the tumor volume change curves of different groups over study days in an HCC827 CDX mouse model.
FIG. 7B shows the mouse body weight change curves of different groups over study days in an HCC827 CDX mouse model.
FIG. 8A shows the tumor volume change curves of different groups over study days in an HPAC CDX mouse model.
FIG. 8B shows the mouse body weight change curves of different groups over study days in an HPAC CDX mouse model.

### DETAILED DESCRIPTION

### Terminology

To facilitate the understanding of the present disclosure, certain technical and scientific terms are described below. Unless otherwise specifically defined herein, all technical and scientific terms used herein have the same meaning as commonly understood by those of ordinary skill in the art.

The singular forms "a", "an", and "the" used in the description and claims include plural reference unless the context clearly dictates otherwise.

Unless otherwise clearly stated in the context, throughout the description and the claims, the words "comprise", "have", "include", and the like, should be construed in an inclusive sense as opposed to an exclusive or exhaustive sense.

The term "cytokine" is a general term for proteins that are released by a cell population to act on other cells as intercellular mediators. Examples of such cytokines include lymphokines, monokines, chemokines, and traditional polypeptide hormones. Exemplary cytokines include mIL-2, IFNγ, TNFα, CCL-2, and IL-6.

The term "and/or" is meant to include the two meanings "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used in the present disclosure are as described in J. Biol. Chem., 243, p3558 (1968).

The term "MUC1" refers to a full-length MUC1 protein. Human MUC1 comprises the amino acid sequence of SEQ ID NO: 2. The amino acid sequences of MUC1 molecules from non-human species (e.g., mice, monkeys, rabbits, dogs, and pigs) may be obtained from public sources.

The term "amino acid" refers to naturally occurring and synthetic amino acids, as well as amino acid analogs and amino acid mimics that function similarly to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have an identical basic chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain an identical basic chemical structure to naturally occurring amino acids. Amino acid mimics refer to chemical compounds that have a structure different from the general chemical structure of amino acids, but function similarly to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., the replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression "position + amino acid residue" may be used to denote an amino acid residue at a specific position. For example, 82aR means that the amino acid residue at position 82a is R. S82aR means that the amino acid residue at position 82a (also referred to as 82A) is mutated from the original S to R.

The term "antigen-binding molecule" is used in the broadest sense and encompasses a variety of molecules that specifically bind to an antigen, including but not limited to antibodies, other polypeptides having antigen binding activity, and antibody fusion proteins in which the two are fused, as long as they exhibit the desired antigen binding activity. The antigen-binding molecule herein comprises a variable region (VH) and a variable region (VL) which together comprise an antigen-binding domain. Illustratively, the antigen-binding molecule herein is a bispecific antigen-binding molecule (e.g., a bispecific antibody).

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), as long as they exhibit the desired antigen-binding activity. For example, a natural IgG antibody is a heterotetrameric glycoprotein of about 150,000 Daltons composed of two light chains and two heavy chains linked by a disulfide bond. From the N-terminus to the C-terminus, each heavy chain comprises one variable region (VH), also known as variable heavy domain or heavy chain variable region, followed by three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of symmetry in their structures. Antibody-fragment-type bispecific antibodies, e.g., Fab fragments lacking Fc fragments, are formed by combining two or more Fab fragments in one molecule. They have relatively low immunogenicity, are small in molecular weight, and have relatively high tumor tissue permeability. Typical antibody structures of this type include, for example, F(ab)₂, scFv-Fab, and (scFv)₂-Fab. IgG-like bispecific antibodies (e.g., comprising Fc fragments) are relatively large in molecular weight. The Fc fragments facilitate the purification of the antibodies and increase their solubility and stability, and the Fc portions may also bind to the receptor FcRn, increasing the serum half-life of the antibodies.

"Natural antibody" refers to a naturally occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain comprises one variable region (VH), also known as variable heavy domain or heavy chain variable region, followed by heavy chain constant regions. Generally, a natural IgG heavy chain constant region comprises three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL). The terms "full-length antibody", "intact antibody", and "whole antibody" are used herein interchangeably and refer to an antibody having a substantially similar structure to a natural antibody structure or comprising heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" of an antibody refers to a domain in an antibody heavy or light chain that is involved in the binding of the antibody to an antigen. Herein, the heavy chain variable region (VH) and light chain variable region (VL) of the antibody each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278), and the like. The corresponding relationships between the various numbering schemes are well known to those skilled in the art and, illustratively, are shown in Table 1 below.

**Table 1. The relationships between CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the variable regions and CDRs in examples of the present disclosure. Although one numbering scheme (e.g., Kabat) is employed to define amino acid residues in specific embodiments, corresponding technical solutions for other numbering schemes are to be considered as equivalent technical solutions.

The term "antibody fragment" refers to a molecule different from an intact antibody, which comprises a moiety of an intact antibody that binds to an antigen to which the intact antibody binds. Examples of antibody fragments include, but are not limited to, Fd, Fv, Fab, dsFv, dAb, Fab', Fab'-SH, F(ab')2, single-domain antibodies, single-chain Fab (scFab), diabodies, linear antibodies, single-chain antibodies (e.g., scFv), and multispecific antibodies formed from antibody fragments.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" describes an antibody in which a portion of the heavy chain and/or the light chain is derived from a particular source or species, while the remainder of the heavy chain and/or the light chain is derived from a different source or species.

The term "humanized" antibody refers to an antibody that retains the reactivity of a non-human antibody while having low immunogenicity in humans. For example, the humanization can be achieved by retaining the non-human CDRs and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable regions and constant regions are human sequences. The term encompasses antibodies that are derived from human genes but have, for example, sequences that have been altered to, e.g., reduce possible immunogenicity, increase affinity, and eliminate cysteines or glycosylation sites that may cause undesired folding. The term encompasses antibodies recombinantly produced in non-human cells (that may confer glycosylation not characteristic of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the 1:1 interaction between members of a binding pair (e.g., an antibody and an antigen). The affinity of molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system (e.g., Biacore), or affinity in a solution is determined by solution equilibrium titration (SET).

The term "surface plasmon resonance" refers to an optical phenomenon that allows for the analysis of real-time interactions by detecting changes in protein concentrations within a biosensor matrix, for example, using the BIAcoreTM system (Biacore Life Sciences division of GE Healthcare, Piscataway, NJ).

The term "effector function" refers to biological activities that can be attributed to the Fc region of an antibody (either the natural sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, a polyclonal antibody formulation generally comprises several different antibodies comprising different amino acid sequences in their variable domains, which are generally specific for different epitopes. "Monoclonal" refers to the characteristics of an antibody obtained from a substantially homogeneous antibody population and should not be construed as requiring the production of the antibody by any particular method. In some embodiments, the antibody provided by the present disclosure is a monoclonal antibody.

The term "antigen" refers to a molecule or molecular portion of an antibody that can be selectively recognized or bound by, for example, an antigen-binding molecule (including, for example, antibodies). An antigen may have one or more epitopes capable of interacting with different antigen-binding molecules (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (Prot. Sci. 9 (2000) 487-496), and cross-blocking. The term "capable of specifically binding", "specifically bind", or "bind" means that an antibody is capable of binding to a certain antigen or an epitope of the antigen with higher affinity than to other antigens or epitopes. Generally, an antibody binds to an antigen or an epitope thereof with an equilibrium dissociation constant (KD) of about 1 × 10⁻⁷ M or less (e.g., about 1 × 10⁻⁸ M, 1 × 10⁻⁹ M, 1 × 10⁻¹⁰ M, 1 × 10⁻¹¹ M, or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE^{®} surface plasmon resonance assay. However, an antibody that specifically binds to an antigen or an epitope thereof may have cross-reactivity to other related antigens, e.g., to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The term "antigen-binding moiety" refers to a polypeptide molecule that specifically binds to an antigen of interest or an epitope thereof. A specific antigen-binding moiety includes an antigen-binding domain of an antibody; for example, it comprises a heavy chain variable region and a light chain variable region. The term "antigen-binding moiety that specifically binds to MUC1" refers to a moiety that is capable of binding to MUC1 or an epitope thereof with sufficient affinity, such that a molecule comprising the moiety can be used as a diagnostic agent and/or a therapeutic agent targeting MUC1. Antigen-binding moieties include antibody fragments as defined herein, e.g., a Fab, a replaced Fab, or an scFv.

The terms "anti-MUC1 antibody" and "antibody that binds to MUC1" refer to an antibody that is capable of binding to MUC1 or an epitope thereof with sufficient affinity.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcy receptor (FcyR) expressed on the effector cells. For example, NK cells express FcγRIIIa, while monocytes express FcyRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis (ADCP)" refers to a mechanism by which antibody-coated target cells are eliminated by internalization of phagocytic cells (such as macrophages or dendritic cells).

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. The activation of a complement may also result in the deposition of complement components on the surface of target cells, and these complement components promote CDC by binding to complement receptors on leukocytes (e.g., CR3).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized similarly to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated nucleic acid" refers to a nucleic acid molecule that has been separated from components of its natural environment. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences. The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. Mutant progenies that have the same function or biological activity as the cells screened or selected from the initially transformed cells are included herein. Host cells include prokaryotic and eukaryotic host cells, wherein the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica, Pichia, Saccharomycescerevisiae, Saccharomyces, Hansenula polymorpha, Kluyveromyces, Kluyveromyces lactis, Candida albicans, Aspergillus nidulans, Aspergillus niger, Aspergillus oryzae, Trichoderma reesei, Chrysosporium lucknowense, Fusarium sp., Fusarium gramineum, Fusarium venenatum, Physcomitrella patens,* and *Neurospora crassa. Pichia,* any *Saccharomyces, Hansenula polymorpha,* any *Kluyveromyces, Candida albicans,* any *Aspergillus, Trichoderma reesei, Chrysosporium lucknowense,* any *Fusarium, Yarrowia lipolytica,* and *Neurospora crassa.*

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). Preferably, the alkyl is an alkyl group having 1 to 12 carbon atoms (i.e., C₁₋₁₂ alkyl); more preferably, the alkyl is an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of deuterium (D), halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl is as defined above, and it has 2 to 12 (e.g., 2, 3, 4, 5, 6, 7, 8, 9, 10, 11, or 12) carbon atoms (i.e., C₂₋₁₂ alkenyl). Preferably, the alkenyl is an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of deuterium (D), alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of deuterium (D), halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e.,3- to 12-membered cycloalkyl), more preferably a cycloalkyl group having 3 to 8 ring atoms (i.e., 3- to 8-membered cycloalkyl), and most preferably a cycloalkyl group having 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, and it may contain in the rings one or more double bonds, or it may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), with the proviso that at least one all-carbon ring is contained and the point of attachment is on the all-carbon ring; it has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). Preferably, the spirocycloalkyl is a spirocycloalkyl group having 6 to 14 ring atoms (i.e., 6- to 14-membered spirocycloalkyl); more preferably, the spirocycloalkyl is a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl); monospirocycloalkyl or bispirocycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl is more preferred. Non-limiting examples include: 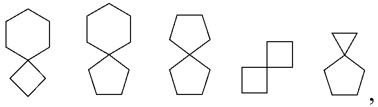 wherein the point of attachment may be at any position; 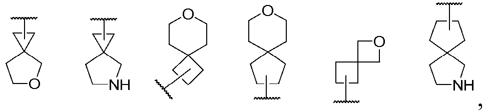 and the like.

The term "fused cycloalkyl" refers to a polycyclic system in which two adjacent carbon atoms are shared between rings, and it is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group, and it may contain one or more double bonds in the rings and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). Preferably, the fused cycloalkyl is a fused cycloalkyl group having 6 to 14 ring atoms (i.e., 6- to 14-membered fused cycloalkyl); more preferably, the fused cycloalkyl is a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl); bicyclic fused cycloalkyl or tricyclic fused cycloalkyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl is more preferred. Non-limiting examples include: 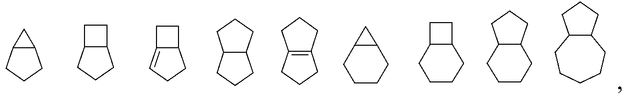 wherein the point of attachment may be at any position; 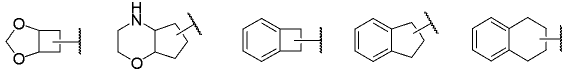 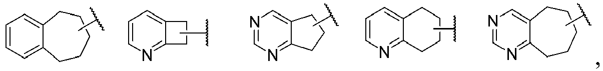 and the like.

The term "bridged cycloalkyl" refers to an all-carbon polycyclic system in which two carbon atoms that are not directly connected are shared between rings, and it may contain one or more double bonds in the rings and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). Preferably, the bridged cycloalkyl is a bridged cycloalkyl group having 6 to 14 carbon atoms (i.e., 6- to 14-membered bridged cycloalkyl); more preferably, the bridged cycloalkyl is a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl); bicyclic bridged cycloalkyl or tricyclic bridged cycloalkyl is preferred. Non-limiting examples include: 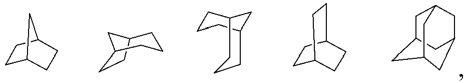 wherein the point of attachment may be at any position.

Cycloalkyl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). Preferably, the heterocyclyl is a heterocyclyl group having 3 to 12 ring atoms (i.e., 3- to 12-membered heterocyclyl), e.g., 4- to 12-membered heterocyclyl containing at least one nitrogen atom; further preferably, the heterocyclyl is a heterocyclyl group having 3 to 8 ring atoms (i.e., 3- to 8-membered heterocyclyl); more preferably, the heterocyclyl is a heterocyclyl group having 3 to 6 ring atoms (i.e., 3- to 6-membered heterocyclyl); most preferably, the heterocyclyl is a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded), with the proviso that at least one monocyclic heterocyclyl group is contained and the point of attachment is on the monocyclic heterocyclyl group; it has 5 to 20 (e.g., 5, 6, 7, 8, 9, 10, 11, 12, 13, 14, 15, 16, 17, 18, 19, or 20) ring atoms (i.e., 5-to 20-membered spiroheterocyclyl). Preferably, the spiroheterocyclyl is a spiroheterocyclyl group having 6 to 14 ring atoms (i.e., 6- to 14-membered spiroheterocyclyl); more preferably, the spiroheterocyclyl is a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl); monospiroheterocyclyl or bispiroheterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl is more preferred. Non-limiting examples include: 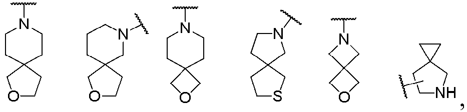 and the like.

The term "fused heterocyclyl" refers to a polycyclic heterocyclic system in which two adjacent atoms are shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded); it is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group, and it has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). Preferably, the fused heterocyclyl is a fused heterocyclyl group having 6 to 14 ring atoms (i.e., 6- to 14-membered fused heterocyclyl); more preferably, the fused heterocyclyl is a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl); bicyclic fused heterocyclyl or tricyclic fused heterocyclyl is preferred, and 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl is more preferred. Non-limiting examples include: 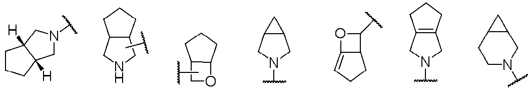 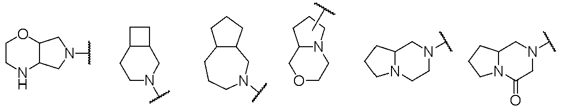 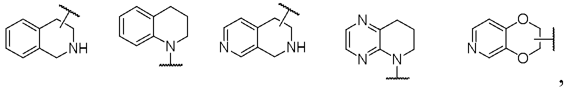 and the like.

The term "bridged heterocyclyl" refers to a polycyclic heterocyclic system in which two atoms that are not directly connected are shared between rings, and it may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded); it has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). Preferably, the bridged heterocyclyl is a bridged heterocyclyl group having 6 to 14 ring atoms (i.e., 6- to 14-membered bridged heterocyclyl); more preferably, the bridged heterocyclyl is a bridged heterocyclyl group having 7 to 10 ring atoms (i.e., 7- to 10-membered bridged heterocyclyl). According to the number of constituent rings, the bridged heterocyclyl can be divided into bicyclic bridged heterocyclyl and polycyclic bridged heterocyclyl (e.g., tricyclic bridged heterocyclyl and tetracyclic bridged heterocyclyl); bicyclic bridged heterocyclyl or tricyclic bridged heterocyclyl is preferred. Non-limiting examples include: 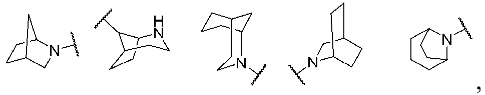 and the like.

Heterocyclyl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, and it has 6 to 14 (e.g., 6, 7, 8, 9, 10, 11, 12, 13, or 14) ring atoms (i.e., 6- to 14-membered aryl). Preferably, the aryl is an aryl group having 6 to 10 ring atoms (i.e., 6- to 10-membered aryl). The monocyclic aryl is, for example, phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include: 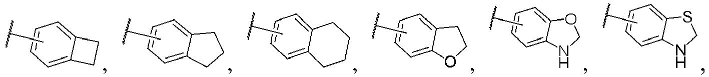 and the like.

Aryl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 5 to 14 (e.g., 5, 6, 7, 8, 9, 10, 11, 12, 13, or 14) ring atoms (i.e., 5- to 14-membered heteroaryl). Preferably, the heteroaryl is a heteroaryl group having 5 to 10 ring atoms (i.e., 5- to 10-membered heteroaryl); more preferably, the heteroaryl is a monocyclic heteroaryl group having 5 or 6 ring atoms (i.e., 5- or 6-membered monocyclic heteroaryl) or a bicyclic heteroaryl group having 8 to 10 ring atoms (i.e., 8- to 10-membered bicyclic heteroaryl); most preferably, the heteroaryl is a 5- or 6-membered monocyclic heteroaryl group containing in the ring 1, 2, or 3 heteroatoms selected from the group consisting of nitrogen, oxygen, and sulfur or an 8-to 10-membered bicyclic heteroaryl group containing in the ring 1, 2, or 3 heteroatoms selected from the group consisting of nitrogen, oxygen, and sulfur.

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridyl, pyrimidinyl, pyridonyl, N-alkylpyridinone (e.g., 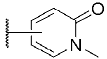 ), pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances the number of ring atoms continues to represent the number of ring atoms in the polycyclic heteroaromatic ring system. Non-limiting examples include: 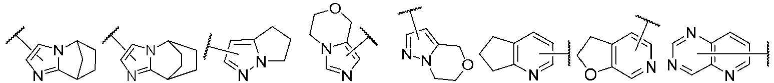 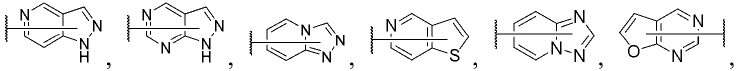 and the like.

Heteroaryl may be substituted or unsubstituted, and when it is substituted, it may be substituted at any accessible point of attachment, and the substituent is preferably selected from the group consisting of one or more of deuterium (D), halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The cycloalkyl, heterocyclyl, aryl, and heteroaryl described above include residues derived by removing one hydrogen atom from a ring atom of the parent structure, or residues derived by removing two hydrogen atoms from the same ring atom or two different ring atoms of the parent structure, i.e., "divalent cycloalkyl", "divalent heterocyclyl", "arylene", and "heteroarylene".

In the chemical structure of the compound of the present disclosure, the bond "/" indicates an unspecified configuration; that is, if chiral isomers exist in the chemical structure, the bond " 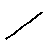 " may be " 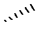 " or " 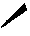 ", or includes both the configurations " 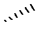 " and " 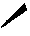 ".

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (tritium, T) , ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I; deuterium is preferred.

Compared to non-deuterated drugs, deuterated drugs have the advantages of reduced toxic and side effects, increased drug stability, enhanced efficacy, prolonged biological half-lives, and the like. All isotopic variations of the compound of the present disclosure, whether radioactive or not, are included within the scope of the present disclosure. Each available hydrogen atom attached to a carbon atom may be independently replaced with a deuterium atom, wherein the replacement with deuterium may be partial or complete. The partial replacement with deuterium refers to the replacement of at least one hydrogen atom with at least one deuterium atom.

"Optional" or "optionally" means that the event or circumstance subsequently described may, but does not necessarily, occur. This description includes the instance where the event or circumstance occurs or does not occur.

The term "pharmaceutical composition" refers to a mixture comprising one or more of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof described herein, and other chemical components. The other components are, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates, sheep, dogs, cows, chickens, amphibians, and reptiles. Unless indicated, the terms "patient" and "subject" are used interchangeably herein. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells, or tissues isolated from a subject, as well as fluids, cells, or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody or the antigen-binding fragment thereof, the antigen-binding molecule, or the antibody-drug conjugate of the present disclosure is used to delay the development of a disease or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

### Anti-MUC1 Antibody or Antigen-Binding Fragment Thereof, Anti-EGFR Antibody or Antigen-Binding Fragment Thereof, and Antigen-binding Molecule That Specifically Binds to EGFR and MUC1 of Present Disclosure

The present disclosure provides an anti-MUC1 antibody or an antigen-binding fragment thereof, an anti-EGFR antibody or an antigen-binding fragment thereof, and an antigen-binding molecule that specifically binds to EGFR and MUC1, which have a number of advantageous properties, such as good *in vitro* killing activity, therapeutic activity, safety, pharmacokinetic properties, and druggability (e.g., yield, purity, and stability).

### Exemplary Anti-MUC1 Antibody or Antigen-Binding Fragment Thereof

An example of the present disclosure discloses antibody series M4, M6, F4-1, and F4-18. Antibody M4 is taken as an example below to describe the antibody or the antigen-binding fragment thereof of the present disclosure.

Illustratively, the anti-MUC1 antibody or the antigen-binding fragment thereof of the present disclosure comprises a heavy chain variable region and a light chain variable region, wherein the heavy chain variable region comprises a HCDR1, a HCDR2, and a HCDR3 that comprise the amino acid sequences of a HCDR1, a HCDR2, and a HCDR3 in SEQ ID NO: 4, respectively, and the light chain variable region comprises a LCDR1, a LCDR2, and a LCDR3 that comprise the amino acid sequences of a LCDR1, a LCDR2, and a LCDR3 in SEQ ID NO: 5, respectively.

Illustratively, the anti-MUC1 antibody or the antigen-binding fragment thereof of the present disclosure comprises a heavy chain variable region and a light chain variable region, wherein the heavy chain variable region comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the light chain variable region comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17.

Illustratively, the anti-MUC1 antibody or the antigen-binding fragment thereof of the present disclosure comprises a heavy chain variable region and a light chain variable region, wherein the heavy chain variable region comprises a HCDR1 whose amino acid sequence is set forth in SEQ ID NO: 12, a HCDR2 whose amino acid sequence is set forth in SEQ ID NO: 13, and a HCDR3 whose amino acid sequence is set forth in SEQ ID NO: 14, and the light chain variable region comprises a LCDR1 whose amino acid sequence is set forth in SEQ ID NO: 15, a LCDR2 whose amino acid sequence is set forth in SEQ ID NO: 16, and a LCDR3 whose amino acid sequence is set forth in SEQ ID NO: 17.

Illustratively, the anti-MUC1 antibody or the antigen-binding fragment thereof of the present disclosure is a murine antibody, a chimeric antibody, a humanized antibody, or a fully human-derived antibody. In some embodiments, the anti-MUC1 antibody or the antigen-binding fragment thereof of the present disclosure is a chimeric antibody or a fully human-derived antibody. In some embodiments, the anti-MUC1 antibody or the antigen-binding fragment thereof is a humanized antibody.

Illustratively, the anti-MUC1 antibody or the antigen-binding fragment thereof of the present disclosure comprises human antibody framework regions (FRs).

Illustratively, provided is the anti-MUC1 antibody or the antigen-binding fragment thereof of the present disclosure, wherein the heavy chain variable region comprises an amino acid sequence having at least 70% sequence identity to SEQ ID NO: 36, 37, or 38, and the light chain variable region comprises an amino acid sequence having at least 70% sequence identity to SEQ ID NO: 39, 40, 41, or 42; or the heavy chain variable region comprises an amino acid sequence having at least 70% sequence identity to SEQ ID NO: 4, and the light chain variable region comprises an amino acid sequence having at least 70% sequence identity to SEQ ID NO: 5.

Illustratively, provided is the anti-MUC1 antibody or the antigen-binding fragment thereof of the present disclosure, wherein the heavy chain variable region comprises a FR1, a FR2, and a FR3 that are derived from IGHV1-46*01 and a FR4 derived from IGHJ6*01, and the FRs are unsubstituted or comprise one or more amino acid substitutions selected from the group consisting of 1E, 28S, 38K, 40R, 48I, 71A, 73K, 76D, and 82aR; and/or the light chain variable region comprises a FR1, a FR2, and a FR3 that are derived from IGKV1-39*01, 1GKV6-21*02, or IGKV3-11*01 and a FR4 derived from IGKJ4*01, and the FRs are unsubstituted or comprise one or more amino acid substitutions selected from the group consisting of 3V, 43S, 47W, 49Y, and 60G. In some embodiments, the variable regions and CDRs described above are defined according to the Kabat numbering scheme.

In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise one or more amino acid substitutions selected from the group consisting of 1E, 28S, 38K, 40R, 48I, 71A, 73K, 76D, and 82aR; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise one or more amino acid substitutions selected from the group consisting of 3V, 43S, 47W, 49Y, and 60G. In some embodiments, the variable regions and CDRs described above are defined according to the Kabat numbering scheme.

In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise one or more amino acid substitutions selected from the group consisting of 1E, 28S, 38K, 40R, 48I, 71A, 73K, 76D, and 82aR; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise one or more amino acid substitutions selected from the group consisting of 3V, 43S, and 47W. In some embodiments, the variable regions and CDRs described above are defined according to the Kabat numbering scheme.

In some embodiments, provided is the anti-MUC1 antibody or the antigen-binding fragment thereof, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14, and the FRs of the heavy chain variable region comprise amino acid substitutions of 1E, 71A, 73K, and 76D; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17, and the FRs of the light chain variable region comprise amino acid substitutions of 43S and 47W. In some embodiments, the variable regions and CDRs described above are defined according to the Kabat numbering scheme.

Illustratively, the anti-MUC1 antibody or the antigen-binding fragment thereof of the present disclosure is an antibody fragment; in some embodiments, the antibody fragment is selected from the group consisting of Fab, Fab', F(ab')2, Fd, Fv, scFv, dsFv, and dAb.

Illustratively, the anti-MUC1 antibody or the antigen-binding fragment thereof of the present disclosure comprises a heavy chain constant region and a light chain constant region. In some embodiments, the heavy chain constant region is a human IgG1, IgG2, IgG3, or IgG4 heavy chain constant region. In some embodiments, the light chain constant region is a human κ or λ light chain constant region. In some embodiments, the heavy chain constant region comprises the amino acid sequence of SEQ ID NO: 69 or 186, and the light chain constant region comprises the amino acid sequence of SEQ ID NO: 70. In some embodiments, the heavy chain constant region comprises the amino acid sequence of SEQ ID NO: 69, and the light chain constant region comprises the amino acid sequence of SEQ ID NO: 70.

Illustratively, provided is the anti-MUC1 antibody or the antigen-binding fragment thereof of the present disclosure, wherein the anti-MUC1 antibody comprises a heavy chain and a light chain, wherein the heavy chain comprises an amino acid sequence having at least 70% sequence identity to SEQ ID NO: 71, 73, 75, or 77, and the light chain comprises an amino acid sequence having at least 70% sequence identity to SEQ ID NO: 72, 74, 76, or 78.

Illustratively, provided is the anti-MUC1 antibody or the antigen-binding fragment thereof of the present disclosure, wherein the anti-MUC1 antibody comprises a heavy chain and a light chain, wherein:
the amino acid sequence of the heavy chain is set forth in SEQ ID NO: 71, and the amino acid sequence of the light chain is set forth in SEQ ID NO: 72; or
the amino acid sequence of the heavy chain is set forth in SEQ ID NO: 73, and the amino acid sequence of the light chain is set forth in SEQ ID NO: 74; or
the amino acid sequence of the heavy chain is set forth in SEQ ID NO: 75, and the amino acid sequence of the light chain is set forth in SEQ ID NO: 76; or
the amino acid sequence of the heavy chain is set forth in SEQ ID NO: 77, and the amino acid sequence of the light chain is set forth in SEQ ID NO: 78.

Illustratively, provided is the anti-MUC1 antibody or the antigen-binding fragment thereof of the present disclosure, wherein the anti-MUC1 antibody comprises a heavy chain and a light chain, wherein the amino acid sequence of the heavy chain is set forth in SEQ ID NO: 71, and the amino acid sequence of the light chain is set forth in SEQ ID NO: 72.

Illustratively, the present disclosure further provides an isolated anti-MUC1 antibody or an antigen-binding fragment thereof that competes for binding to human MUC1 with the anti-MUC1 antibody according to any one of the foregoing.

Illustratively, the isolated anti-MUC1 antibody or the antigen-binding fragment thereof of the present disclosure binds to human MUC1 with a KD of less than 5 × 10⁻⁸ M (e.g., less than 4 × 10⁻⁸ M, less than 3 × 10⁻⁸ M, less than 2.5 × 10⁻⁸ M, less than 2 × 10⁻⁸ M, less than 1.5 × 10⁻⁸ M, less than 1 × 10⁻⁸ M, less than 9 × 10⁻⁹ M, less than 8 × 10⁻⁹ M, less than 7 × 10⁻⁹ M, less than 6 × 10⁻⁹ M, less than 5 × 10⁻⁹ M, less than 4 × 10⁻⁹ M, less than 3 × 10⁻⁹ M, or less than 2 × 10⁻⁹ M), as measured by Biacore.

### Exemplary Anti-EGFR Antibody or Antigen-Binding Fragment Thereof

An example of the present disclosure discloses an anti-EGFR antibody or an antigen-binding fragment thereof.

Illustratively, the anti-EGFR antibody or the antigen-binding fragment thereof of the present disclosure comprises a heavy chain variable region and a light chain variable region, wherein the heavy chain variable region comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the light chain variable region comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

Illustratively, the anti-EGFR antibody or the antigen-binding fragment thereof of the present disclosure is a murine antibody, a chimeric antibody, a humanized antibody, or a fully human-derived antibody. In some embodiments, the anti-EGFR antibody or the antigen-binding fragment thereof of the present disclosure is a chimeric antibody or a fully human-derived antibody. In some embodiments, the anti-EGFR antibody or the antigen-binding fragment thereof is a humanized antibody.

Illustratively, the anti-EGFR antibody or the antigen-binding fragment thereof of the present disclosure comprises human antibody framework regions (FRs).

Illustratively, the anti-EGFR antibody or the antigen-binding fragment thereof of the present disclosure comprises a heavy chain variable region and a light chain variable region, wherein the heavy chain variable region comprises the amino acid sequence of SEQ ID NO: 138, and the light chain variable region comprises the amino acid sequence of SEQ ID NO: 149.

Illustratively, the anti-EGFR antibody or the antigen-binding fragment thereof of the present disclosure comprises a heavy chain and a light chain, wherein the heavy chain comprises the amino acid sequence of SEQ ID NO: 153, and the light chain comprises the amino acid sequence of SEQ ID NO: 164.

### Exemplary Antigen-Binding Molecule That Specifically Binds to EGFR and MUC1

An example of the present disclosure discloses an antigen-binding molecule that specifically binds to EGFR and MUC1.

Illustratively, the antigen-binding molecule that specifically binds to EGFR and MUC1 of the present disclosure comprises at least one antigen-binding moiety that specifically binds to EGFR and at least one antigen-binding moiety that specifically binds to MUC1, wherein the antigen-binding moiety that specifically binds to EGFR comprises a heavy chain variable region EGFR-VH and a light chain variable region EGFR-VL, and the antigen-binding moiety that specifically binds to MUC1 comprises a heavy chain variable region MUC1-VH and a light chain variable region MUC1-VL, wherein: the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

Illustratively, the antigen-binding molecule that specifically binds to EGFR and MUC1 of the present disclosure comprises one first chain having a structure represented by formula (a), one second chain having a structure represented by formula (b), one third chain having a structure represented by formula (c), and one fourth chain having a structure represented by formula (d):

(a) [MUC1-VH]-[CH1]-[Fc1],

(b) [MUC1-VL]-[CL],

(c) [EGFR-VH]-[GGGGS]-[Titin]-[Fc2], and

(d) [EGFR-VL]-[GGGGS]-[Obscurin],

wherein the structures represented by formulas (a), (b), (c), and (d) are arranged from the N-terminus to the C-terminus.

Illustratively, the format of the antigen-binding molecule that specifically binds to EGFR and MUC1 of the present disclosure is an asymmetrically structured molecule comprising four chains, as shown in FIG. 5, wherein the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

Illustratively, the format of the antigen-binding molecule that specifically binds to EGFR and MUC1 of the present disclosure is an asymmetrically structured molecule comprising four chains, as shown in FIG. 5, wherein the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149.

Illustratively, the antigen-binding molecule that specifically binds to EGFR and MUC1 of the present disclosure comprises one first chain set forth in SEQ ID NO: 171, one second chain set forth in SEQ ID NO: 74, one third chain set forth in SEQ ID NO: 174, and one fourth chain set forth in SEQ ID NO: 173.

Illustratively, the antigen-binding molecule that specifically binds to EGFR and MUC1 of the present disclosure comprises one first chain set forth in SEQ ID NO: 178, one second chain set forth in SEQ ID NO: 74, one third chain set forth in SEQ ID NO: 179, and one fourth chain set forth in SEQ ID NO: 173.

### Exemplary Antibody-Drug Conjugate or Pharmaceutically Acceptable Salt Thereof

An example of the present disclosure discloses an antibody-drug conjugate represented by general formula (Pc-L-Y-D) or a pharmaceutically acceptable salt thereof: 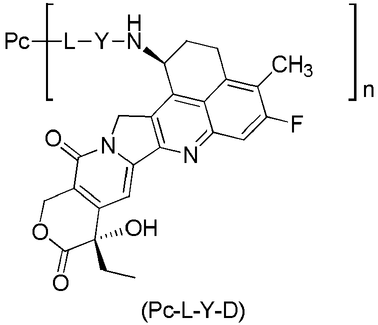 wherein:
Y is

   -O-(CR^{a}R^{b})ₘ-CR¹R²-C(O)-;
R^{a} and R^{b} are identical or different and are each independently selected from the group consisting of hydrogen, deuterium, halogen, and C₁₋₆ alkyl;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
m is 0, 1, 2, 3, or 4;
n is 2 to 8; preferably, n is 4 to 8; more preferably, n is 4 to 6;
L is a linker unit;
Pc is an antigen-binding molecule that specifically binds to EGFR and MUC1, wherein the antigen-binding molecule comprises one antigen-binding moiety that specifically binds to EGFR and one antigen-binding moiety that specifically binds to MUC1; the antigen-binding moiety that specifically binds to EGFR comprises a heavy chain variable region EGFR-VH and a light chain variable region EGFR-VL, and the antigen-binding moiety that specifically binds to MUC1 comprises a heavy chain variable region MUC1-VH and a light chain variable region MUC1-VL, wherein:
   the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
   preferably, the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149;
   more preferably, the antigen-binding molecule comprises one first chain comprising the amino acid sequence of SEQ ID NO: 171, one second chain comprising the amino acid sequence of SEQ ID NO: 74, one third chain comprising the amino acid sequence of SEQ ID NO: 174, and one fourth chain comprising the amino acid sequence of SEQ ID NO: 173; or one first chain comprising the amino acid sequence of SEQ ID NO: 178, one second chain comprising the amino acid sequence of SEQ ID NO: 74, one third chain comprising the amino acid sequence of SEQ ID NO: 179, and one fourth chain comprising the amino acid sequence of SEQ ID NO: 173.

Illustratively, the antibody-drug conjugate represented by general formula (Pc-L-Y-D) or the pharmaceutically acceptable salt thereof of the present disclosure is an antibody-drug conjugate represented by general formula (Pc-Lₐ-Y-D) or a pharmaceutically acceptable salt thereof: 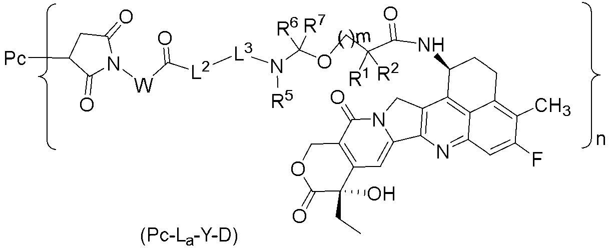 wherein:
Pc is the antigen-binding molecule that specifically binds to EGFR and MUC1 according to the foregoing;
m is 0, 1, 2, 3, or 4;
n is 2 to 8; preferably, n is 4 to 8; more preferably, n is 4 to 6;
R¹ is 3- to 6-membered cycloalkyl-C₁₋₆ alkyl or 3- to 6-membered cycloalkyl;
R² is selected from the group consisting of hydrogen, C₁₋₆ haloalkyl, and 3- to 6-membered cycloalkyl;
or, R¹ and R², together with the carbon atom to which they are attached, form 3- to 6-membered cycloalkyl;
W is selected from the group consisting of C₁₋₆ alkylene and C₁₋₆ alkylene-3- to 6-membered cycloalkyl;
L² is a chemical bond;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, C₁₋₆ alkyl, C₁₋₆ haloalkyl, C₁₋₆ deuterated alkyl, C₁₋₆ alkoxy, and 3- to 6-membered cycloalkyl;
R⁵ is hydrogen or C₁₋₆ alkyl;
R⁶ and R⁷ are identical or different and are each independently hydrogen or C₁₋₆ alkyl. Illustratively, the antibody-drug conjugate represented by general formula (Pc-L-Y-D) or the pharmaceutically acceptable salt thereof of the present disclosure is an antibody-drug conjugate represented by general formula (Pc-9-A) or a pharmaceutically acceptable salt thereof: 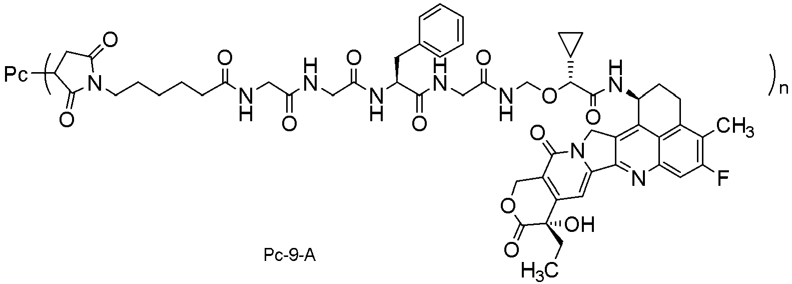 wherein:
   n is 2 to 8; preferably, n is 4 to 8; more preferably, n is 4 to 6;
   Pc is the antigen-binding molecule that specifically binds to EGFR and MUC1 according to the foregoing.

### Antibody Structure

In certain embodiments, the antibody provided herein is a full-length antibody.

In certain embodiments, the antibody provided herein is an antibody fragment.

In one embodiment, the antibody fragment is a Fab, Fab', Fab'-SH, or F(ab')₂ fragment, particularly a Fab fragment. "Fab" is a monovalent fragment consisting of the VL, VH, CL, and CH1 domains. "Fab fragment" may be produced by papain cleavage of an antibody. "Fab'" comprises VL, CL, and VH and CH1, and further comprises the region between the CH1 and CH2 domains, so that an interchain disulfide bond can be formed between the two heavy chains of two Fab' fragments to form a F(ab')2 molecule. "Fab'-SH" is a Fab' fragment in which the cysteine residues of the constant regions have a free sulfhydryl group. "F(ab')₂" is a bivalent fragment comprising two Fab fragments linked by a disulfide bond at the hinge region.

In another embodiment, the antibody fragment is a diabody, a triabody, or a tetrabody. Diabodies are antibody fragments comprising two antigen-binding sites. The fragment comprises a VH and a VL linked in the same polypeptide chain (VH-VL). By using a linker that is too short to allow pairing between two domains on the same chain, the domains are forced to pair with the complementary domains of another chain, thereby resulting in two antigen-binding sites. The two antigens may be identical or different.

In another embodiment, the antibody fragment is a single-chain Fab fragment. "Single-chain Fab fragment" or "scFab" is a polypeptide consisting of VH, CH1, VL, CL, and a linker, wherein the antibody domains and the linker have one of the following orders in the N-terminus to C-terminus direction: a) VH-CH1-linker-VL-CL, b) VL-CL-linker-VH-CH1, c) VH-CL-linker-VL-CH1, or d) VL-CH1-linker-VH-CL. In one embodiment, the linker is a polypeptide having at least 30 amino acids. In another embodiment, the linker is a polypeptide having between 32 and 50 amino acids. The single-chain Fab fragment is stabilized via the natural disulfide bond between CL and CH1. In addition, these single-chain Fab molecules may be further stabilized by generating interchain disulfide bonds through insertion of cysteine residues (e.g., position 44 in the heavy chain variable region and position 100 in the light chain variable region, according to Kabat numbering).

In another embodiment, the antibody fragment is an Fv fragment consisting of the VH and VL domains of a single arm of the antibody.

In another embodiment, the antibody fragment is a single-chain variable fragment (scFv). "scFv" is a fusion protein comprising at least one antibody fragment comprising a light chain variable region and at least one antibody fragment comprising a heavy chain variable region, wherein the light and heavy chain variable regions are contiguously linked by a short flexible peptide linker and are capable of being expressed as a single-chain polypeptide, and wherein the scFv retains the specificity of the intact antibody from which it is derived. Unless specifically indicated, an scFv may comprise VL and VH variable regions in any order herein; for example, with respect to the N-terminus and C-terminus of the polypeptide, the scFv may comprise VL-linker-VH or may comprise VH-linker-VL.

In another embodiment, the antibody fragment is a dsFv obtained by linking polypeptides in which one amino acid residue of each of VH and VL is substituted with a cysteine residue by a disulfide bond between the cysteine residues. The amino acid residues substituted with cysteine residues can be selected according to known methods (Protein Engineering, 7:697 (1994)) based on the prediction of the three-dimensional structure of the antibody.

In another embodiment, the antibody fragment is a single-domain antibody (dAb). Single-domain antibodies are antibody fragments comprising all or a portion of the heavy chain variable domain or all or a portion of the light chain variable domain of an antibody.

In certain embodiments, the antibody provided herein is a chimeric antibody. In one example, a chimeric antibody comprises a non-human variable region (e.g., a variable region derived from a mouse, rat, hamster, domestic rabbit, or non-human primate, such as a monkey) and a human constant region. In another example, a chimeric antibody is a "class switched" antibody in which the class or subclass has been changed from the class or subclass of the parent antibody.

In certain embodiments, the antibody is a humanized antibody. Typically, non-human antibodies are humanized to reduce immunogenicity to humans while retaining the specificity and affinity of the parent non-human antibody. Generally, the humanized antibody comprises one or more variable regions in which the CDRs or portions thereof are derived from a non-human antibody and the FRs or portions thereof are derived from a human antibody. Optionally, the humanized antibody can further comprise a portion of a human constant region. In some embodiments, some FR residues in a humanized antibody may be replaced with corresponding residues from a non-human antibody (e.g., an antibody that provides CDR sequences).

Humanized antibodies and methods for their production are reviewed, e.g., in Almagro and Fransson, Front. Biosci. 13:1619-1633 (2008), and further described, e.g., in Riechmann et al., Nature 332:323-329 (1988); Queen et al., Proc. Nat'l Acad. Sci. USA 86:10029-10033 (1989); U.S. Patent Nos. 5,821,337, 7,527,791, 6,982,321, and 7,087,409; Kashmiri et al., Methods 36:25-34 (2005) (describing specificity-determining region (SDR) grafting); Padlan, Mol. Immunol. 28:489-498 (1991) (describing "resurfuacing"); Dall' Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" method for FR shuffling).

Human framework regions that can be used for humanization include, but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA, 89:4285 (1992); and Presta et al., J. Immunol., 151:2623 (1993)); human mature (somatically mutated) framework regions or human germline framework regions (see, e.g., Almagro and Fransson, Front. Biosci. 13:1619-1633 (2008)); and framework regions obtained by screening FR libraries (see, e.g., Baca et al., J. Biol. Chem. 272:10678-10684 (1997) and Rosok et al., J. Biol. Chem. 271:22611-22618 (1996)).

### Variants of Anti-MUC1 Antibody or Antigen-Binding Fragment Thereof and Anti-EGFR Antibody or Antigen-Binding Fragment Thereof

In certain embodiments, amino acid sequence variants of the anti-MUC1 antibody or the antigen-binding fragment thereof and the anti-EGFR antibody or the antigen-binding fragment thereof provided herein are encompassed. For example, it may be desirable to improve the binding affinity and/or other biological properties of the antibodies. The amino acid sequence variants of the antibodies can be prepared by introducing appropriate modifications into the nucleotide sequences that encode the antibodies, or by peptide synthesis. Such modifications include, for example, deletions and/or insertions and/or substitutions of residues within the amino acid sequences of the anti-MUC1 antibody or the antigen-binding fragment thereof and the anti-EGFR antibody or the antigen-binding fragment thereof. Any combination of deletions, insertions, and substitutions can be made to obtain the final construct, as long as the final construct possesses the desired characteristics, e.g., antigen binding properties.

### Substitution, Insertion, and Deletion Variants

In certain embodiments, antibody variants comprising one or more amino acid substitutions are provided. Positions of interest for substitutional mutagenesis include CDRs and FRs. Conservative substitutions are shown in Table 2 under the heading of "preferred substitution". More substantial changes are provided in Table 2 under the heading of "exemplary substitution", and as further described below with reference to amino acid side chain classes. Amino acid substitutions can be introduced into an antibody of interest, and the products are screened for a desired activity, e.g., retained/improved antigen binding, reduced immunogenicity, or improved ADCC or CDC.

**Table 2. Amino acid substitutions**

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitution variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitution variant is an affinity-matured antibody, which can be conveniently produced, for example, using phage display-based affinity maturation techniques such as those described herein. Briefly, one or more CDR residues are mutated, and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling. Particularly, HCDR3 and LCDR3 are often targeted. In certain embodiments, substitutions, insertions, or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or group of target residues (e.g., charged residues such as Arg, Asp, His, Lys, and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertion variants of the antibody molecule include the fusion of the N- or C-terminus of the antibody to an enzyme or a polypeptide that increases the serum half-life of the antibody.

### Recombination Method

The anti-MUC1 antibody or the antigen-binding fragment thereof and the anti-EGFR antibody or the antigen-binding fragment thereof can be produced using recombination methods. For these methods, one or more isolated nucleic acids encoding the anti-MUC1 antibody or the antigen-binding fragment thereof or the anti-EGFR antibody or the antigen-binding fragment thereof are provided.

In one embodiment, the present disclosure provides an isolated nucleic acid encoding the anti-MUC1 antibody or the antigen-binding fragment thereof or the anti-EGFR antibody or the antigen-binding fragment thereof according to the foregoing. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing a polypeptide or fusion protein, wherein the method comprises culturing a host cell comprising a nucleic acid encoding the polypeptide or fusion protein, as provided above, under conditions suitable for expression, and optionally recovering the anti-MUC1 antibody or the antigen-binding fragment thereof from the host cell (or host cell culture medium).

For recombinant production of the anti-MUC1 antibody or the antigen-binding fragment thereof or the anti-EGFR antibody or the antigen-binding fragment thereof, the nucleic acid encoding the protein is isolated and inserted into one or more vectors for further cloning and/or expression in a host cell. Such nucleic acids can be readily isolated and sequenced using conventional procedures, or produced by recombination methods, or obtained by chemical synthesis.

Suitable host cells for cloning or expressing a vector encoding the anti-MUC1 antibody or the antigen-binding fragment thereof or the anti-EGFR antibody or the antigen-binding fragment thereof include the prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

In addition to prokaryotes, eukaryotic microorganisms such as filamentous fungi or yeast are suitable cloning or expression hosts for vectors encoding fusion proteins, including fungal and yeast strains. Suitable host cells for expression of the fusion protein may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978, and US6417429; vertebrate cells can also be used as hosts, e.g., mammalian cell lines adapted for growth in a suspension. Other examples of suitable mammalian host cell lines include an SV40-transformed monkey kidney CVlline (COS-7); a human embryonic kidney line (293 or 293T cells); baby hamster kidney cells (BHK); mouse Sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for antibody production see, e.g., Yazaki, P. and Wu, A.M., Methods in Molecular Biology, Vol. 248, Lo, B.K.C. (eds.), Humana Press, Totowa, NJ (2004), pp. 255-268.

### Assays

The physical/chemical characteristics and/or biological activity of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof provided herein can be identified, screened, or characterized by a variety of assays known in the art. In one aspect, the activity of the anti-MUC1 antibody or the antigen-binding fragment thereof or the anti-EGFR antibody or the antigen-binding fragment thereof of the present disclosure is tested, for example, by known methods such as ELISA and western blot.

### Treatment Method and Route of Administration

Any of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof provided in the present disclosure can be used for a treatment method. In yet another aspect, the present disclosure provides use of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof in the manufacture or preparation of a medicament. In some embodiments, the disease is an MUC1 or EGFR-associated disease or disorder. In some embodiments, the disease is a tumor. In some embodiments, the disease is selected from the group consisting of astrocytoma (e.g., anaplastic astrocytoma), glioblastoma, bladder cancer, bone cancer, brain cancer, breast cancer (e.g., breast cancer characterized by BRCA1 and/or BRCA2 mutation), cervical cancer, colorectal cancer (e.g., colon cancer and rectal cancer), fallopian tube cancer, gallbladder cancer, gastric cancer, head and neck cancer, idiopathic myelofibrosis, renal cancer (e.g., renal cell carcinoma, rhabdoid tumor of the kidney, and Wilms tumor), leukemia, liver cancer (e.g., hepatocellular carcinoma), esophageal cancer (e.g., esophageal squamous cell carcinoma), lung cancer (e.g., non-small cell lung cancer and small cell lung cancer), medulloblastoma, melanoma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, sarcoma (e.g., chondrosarcoma, Ewing sarcoma, osteosarcoma, rhabdomyosarcoma, synovial sarcoma, and soft tissue sarcoma), squamous cell carcinoma (e.g., cutaneous squamous cell carcinoma), thyroid cancer, endometrial cancer, vestibular schwannoma, blastoma, vulvar cancer, thymoma, testicular cancer, cholangiocarcinoma, pheochromocytoma, paraganglioma, and adenoid cystic carcinoma; in some embodiments, the cancer is selected from the group consisting of lung cancer, head and neck cancer, esophageal cancer, breast cancer, pancreatic cancer, prostate cancer, thyroid cancer, gastric cancer, ovarian cancer, colorectal cancer, liver cancer, gallbladder cancer, renal cancer, cervical cancer, and bladder cancer. In some embodiments, the cancer is lung cancer, preferably non-small cell lung cancer.

In yet another aspect, provided is a pharmaceutical composition comprising the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof, for example, for use in any of the above pharmaceutical uses or treatment methods. In one embodiment, the pharmaceutical composition comprises any of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof provided herein and a pharmaceutically acceptable carrier.

The anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof of the present disclosure can be used alone or in combination with other agents for treatment. For example, the antibody of the present disclosure can be co-administered with at least one additional therapeutic agent.

The anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof (and any additional therapeutic agent) of the present disclosure can be administered by any suitable means, including parenteral administration, intrapulmonary administration, and intranasal administration, and, if local treatment is required, intralesional administration. Parenteral infusion includes intramuscular, intravenous, intra-arterial, intraperitoneal, or subcutaneous administration. Administration may be performed via any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus injection administration, and pulse infusion.

The anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the administration method, the timing of administration, and other factors known to medical practitioners. The anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof can be formulated together with or without one or more agents currently used for preventing or treating the disorder. The effective amount of such additional agents depends on the amount present in the pharmaceutical composition, the type of the disorder or treatment, and other factors. These are generally used in the same dosages and routes of administration as described herein, or in about 1% to 99% of the dosages described herein, or in other dosages, and by any route empirically/clinically determined to be appropriate.

For the prevention or treatment of disease, the appropriate dosage of the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to a patient in one or a series of treatments.

### Product

In another aspect of the present disclosure, provided is a product (e.g., a medication box) comprising materials useful for the treatment, prevention, and/or diagnosis of the above disorder. The product comprises a container and a label or package insert on or associated with the container. Suitable containers include, for example, bottles, vials, syringes, IV solution bags, and the like. The container may be formed of a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the anti-MUC1 antibody or the antigen-binding fragment thereof, the anti-EGFR antibody or the antigen-binding fragment thereof, the antigen-binding molecule that specifically binds to EGFR and MUC1, and the antibody-drug conjugates thereof or the pharmaceutically acceptable salts thereof of the present disclosure. The label or package insert indicates that the composition is used to treat the selected condition. In addition, the product may comprise: (a) a first container containing a composition, wherein the composition comprises the active molecule of the present disclosure; and (b) a second container containing a composition, wherein the composition comprises an additional cytotoxic agent or therapeutic agents of other aspects. The product in this embodiment of the present disclosure may further comprise a package insert indicating that the composition may be used to treat a particular condition. Alternatively or additionally, the product may further comprise a second (or third) container containing a pharmaceutically acceptable buffer. From a commercial and user standpoint, it may further comprise other required materials, including other buffers, diluents, filters, needles, and syringes.

The present disclosure is further described below with reference to examples and test examples. However, these examples and test examples do not limit the scope of the present disclosure. In the examples or test examples of the present disclosure, the experimental methods whose specific conditions are not specified were generally performed under conventional conditions such as Antibodies: A Laboratory Manual and Molecular Cloning: A Laboratory Manual by Cold Spring Harbor Laboratory, or under conditions recommended by the manufacturer of the starting material or commercial product. The reagents whose specific sources are not specified were commercially available.

### Examples

### Example 1: Preparation of MUC1 Antigens

With a UniProt MUC1 antigen (human MUC1 protein, Uniprot number: P15941) used as a template for MUC1, the amino acid sequences of the antigen and the protein for detection used in the present disclosure were designed, and optionally, different tags such as His tags or Fc were fused on the basis of the MUC1 protein. After cloning into a pTT5 vector (Biovector, CAT#102762), 293 cells were transiently transfected with the vector. After expression and purification, the antigen and the protein for detection of the present disclosure were obtained.

A His-tagged MUC1-C protein extracellular domain (abbreviated as MUC1-C-L-6xHis) sequence was used as an immunization antigen and a detection reagent: 
ASGSASGSASTLVHNGTSARATTTPASKSTPFSIPSHHSDTPTTLASHSTKTD
ASSTHHSSVPPLTSSNHSTSPQLSTGVSFFFLSFHISNLQFNSSLEDPSTDYYQ
ELQRDISEMFLQIYKQGGFLGLSNIKFRPGSVVVQLTLAFREGTINVHDVET
QFNQYKTEAASRYNLTISDVSVSDVPFPFSAQSHHHHHH
SEQ ID NO: 1
 Note: The 6×His tag is underlined, and the MUC1 protein extracellular domain is in bold type.

The sequence of a fusion protein of the MUC1-C protein extracellular domain and human-IgG1-Fc (abbreviated as hMUC1-C-L-Fc) was used as an immunogen: 
ASGSASGSASTLVHNGTSARATTTPASKSTPFSIPSHHSDTPTTLASHSTKTD
ASSTHHSSVPPLTSSNHSTSPQLSTGVSFFFLSFHISNLQFNSSLEDPSTDYYQ
ELQRDISEMFLQIYKQGGFLGLSNIKFRPGSVVVQLTLAFREGTINVHDVET
QFNQYKTEAASRYNLTISDVSVSDVPFPFSAQSDKTHTCPPCPAPELLGGPSVF
LFPPKPKDTLMISRTPEVTCVVVDVSHEDPEVKFNWYVDGVEVHNAKTKPREE
QYNSTYRVVSVLTVLHQDWLNGKEYKCKVSNKALPAPIEKTISKAKGQPREPQ
VYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK
SEQ ID NO: 2
 Note: Human-IgG1-Fc is underlined, and the MUC1 protein extracellular domain is in bold type.

In addition, the sequence of a fusion protein of the cyno-MUC1-C protein extracellular domain and human-IgG1-Fc (abbreviated as Cyno MUC1-C-Fc) was used as a detection reagent: 
LSLGVSFFFLSFHISNLQFNSSLEDPSTNYYQQLQRDISELFLQIYKQGDFLG
LSNIMFRPGSVVVQSTLVFREGTTNVHDVETQFNQRKTEAASRYNLTISDIS
VRDVPFPFSAQTGAGDKTHTCPPCPAPELLGGPSVFLFPPKPKDTLMISRTPEVT
 
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 3
 Note: Human-IgG1-Fc is underlined, and the cyno-MUC1 protein extracellular domain is in bold type.

### Example 2: Purification of MUC1-Associated Recombinant Proteins

### 1. Purification of His-tagged recombinant protein

A cell expression supernatant sample was centrifuged at high speed to remove impurities. A nickel column was equilibrated with a PBS solution containing 20 mM imidazole and rinsed with 2-5 column volumes. The cell supernatant sample after exchange was loaded onto the Ni Sepharose excel column (GE, 17-3712-02). The column was rinsed with a PBS solution until the A280 reading dropped to the baseline. Subsequently, the chromatography column was rinsed with PBS + 20 mM imidazole to remove non-specifically bound protein impurities, and the eluate was collected. The target protein was eluted with a PBS solution containing 300 mM imidazole, and the elution peak was collected. The target protein was buffer-exchanged into PBS using a concentration tube and concentrated to an appropriate concentration. After it was confirmed by electrophoresis, peptide mapping, and LC-MS that the obtained protein was the desired protein, the protein was aliquoted for later use. MUC1-C-L-6xHis, which contains a His tag, was obtained and used as a detection reagent for the antibodies of the present disclosure.

### 2. Purification of MUC1-C-L-Fc fusion protein

The cell expression supernatant sample was centrifuged at high speed to remove impurities, and the supernatant was purified by MabSelect Sure (GE, 17-5438-01) affinity chromatography. The MabSelect Sure chromatography column was first regenerated with 0.2 M NaOH and then equilibrated with PBS. After the supernatant was bound, the column was washed with PBS until the A280 reading dropped to the baseline. The target protein was eluted with a 0.1 M acetic acid buffer at pH 3.5 and neutralized with 1 M Tris-HCl. The target protein was buffer-exchanged into PBS using a concentration tube and concentrated to an appropriate concentration. After it was confirmed by electrophoresis and LC-MS that the obtained protein was the desired protein, the protein was aliquoted for later use. This method was used to purify the MUC1-C-L-Fc fusion protein. This method can also be used to purify the antibody proteins in the present disclosure.

### 3. Purification of hybridoma screening antibody small sample expression:

The cell expression supernatant sample was centrifuged at high speed to remove impurities, and a Protein A magnetic bead packing material (SM003100) was added to the supernatant. The mixture was then shaken at room temperature for 3 h. The packing material was washed 3 times with PBS and once with ultrapure water. The target protein was eluted with a 0.1 M acetic acid buffer at pH 3.0 and neutralized with 1 M Tris-HCl. The target protein was buffer-exchanged into PBS using a concentration tube and concentrated to an appropriate concentration. After it was confirmed by electrophoresis and LC-MS that the obtained protein was the desired protein, the protein was aliquoted for later use.

### Example 3: Mouse Immunization Scheme for Murine Anti-MUC1-C Antibodies and Hybridoma Antibody Acquisition

### 1. Immunization of mice

Anti-human MUC1-C antibodies were produced by immunizing mice. Laboratory Balb/c and SJL white mice, female, aged 6-8 weeks (Shanghai SLAC Laboratory Animal Co., Ltd., animal production license number: SCXK (Shanghai) 2017-0005). Housing environment: SPF. The purchased mice were housed for 1 week in a laboratory environment with a 12/12-hour light/dark cycle at a temperature of 20-25 °C with humidity at 40-60%. The acclimatized mice were immunized according to the following scheme.

Immunization scheme 1: Immunization was performed using a protein antigen (hMUC1 C-L-Fc). For the protein antigen (hMUC1 C-L-Fc), the TiterMax^{®} Gold Adjuvant (Sigma) and Thermo Imject^{®} Alum (Thermo) adjuvants were used for cross-immunization. The protein antigen (hMUC1 C-L-Fc) and the adjuvant (TiterMax^{®} Gold Adjuvant) were mixed at a 1:1 ratio, and the mixture was emulsified and then inoculated into mice. The protein antigen (hMUC1 C-L-Fc) and the adjuvant (Thermo Imject^{®} Alum) were mixed at a 3:1 ratio. After shaking for thorough mixing, the mixture was inoculated into mice. Immunizations were performed at 50 µg/mouse/immunization (primary immunization), 25 µg/mouse/immunization (conventional immunization), and 50 µg/mouse/immunization (boost immunization). The mice were immunized on days 0, 14, 34, 48, and 78. Blood samples were collected on days 26, 40, 57, and 82, and the antibody titer in the serum of the mice was determined. After 4-5 immunizations, the antibody titer in the serum of the mice was determined by ELISA, and the mice in which the antibody titer in the serum was high and tended to plateau were selected for splenocyte fusion. Boost immunization was performed 3 days before splenocyte fusion. A solution of the protein antigen (hMUC1 C-L-Fc) prepared using normal saline was injected intraperitoneally (i.p.) at 50 µg/mouse, or a cell antigen suspension prepared using a phosphate buffer solution was injected intraperitoneally (i.p.) at 1 × 10⁷ cells/mouse.

Immunization scheme 2: Cross-immunization was performed using a protein antigen (hMUC1-C-L-His) and a cell antigen (CHO-K1-MUC1-C). For the protein antigen (hMUC1-C-L-His), the TiterMax^{®} Gold Adjuvant (Sigma) and Thermo Imject^{®} Alum (Thermo) adjuvants were used for cross-immunization. The protein antigen (hMUC1-C-L-His) and the adjuvant (TiterMax^{®} Gold Adjuvant) were mixed at a 1:1 ratio, and the mixture was emulsified and then inoculated into mice. The antigen (hMUC1-C-L-Fc) and the adjuvant (Thermo Imject^{®} Alum) were mixed at a 3:1 ratio. After shaking for thorough mixing, the mixture was inoculated into mice. Immunizations were performed at 50 µg/mouse/immunization (primary immunization), 25 µg/mouse/immunization (conventional immunization), and 50 µg/mouse/immunization (boost immunization). For the cell antigen (CHO-K1-MUC1-C), immunizations were performed at 1 × 10⁷ cells/mouse/immunization. The cell antigen was resuspended in a phosphate buffer solution before inoculation. The mice were inoculated on days 0, 14, 34, 48, 69, and 86. Blood samples were collected on days 26, 40, 57, 82, and 96. After 5-9 immunizations, the antibody titer in the serum of the mice was determined by ELISA, and the spleens of the mice in which the antibody titer in the serum was high and tended to plateau were collected for splenocyte fusion. Boost immunization was performed 3 days before splenocyte fusion. A cell antigen suspension prepared using a phosphate buffer solution was injected intraperitoneally (i.p.) at 1 × 10⁷ cells/mouse.

Immunization scheme 3: Cross-immunization was performed using a protein antigen (hMUC1-C-L-Fc) and a cell antigen (CHO-K1-MUC1-C). For the protein antigen (hMUC1-C-L-Fc), the TiterMax^{®} Gold Adjuvant (Sigma) and Thermo Imject^{®} Alum (Thermo) adjuvants were used for cross-immunization. The protein antigen (hMUC1-C-L-Fc) and the adjuvant (TiterMax^{®} Gold Adjuvant) were mixed at a 1:1 ratio, and the mixture was emulsified and then inoculated into mice. The antigen (hMUC1-C-L-Fc) and the adjuvant (Thermo Imject^{®} Alum) were mixed at a 3:1 ratio. After shaking for thorough mixing, the mixture was inoculated into mice. Immunizations were performed at 50 µg/mouse/immunization (primary immunization), 25 µg/mouse/immunization (conventional immunization), and 50 µg/mouse/immunization (boost immunization). For the cell antigen (CHO-K1-MUC1-C), immunizations were performed at 1 × 10⁷ cells/mouse/immunization. The cell antigen was resuspended in a phosphate buffer solution before inoculation. The mice were inoculated on days 0, 12, 25, 40, 55, 112, 127, and 156. Blood samples were collected on days 21, 35, 49, 63, 108, and 154. After 5-8 immunizations, the antibody titer in the serum of the mice was determined by ELISA, and the spleens of the mice in which the antibody titer in the serum was high and tended to plateau were collected for splenocyte fusion. Boost immunization was performed 3 days before splenocyte fusion. A solution of the protein antigen (hMUC1-C-L-Fc) prepared using normal saline was injected intraperitoneally (i.p.) at 50 µg/mouse.

Immunization scheme 4: Cross-immunization was performed using a protein antigen (hMUC1-C-L-Fc) and a cell antigen (HEK293-MUC1-C). For the protein antigen (hMUC1-C-L-Fc), the TiterMax^{®} Gold Adjuvant (Sigma) and Thermo Imject^{®} Alum (Thermo) adjuvants were used for cross-immunization. The protein antigen (hMUC1-C-L-Fc) and the adjuvant (TiterMax^{®} Gold Adjuvant) were mixed at a 1:1 ratio, and the mixture was emulsified and then inoculated into mice. The antigen (hMUC1-C-L-Fc) and the adjuvant (Thermo Imject^{®} Alum) were mixed at a 3:1 ratio. After shaking for thorough mixing, the mixture was inoculated into mice. Immunizations were performed at 50 µg/mouse/immunization (primary immunization), 25 µg/mouse/immunization (conventional immunization), and 50 µg/mouse/immunization (boost immunization). For the cell antigen (HEK293-MUC1-C), immunizations were performed at 1 × 10⁷ cells/mouse/immunization. The cell antigen was resuspended in a phosphate buffer solution before inoculation. The mice were inoculated on days 0, 14, 28, and 42. Blood samples were collected on days 10 and 38. After 3-4 immunizations, the antibody titer in the serum of the mice was determined by ELISA, and the spleens of the mice in which the antibody titer in the serum was high and tended to plateau were collected for splenocyte fusion. Boost immunization was performed 3 days before splenocyte fusion. A solution of the protein antigen (hMUC1-C-L-Fc) prepared using normal saline was injected intraperitoneally (i.p.) at 50 µg/mouse.

### 2. Splenocyte fusion

Spleen lymphocytes and Sp2/0 cells (myeloma cells, ATCC^{®} CRL-8287^{™}) were fused using an optimized electrofusion method to obtain hybridoma cells.

The resulting hybridoma cells were resuspended in a complete culture medium (an IMDM culture medium containing 20% FBS, 1× HAT, and 1× OPI) at a density of 3-4 × 10^5/mL according to the spleen cell count, and the suspension was seeded in a 96-well plate at 150 µL/well. After 4-5 days of incubation at 37 °C with 5% CO₂, the supernatant was removed, and an HT complete culture medium (an IMDM culture medium containing 20% FBS, 1× HT, and 1× OPI) was added at 200 µL/well. After 2 days of culture at 37 °C with 5% CO₂, an ELISA assay was performed.

### Example 4: Screening of Murine Anti-MUC1-C Hybridoma Antibodies

### 1. ELISA assay for binding of hybridoma supernatant antibodies to hMUC1-C-L-his protein

Human hMUC1-C-L-his protein was diluted to a concentration of 1 µg/mL in a PBS buffer (pH 7.4), and the dilution was added to a 96-well microplate (Corning, Cat. No. CLS3590-100EA) at 100 µL/well. The plate was then placed in a refrigerator at 4 °C for 16-18 h. After the liquid was discarded, a PBS-diluted 5% skim milk powder (Sangon Biotech, Cat. No. A600669-0250) blocking solution was added at 200 µL/well, and the plate was incubated in an incubator at 37 °C for 1.5 h. After the blocking, the blocking solution was discarded, and the plate was washed 3 times with a PBST buffer (pH 7.4 PBS containing 0.1% tween-20). The hybridoma supernatants were then added at 100 µL/well, and the plate was incubated in an incubator at 37 °C for 1 h. After the incubation, the plate was washed 5 times with PBST, and a secondary antibody (Jackson ImmunoResearch, Cat. No. 1115-035-003) diluted in 2% MPBS was added at 50 µL/well. The plate was then incubated at 37 °C for 1 h. The plate was washed 5 times with PBST, and the TMB chromogenic substrate (KPL, Cat. No. 52-00-03) was added at 50 µL/well. The plate was then incubated at room temperature for 5-10 min, and 1 M H₂SO₄ was added at 50 µL/well to stop the reaction. The absorbance at a wavelength of 450 nm was measured using a VERSAmax microplate reader (Molecular Devices). The results are shown in the table below.

**Table 3. The results of the ELISA assay for the binding of the hybridoma supernatants to the human MUC1-C-L-his antigen**

| Hybridoma supernatant No. | Microplate reader OD450nm value |
|---|---|
| 17F3 | 1.896 |
| 28E4 | 1.521 |
| 3H6 | 2.335 |
| 36G9 | 2.053 |

### 2. Mirrorball assay for binding of hybridoma supernatant antibodies to human MUC1-C cell strain

HCC827-human-MUC1-C cells (in-house constructed overexpression stably transfected cell strain), CHOK1-cyno-MUC1-C cells (in-house constructed overexpression stably transfected cell strain), or CHOK1-WT cells were digested and washed once with a PBS buffer. The cells were then centrifuged at 1000 rpm for 5 min and resuspended in CellTracker^{™} Green CMFDA dye (Thermo Fisher Scientific (China) Co., Ltd., Cat. No. C7025), diluted in a PBS buffer and having a final concentration of 50 nM, and the suspension (cell density: 1E6/mL) was incubated in an incubator for 30 min. After the incubation, the suspension was centrifuged at 1000 rpm for 5 min, and the supernatant was discarded. The cells were washed once with PBS (containing 1% FBS (Gibco, Cat. No. 10100147)) and centrifuged, and a PBS buffer was then added to resuspend the cells (final density: 1-2E5/mL). An APC-labeled fluorescent secondary antibody (BD biosciences, Cat. No. 550826) was added at a dilution ratio of 1:200. The mixed solution of cells was added to a 384-well plate (Corning, Cat. No. 3764) at 20 µL of cells/well, i.e., 2000 to 4000 cells/well. 20 µL of hybridoma supernatant was added to each well of the 384-well plate except for the positive and negative control wells. The plate was then placed in a dark place at room temperature for 2 h, and Mirrorball cytometer (Sptlabtech) readings were taken.

**Table 4. The results of the mirrorball assay for the hybridoma supernatants**

| Hybridoma supernatant No. | Human MUC1-CT binding | Monkey MUC1-CT binding | Non-specific binding |
|---|---|---|---|
| 17F3 | + | + | - |
| 28E4 | + | + | - |
| 3H6 | + | + | - |
| 36G9 | + | + | - |

| | | | |
|---|---|---|---|
| Note: "+" means that there was binding, and "-" means that there was no binding. | | | |

### 3. Screening of MUC1-C antibodies based on hybridoma cells

The four hybridoma strains obtained after the qualitative ELISA and mirrorball assays were sequenced, and the corresponding antibodies were named after the sequencing. After the sequencing, the antibody of hybridoma 17F3 was designated M4, the antibody of hybridoma 28E4 was designated M6, the antibody of hybridoma 3H6 was designated F4-1, and the antibody of hybridoma 36G9 was designated F4-18.

The four antibodies obtained were sequenced, and the obtained sequences were subjected to CDR classification. The murine variable region sequences were selected and linked to human antibody constant region sequences, and chimeric antibodies were expressed. The amino acid sequences of the heavy and light chain variable regions of the obtained antibodies are shown below:
> Heavy chain variable region sequence of M4 (M4 mVH): 
QVQLQQSGAELARPGASVKLSCKASGYSFTTYGVPWVKQRTGQGLEWIGDIYP
RSGNTYYNEKFKGKATLTADKSSDTVFMELRSLSSEDSAVYFCAREDYDNYPYA
LDYWGQGTSVTVSS
SEQ ID NO: 4
> Light chain variable region sequence of M4 (M4 mVL): 
EIVLTQSPAIIAASPGEKVTITCSASSSVFNMNWYQQKPGSSPKIWIYDISKLASG
VPGRFSGSGSGTSFSLTINSMEAEDVGTYYCQQRSFYPPTFGGGTKVEIK
SEQ ID NO: 5
> Heavy chain variable region sequence of M6 (M6 mVH): 
QVQLQQSGAELMKPGASVKLSCKATGYRFIPYWIEWIKERPGHGLEWIGEILPG
TGRTNYNEKFKGKATFTADTSSNTAYMQLSSLTTEDSAIYYCARYGDDTSGGYY
 
AVDYWGQGTSVTVSS
SEQ ID NO: 6
> Light chain variable region sequence of M6 (M6 mVL): 
DIVMTQSSASLAMSLGQRATISCRASESVNILGTNLIHWYQQKPGQPPKLLIYHA
SNLETGVPARFSGSGSRTDFTLTIDPVEEDDVAIYYCLQSRKIPWTFGGGTKLEIK
SEQ ID NO: 7
> Heavy chain variable region sequence of F4-1 (F4-1 mVH): 
QIQLQQSGPELVKPGASVKISCKASGYTFTDNYINWVKQRPGQGLEYIGWIYPG
SGNNKFNEKFKGKATLTVDTSSRTAFMQLSSLTFEDSAVYFCARDYPFPSYHYG
MDYWGQGTSVTVSS
SEQ ID NO: 8
> Light chain variable region sequence of F4-1 (F4-1 mVL): 
QILLTQSPAIMSASPGERVTITCSASSSVSYIHWFQQKPGTSPKLWIYSTSNLASG
VPPRFSGSGSGTSFSLTVSRMEAEDAATYYCQQRSSYPPTFGGGTKLEIK
SEQ ID NO: 9
> Heavy chain variable region sequence of F4-18 (F4-18 mVH): 
EVQLQQSGAELVRPGSSVKMSCKTSGYTFTSYGINWVKQRPGQGLEWIGYIYL
GSDYTEYNEKFKGKATLTSDTTSSTAYMQLSSLTSEDSAIYFCARSAGSLFAYWG
QGTLVTVSA
SEQ ID NO: 10
> Light chain variable region sequence of F4-18 (F4-18 mVL): 
DIQLTQSPSSLSASLGERVSLTCRASQDIGITLNWLQQEPDGTIKRLIYATSSLDSG
VPKRFSGSRSGSDYSLTISSLESEDFVDYYCLQYASSPYTFGGGTKLEIK
SEQ ID NO: 11
Note: In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

The CDR sequences of the heavy and light chains of murine antibodies M4, M6, F4-1, and F4-18 are shown in the table below:

**Table 5. The CDR sequences of the heavy and light chains of the antibodies**

| Antibody | Heavy chain | | Light chain | |
|---|---|---|---|---|
| M4 | HCDR1 | TYGVP (SEQ ID NO: 12) | LCDR1 | SASSSVFNMN (SEQ ID NO: 15) |
| | HCDR2 | DIYPRSGNTYYNEKFKG (SEQ ID NO: 13) | LCDR2 | DISKLAS (SEQ ID NO: 16) |
| | HCDR3 | EDYDNYPYALDY (SEQ ID NO: 14) | LCDR3 | QQRSFYPPT SEQ ID NO: 17) |
| M6 | HCDR1 | PYWIE (SEQ ID NO: 18) | LCDR1 | RASESVNILGTNLIH (SEQ ID NO: 21) |
| | HCDR2 | EILPGTGRTNYNEKFKG (SEQ ID NO: 19) | LCDR2 | HASNLET (SEQ ID NO: 22 |
| | HCDR3 | YGDDTSGGYYAVDY (SEQ ID NO: 20) | LCDR3 | LQSRKIPWT (SEQ ID NO: 23) |
| F4-1 | HCDR1 | DNYIN (SEQ ID NO: 24) | LCDR1 | SASSSVSYIH (SEQ ID NO: 27) |
| | HCDR2 | WIYPGSGNNKFNEKFKG (SEQ ID NO: 25) | LCDR2 | STSNLAS (SEQ ID NO: 28) |
| | HCDR3 | DYPFPSYHYGMDY (SEQ ID NO: 26) | LCDR3 | QQRSSYPPT (SEQ ID NO: 29) |
| F4-18 | HCDR1 | SYGIN (SEQ ID NO: 30) | LCDR1 | RASQDIGITLN (SEQ ID NO: 33) |
| | HCDR2 | YIYLGSDYTEYNEKFKG (SEQ ID NO: 31) | LCDR2 | ATSSLDS (SEQ ID NO: 34) |
| | HCDR3 | SAGSLFAY (SEQ ID NO: 32) | LCDR3 | LQYASSPYT (SEQ ID NO: 35) |

| | | | | |
|---|---|---|---|---|
| Note: The CDRs in the table are CDRs determined according to the Kabat numbering scheme. | | | | |

### Example 5: Humanization of Anti-MUC1-C Murine Antibodies

By alignment with the IMGT human antibody heavy and light chain variable region germline gene database through MOE software, heavy and light chain variable region germline genes highly homologous with M4, M6, F4-1, and F4-18 were selected as templates. The CDRs of the four murine antibodies were grafted into corresponding human templates to form variable region sequences in the following order: FR1-CDR1-FR2-CDR2-FR3-CDR3-FR4. Illustratively, the CDR amino acid residues in the specific examples below were determined and annotated using the Kabat numbering scheme.

### 1. Humanization of murine antibody M4

For murine antibody M4, the humanized light chain templates were IGKV1-39*01/IGKV6-21*02/IGKV3-11*01 and IGKJ4*01, and the humanized heavy chain templates were IGHV1-46*01 and IGHJ6*01. The CDRs of murine antibody M4 were grafted into its humanized templates. Further, amino acids of the FR portions of the humanized antibodies were back-mutated, and it was contemplated to remove potential chemical modification sites such as isomerization, eliminate the formation of N-terminal pyroglutamic acid, reduce potential immunogenicity, etc. The light chain FR portions contained one or more mutations of 3, 43, 47, 49, or 60 (the positions of the mutation sites were determined according to the Kabat numbering scheme). The heavy chain FR portions contained one or more mutations of 1, 28, 38, 40, 48, 71, 73, 76, and 82a (the positions of the mutation sites were determined according to the Kabat numbering scheme). The amino acid substitutions in the variable regions of the humanized antibodies of antibody M4 are shown in the table below.

**Table 6. The amino acid substitutions in the variable regions of the humanized antibodies of M4**

| **VL** | | **VH** | |
|---|---|---|---|
| huM4VL1 | Graft(IGKV1-39*01)+ A43S, L47W | huM4VH1 | Graft(IGHV1-46*01)+Q1E, R71A, T73K, S76D |
| huM4VL2 | Graft(IGKV1-39*01)+ Q3V, A43S, L47W | huM4VH2 | Graft(IGHV1-46*01)+Q1E, T28S, A40R, M48I, R71A, T73K, S76D |
| huM4VL3 | Graft(IGKV6-21 *02)+ L47W, K49Y | huM4VH3 | Graft(IGHV1-46*01)+ Q1E, T28S, R38K, A40R, M48I, R71A, T73K, S76D, S82aR |
| huM4VL4 | Graft(IGKV3-11*01)+ A43S, L47W, A60G | | |

| | | | |
|---|---|---|---|
| Note: Graft means that the CDRs of the murine antibody were grafted into the human germline FRs; the positions of the mutation sites were determined according to the Kabat numbering scheme; for example, "S82aR" means that the S at position 82a (also referred to as 82A) was mutated to R, according to the Kabat numbering scheme. | | | |

The heavy chain variable region/light chain variable region sequences of the humanized antibodies of M4 are shown below:
> huM4VH1 
EVQLVQSGAEVKKPGASVKVSCKASGYTFTTYGVPWVRQAPGQGLEWMGDI
YPRSGNTYYNEKFKGRVTMTADKSTDTVYMELSSLRSEDTAVYYCAREDYDN
YPYALDYWGQGTTVTVSS
SEQ ID NO: 36
> huM4VH2 
EVQLVQSGAEVKKPGASVKVSCKASGYSFTTYGVPWVRQRPGQGLEWIGDIY
PRSGNTYYNEKFKGRVTMTADKSTDTVYMELSSLRSEDTAVYYCAREDYDNY
PYALDYWGQGTTVTVSS
 SEQ ID NO: 37
> huM4VH3 
EVQLVQSGAEVKKPGASVKVSCKASGYSFTTYGVPWVKQRPGQGLEWIGDIY
PRSGNTYYNEKFKGRVTMTADKSTDTVYMELRSLRSEDTAVYYCAREDYDNY
PYALDYWGQGTTVTVSS
SEQ ID NO: 38
> huM4VL1 
DIQMTQSPSSLSASVGDRVTITCSASSSVFNMNWYQQKPGKSPKLWIYDISKLA
SGVPSRFSGSGSGTDFTLTISSLQPEDFATYYCQQRSFYPPTFGGGTKVEIK
SEQ ID NO: 39
> huM4VL2 
DIVMTQSPSSLSASVGDRVTITCSASSSVFNMNWYQQKPGKSPKLWIYDISKLA
SGVPSRFSGSGSGTDFTLTISSLQPEDFATYYCQQRSFYPPTFGGGTKVEIK
SEQ ID NO: 40
> huM4VL3 
EIVLTQSPDFQSVTPKEKVTITCSASSSVFNMNWYQQKPDQSPKLWIYDISKLAS
GVPSRFSGSGSGTDFTLTINSLEAEDAATYYCQQRSFYPPTFGGGTKVEIK
SEQ ID NO: 41
> huM4VL4

EIVLTQSPATLSLSPGERATLSCSASSSVFNMNWYQQKPGQSPRLWIYDISKLAS
GIPGRFSGSGSGTDFTLTISSLEPEDFAVYYCQQRSFYPPTFGGGTKVEIK
SEQ ID NO: 42

In the above sequences, the regions are arranged in the following order: FR1-CDR1-FR2-CDR2-FR3-CDR3-FR4; in the sequences, the CDR sequences determined according to the Kabat numbering scheme are underlined, and the portions that are not underlined are the FR sequences.

### 2. Humanization of murine antibody M6

For murine antibody M6, the humanized light chain templates were IGKV4-1*01/IGKV3-11*01 and IGKJ4*01, and the humanized heavy chain templates were IGHV1-46*01 and IGHJ6*01. The CDRs of murine antibody M6 were grafted into its humanized templates. Further, amino acids of the FR portions of the humanized antibodies were back-mutated, and it was contemplated to remove potential chemical modification sites such as isomerization, eliminate the formation of N-terminal pyroglutamic acid, reduce potential immunogenicity, etc. The light chain FR portions contained one or more mutations of 1, 4, 45, 68, or 83 (the positions of the mutation sites were determined according to the Kabat numbering scheme). The heavy chains contained one or more mutations of 1, 28, 30, 39, 40, 43, 69, 71, 76, 82b, 83, 84, and 97 (the positions of the mutation sites were determined according to the Kabat numbering scheme). The amino acid substitutions in the variable regions of the humanized antibodies of antibody M6 are shown in the table below.

**Table 7-1. The amino acid substitutions in the variable regions of the humanized antibodies of M6**

| **VL** | | **VH** | |
|---|---|---|---|
| huM6VL1 | Graft(IGKV4-1*01) + G68R | huM6VH1 | Graft(IGKV1-46*01) + Q1E, M69F, R71A, S76N |
| huM6VL2 | Graft(IGKV3-11*01) + R45K, G68R | huM6VH2 | Graft(IGKV1-46*01) + Q1E, T28R, T30I, M69F, R71A, S76N |
| huM6VL3 | Graft(IGKV3-11*01) + E1D, L4M, R45K, G68R, F83V | huM6VH3 | Graft(IGKV1-46*01) +Q1E, T28R, T30I, A40R, M69F, R71A, S76N |
| | | huM6VH4 | Graft(IGKV1-46*01) +Q1E, T28R, T30I, Q39E, A40R, Q43H, M69F, R71A, S76N, R83T |
| | | huM6VH5 | Graft(IGKV1-46*01) + Q1E, T28R, T30I, Q39E, A40R, Q43H, M69F, R71A, S76N, R83T+D97E |
| | | huM6VH6 | Graft(IGKV1-46*01) + Q1E, T28R, T30I, Q39E, A40R, Q43H, M69F, R71A, S76N, S82bQ, R83T+D97E |
| | | huM6VH7 | Graft(IGKV1-46*01) + Q1E, T28R, T30I, Q39E, A40R, Q43H, M69F, R71A, S76N, R83T, S84N+ D97E |

| | | | |
|---|---|---|---|
| Note: Graft means that the CDRs of the murine antibody were grafted into the human germline FRs; the positions of the mutation sites were determined according to the Kabat numbering scheme; for example, "G68R" means that the G at position 68 was mutated to R, according to the Kabat numbering scheme. | | | |

The heavy chain variable region/light chain variable region sequences of the humanized antibodies of M6 are shown below:
> huM6VH1 
EVQLVQSGAEVKKPGASVKVSCKASGYTFTPYWIEWVRQAPGQGLEWMGEIL
PGTGRTNYNEKFKGRVTFTADTSTNTVYMELSSLRSEDTAVYYCARYGDDTSG
GYYAVDYWGQGTTVTVSS
SEQ ID NO: 43
> huM6VH2 
EVQLVQSGAEVKKPGASVKVSCKASGYRFIPYWIEWVRQAPGQGLEWMGEIL
PGTGRTNYNEKFKGRVTFTADTSTNTVYMELSSLRSEDTAVYYCARYGDDTSG
GYYAVDYWGQGTTVTVSS
SEQ ID NO: 44
> huM6VH3 
EVQLVQSGAEVKKPGASVKVSCKASGYRFIPYWIEWVRQRPGQGLEWMGEIL
PGTGRTNYNEKFKGRVTFTADTSTNTVYMELSSLRSEDTAVYYCARYGDDTSG
GYYAVDYWGQGTTVTVSS
SEQ ID NO: 45
> huM6VH4 
EVQLVQSGAEVKKPGASVKVSCKASGYRFIPYWIEWVRERPGHGLEWMGEIL
PGTGRTNYNEKFKGRVTFTADTSTNTVYMELSSLTSEDTAVYYCARYGDDTSG
GYYAVDYWGQGTTVTVSS
SEQ ID NO: 46
> huM6VH5 
EVQLVQSGAEVKKPGASVKVSCKASGYRFIPYWIEWVRERPGHGLEWMGEIL
PGTGRTNYNEKFKGRVTFTADTSTNTVYMELSSLTSEDTAVYYCARYGEDTSG
GYYAVDYWGQGTTVTVSS
SEQ ID NO: 47
> huM6VH6 
EVQLVQSGAEVKKPGASVKVSCKASGYRFIPYWIEWVRERPGHGLEWMGEIL
PGTGRTNYNEKFKGRVTFTADTSTNTVYMELSQLTSEDTAVYYCARYGEDTS
GGYYAVDYWGQGTTVTVSS
SEQ ID NO: 48
> huM6VH7 
EVQLVQSGAEVKKPGASVKVSCKASGYRFIPYWIEWVRERPGHGLEWMGEIL
PGTGRTNYNEKFKGRVTFTADTSTNTVYMELSSLTNEDTAVYYCARYGEDTS
GGYYAVDYWGQGTTVTVSS
SEQ ID NO: 49
> huM6VL1 DIVMTQSPDSLAVSLGERATINCRASESVNILGTNLIHWYQQKPGQPPKLLIYHA 
SNLETGVPDRFSGSGSRTDFTLTISSLQAEDVAVYYCLQSRKIPWTFGGGTKVEI
K
SEQ ID NO: 50
> huM6VL2 
EIVLTQSPATLSLSPGERATLSCRASESVNILGTNLIHWYQQKPGQAPKLLIYHA
SNLETGIPARFSGSGSRTDFTLTISSLEPEDFAVYYCLQSRKIPWTFGGGTKVEIK
SEQ ID NO: 51
> huM6VL3

DIVMTQSPATLSLSPGERATLSCRASESVNILGTNLIHWYQQKPGQAPKLLIYHA
SNLETGIPARFSGSGSRTDFTLTISSLEPEDVAVYYCLQSRKIPWTFGGGTKVEIK
SEQ ID NO: 52

The CDRs of the humanized antibodies of M6 are shown below:

**Table 7-2. The CDRs of the humanized antibodies of M6**

| Variable region | CDR | Sequence | SEQ ID NO |
|---|---|---|---|
| huM6VH5/ huM6VH6/ huM6VH7 | HCDR3 | YGEDTSGGYYAVDY | 113 |

In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

### 3. Humanization of murine antibody F4-1

For murine antibody F4-1, the humanized light chain templates were IGKV1-39*01 and IGKJ4*01, and the humanized heavy chain templates were IGHV1-3*01 and IGHJ6*01. The CDRs of murine antibody F4-1 were grafted into its humanized templates. Further, amino acids of the FR portions of the humanized antibodies were back-mutated, and it was contemplated to remove potential chemical modification sites such as isomerization, eliminate the formation of N-terminal pyroglutamic acid, reduce potential immunogenicity, etc. The light chain FR portions contained mutations of 4, 36, 42, 43, 47, 60, 70, and 75 (the positions of the mutation sites were determined according to the Kabat numbering scheme). The heavy chain FR portions contained one or more mutations of 1, 2, 12, 40, 44, 47, 48, 69, 71, and 76 (the positions of the mutation sites were determined according to the Kabat numbering scheme). The amino acid substitutions in the variable regions of the humanized antibodies of antibody F4-1 are shown in the table below.

**Table 8. The amino acid substitutions in the variable regions of the humanized antibodies of F4-1**

| **VL** | | **VH** | |
|---|---|---|---|
| huF4-1VL1 | Graft(IGKV1-39*01) + Y36F, L47W | huF4-1VH1 | Graft(IGHV1-3*01) + Q1E, W47Y, R71V |
| huF4-1VL2 | Graft(IGKV1-39*01) + M4L, Y36F, L47W, I75V | huF4-1VH2 | Graft(IGHV1-3*01) + Q1E, W47Y, M48I, I69L, R71V, S76R |
| huF4-1VL3 | Graft(IGKV1-39*01) + M4L, Y36F, K42T, A43S, L47W, I75V | huF4-1VH3 | Graft(IGHV1-3*01) + Q1E, V2I, K12V, A40R, R44G, W47Y, M48I, I69L, R71V, S76R |
| huF4-1VL4 | Graft(IGKV1-39*01) + M4L, Y36F, K42T, A43S, L47W, S60P, D70S, I75V | | |

| | | | |
|---|---|---|---|
| Note: Graft means that the CDRs of the murine antibody were grafted into the human germline FRs; the positions of the mutation sites were determined according to the Kabat numbering scheme. For example, "Y36F" means that the Y at position 36 was mutated to F, according to the Kabat numbering scheme. | | | |

The heavy chain variable region/light chain variable region sequences of the humanized antibodies of F4-1 are shown below:
> huF4-1VH1 
EVQLVQSGAEVKKPGASVKVSCKASGYTFTDNYINWVRQAPGQRLEYMGWIY
PGSGNNKFNEKFKGRVTITVDTSASTAYMELSSLRSEDTAVYYCARDYPFPSYH
YGMDYWGQGTTVTVSS
SEQ ID NO: 53
> huF4-1VH2 
EVQLVQSGAEVKKPGASVKVSCKASGYTFTDNYINWVRQAPGQRLEYIGWIYP
GSGNNKFNEKFKGRVTLTVDTSARTAYMELSSLRSEDTAVYYCARDYPFPSYH
YGMDYWGQGTTVTVSS
SEQ ID NO: 54
> huF4-1VH3 
EIQLVQSGAEVVKPGASVKVSCKASGYTFTDNYINWVRQRPGQGLEYIGWIYP
GSGNNKFNEKFKGRVTLTVDTSARTAYMELSSLRSEDTAVYYCARDYPFPSYH
YGMDYWGQGTTVTVSS
 SEQ ID NO: 55
> huF4-1VL1 
DIQMTQSPSSLSASVGDRVTITCSASSSVSYIHWFQQKPGKAPKLWIYSTSNLAS
GVPSRFSGSGSGTDFTLTISSLQPEDFATYYCQQRSSYPPTFGGGTKVEIK
SEQ ID NO: 56
> huF4-1VL2 
DIQLTQSPSSLSASVGDRVTITCSASSSVSYIHWFQQKPGKAPKLWIYSTSNLAS
GVPSRFSGSGSGTDFTLTVSSLQPEDFATYYCQQRSSYPPTFGGGTKVEIK
SEQ ID NO: 57
> huF4-1VL3 
DIQLTQSPSSLSASVGDRVTITCSASSSVSYIHWFQQKPGTSPKLWIYSTSNLASG
VPSRFSGSGSGTDFTLTVSSLQPEDFATYYCQQRSSYPPTFGGGTKVEIK
SEQ ID NO: 58
> huF4-1VL4

DIQLTQSPSSLSASVGDRVTITCSASSSVSYIHWFQQKPGTSPKLWIYSTSNLASG
VPPRFSGSGSGTSFTLTVSSLQPEDFATYYCQQRSSYPPTFGGGTKVEIK
SEQ ID NO: 59

In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

### 4. Humanization of murine antibody F4-18

For murine antibody F4-18, the humanized light chain templates were IGKV1-39*01 and IGKJ4*01, and the humanized heavy chain templates were IGHV1-3*01 and IGHJ1*01. The CDRs of murine antibody F4-18 were grafted into its humanized templates. Further, amino acids of the FR portions of the humanized antibodies were back-mutated, and it was contemplated to remove potential chemical modification sites such as isomerization, eliminate the formation of N-terminal pyroglutamic acid, reduce potential immunogenicity, etc. The light chain FR portions contained mutations of 4, 36, 39, 42, 44, 46, 60, 66, 69, and 71 (the positions of the mutation sites were determined according to the Kabat numbering scheme). The heavy chains contained one or more mutations of 12, 20, 24, 40, 44, 48, 69, 71, 96, and 101 (the positions of the mutation sites were determined according to the Kabat numbering scheme). The amino acid substitutions in the variable regions of the humanized antibodies of antibody F4-18 are shown in the table below.

**Table 9-1. The amino acid substitutions in the variable regions of the humanized antibodies of F4-18**

| **VL** | | **VH** | |
|---|---|---|---|
| huF4-18VL1 | Graft(IGKV1-39*01) + L46R, G66R, F71Y | huF4-18VH1 | Graft(IGHV1-3*01) + I69L, R71S |
| huF4-18VL2 | Graft(IGKV1-39*01) + Y36L, L46R, G66R, F71Y | huF4-18VH2 | Graft(IGHV1-3*01) + A40R, R44G, M48I, I69L, R71S |
| huF4-18VL3 | Graft(IGKV1-39*01) + M4L, Y36L, P44I, L46R, G66R, T69S, F71Y | huF4-18VH3 | Graft(IGHV1-3*01) + K12V, V20M, A24T, A40R, R44G, M48I, I69L, R71S |
| huF4-18VL4 | Graft(IGKV1-39*01) + M4L, Y36L, K39E, K42G, P44I, L46R, S60K, G66R, T69S, F71Y | huF4-18VH4 | Graft(IGHV1-3*01) + K12V, V20M, A24T, A40R, R44G, M48I, I69L, R71S+A96G |
| | | huF4-18VH5 | Graft(IGHV1-3*01) + K12V, V20M, A24T, A40R, R44G, M48I, I69L, R71S+A101G |

| | | | |
|---|---|---|---|
| Note: Graft means that the CDRs of the murine antibody were grafted into the human germline FRs; the positions of the mutation sites were determined according to the Kabat numbering scheme. For example, "M4L" means that the M at position 4 was mutated to L, according to the Kabat numbering scheme. | | | |

The heavy chain variable region/light chain variable region sequences of the humanized antibodies of F4-18 are shown below:
> huF4-18VH1 
EVQLVQSGAEVKKPGASVKVSCKASGYTFTSYGINWVRQAPGQRLEWMGYIY
LGSDYTEYNEKFKGRVTLTSDTSASTAYMELSSLRSEDTAVYYCARSAGSLFAY
WGQGTLVTVSS
SEQ ID NO: 60
> huF4-18VH2 
EVQLVQSGAEVKKPGASVKVSCKASGYTFTSYGINWVRQRPGQGLEWIGYIYL
GSDYTEYNEKFKGRVTLTSDTSASTAYMELSSLRSEDTAVYYCARSAGSLFAYW
GQGTLVTVSS
SEQ ID NO: 61
> huF4-18VH3 
EVQLVQSGAEVVKPGASVKMSCKTSGYTFTSYGINWVRQRPGQGLEWIGYIYL
GSDYTEYNEKFKGRVTLTSDTSASTAYMELSSLRSEDTAVYYCARSAGSLFAYW
GQGTLVTVSS
SEQ ID NO: 62
> huF4-18VH4 
EVQLVQSGAEVVKPGASVKMSCKTSGYTFTSYGINWVRQRPGQGLEWIGYIYL
GSDYTEYNEKFKGRVTLTSDTSASTAYMELSSLRSEDTAVYYCARSGGSLFAYW
GQGTLVTVSS
SEQ ID NO: 63
> huF4-18VH5 
EVQLVQSGAEVVKPGASVKMSCKTSGYTFTSYGINWVRQRPGQGLEWIGYIYL
GSDYTEYNEKFKGRVTLTSDTSASTAYMELSSLRSEDTAVYYCARSAGSLFGYW
GQGTLVTVSS
SEQ ID NO: 64
> huF4-18VL1 
DIQMTQSPSSLSASVGDRVTITCRASQDIGITLNWYQQKPGKAPKRLIYATSSLDS
GVPSRFSGSRSGTDYTLTISSLQPEDFATYYCLQYASSPYTFGGGTKVEIK
SEQ ID NO: 65
> huF4-18VL2 
DIQMTQSPSSLSASVGDRVTITCRASQDIGITLNWLQQKPGKAPKRLIYATSSLDS
GVPSRFSGSRSGTDYTLTISSLQPEDFATYYCLQYASSPYTFGGGTKVEIK
SEQ ID NO: 66
> huF4-18VL3 
DIQLTQSPSSLSASVGDRVTITCRASQDIGITLNWLQQKPGKAIKRLIYATSSLDS
GVPSRFSGSRSGSDYTLTISSLQPEDFATYYCLQYASSPYTFGGGTKVEIK
SEQ ID NO: 67
> huF4-18VL4

DIQLTQSPSSLSASVGDRVTITCRASQDIGITLNWLQQEPGGAIKRLIYATSSLDS
GVPKRFSGSRSGSDYTLTISSLQPEDFATYYCLQYASSPYTFGGGTKVEIK
SEQ ID NO: 68

The CDRs of the humanized antibodies of F4-18 are shown below:

**Table 9-2. The CDRs of the humanized antibodies of F4-18**

| Variable region | CDR | Sequence | SEQ ID NO |
|---|---|---|---|
| huF4-18VH4 | HCDR3 | SGGSLFAY | 114 |
| huF4-18VH5 | HCDR3 | SAGSLFGY | 115 |

In the above sequences, the regions are arranged in the following order: FR1-CDR1-FR2-CDR2-FR3-CDR3-FR4; the CDR sequences determined according to the Kabat numbering scheme are underlined, and the portions that are not underlined are the FR sequences.

5. Construction and expression of IgG1 formats of anti-MUC1-C humanized antibodies Primers were designed, and VH/VK gene fragments of the humanized antibodies were constructed by PCR and then homologously recombined with expression vector pTT5 (with a signal peptide and a constant region gene (CH1-FC/CL) fragment, constructed in the laboratory) to construct antibody full-length expression vector VH-CH1-FC-pTT5/VK-CL-pTT5. The heavy chain constant regions of the antibodies may be selected from the group consisting of the heavy chain constant regions of human IgG1, IgG2, IgG3, and IgG4, and variants thereof, and the light chain constant regions may be selected from the group consisting of the light chain constant regions of human κ and λ chains and variants thereof. Illustratively, in the following examples, the heavy chain constant regions of the antibodies were selected from the group consisting of human IgG1 heavy chain constant regions set forth in SEQ ID NOs: 69 and 186, and the light chain constant regions were selected from the group consisting of a human light chain constant region set forth in SEQ ID NO: 70.

Human IgG1 heavy chain constant region sequence:








SEQ ID NO: 69

Human IgG1 heavy chain constant region sequence (a format with the LALA mutations):

ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK

SEQ ID NO: 186;

Human light chain constant region sequence:



SEQ ID NO: 70

The carboxy-termini of the heavy chain variable regions of the murine antibodies M4, M6, F4-1, and F4-18 obtained above were linked to the amino-terminus of the human heavy chain constant region set forth in SEQ ID NO: 69, and the carboxy-termini of the light chain variable regions of the murine antibodies were linked to the amino-terminus of the human light chain constant region set forth in SEQ ID NO: 70. Thus, their corresponding chimeric antibodies can be obtained. Specifically, the chimeric antibodies of M4, M6, F4-1, and F4-18 were denoted by ChiM4, ChiM6, ChiF4-1, and ChiF4-18, respectively.

The carboxy-termini of the heavy chain variable regions of the humanized antibodies of M4, M6, F4-1, and F4-18 constructed above were linked to the amino-terminus of the human heavy chain constant region set forth in SEQ ID NO: 69 to form antibody full-length heavy chains, and the carboxy-termini of the light chain variable regions of the humanized antibodies of M4, M6, F4-1, and F4-18 were linked to the amino-terminus of the human light chain constant region set forth in SEQ ID NO: 70 to form antibody full-length light chains. Thus, the humanized antibodies shown in Tables 10-13 below can be obtained.

**Table 10. The humanized antibodies of M4**

| Variable region | huM4VL1 | huM4VL2 | huM4VL3 | huM4VL4 |
|---|---|---|---|---|
| huM4VH1 | M4-H1L1 | M4-H1L2 | M4-H1L3 | M4-H1L4 |
| huM4VH2 | M4-H2L1 | M4-H2L2 | M4-H2L3 | M4-H2L4 |
| huM4VH3 | M4-H3L1 | M4-H3L2 | M4-H3L3 | M4-H3L4 |

| | | | | |
|---|---|---|---|---|
| Note: In the table, "M4-H1L1" represents a humanized antibody whose heavy chain variable region is huM4VH1 (SEQ ID NO: 36), whose light chain variable region is huM4VL1 (SEQ ID NO: 39), whose heavy chain constant region is set forth in SEQ ID NO: 69, and whose light chain constant region is set forth in SEQ ID NO: 70; this similarly applies to the other antibodies. | | | | |

**Table 11. The humanized antibodies of M6**

| Variable region | huM6VL1 | huM6VL2 | huM6VL3 |
|---|---|---|---|
| huM6VH1 | M6-H1L1 | M6-H1L2 | M6-H1L3 |
| huM6VH2 | M6-H2L1 | M6-H2L2 | M6-H2L3 |
| huM6VH3 | M6-H3L1 | M6-H3L2 | M6-H3L3 |
| huM6VH4 | M6-H4L1 | M6-H4L2 | M6-H4L3 |
| huM6VH5 | M6-H5L1 | M6-H5L2 | M6-H5L3 |
| huM6VH6 | M6-H6L1 | M6-H6L2 | M6-H6L3 |
| huM6VH7 | M6-H7L1 | M6-H7L2 | M6-H7L3 |

| | | | |
|---|---|---|---|
| Note: In the table, "M6-H1L1" represents a humanized antibody whose heavy chain variable region is huM6VH1 (SEQ ID NO: 43), whose light chain variable region is huM6VL1 (SEQ ID NO: 50), whose heavy chain constant region is set forth in SEQ ID NO: 69, and whose light chain constant region is set forth in SEQ ID NO: 70; this similarly applies to the other antibodies. | | | |

**Table 12. The humanized antibodies of F4-1**

| Variable region | huF4-1VL1 | huF4-1VL2 | huF4-1VL3 | huF4-1VL4 |
|---|---|---|---|---|
| huF4-1VH1 | F4-1-H1L1 | F4-1-H1L2 | F4-1-H1L3 | F4-1-H1L4 |
| huF4-1VH2 | F4-1-H2L1 | F4-1-H2L2 | F4-1-H2L3 | F4-1-H2L4 |
| huF4-1VH3 | F4-1-H3L1 | F4-1-H3L2 | F4-1-H3L3 | F4-1-H3L4 |

| | | | | |
|---|---|---|---|---|
| Note: In the table, "F4-1-H1L1" represents a humanized antibody whose heavy chain variable region is huF4-1VH1 (SEQ ID NO: 53), whose light chain variable region is huF4-1VL1 (SEQ ID NO: 56), whose heavy chain constant region is set forth in SEQ ID NO: 69, and whose light chain constant region is set forth in SEQ ID NO: 70; this similarly applies to the other antibodies. | | | | |

**Table 13. The humanized antibodies of F4-18**

| Variable region | huF4-18VL1 | huF4-18VL2 | huF4-18VL3 | huF4-18VL4 |
|---|---|---|---|---|
| huF4-18VH1 | F4-18-H1L1 | F4-18-H1L2 | F4-18-H1L3 | F4-18-H1L4 |
| huF4-18VH2 | F4-18-H2L1 | F4-18-H2L2 | F4-18-H2L3 | F4-18-H2L4 |
| huF4-18VH3 | F4-18-H3L1 | F4-18-H3L2 | F4-18-H3L3 | F4-18-H3L4 |
| huF4-18VH4 | F4-18-H4L1 | F4-18-H4L2 | F4-18-H4L3 | F4-18-H4L4 |
| huF4-18VH5 | F4-18-H5L1 | F4-18-H5L2 | F4-18-H5L3 | F4-18-H5L4 |

| | | | | |
|---|---|---|---|---|
| Note: In the table, "F4-18-H1L1" represents a humanized antibody whose heavy chain variable region is huF4-18VH1 (SEQ ID NO: 60), whose light chain variable region is huF4-18VL1 (SEQ ID NO: 65), whose heavy chain constant region is set forth in SEQ ID NO: 69, and whose light chain constant region is set forth in SEQ ID NO: 70; this similarly applies to the other antibodies. | | | | |

Exemplary humanized antibody heavy chain/light chain full-length sequences are shown below:
> Heavy chain sequence of M4-H1L1: 
EVQLVQSGAEVKKPGASVKVSCKASGYTFTTYGVPWVRQAPGQGLEWMGDIY
PRSGNTYYNEKFKGRVTMTADKSTDTVYMELSSLRSEDTAVYYCAREDYDNYP
YALDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
 
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK
SEQ ID NO: 71
> Light chain sequence of M4-H1L1: 
DIQMTQSPSSLSASVGDRVTITCSASSSVFNMNWYQQKPGKSPKLWIYDISKLAS
GVPSRFSGSGSGTDFTLTISSLQPEDFATYYCQQRSFYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 72
> Heavy chain sequence of M4-H2L1: 
EVQLVQSGAEVKKPGASVKVSCKASGYSFTTYGVPWVRQRPGQGLEWIGDIYP
RSGNTYYNEKFKGRVTMTADKSTDTVYMELSSLRSEDTAVYYCAREDYDNYPY
ALDYWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 73
> Light chain sequence of M4-H2L1: 
DIQMTQSPSSLSASVGDRVTITCSASSSVFNMNWYQQKPGKSPKLWIYDISKLAS
GVPSRFSGSGSGTDFTLTISSLQPEDFATYYCQQRSFYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 74
> Heavy chain sequence of M4-H1L2: 
EVQLVQSGAEVKKPGASVKVSCKASGYTFTTYGVPWVRQAPGQGLEWMGDIY
PRSGNTYYNEKFKGRVTMTADKSTDTVYMELSSLRSEDTAVYYCAREDYDNYP
YALDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
 
FYPSDIAVEWESNGQPENNYKTTPPVLDSDGSFFLYSKLTVDKSRWQQGNVFSC
SVMHEALHNHYTQKSLSLSPGK
SEQ ID NO: 75
> Light chain sequence of M4-H1L2: 
DIVMTQSPSSLSASVGDRVTITCSASSSVFNMNWYQQKPGKSPKLWIYDISKLAS
GVPSRFSGSGSGTDFTLTISSLQPEDFATYYCQQRSFYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 76
> Heavy chain sequence of M4-H2L2: 
EVQLVQSGAEVKKPGASVKVSCKASGYSFTTYGVPWVRQRPGQGLEWIGDIYP
RSGNTYYNEKFKGRVTMTADKSTDTVYMELSSLRSEDTAVYYCAREDYDNYPY
ALDYWGQGTTVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSV
MHEALHNHYTQKSLSLSPGK
SEQ ID NO: 77
> Light chain sequence of M4-H2L2: 
DIVMTQSPSSLSASVGDRVTITCSASSSVFNMNWYQQKPGKSPKLWIYDISKLAS
GVPSRFSGSGSGTDFTLTISSLQPEDFATYYCQQRSFYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 78
> Heavy chain sequence of M6-H1L1: 
EVQLVQSGAEVKKPGASVKVSCKASGYTFTPYWIEWVRQAPGQGLEWMGEIL
PGTGRTNYNEKFKGRVTFTADTSTNTVYMELSSLRSEDTAVYYCARYGDDTSG
GYYAVDYWGQGTTVTVSSASTKGPSVFPLAPSSKSTSGGTAALGCLVKDYFPEP
VTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPSRDELTKNQVSLTCLV
KGFYPSDIAVEWESNGQPENNYKTTPPVLDSDGSFFLYSKLTVDKSRWQQGNVF
SCSVMHEALHNHYTQKSLSLSPGK
 SEQ ID NO: 79
> Light chain sequence of M6-H1L1: 
DIVMTQSPDSLAVSLGERATINCRASESVNILGTNLIHWYQQKPGQPPKLLIYHA
SNLETGVPDRFSGSGSRTDFTLTISSLQAEDVAVYYCLQSRKIPWTFGGGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
SEQ ID NO: 80
> Heavy chain sequence of M6-H3L1: 
EVQLVQSGAEVKKPGASVKVSCKASGYRFIPYWIEWVRQRPGQGLEWMGEILP
GTGRTNYNEKFKGRVTFTADTSTNTVYMELSSLRSEDTAVYYCARYGDDTSGG
YYAVDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 81
> Light chain sequence of M6-H3L1: 
DIVMTQSPDSLAVSLGERATINCRASESVNILGTNLIHWYQQKPGQPPKLLIYHA
SNLETGVPDRFSGSGSRTDFTLTISSLQAEDVAVYYCLQSRKIPWTFGGGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC
SEQ ID NO: 82
> Heavy chain sequence of M6-H2L2: 
EVQLVQSGAEVKKPGASVKVSCKASGYRFIPYWIEWVRQAPGQGLEWMGEILP
GTGRTNYNEKFKGRVTFTADTSTNTVYMELSSLRSEDTAVYYCARYGDDTSGG
YYAVDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 83
> Light chain sequence of M6-H2L2: 
EIVLTQSPATLSLSPGERATLSCRASESVNILGTNLIHWYQQKPGQAPKLLIYHAS
NLETGIPARFSGSGSRTDFTLTISSLEPEDFAVYYCLQSRKIPWTFGGGTKVEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 84
> Heavy chain sequence of M6-H3L2: 
EVQLVQSGAEVKKPGASVKVSCKASGYRFIPYWIEWVRQRPGQGLEWMGEILP
GTGRTNYNEKFKGRVTFTADTSTNTVYMELSSLRSEDTAVYYCARYGDDTSGG
YYAVDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 85
> Light chain sequence of M6-H3L2: 
EIVLTQSPATLSLSPGERATLSCRASESVNILGTNLIHWYQQKPGQAPKLLIYHAS
NLETGIPARFSGSGSRTDFTLTISSLEPEDFAVYYCLQSRKIPWTFGGGTKVEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 86
> Heavy chain sequence of M6-H4L2: 
EVQLVQSGAEVKKPGASVKVSCKASGYRFIPYWIEWVRERPGHGLEWMGEILP
GTGRTNYNEKFKGRVTFTADTSTNTVYMELSSLTSEDTAVYYCARYGDDTSGG
YYAVDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 87
> Light chain sequence of M6-H4L2: 
EIVLTQSPATLSLSPGERATLSCRASESVNILGTNLIHWYQQKPGQAPKLLIYHAS
NLETGIPARFSGSGSRTDFTLTISSLEPEDFAVYYCLQSRKIPWTFGGGTKVEIKRT
 
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 88
> Heavy chain sequence of F4-1-H1L1: 
EVQLVQSGAEVKKPGASVKVSCKASGYTFTDNYINWVRQAPGQRLEYMGWIY
PGSGNNKFNEKFKGRVTITVDTSASTAYMELSSLRSEDTAVYYCARDYPFPSYH
YGMDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 89
> Light chain sequence of F4-1-H1L1: 
DIQMTQSPSSLSASVGDRVTITCSASSSVSYIHWFQQKPGKAPKLWIYSTSNLAS
GVPSRFSGSGSGTDFTLTISSLQPEDFATYYCQQRSSYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 90
> Heavy chain sequence of F4-1-H1L2: 
EVQLVQSGAEVKKPGASVKVSCKASGYTFTDNYINWVRQAPGQRLEYMGWIY
PGSGNNKFNEKFKGRVTITVDTSASTAYMELSSLRSEDTAVYYCARDYPFPSYH
YGMDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 91
> Light chain sequence of F4-1-H1L2: 
DIQLTQSPSSLSASVGDRVTITCSASSSVSYIHWFQQKPGKAPKLWIYSTSNLASG
VPSRFSGSGSGTDFTLTVSSLQPEDFATYYCQQRSSYPPTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
 SEQ ID NO: 92
> Heavy chain sequence of F4-1-H2L2: 
EVQLVQSGAEVKKPGASVKVSCKASGYTFTDNYINWVRQAPGQRLEYIGWIYP
GSGNNKFNEKFKGRVTLTVDTSARTAYMELSSLRSEDTAVYYCARDYPFPSYHY
GMDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSC
SVMHEALHNHYTQKSLSLSPGK
SEQ ID NO: 93
> Light chain sequence of F4-1-H2L2: 
DIQLTQSPSSLSASVGDRVTITCSASSSVSYIHWFQQKPGKAPKLWIYSTSNLASG
VPSRFSGSGSGTDFTLTVSSLQPEDFATYYCQQRSSYPPTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 94
> Heavy chain sequence of F4-1-H1L4: 
EVQLVQSGAEVKKPGASVKVSCKASGYTFTDNYINWVRQAPGQRLEYMGWIY
PGSGNNKFNEKFKGRVTITVDTSASTAYMELSSLRSEDTAVYYCARDYPFPSYH
YGMDYWGQGTTVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNT
KVDKKVEPKSCDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPSRDELTKNQVSLTCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 95
> Light chain sequence of F4-1-H1L4: 
DIQLTQSPSSLSASVGDRVTITCSASSSVSYIHWFQQKPGTSPKLWIYSTSNLASG
VPPRFSGSGSGTSFTLTVSSLQPEDFATYYCQQRSSYPPTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 96
> Heavy chain sequence of F4-18-H1L1: 
EVQLVQSGAEVKKPGASVKVSCKASGYTFTSYGINWVRQAPGQRLEWMGYIY
LGSDYTEYNEKFKGRVTLTSDTSASTAYMELSSLRSEDTAVYYCARSAGSLFAY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK
SEQ ID NO: 97
> Light chain sequence of F4-18-H1L1: 
DIQMTQSPSSLSASVGDRVTITCRASQDIGITLNWYQQKPGKAPKRLIYATSSLDS
GVPSRFSGSRSGTDYTLTISSLQPEDFATYYCLQYASSPYTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 98
> Heavy chain sequence of F4-18-H2L1: 
EVQLVQSGAEVKKPGASVKVSCKASGYTFTSYGINWVRQRPGQGLEWIGYIYL
GSDYTEYNEKFKGRVTLTSDTSASTAYMELSSLRSEDTAVYYCARSAGSLFAYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 99
> Light chain sequence of F4-18-H2L1: 
DIQMTQSPSSLSASVGDRVTITCRASQDIGITLNWYQQKPGKAPKRLIYATSSLDS
GVPSRFSGSRSGTDYTLTISSLQPEDFATYYCLQYASSPYTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 100
> Heavy chain sequence of F4-18-H2L2: 
EVQLVQSGAEVKKPGASVKVSCKASGYTFTSYGINWVRQRPGQGLEWIGYIYL
GSDYTEYNEKFKGRVTLTSDTSASTAYMELSSLRSEDTAVYYCARSAGSLFAYW
 
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 101
> Light chain sequence of F4-18-H2L2: 
DIQMTQSPSSLSASVGDRVTITCRASQDIGITLNWLQQKPGKAPKRLIYATSSLDS
GVPSRFSGSRSGTDYTLTISSLQPEDFATYYCLQYASSPYTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 102
> Heavy chain sequence of F4-18-H2L3: 
EVQLVQSGAEVKKPGASVKVSCKASGYTFTSYGINWVRQRPGQGLEWIGYIYL
GSDYTEYNEKFKGRVTLTSDTSASTAYMELSSLRSEDTAVYYCARSAGSLFAYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 103
> Light chain sequence of F4-18-H2L3: 
DIQLTQSPSSLSASVGDRVTITCRASQDIGITLNWLQQKPGKAIKRLIYATSSLDSG
VPSRFSGSRSGSDYTLTISSLQPEDFATYYCLQYASSPYTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 104
> Heavy chain sequence of F4-18-H4L3: 
EVQLVQSGAEVVKPGASVKMSCKTSGYTFTSYGINWVRQRPGQGLEWIGYIYL
GSDYTEYNEKFKGRVTLTSDTSASTAYMELSSLRSEDTAVYYCARSGGSLFAYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
 
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 105
> Light chain sequence of F4-18-H4L3: 
DIQLTQSPSSLSASVGDRVTITCRASQDIGITLNWLQQKPGKAIKRLIYATSSLDSG
VPSRFSGSRSGSDYTLTISSLQPEDFATYYCLQYASSPYTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 106
> Heavy chain sequence of F4-18-H1L4: 
EVQLVQSGAEVKKPGASVKVSCKASGYTFTSYGINWVRQAPGQRLEWMGYIY
LGSDYTEYNEKFKGRVTLTSDTSASTAYMELSSLRSEDTAVYYCARSAGSLFAY
WGQGTLVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK
SEQ ID NO: 107
> Light chain sequence of F4-18-H1L4: 
DIQLTQSPSSLSASVGDRVTITCRASQDIGITLNWLQQEPGGAIKRLIYATSSLDSG
VPKRFSGSRSGSDYTLTISSLQPEDFATYYCLQYASSPYTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 108
> Heavy chain sequence of F4-18-H2L4: 
EVQLVQSGAEVKKPGASVKVSCKASGYTFTSYGINWVRQRPGQGLEWIGYIYL
GSDYTEYNEKFKGRVTLTSDTSASTAYMELSSLRSEDTAVYYCARSAGSLFAYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
 
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 109
> Light chain sequence of F4-18-H2L4: 
DIQLTQSPSSLSASVGDRVTITCRASQDIGITLNWLQQEPGGAIKRLIYATSSLDSG
VPKRFSGSRSGSDYTLTISSLQPEDFATYYCLQYASSPYTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 110
> Heavy chain sequence of F4-18-H4L4: 
EVQLVQSGAEVVKPGASVKMSCKTSGYTFTSYGINWVRQRPGQGLEWIGYIYL
GSDYTEYNEKFKGRVTLTSDTSASTAYMELSSLRSEDTAVYYCARSGGSLFAYW
GQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPELLGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 111
> Light chain sequence of F4-18-H4L4: 
DIQLTQSPSSLSASVGDRVTITCRASQDIGITLNWLQQEPGGAIKRLIYATSSLDSG
VPKRFSGSRSGSDYTLTISSLQPEDFATYYCLQYASSPYTFGGGTKVEIKRTVAAP
SVFIFPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC
SEQ ID NO: 112
Note: In the above antibody full-length sequences, the antibody variable region sequences are underlined, and the portions that are not underlined are the antibody constant region sequences.

### Example 6: Engineering of Anti-EGFR Antibody

Molecules that specifically bind to EGFR may be derived from any suitable antibody, such as zalutumumab or a variant thereof:

**Table 14. The CDR sequences of zalutumumab**

| Zalutumumab | | | |
|---|---|---|---|
| HCDR1 | TYGMH (SEQ ID NO: 116) | LCDR1 | RASQDISSALV(SEQ ID NO: 119) |
| HCDR2 | VIWDDGSYKYYGDSVKG (SEQ ID NO: 117) | LCDR2 | DASSLES (SEQ ID NO: 120) |
| HCDR3 | DGITMVRGVMKDYFDY (SEQ ID NO: 118) | LCDR3 | QQFNSYPLT (SEQ ID NO: 121) |

> Heavy chain variable region sequence of zalutumumab (abbreviated as "ZalVH"): 



SEQ ID NO: 122
> Light chain variable region sequence of zalutumumab (abbreviated as "ZalVL"): 


SEQ ID NO: 123
> Heavy chain sequence of zalutumumab: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKRVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 124
> Light chain sequence of zalutumumab:

AIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 125

By mutating the amino acid(s) at position(s) 31, 33, 52A, 56, 60, 97, and/or 99 of the heavy chain variable region and/or the amino acid at position 1 of the light chain variable region of zalutumumab, a total of 15 anti-EGFR antibodies were obtained, and they were ZalH', ZalH1, ZalH2, ZalH3, ZalH4, ZalH5, ZalH6, ZalH7, ZalH8, ZalH9, ZalH10, ZalH11, ZalH12, ZalH13, and ZalH14. Their specific sequences are shown below:

**Table 15. The amino acid sequences of replaced CDRs**

| Antibody | CDR | Amino acid sequence | SEQ ID NO |
|---|---|---|---|
| ZalH1 | HCDR1 | SYGMH | 126 |
| ZalH2 | HCDR2 | VIWEDGSYKYYGDSVKG | 127 |
| ZalH3 | HCDR3 | DGLTMVRGVMKDYFDY | 128 |
| ZalH4 | HCDR3 | DGVTMVRGVMKDYFDY | 129 |
| ZalH5 | HCDR3 | DGATMVRGVMKDYFDY | 130 |
| ZalH6 | HCDR3 | DGITVVRGVMKDYFDY | 131 |
| ZalH7 | HCDR1 | SYGMH | 126 |
| | HCDR2 | VIWEDGSYKYYGDSVKG | 127 |
| ZalH8 | HCDR1 | SYGMH | 126 |
| | HCDR3 | DGLTMVRGVMKDYFDY | 128 |
| ZalH9 | HCDR1 | SYGMH | 126 |
| | HCDR3 | DGVTMVRGVMKDYFDY | 129 |
| ZalH10 | HCDR1 | SYGMH | 126 |
| | HCDR3 | DGATMVRGVMKDYFDY | 130 |
| ZalH11 | HCDR1 | SYGMH | 126 |
| | HCDR3 | DGITVVRGVMKDYFDY | 131 |
| ZalH12 | HCDR2 | VIWDDGSNKYYGDSVKG | 132 |
| ZalH13 | HCDR1 | SYAMH | 133 |
| ZalH14 | HCDR2 | VIWYDGSNKYYADSVKG | 134 |

| | | | |
|---|---|---|---|
| Note: Antibody ZalH1 means that amino acid mutations were made at position 31 of the heavy chain variable region (i.e., HCDR1) and position 1 of the light chain variable region of zalutumumab; antibody ZalH7 means that mutations were made at positions 31 and 52A of the heavy chain variable region (i.e., HCDR1 and HCDR2) and position 1 of the light chain variable region of zalutumumab simultaneously; ZalH' means that a mutation was made only at position 1 of the light chain variable region of zalutumumab; the antibody heavy chain constant region was selected from the group consisting of the human IgG1 heavy chain constant region set forth in SEQ ID NO: 69, and the light chain constant region was selected from the group consisting of the human light chain constant region set forth in SEQ ID NO: 70. This similarly applies to the other antibodies. | | | |

> Heavy chain variable region sequence of ZalH1 (abbreviated as "Za1VH1"): 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSS
 SEQ ID NO: 135
> Heavy chain variable region sequence of ZalH2 (abbreviated as "ZalVH2"): 
QVQLVESGGGVVQPGRSLRLSCAASGFTFSTYGMHWVRQAPGKGLEWVAVIW
EDGSYKYYGDSVKGRFTISRDNSKNTLYLQMNSLRAEDTAVYYCARDGITMVR
GVMKDYFDYWGQGTLVTVSS
SEQ ID NO: 136
> Heavy chain variable region sequence of ZalH3 (abbreviated as "ZalVH3"): 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGLTMV
RGVMKDYFDYWGQGTLVTVSS
SEQ ID NO: 137
> Heavy chain variable region sequence of ZalH4 (abbreviated as "ZalVH4"): 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGVTMV
RGVMKDYFDYWGQGTLVTVSS
SEQ ID NO: 138
> Heavy chain variable region sequence of ZalH5 (abbreviated as "ZalVH5"): 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGATMV
RGVMKDYFDYWGQGTLVTVSS
SEQ ID NO: 139
> Heavy chain variable region sequence of ZalH6 (abbreviated as "ZalVH6"): 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITVVR
GVMKDYFDYWGQGTLVTVSS
SEQ ID NO: 140
> Heavy chain variable region sequence of ZalH7 (abbreviated as "ZalVH7"): 
QVQLVESGGGVVQPGRSLRLSCAASGFTFSSYGMHWVRQAPGKGLEWVAVIW
EDGSYKYYGDSVKGRFTISRDNSKNTLYLQMNSLRAEDTAVYYCARDGITMVR
GVMKDYFDYWGQGTLVTVSS
SEQ ID NO: 141
> Heavy chain variable region sequence of ZalH8 (abbreviated as "ZalVH8"): 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGLTMV
RGVMKDYFDYWGQGTLVTVSS
SEQ ID NO: 142
> Heavy chain variable region sequence of ZalH9 (abbreviated as "ZalVH9"): 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGVTMV
RGVMKDYFDYWGQGTLVTVSS
SEQ ID NO: 143
> Heavy chain variable region sequence of ZalH10 (abbreviated as "ZalVH10"): 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGATMV
RGVMKDYFDYWGQGTLVTVSS
SEQ ID NO: 144
> Heavy chain variable region sequence of ZalH11 (abbreviated as "ZalVH11"): 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGITVVR
GVMKDYFDYWGQGTLVTVSS
SEQ ID NO: 145
> Heavy chain variable region sequence of ZalH12 (abbreviated as "ZalVH12"): 
QVQLVESGGGVVQPGRSLRLSCAASGFTFSTYGMHWVRQAPGKGLEWVAVIW
DDGSNKYYGDSVKGRFTISRDNSKNTLYLQMNSLRAEDTAVYYCARDGITMVR
GVMKDYFDYWGQGTLVTVSS
SEQ ID NO: 146
> Heavy chain variable region sequence of ZalH13 (abbreviated as "ZalVH13"): 
QVQLVESGGGVVQPGRSLRLSCAASGFTFSSYAMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSS
SEQ ID NO: 147
> Heavy chain variable region sequence of ZalH14 (abbreviated as "ZalVH14"): 
QVQLVESGGGVVQPGRSLRLSCAASGFTFSTYGMHWVRQAPGKGLEWVAVIW
YDGSNKYYADSVKGRFTISRDNSKNTLYLQMNSLRAEDTAVYYCARDGITMVR
GVMKDYFDYWGQGTLVTVSS
SEQ ID NO: 148
> Light chain variable region sequences of ZalH' and ZalH1 to ZalH14 (hereinafter abbreviated as "Za1VL1"): 
DIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIK
SEQ ID NO: 149
> Heavy chain sequence of ZalH': SEQ ID NO: 124
> Heavy chain sequence of ZalH1: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 150
> Heavy chain sequence of ZalH2: 
QVQLVESGGGVVQPGRSLRLSCAASGFTFSTYGMHWVRQAPGKGLEWVAVIW
EDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 151
> Heavy chain sequence of ZalH3: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGLTMV
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
GNVFSCSVMHEALHNHYTQKSLSLSPGK
SEQ ID NO: 152
> Heavy chain sequence of ZalH4: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGVTMV
 
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
GNVFSCSVMHEALHNHYTQKSLSLSPGK
SEQ ID NO: 153
> Heavy chain sequence of ZalH5: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGATMV
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
GNVFSCSVMHEALHNHYTQKSLSLSPGK
SEQ ID NO: 154
> Heavy chain sequence of ZalH6: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITVVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 155
> Heavy chain sequence of ZalH7: 
QVQLVESGGGVVQPGRSLRLSCAASGFTFSSYGMHWVRQAPGKGLEWVAVIW
EDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
 
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 156
> Heavy chain sequence of ZalH8: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGLTMV
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
GNVFSCSVMHEALHNHYTQKSLSLSPGK
SEQ ID NO: 157
> Heavy chain sequence of ZalH9: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGVTMV
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
GNVFSCSVMHEALHNHYTQKSLSLSPGK
SEQ ID NO: 158
> Heavy chain sequence of ZalH10: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGATMV
RGVMKDYFDYWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEV
TCVVVDVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQ
 
GNVFSCSVMHEALHNHYTQKSLSLSPGK
SEQ ID NO: 159
> Heavy chain sequence of ZalH11: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGITVVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 160
> Heavy chain sequence of ZalH12: 
QVQLVESGGGVVQPGRSLRLSCAASGFTFSTYGMHWVRQAPGKGLEWVAVIW
DDGSNKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 161
> Heavy chain sequence of ZalH13: 
QVQLVESGGGVVQPGRSLRLSCAASGFTFSSYAMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 162
> Heavy chain sequence of ZalH14: 
QVQLVESGGGVVQPGRSLRLSCAASGFTFSTYGMHWVRQAPGKGLEWVAVIW
YDGSNKYYADSVKGRFTISRDNSKNTLYLQMNSLRAEDTAVYYCARDGITMVR
GVMKDYFDYWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK
SEQ ID NO: 163
> Light chain sequences of ZalH' and ZalH1 to ZalH14: 
DIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 164
Note: In the above antibody sequences, the antibody variable region sequences are underlined, the antibody CDR sequences are double underlined, the portions that are not underlined are the antibody constant region sequences, and the letters in bold type are mutated amino acids.

### Example 7: Construction of Anti-EGFR-MUC1 Bispecific Antibodies

A 1:1 molecular format was used for EGFR-MUC1 bispecific antibodies. M4-H1L1 (hereinafter referred to as "M4H1L1") was selected as the MUC1 arm and combined with EGFR antibody ZalH', ZalH4, ZalH8, ZalH10, or ZalH13 into a bispecific antibody in which the VH of the EGFR antibody is combined with Titin and its VL is combined with Obscurin. In addition, mutations S358C and T370W (knobs) were introduced into the heavy chain of the MUC1 antibody, and mutations Y357C, T374S, L376A, and Y415V (holes) were introduced into the heavy chain of the EGFR antibody. The format was an asymmetrically structured molecule containing four chains:

chain 1: [VH (anti-MUC1)]-[IgG1 (CH1)]-[Fc (Knob)];

chain 2: [VL (anti-MUC1)]-[CL];

chain 3: [VH (anti-EGFR)]-[linker 1]-[Titin]-[Fc (Hole)];

and

chain 4: [VL (anti-EGFR)]-[linker 2]-[Obscurin].

The format is schematically shown in FIG. 5 (where T stands for Titin and O stands for Obscurin).

**Table 16. The bispecific antibodies of the present disclosure**

| Bispecific antibody | Chain 1 | Chain 2 | Chain 3 | Chain 4 |
|---|---|---|---|---|
| M4H1L1-ZalH' | huM4VH1-CH1-Fc(Knob) (SEQ ID NO: 171) | huM4VL1-CL (SEQ ID NO: 74) | ZalVH-linker 1-Titin-Fc (Hole) (SEQ ID NO: 172) | ZalVL1-linker 2 -Obscurin (SEQ ID NO: 173) |
| M4H1L1-ZalH4 | | | ZalVH4-linker 1-Titin-Fc (Hole) (SEQ ID NO: 174) | |
| M4H1L1-ZalH8 | | | ZalVH8-linker 1-Titin-Fc (Hole) (SEQ ID NO: 175) | |
| M4H1L1-ZalH10 | | | ZalVH10-linker 1-Titin-Fc (Hole) (SEQ ID NO: 176) | |
| M4H1L1-ZalH13 | | | ZalVH13-linker 1-Titin-Fc (Hole) (SEQ ID NO: 177) | |

| | | | | |
|---|---|---|---|---|
| Note: Illustratively, M4H1L1-ZalH' means that the molecule uses the variable regions of M4-H1L1 as its MUC1-binding domain, the variable regions of ZalH' as its EGFR-binding domain, and the format shown in FIG. 5 as its molecular structure. This similarly applies to the other molecules. | | | | |

> Titin chain: 
GIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI
SEQ ID NO: 165
> Obscurin chain: 
SGAPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA
SEQ ID NO: 166
> CH1: 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKV
SEQ ID NO: 167
> CL: SEQ ID NO: 70
> Linker 1 and linker 2: GGGGS (SEQ ID NO: 168)
> Fc (knob): 
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPCRDELTKNQVSLWCLVKGFYPSD
 
IAVEWESNGQPENNYKTTPPVLDSDGSFFLYSKLTVDKSRWQQGNVFSCSVMH
EALHNHYTQKSLSLSPGK
SEQ ID NO: 169
> Fc (hole):

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSRDELTKNQVSLSCAVKGFYPSDIAVEWE
SNGQPENNYKTTPPVLDSDGSFFLVSKLTVDKSRWQQGNVFSCSVMHEALHNH
YTQKSLSLSPGK
SEQ ID NO: 170

Their full-length sequences are shown below: Sequences of M4H1L1-ZalH':
Chain 1 (huM4VH1-CH1-Fc (Knob)): 
EVQLVQSGAEVKKPGASVKVSCKASGYTFTTYGVPWVRQAPGQGLEWMGDIY
PRSGNTYYNEKFKGRVTMTADKSTDTVYMELSSLRSEDTAVYYCAREDYDNYP
YALDYWGQGTTVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNTK
VDKKVEPKSCDKTHTCPPCPAPELLGGPSVFLFPPKPKDTLMISRTPEVTCVVVD
VSHEDPEVKFNWYVDGVEVHNAKTKPREEQYNSTYRVVSVLTVLHQDWLNG
KEYKCKVSNKALPAPIEKTISKAKGQPREPQVYTLPPCRDELTKNQVSLWCLVK
GFYPSDIAVEWESNGQPENNYKTTPPVLDSDGSFFLYSKLTVDKSRWQQGNVFS
CSVMHEALHNHYTQKSLSLSPGK
SEQ ID NO: 171
> Chain 2 (huM4VL1-CL): 
DIQMTQSPSSLSASVGDRVTITCSASSSVFNMNWYQQKPGKSPKLWIYDISKLAS
GVPSRFSGSGSGTDFTLTISSLQPEDFATYYCQQRSFYPPTFGGGTKVEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
SEQ ID NO: 74
Chain 3: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSGGGGSGIPPKIECLPIDISIDEGKVLTVASAFTGE
PTPEVTWSTGGRKIHSQEQGRFHIENTDDSTTLTIKDVQKQDGGLYTLTLRNEFG
SDSATVNIHIRSIDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
 
GKEYKCKVSNKALPAPIEKTISKAKGQPREPQVCTLPPSRDELTKNQVSLSCAVK
GFYPSDIAVEWESNGQPENNYKTTPPVLDSDGSFFLVSKLTVDKSRWQQGNVFS
CSVMHEALHNHYTQKSLSLSPGK
SEQ ID NO: 172
Chain 4:

DIQLTQSPSSLSASVGDRVTITCRASQDISSALVWYQQKPGKAPKLLIYDASSLES
GVPSRFSGSESGTDFTLTISSLQPEDFATYYCQQFNSYPLTFGGGTKVEIKGGGGS
SGAPRFLTRPKASVVSVGKDATLSCQIVGNPFPQVSWEKDKQPVTAGVRFRLAQ
DGDLYRLKILDLQLSDSGQYVCRARNAHGEAFACLGLQVDAEA
SEQ ID NO: 173

Sequences of M4H1L1-ZalH4:
Chain 1: SEQ ID NO: 171
Chain 2: SEQ ID NO: 74
Chain 3: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGVTMV
RGVMKDYFDYWGQGTLVTVSSGGGGSGIPPKIECLPIDISIDEGKVLTVASAFTG
EPTPEVTWSTGGRKIHSQEQGRFHIENTDDSTTLTIKDVQKQDGGLYTLTLRNEF
GSDSATVNIHIRSIDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVF
SCSVMHEALHNHYTQKSLSLSPGK
SEQ ID NO: 174
Chain 4: SEQ ID NO: 173

Sequences of M4H1L1-ZalH8:
Chain 1: SEQ ID NO: 171
Chain 2: SEQ ID NO: 74
Chain 3: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGLTMV
RGVMKDYFDYWGQGTLVTVSSGGGGSGIPPKIECLPIDISIDEGKVLTVASAFTG
EPTPEVTWSTGGRKIHSQEQGRFHIENTDDSTTLTIKDVQKQDGGLYTLTLRNEF
GSDSATVNIHIRSIDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVCTLPPSRDELTKNQVSLSCAV
 
KGFYPSDIAVEWESNGQPENNYKTTPPVLDSDGSFFLVSKLTVDKSRWQQGNVF
SCSVMHEALHNHYTQKSLSLSPGK
SEQ ID NO: 175
> Chain 4: SEQ ID NO: 173

Sequences of M4H1L1-ZalH10:
Chain 1: SEQ ID NO: 171
Chain 2: SEQ ID NO: 74
Chain 3: 
QVQLVESGGGVVQPGRSLRLSCAASGFTFSSYGMHWVRQAPGKGLEWVAVIW
DDGSYKYYGDSVKGRFTISRDNSKNTLYLQMNSLRAEDTAVYYCARDGATMV
RGVMKDYFDYWGQGTLVTVSSGGGGSGIPPKIECLPIDISIDEGKVLTVASAFTG
EPTPEVTWSTGGRKIHSQEQGRFHIENTDDSTTLTIKDVQKQDGGLYTLTLRNEF
GSDSATVNIHIRSIDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVCTLPPSRDELTKNQVSLSCAV
KGFYPSDIAVEWESNGQPENNYKTTPPVLDSDGSFFLVSKLTVDKSRWQQGNVF
SCSVMHEALHNHYTQKSLSLSPGK
SEQ ID NO: 176
> Chain 4: SEQ ID NO: 173

Sequences of M4H1L1-ZalH13:
Chain 1: SEQ ID NO: 171
Chain 2: SEQ ID NO: 74
Chain 3: 
QVQLVESGGGVVQPGRSLRLSCAASGFTFSSYAMHWVRQAPGKGLEWVAVIW
DDGSYKYYGDSVKGRFTISRDNSKNTLYLQMNSLRAEDTAVYYCARDGITMVR
GVMKDYFDYWGQGTLVTVSSGGGGSGIPPKIECLPIDISIDEGKVLTVASAFTGE
PTPEVTWSTGGRKIHSQEQGRFHIENTDDSTTLTIKDVQKQDGGLYTLTLRNEFG
SDSATVNIHIRSIDKTHTCPPCPAPELLGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVCTLPPSRDELTKNQVSLSCAVK
GFYPSDIAVEWESNGQPENNYKTTPPVLDSDGSFFLVSKLTVDKSRWQQGNVFS
CSVMHEALHNHYTQKSLSLSPGK
SEQ ID NO: 177
> Chain 4: SEQ ID NO: 173
Note: In the above antibody sequences, the antibody variable region sequences are underlined, the portions that are not underlined are the antibody constant region sequences, and the linker sequences are wavy-underlined.

The VH/VL sequences of the negative control antibody used in the present disclosure, **IgG1,** were from the patent US6114143A, and its heavy and light chain constant region sequences were SEQ ID NO: 69 and SEQ ID NO: 70, respectively. Its full-length sequence is shown below:
Heavy chain **of IgG1:** 
QVQLVQSGAEVKKPGASVKVSCKASGYTFTNSWIGWFRQAPGQGLEWIGDIYP
GGGYTNYNEIFKGKATMTADTSTNTAYMELSSLRSEDTAVYYCSRGIPGYAMDY
WGQGTLVTVSSASTKGPSVFPLAPSSKSTSGGTAALGCLVKDYFPEPVTVSWNSGAL
TSGVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK
SEQ ID NO: 184
Light chain of **IgG1:** 
DIQMTQSPSSLSASVGDRVTMSCKSSQSLLNSGDQKNYLTWYQQKPGKAPKLLI
YWASTGESGVPSRFSGSGSGTDFTFTISSLQPEDIATYYCQNDYSYPWTFGQGTK
VEIKRTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGEC
SEQ ID NO: 185
Note: In the sequence, the variable regions are underlined, and the constant regions are italicized.

### Example 8: Bispecific Antibody M4H1L1-ZalH4-LALA

Bispecific antibody M4H1L1-ZalH4-LALA was obtained by introducing mutations L238A and L239A into chain 1 of bispecific antibody M4H1L1-ZalH4 and mutations L242A and L243A into chain 3 and keeping the sequences of chains 2 and 4 unchanged. M4H1L1-ZalH4-LALA contained four chains, and their specific sequences are shown below:
Sequences of M4H1L1-ZalH4-LALA:
Chain 1: 
EVQLVQSGAEVKKPGASVKVSCKASGYTFTTYGVPWVRQAPGQGLEWMGDIY
PRSGNTYYNEKFKGRVTMTADKSTDTVYMELSSLRSEDTAVYYCAREDYDNYP
YALDYWGQGTTVTVSSASTKGPSVFPLAPSSKSTSGGTAALGCLVKDYFPEPVT
 
VSWNSGALTSGVHTFPAVLQSSGLYSLSSVVTVPSSSLGTQTYICNVNHKPSNTK
VDKKVEPKSCDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQDWLN
GKEYKCKVSNKALPAPIEKTISKAKGQPREPQVYTLPPCRDELTKNQVSLWCLV
KGFYPSDIAVEWESNGQPENNYKTTPPVLDSDGSFFLYSKLTVDKSRWQQGNVF
SCSVMHEALHNHYTQKSLSLSPGK
SEQ ID NO: 178
Chain 2: SEQ ID NO: 74
Chain 3: 
QVQLVESGGGVVQPGRSLRLSCAASGFTFSTYGMHWVRQAPGKGLEWVAVIW
DDGSYKYYGDSVKGRFTISRDNSKNTLYLQMNSLRAEDTAVYYCARDGVTMV
RGVMKDYFDYWGQGTLVTVSSGGGGSGIPPKIECLPIDISIDEGKVLTVASAFTG
EPTPEVTWSTGGRKIHSQEQGRFHIENTDDSTTLTIKDVQKQDGGLYTLTLRNEF
GSDSATVNIHIRSIDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQDW
LNGKEYKCKVSNKALPAPIEKTISKAKGQPREPQVCTLPPSRDELTKNQVSLSCA
VKGFYPSDIAVEWESNGQPENNYKTTPPVLDSDGSFFLVSKLTVDKSRWQQGN
VFSCSVMHEALHNHYTQKSLSLSPGK
SEQ ID NO: 179
Chain 4: SEQ ID NO: 173
> Fc (knob)': 
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPCRDELTKNQVSLWCLVKGFYPSD
IAVEWESNGQPENNYKTTPPVLDSDGSFFLYSKLTVDKSRWQQGNVFSCSVMH
EALHNHYTQKSLSLSPGK
SEQ ID NO: 182
> Fc (hole)': 
DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVCTLPPSRDELTKNQVSLSCAVKGFYPSDIAVEWE
SNGQPENNYKTTPPVLDSDGSFFLVSKLTVDKSRWQQGNVFSCSVMHEALHNH
YTQKSLSLSPGK
SEQ ID NO: 183
Note: In the above antibody sequences, the antibody variable region sequences are underlined, the portions that are not underlined are the antibody constant region sequences, the linker sequences are wavy-underlined, and the letters in bold type are mutated amino acids.

### Example 9: Conjugation of EGFR-MUC1 Bispecific Antibodies to Toxin M M4H1L1-ZalH'-M

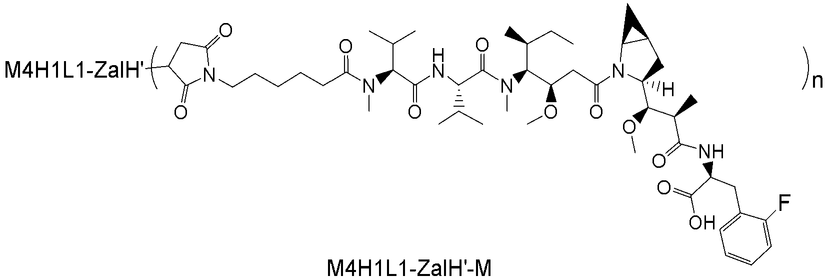

A prepared aqueous solution of tris(2-carboxyethyl)phosphine hydrochloride (TCEP.HCl) (10 mM, 6.3 µL, 63 nmol) was added to antibody **M4H1L1-ZalH'** in an aqueous PBS buffer solution (a 0.05 M aqueous PBS buffer solution (pH 6.3); 10.0 mg/mL, 0.38 mL, 25.7 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL. Compound **M** (0.245 mg, 257 nmol) was dissolved in 24 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **M4H1L1-ZalH'-M** in the histidine-acetic acid buffer (0.3 mg/mL, 10.2 mL). The product was then refrigerated at 4 °C. The average DAR value was calculated by MS: n = 3.87.

### M4H1L1-ZalH4-M

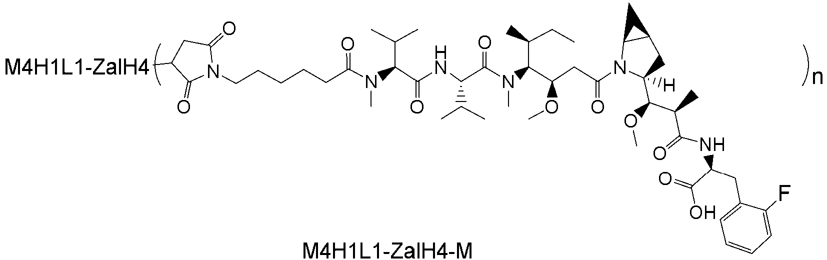

A prepared aqueous solution of tris(2-carboxyethyl)phosphine hydrochloride (TCEP.HCl) (10 mM, 8.4 µL, 84 nmol) was added to antibody **M4H1L1-ZalH4** in an aqueous PBS buffer solution (a 0.05 M aqueous PBS buffer solution (pH 6.3); 10.0 mg/mL, 0.5 mL, 33.8 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **M** (0.32 mg, 338 nmol) was dissolved in 32 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **M4H1L1-ZalH4-M** in the histidine-acetic acid buffer (0.42 mg/mL, 10.7 mL). The product was then refrigerated at 4 °C. The average DAR value was calculated by MS: n = 4.8.

### M4H1L1-ZalH8-M

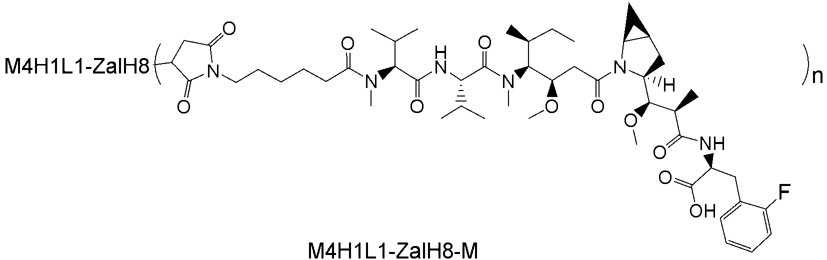

A prepared aqueous solution of tris(2-carboxyethyl)phosphine hydrochloride (TCEP.HCl) (10 mM, 8.25 µL, 82.5 nmol) was added to antibody **M4H1L1-ZalH8** in an aqueous PBS buffer solution (a 0.05 M aqueous PBS buffer solution (pH 6.3); 10.0 mg/mL, 0.5 mL, 33.8 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **M** (0.32 mg, 338 nmol) was dissolved in 32 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **M4H1L1-ZalH8-M** in the histidine-acetic acid buffer (0.39 mg/mL, 10.7 mL). The product was then refrigerated at 4 °C. The average DAR value was calculated by MS: n = 4.13.

### M4H1L1-ZalH10-M

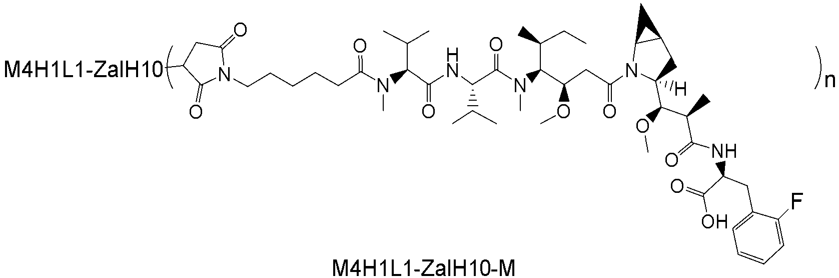

A prepared aqueous solution of tris(2-carboxyethyl)phosphine hydrochloride (TCEP.HCl) (10 mM, 4.57 µL, 45.7 nmol) was added to antibody **M4H1L1-ZalH10** in an aqueous PBS buffer solution (a 0.05 M aqueous PBS buffer solution (pH 6.3); 10.0 mg/mL, 0.276 mL, 18.6 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **M** (0.18 mg, 186 nmol) was dissolved in 18 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give the title product **M4H1L1-ZalH10-M** in the histidine-acetic acid buffer (0.22 mg/mL, 10.2 mL). The product was then refrigerated at 4 °C. The average DAR value was calculated by MS: n = 4.33.

### M4H1L1-ZalH13-M

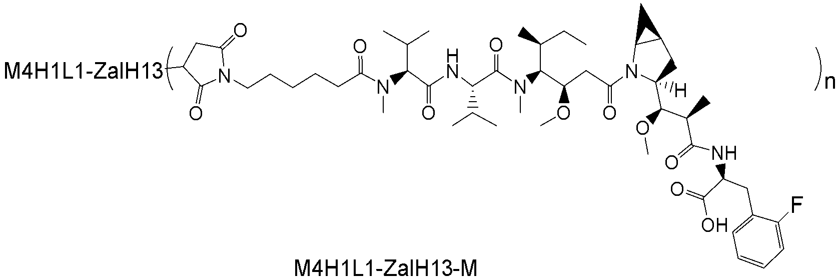

A prepared aqueous solution of tris(2-carboxyethyl)phosphine hydrochloride (TCEP.HCl) (10 mM, 7.85 µL, 78.5 nmol) was added to antibody **M4H1L1-ZalH13** in an aqueous PBS buffer solution (a 0.05 M aqueous PBS buffer solution (pH 6.3); 10.0 mg/mL, 0.47 mL, 32 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **M** (0.306 mg, 320 nmol) was dissolved in 31 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **M4H1L1-ZalH13-M** in the histidine-acetic acid buffer (0.42 mg/mL, 10.2 mL). The product was then refrigerated at 4 °C. The average DAR value was calculated by MS: n = 4.3.

### IgG1-M

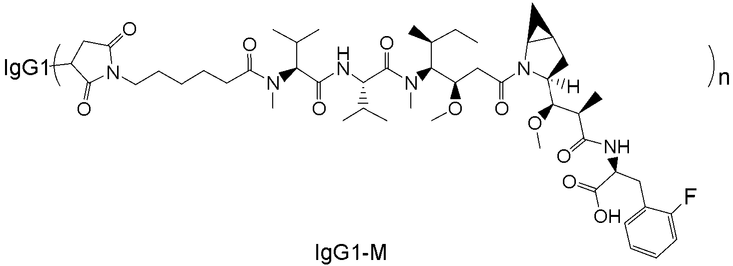

A prepared aqueous solution of tris(2-carboxyethyl)phosphine hydrochloride (TCEP.HCl) (10 mM, 8.11 µL, 81.1 nmol) was added to antibody **IgG1** in an aqueous PBS buffer solution (a 0.05 M aqueous PBS buffer solution (pH 6.3); 10.0 mg/mL, 0.5 mL, 33.8 nmol) at 37 °C. The mixture was shaken on a water bath shaker at 37 °C for 3 h, and the reaction was stopped. The reaction mixture was cooled to 25 °C in a water bath.

Compound **M** (0.32 mg, 338 nmol) was dissolved in 32 µL of acetonitrile, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **IgG1-M** in the histidine-acetic acid buffer (0.48 mg/mL, 10.2 mL). The product was then refrigerated at 4 °C. The average DAR value was calculated by MS: n = 4.37.

### Example 10: Conjugation of Antibodies to Toxin 9-A

### 1. Conjugation of EGFR-MUC1 bispecific antibodies to toxin 9-A

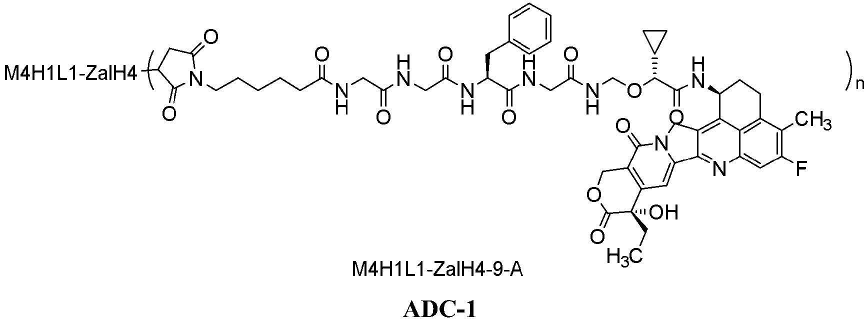

A prepared aqueous solution of tris(2-carboxyethyl)phosphine hydrochloride (TCEP.HCl) (10 mM, 851 µL, 8.51 µmol) was added to antibody **M4H1L1-ZalH4** in an aqueous PBS buffer solution (a 0.05 M aqueous PBS buffer solution (pH 6.3); 10.0 mg/mL, 30 mL, 2.027 µmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound 9-A (26.1 mg, 24.32 µmol) was dissolved in 1.5 mL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **ADC-1** in the histidine-acetic acid buffer (7.1 mg/mL, 39 mL). The product was then refrigerated at 4 °C. The average DAR value was calculated by HIC: n = 5.78. **ADC-2** A prepared aqueous solution of tris(2-carboxyethyl)phosphine hydrochloride (TCEP.HCl) (10 mM, 509.5 µL, 5.095 µmol) was added to antibody **M4H1L1-ZalH4** in an aqueous PBS buffer solution (a 0.05 M aqueous PBS buffer solution (pH 6.3); 10.0 mg/mL, 30.8 mL, 2.074 µmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **9-A** (22.3 mg, 20.74 µmol) was dissolved in 1.5 mL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **ADC-2** in the histidine-acetic acid buffer (5.8 mg/mL, 47.6 mL). The product was then refrigerated at 4 °C. The average DAR value was calculated by HIC: n = 4.5.

### ADC-3

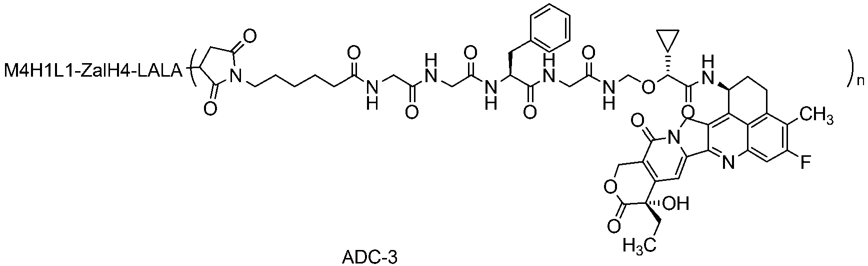

A prepared aqueous solution of tris(2-carboxyethyl)phosphine hydrochloride (TCEP.HCl) (10 mM, 29.3 µL, 293 nmol) was added to antibody **M4H1L1-ZalH4-LALA** in an aqueous PBS buffer solution (a 0.05 M aqueous PBS buffer solution (pH 6.3); 10.0 mg/mL, 1.11 mL, 75 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound 9-A (0.967 mg, 900 nmol) was dissolved in 55 µL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give ADC-3 in the histidine-acetic acid buffer (0.96 mg/mL, 10.5 mL). The product was then refrigerated at 4 °C. The average DAR value was calculated by MS: n = 5.51.

### 2. Conjugation of EGFR antibodies to toxin 9-A

### ZalH4-9-A

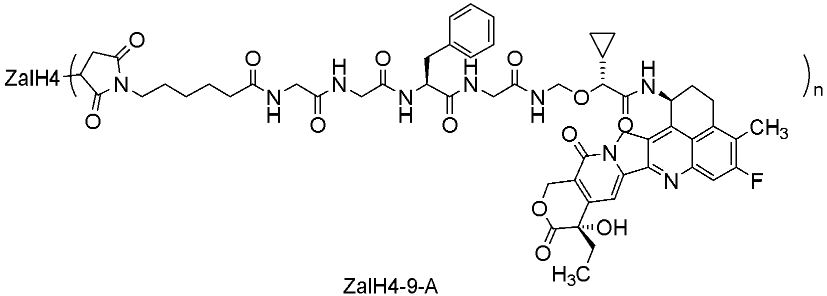

A prepared aqueous solution of tris(2-carboxyethyl)phosphine hydrochloride (TCEP.HCl) (10 mM, 35 µL, 350 nmol) was added to antibody **ZalH4** in an aqueous PBS buffer solution (a 0.05 M aqueous PBS buffer solution (pH 6.3); 10.0 mg/mL, 1.57 mL, 106 nmol) at 37 °C. The mixture was shaken on a water bath shaker at 37 °C for 3 h, and the reaction was stopped. The reaction mixture was cooled to 25 °C in a water bath, buffer-exchanged into a 30 mM histidine-acetic acid buffer (pH 5.0) using a Sephadex G25 gel column, and concentrated to 10 mg/mL.

Compound **9-A** (1.37 mg, 1.272 µmol) was dissolved in 80 µL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **ZalH4-9-A** in the histidine-acetic acid buffer (1.1 mg/mL, 12.8 mL). The product was then refrigerated at 4 °C. The average DAR value was calculated by RP-HPLC: n = 5.94.

### 3. Conjugation of MUC1 antibody to toxin 9-A

### M4H1L1-9-A

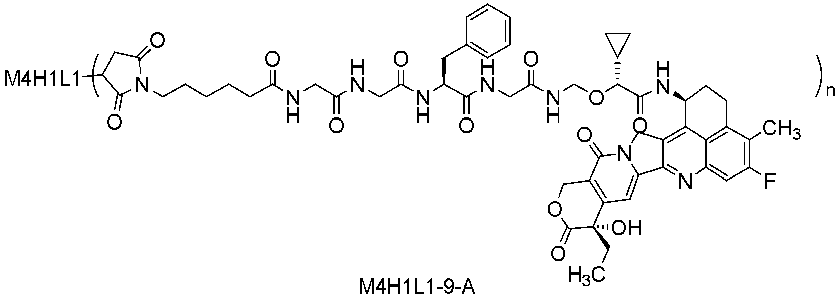

A prepared aqueous solution of tris(2-carboxyethyl)phosphine hydrochloride (TCEP.HCl) (10 mM, 31.2 µL, 312 nmol) was added to antibody **M4H1L1** in an aqueous PBS buffer solution (a 0.05 M aqueous PBS buffer solution (pH 6.3); 10.0 mg/mL, 1.4 mL, 94.6 nmol) at 37 °C. The mixture was shaken on a water bath shaker at 37 °C for 3 h, and the reaction was stopped. The reaction mixture was cooled to 25 °C in a water bath,
buffer-exchanged into a 30 mM histidine-acetic acid buffer (pH 5.0) using a Sephadex G25 gel column, and concentrated to 10 mg/mL.

Compound **9-A** (1.22 mg, 1.135 µmol) was dissolved in 70 µL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 30 mM histidine-acetic acid buffer (pH 5.0)) to give **M4H1L1-9-A** in the histidine-acetic acid buffer (0.94 mg/mL, 12.5 mL). The product was then refrigerated at 4 °C. The average value was calculated by RP-HPLC: n = 6.6.

### 4. Conjugation of negative control antibody to toxin 9-A

### IgG1-9-A

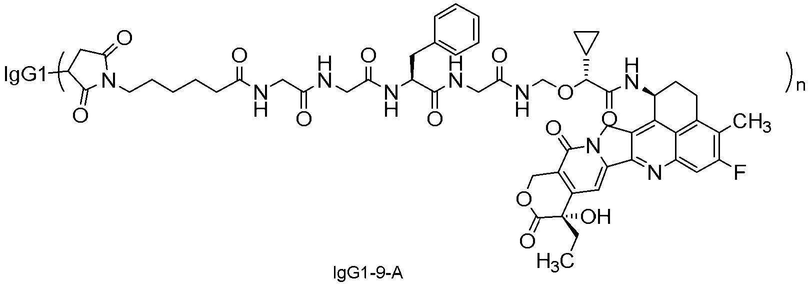

A prepared aqueous solution of tris(2-carboxyethyl)phosphine hydrochloride (TCEP.HCl) (10 mM, 81.6 µL, 816 nmol) was added to antibody **IgG1** in an aqueous PBS buffer solution (a 0.05 M aqueous PBS buffer solution (pH 6.3); 10.0 mg/mL, 3.9 mL, 263 nmol) at 37 °C. The mixture was shaken on a water bath shaker at 37 °C for 3 h, and the reaction was stopped. The reaction mixture was cooled to 25 °C in a water bath.

Compound **9-A** (3.4 mg, 3.162 µmol) was dissolved in 200 µL of dimethyl sulfoxide, and the resulting solution was added dropwise to the above reaction mixture. The mixture was shaken on a water bath shaker at 25 °C for 3 h, and the reaction was stopped. The reaction mixture was subjected to desalting purification using a Sephadex G25 gel column (elution phase: a 0.05 M aqueous PBS buffer solution (pH 6.3), containing 0.001 M EDTA) to give the title product **IgG-9-A** in the PBS buffer (2.42 mg/mL, 14.7 mL). The product was then refrigerated at 4 °C. The average value was calculated by RP-HPLC: n = 6.15.

### Test Example 1: FACS-Based Determination of Affinity of Chimeric Antibodies

HCC827-human-MUC1-C cells (in-house constructed overexpression stably transfected cell strain), CHOK1-cyno-MUC1-C cells (in-house constructed overexpression stably transfected cell strain), and T47D cells (human ductal breast cancer cells) were separately digested and added to a 96-well plate at 1E5 cells/well. After the plate was centrifuged at 300 g for 5 min, the supernatant was discarded, and the cells were washed once with a PBS buffer containing 2% FBS. The antibodies were 5-fold diluted from a starting concentration of 20 µg/mL using a PBS buffer containing 2% FBS to obtain a total of 8 concentration gradients. The antibody samples were added at 100 µL/well, and the cells were resuspended and incubated at 4 °C for 1 h. After the plate was centrifuged at 300 g for 5 min, the supernatant was discarded, and the cells were washed twice with a PBS buffer containing 2% FBS. A secondary antibody (Alexa Fluor^{®} 488 goat anti-human IgG (H+L) 1:1000, Invitrogen, A11013) was added, and the plate was incubated at 4 °C for 40 min. After the plate was centrifuged at 300 g for 5 min, the supernatant was discarded, and the cells were washed twice with a PBS buffer containing 2% FBS. The cells were resuspended in 100 µL of a PBS buffer containing 2% FBS and analyzed using an instrument. The FACS results are shown below.

**Table 17. The experimental results of the FACS-based determination of the affinity of chimeric antibodies for cells**

| Antibody | CHOK1-cyno-MUC1-C | | HCC827-human-MUC1-C | | T47D | |
|---|---|---|---|---|---|---|
| | Emax | EC₅₀ | Emax | EC₅₀ | Emax | EC₅₀ |
| ChiF4-18 | 79783 | 1.944 | 20887 | 0.9456 | 56332 | 3.139 |
| ChiF4-1 | 41700 | 1.016 | 21455 | 0.4288 | 78020 | 0.4632 |
| ChiM4 | 79481 | 5.484 | 13223 | 2.564 | 15536 | 3.075 |
| ChiM6 | 70875 | 5.369 | 14833 | 2.584 | 21775 | 1.519 |

The results show that the chimeric antibodies of the present disclosure exhibited relatively strong affinity for the stably transfected cell strains or the tumor cell strain.

### Test Example 2: Test on Internalization of Anti-MUC1-C Chimeric Antibodies by Tumor Cells

### I. Objective

The objective of this experiment was to determine the endocytic activity of MUC1 chimeric monoclonal antibody drugs on tumor cells. The cells were treated *in vitro* with the MUC1 chimeric monoclonal antibody drugs, at different concentrations. After 3 days of culture, the proliferation of the tumor cells was measured using a CTG (CellTiter-Glo^{®} Luminescent Cell Viability Assay, Promega, Cat. No. G7573) reagent, and the endocytic activity of the antibodies was evaluated based on their IC₅₀ values.

### II. Method

A test method of inhibiting the *in vitro* proliferation of T47D cells was used below to exemplify the method of assessing the endocytic activity of the MUC1 chimeric monoclonal antibody drugs of the present disclosure on tumor cells. This method also applies to, without limitation, the assessment of their activity in inhibiting the *in vitro* proliferation of other tumor cells.
1. Cell culture: T47D cells were cultured in a 1640 culture medium (GE, Cat. No. SH30024.01) containing 10% FBS and 2 µg/mL human insulin (Yeasen, Cat. No. 40112ES60).
2. Cell preparation: T47D cells in the logarithmic growth phase were collected, washed once with PBS (phosphate-buffered saline, Shanghai BasalMedia Technologies Co., Ltd.), and then digested with 2-3 mL of trypsin (0.25% trypsin-EDTA (1×), Gibco, Life Technologies) for 2-3 min. After the cells were fully digested, 10-15 mL of the cell culture medium was added to wash off the digested cells. The mixture was centrifuged at 1000 rpm for 5 min, and the supernatant was discarded. Subsequently, 10-20 mL of a cell culture medium containing 20% ultra-low IgG fetal bovine serum (Biosun, Cat. No. BS-0007-500) was added to resuspend the cells, resulting in a single-cell suspension.
3. Cell plating: The T47D single-cell suspension was well mixed, and its viable cell density was adjusted to 4 × 10⁴ cells/mL using the cell culture medium. The cell suspension with the adjusted density was well mixed and added to a 96-well cell culture plate at 50 µL/well. Only 100 µL of the culture medium was added to each peripheral well of the 96-well plate. The plate was incubated in an incubator for 24 h (37 °C, 5% CO₂).
4. Preparation of MUC1 chimeric monoclonal antibody drugs: A × DT3C (formed by the fusion of Fragment A of diphtheria toxin and the 3C fragment of group G streptococcus; at a molar concentration that was 6 times the molar concentration of the antibody) solution was prepared using a serum-free RPMI 1640 culture medium. A 4× antibody solution was prepared using the same culture medium. The DT3C solution and the antibody solution were well mixed at a 1:1 volume ratio, and the mixture was left to stand at room temperature for 30 min. Then, the mixture was serially diluted.
5. Sample addition: Each antibody dilution was added to cells at a 1:1 ratio, i.e., 50 µL/well. Each sample was added in duplicate. The culture plate was incubated in an incubator for 3 days (37 °C, 5% CO₂).
6. Color development: The 96-well cell culture plate was taken out, and 50 µL of CTG solution was added to each well. The plate was then incubated at room temperature for 10 min.
7. Plate reading: The 96-well cell culture plate was taken out and placed in a microplate reader (PE, Envision), and the chemiluminescence was measured using the microplate reader.

### III. Data analysis

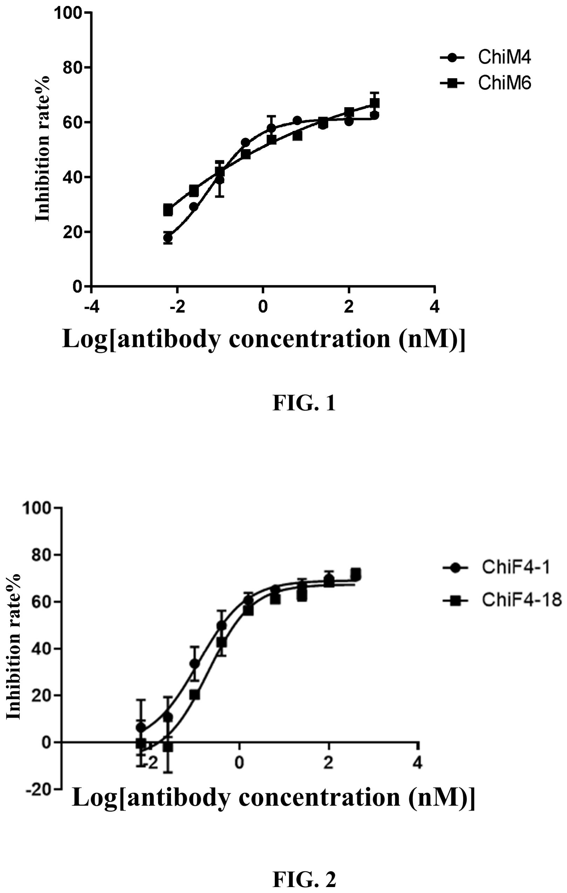

The data were processed and analyzed using Microsoft Excel and Graphpad Prism 5. The results of this example are shown in the tables below and FIGs. 1 and 2.

**Table 18-1. The in vitro internalization experiment of MUC1-C chimeric antibodies on T47D cells**

| Antibody | ChiM4 | ChiM6 |
|---|---|---|
| IC₅₀ (nM) | 0.2102 | 0.4510 |
| Imax (%) | 48.76 | 49.59 |

**Table 18-2. The in vitro internalization experiment of MUC1-C chimeric antibodies on T47D cells**

| Antibody | ChiF4-1 | ChiF4-18 |
|---|---|---|
| IC₅₀ (nM) | 0.1328 | 0.2040 |
| Imax (%) | 70.94 | 72.02 |

The results show that the chimeric antibodies of the present disclosure exhibited relatively strong endocytic activity on the T47D tumor cell strain.

### Test Example 3: FACS Evaluation of Chimeric Antibodies and Humanized Antibodies

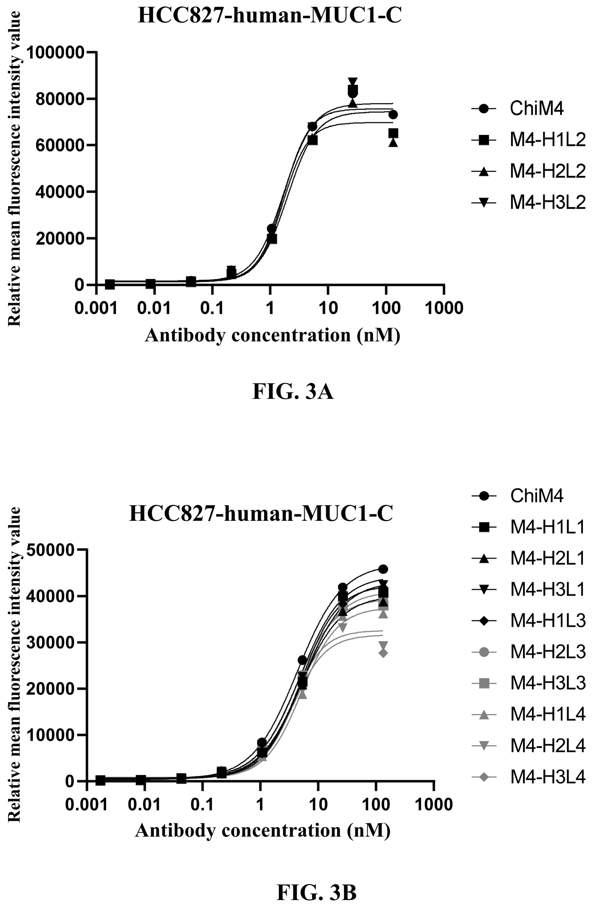

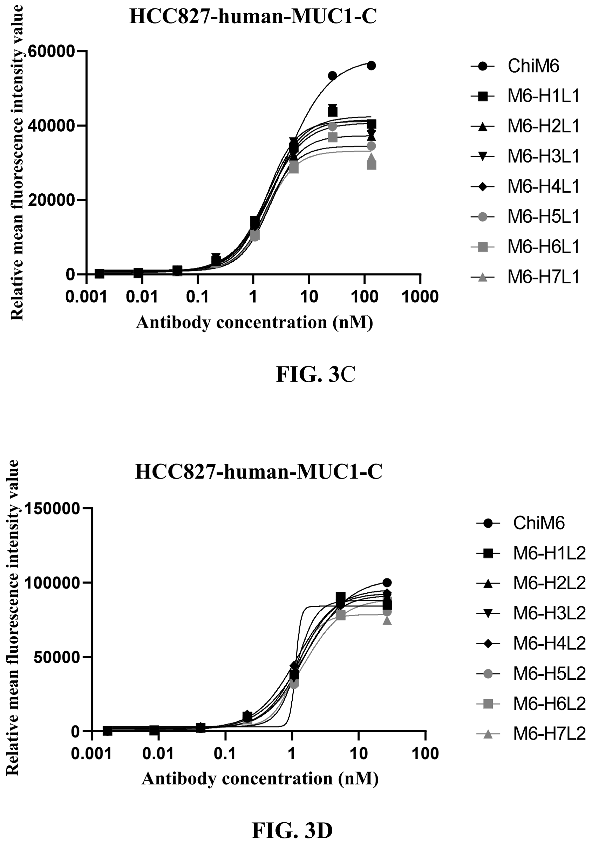

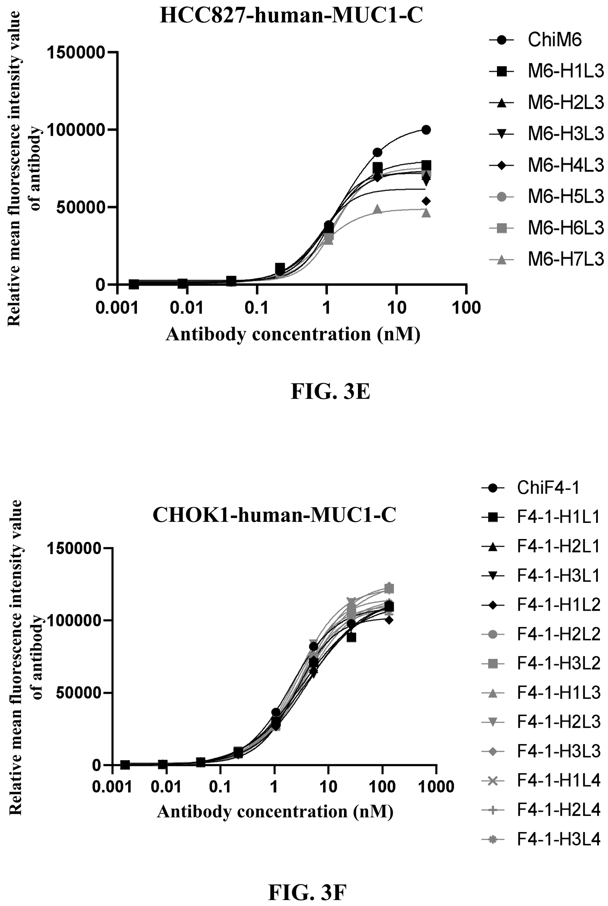

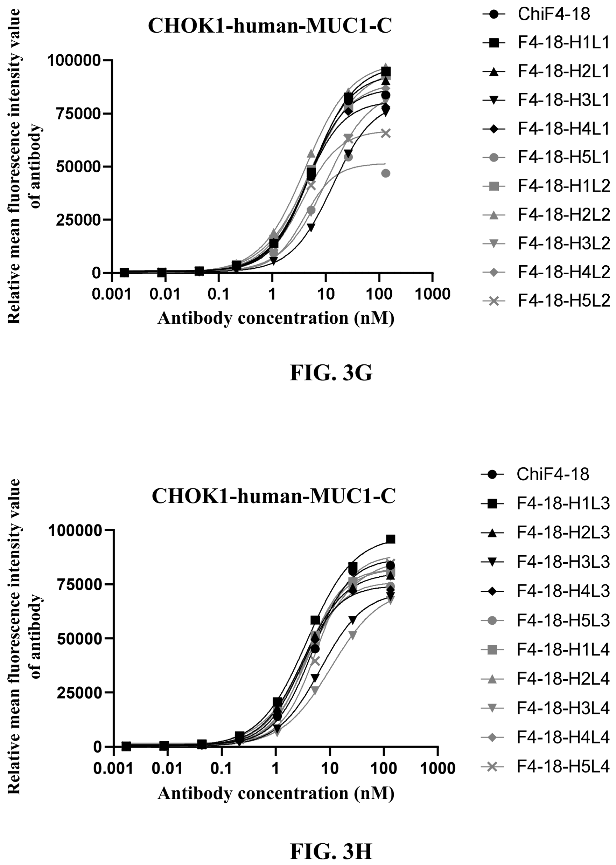

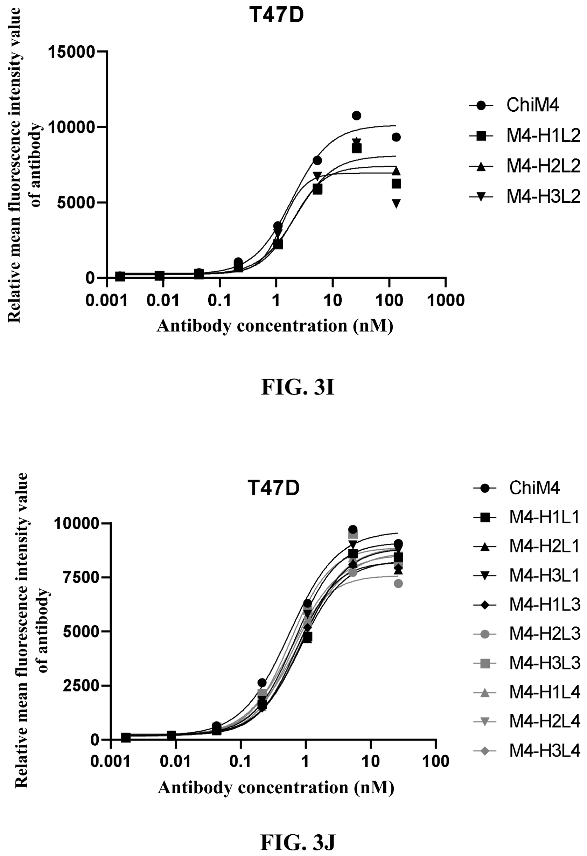

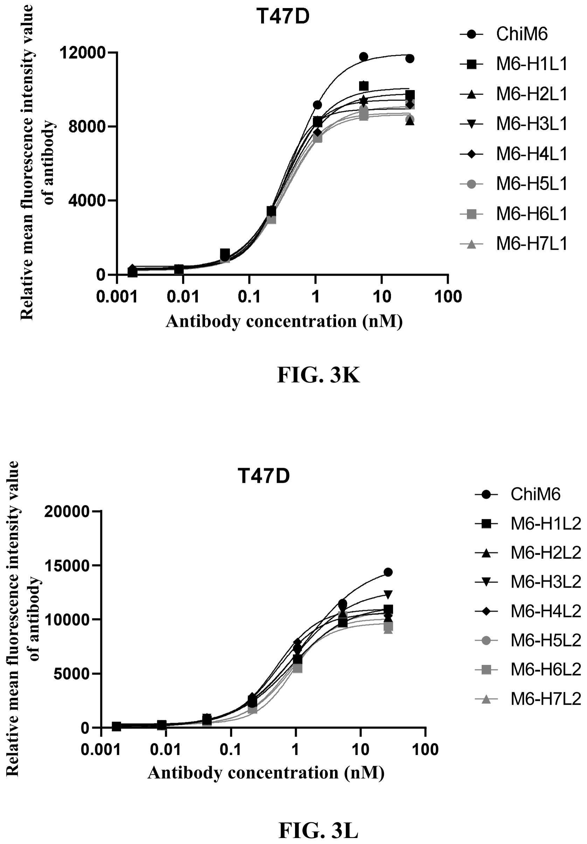

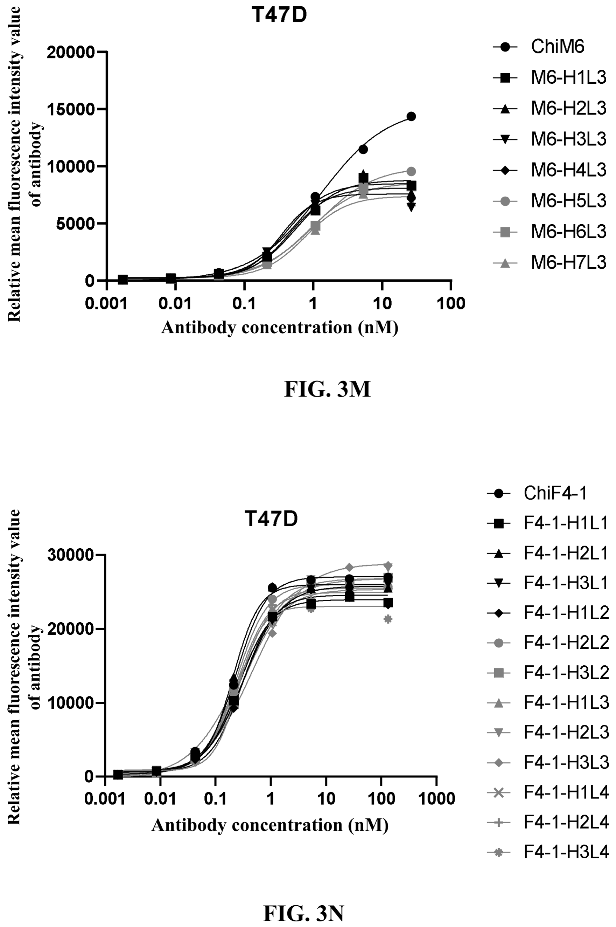

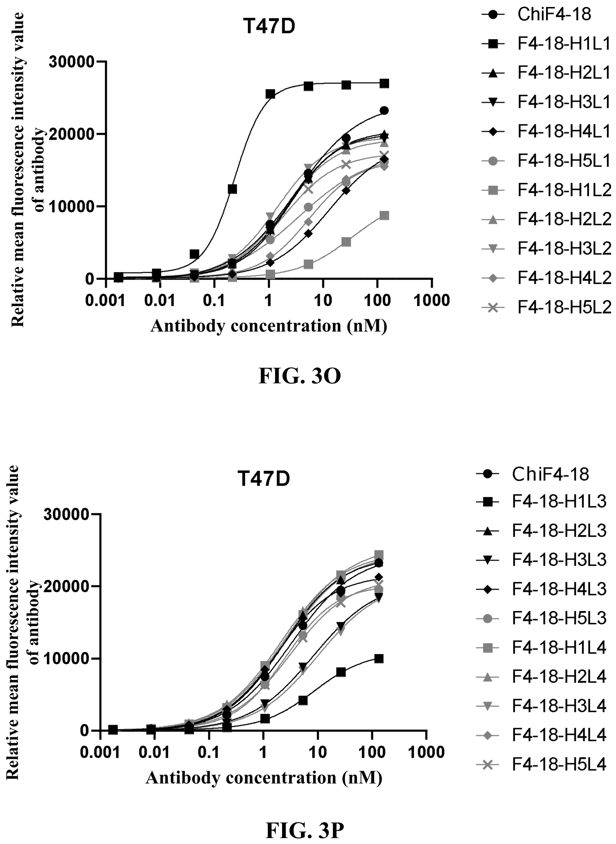

The experimental process was the same as that in Test Example 1 "FACS-Based Determination of Affinity of Chimeric Antibodies". In addition, CHOK1-human-MUC1-C cells (in-house constructed overexpression stably transfected cell strain) were used for evaluation. The experimental results are shown in the tables below and FIGs. 3A to 3P.

**Table 19-1. FACS results for a chimeric antibody and humanized antibodies**

| Antibody | T47D | | HCC827-human-MUC1-C | | CHOK1-cyno-MUC1-C |
|---|---|---|---|---|---|
| | Emax | EC₅₀ (nM) | Emax | EC₅₀ (nM) | Qualitative determination |
| Test 1 | | | | | |
| **ChiM4** | 10136 | 1.906 | 78048 | 1.735 | + |
| **M4-H1L2** | 7397 | 1.974 | 74421 | 1.986 | + |
| **M4-H2L2** | 8093 | 2.268 | 69779 | 1.674 | + |
| **M4-H3L2** | 6945 | 1.291 | 75715 | 1.721 | + |

| Test 2 | | | | | |
|---|---|---|---|---|---|
| **ChiM4** | 9648 | 0.5606 | 46984 | 4.355 | + |
| **M4-H1L1** | 8875 | 0.8962 | 42314 | 4.998 | + |
| **M4-H1L3** | 8218 | 0.7261 | 43129 | 5.425 | + |
| **M4-H1L4** | 8928 | 0.8794 | 37570 | 5.118 | + |
| **M4-H2L1** | 8292 | 0.8224 | 40122 | 4.824 | + |
| **M4-H2L3** | 7585 | 0.578 | 41020 | 4.865 | + |
| **M4-H2L4** | 8696 | 0.7860 | 31612 | 3.591 | + |
| **M4-H3L1** | 9122 | 0.6901 | 44391 | 4.831 | + |
| **M4-H3L3** | 8892 | 0.5826 | 39538 | 3.967 | + |
| **M4-H3L4** | 8579 | 0.6347 | 32581 | 3.499 | + |

| | | | | | |
|---|---|---|---|---|---|
| Note: "+" means that there was binding. | | | | | |

**Table 19-2. FACS results for a chimeric antibody and humanized antibodies**

| Antibody | T47D | | HCC827-human-MUC1-C | | CHOK1-cyno-MUC1-C |
|---|---|---|---|---|---|
| | Emax | EC₅₀ (nM) | Emax | EC₅₀ (nM) | Qualitative determination |
| Test 1 | | | | | |
| **ChiM6** | 11933 | 0.4394 | 58061 | 3.488 | + |
| **M6-H1L1** | 10087 | 0.3598 | 42476 | 1.809 | + |
| **M6-H2L1** | 8948 | 0.2867 | 40705 | 1.844 | + |
| **M6-H3L1** | 9452 | 0.3269 | 41160 | 1.745 | + |
| **M6-H4L1** | 9791 | 0.3652 | 41562 | 1.95 | + |
| **M6-H5L1** | 8737 | 0.3243 | 37259 | 2.064 | + |
| **M6-H6L1** | 9113 | 0.3733 | 33133 | 1.729 | + |
| **M6-H7L1** | 8640 | 0.3377 | 34493 | 1.744 | + |

| Test 2 | | | | | |
|---|---|---|---|---|---|
| **ChiM6** | 15177 | 1.282 | 103050 | 1.6 | + |
| **M6-H1L2** | 11328 | 0.8545 | 88089 | 1.192 | + |
| **M6-H2L2** | 10620 | 0.5207 | 92737 | 1.284 | + |
| **M6-H3L2** | 12749 | 0.9201 | 91603 | 1.398 | + |
| **M6-H4L2** | 10982 | 0.5081 | 95691 | 1.169 | + |
| **M6-H5L2** | 10812 | 0.9095 | 84135 | 1.129 | + |
| **M6-H6L2** | 10074 | 0.9024 | 89200 | 1.515 | + |
| **M6-H7L2** | 9690 | 0.7127 | 78429 | 1.135 | + |
| **M6-H1L3** | 8807 | 0.5553 | 79905 | 1.156 | + |
| **M6-H2L3** | 8504 | 0.4181 | 73548 | 0.9725 | + |
| **M6-H3L3** | 7597 | 0.3382 | 71949 | 1.044 | + |
| **M6-H4L3** | 8117 | 0.4626 | 61807 | 0.8193 | + |
| **M6-H5L3** | 9987 | 1.142 | 73039 | 1.283 | + |
| **M6-H6L3** | 8577 | 0.8491 | 75677 | 1.261 | + |
| **M6-H7L3** | 7403 | 0.7584 | 48940 | 0.803 | + |

| | | | | | |
|---|---|---|---|---|---|
| Note: "+" means that there was binding. | | | | | |

**Table 19-3. FACS results for a chimeric antibody and humanized antibodies**

| Antibody | T47D | | CHOK1-human-MUC1-C | | CHOK1-cyno-MUC1-C |
|---|---|---|---|---|---|
| | Emax | EC₅₀ (nM) | Emax | EC₅₀ (nM) | Qualitative determination |
| **ChiF4-1** | 27068 | 0.2367 | 109218 | 2.02 | + |
| **F4-1-H1L1** | 23966 | 0.2603 | 111735 | 3.243 | + |
| **F4-1-H2L1** | 26021 | 0.2064 | 117364 | 4.155 | + |
| **F4-1-H3L1** | 25786 | 0.3197 | 114681 | 4.252 | + |
| **F4-1-H1L2** | 24585 | 0.3059 | 102108 | 2.546 | + |
| **F4-1-H2L2** | 26042 | 0.2414 | 108994 | 2.62 | + |
| **F4-1-H3L2** | 26810 | 0.3394 | 126219 | 3.779 | + |
| **F4-1-H1L3** | 25336 | 0.3137 | 114624 | 3.694 | + |
| **F4-1-H2L3** | 26804 | 0.2638 | 122468 | 2.636 | + |
| **F4-1-H3L3** | 28853 | 0.4722 | 126077 | 3.663 | + |
| **F4-1-H1L4** | 25529 | 0.2696 | 114929 | 2.933 | + |
| **F4-1-H2L4** | 24955 | 0.2457 | 110389 | 2.401 | + |
| **F4-1-H3L4** | 23045 | 0.2554 | 115566 | 3.349 | + |

| | | | | | |
|---|---|---|---|---|---|
| Note: "+" means that there was binding. | | | | | |

**Table 19-4. FACS results for a chimeric antibody and humanized antibodies**

| Antibody | T47D | | CHOK1-human-MUC1-C | | CHOK1-cyno-MUC1-C |
|---|---|---|---|---|---|
| | Emax | EC₅₀ (nM) | Emax | EC₅₀ (nM) | Qualitative determination |
| **ChiF4-18** | 24374 | 3.259 | 86943 | 4.76 | + |
| **F4-18-H1L1** | 27068 | 0.2367 | 97944 | 5.676 | + |
| **F4-18-H2L1** | 20493 | 2.338 | 93664 | 5.248 | + |
| **F4-18-H3L1** | 20093 | 2.357 | 81304 | 13.45 | + |
| **F4-18-H4L1** | 18980 | 13.05 | 80876 | 4.172 | + |
| **F4-18-H5L1** | 17398 | 3.628 | 51523 | 3.998 | + |
| **F4-18-H1L2** | 11573 | 35.57 | 96745 | 5.494 | + |
| **F4-18-H2L2** | 19312 | 1.943 | 99093 | 4.21 | + |
| **F4-18-H3L2** | 19631 | 1.433 | 86369 | 10.43 | + |
| **F4-18-H4L2** | 16382 | 5.527 | 89606 | 4.59 | + |
| **F4-18-HSL2** | 17396 | 1.901 | 67145 | 3.652 | + |
| **F4-18-H1L3** | 10861 | 8.616 | 97369 | 3.766 | + |
| **F4-18-H2L3** | 24099 | 2.371 | 80408 | 3.256 | + |
| **F4-18-H3L3** | 20725 | 8.281 | 71880 | 6.819 | + |
| **F4-18-H4L3** | 21550 | 1.8 | 74415 | 3.045 | + |
| **F4-18-H5L3** | 20219 | 2.559 | 76219 | 3.537 | + |
| **F4-18-H1L4** | 25575 | 2.546 | 82544 | 3.427 | + |
| **F4-18-H2L4** | 24688 | 2.141 | 86942 | 4.304 | + |
| **F4-18-H3L4** | 20748 | 10.03 | 73373 | 10.64 | + |
| **F4-18-H4L4** | 24598 | 2.528 | 82159 | 3.766 | + |
| **F4-18-H5L4** | 21153 | 3.145 | 88664 | 5.822 | + |

| | | | | | |
|---|---|---|---|---|---|
| Note: "+" means that there was binding. | | | | | |

The results show that the chimeric antibodies and humanized antibodies of the present disclosure exhibited different levels of affinity for the stably transfected cell strains or the tumor cell strain, and that they all exhibited human-monkey cross-binding activity.

### Test Example 4: Test on Internalization of Anti-MUC1-C Humanized Antibodies by Tumor Cells

### I. Objective

The objective of this experiment was to determine the endocytic activity of MUC1 humanized monoclonal antibody drugs on tumor cells. The cells were treated *in vitro* with the MUC1 humanized monoclonal antibody drugs, at different concentrations. After 3 days of culture, the proliferation of the tumor cells was measured using a CTG (CellTiter-Glo^{®} Luminescent Cell Viability Assay, Promega, Cat. No. G7573) reagent, and the endocytic activity of the antibodies was evaluated based on their IC₅₀ values.

### II. Method

A test method of inhibiting the *in vitro* proliferation of T47D cells was used below to exemplify the method of assessing the endocytic activity of the MUC1 humanized monoclonal antibody drugs of the present disclosure on tumor cells. This method also applies to, without limitation, the assessment of their activity in inhibiting the *in vitro* proliferation of other tumor cells.
1. Cell culture: T47D cells were cultured in a 1640 culture medium (GE, Cat. No. SH30024.01) containing 10% FBS and 2 µg/mL human insulin (Yeasen, Cat. No. 40112ES60).
2. Cell preparation: T47D cells in the logarithmic growth phase were collected, washed once with PBS (phosphate-buffered saline, Shanghai BasalMedia Technologies Co., Ltd.), and then digested with 2-3 mL of trypsin (0.25% trypsin-EDTA (1×), Gibco, Life Technologies) for 2-3 min. After the cells were fully digested, 10-15 mL of the cell culture medium was added to wash off the digested cells. The mixture was centrifuged at 1000 rpm for 5 min, and the supernatant was discarded. Subsequently, 10-20 mL of a cell culture medium containing 20% ultra-low IgG fetal bovine serum (Biosun, Cat. No. BS-0007-500) was added to resuspend the cells, resulting in a single-cell suspension.
3. Cell plating: The T47D single-cell suspension was well mixed, and its viable cell density was adjusted to 4 × 10⁴ cells/mL using the cell culture medium. The cell suspension with the adjusted density was well mixed and added to a 96-well cell culture plate at 50 µL/well. Only 100 µL of the culture medium was added to each peripheral well of the 96-well plate. The plate was incubated in an incubator for 24 h (37 °C, 5% CO₂).
4. Preparation of MUC1 chimeric monoclonal antibody drugs: A 4× DT3C (formed by the fusion of Fragment A of diphtheria toxin and the 3C fragment of group G streptococcus; at a molar concentration that was 6 times the molar concentration of the antibody) solution was prepared using a serum-free RPMI 1640 culture medium. A 4× antibody solution was prepared using the same culture medium. The DT3C solution and the antibody solution were well mixed at a 1:1 volume ratio, and the mixture was left to stand at room temperature for 30 min. Then, the mixture was serially diluted.
5. Sample addition: Each antibody dilution was added to cells at a 1:1 ratio, i.e., 50 µL/well. Each sample was added in duplicate. The culture plate was incubated in an incubator for 3 days (37 °C, 5% CO₂).
6. Color development: The 96-well cell culture plate was taken out, and 50 µL of CTG solution was added to each well. The plate was then incubated at room temperature for 10 min.
7. Plate reading: The 96-well cell culture plate was taken out and placed in a microplate reader (PE, Envision), and the chemiluminescence was measured using the microplate reader.

### III. Data analysis

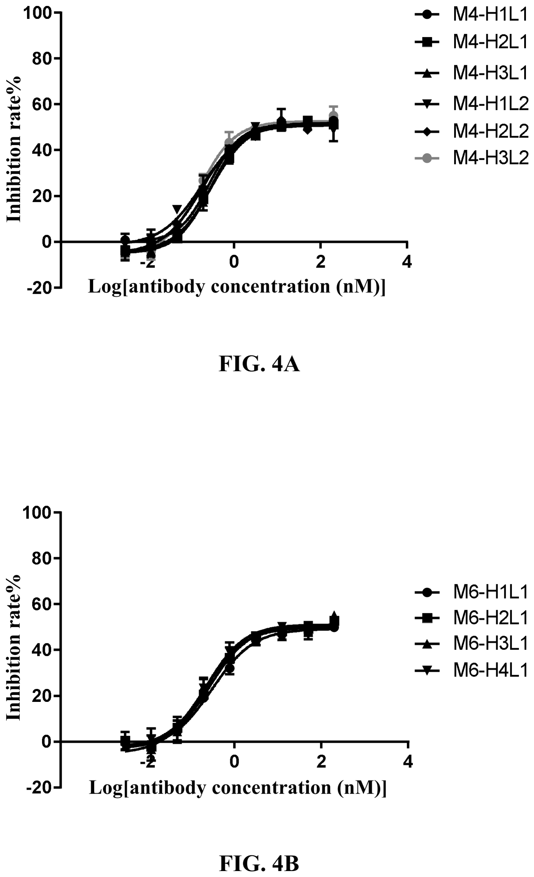

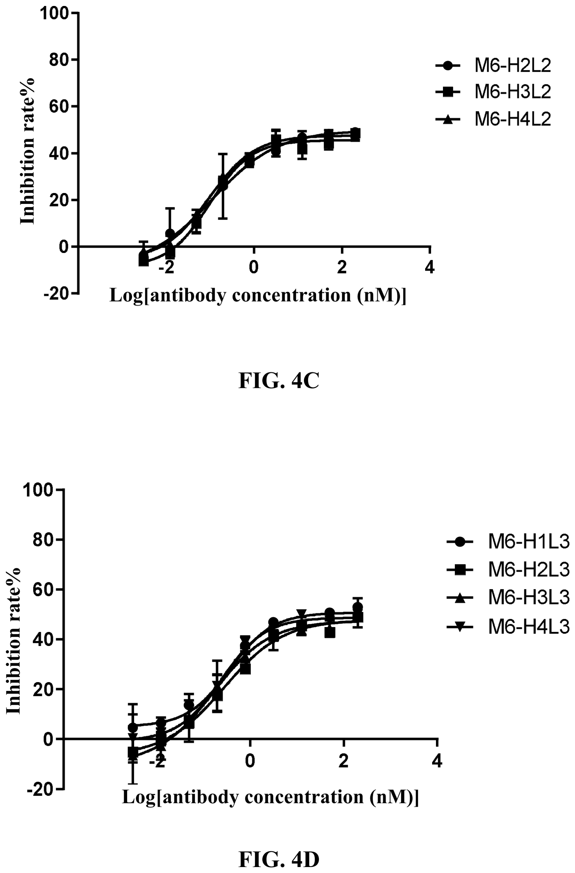

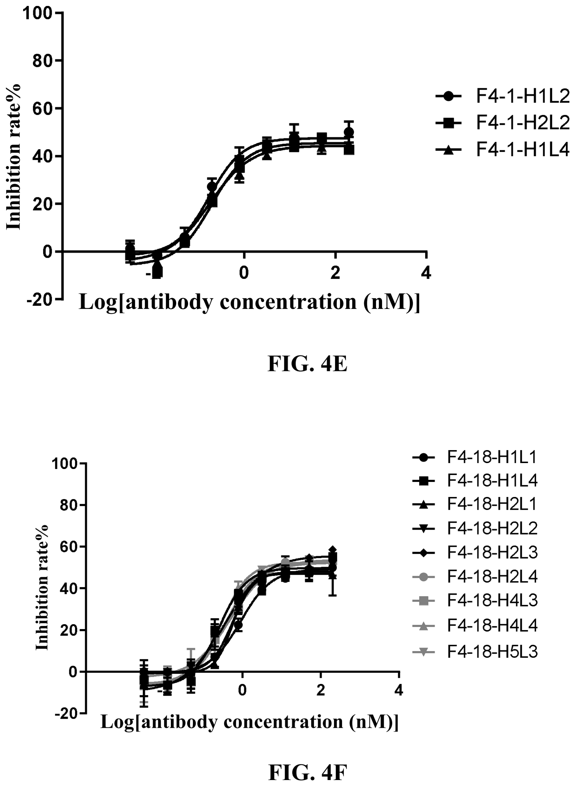

The data were processed and analyzed using Microsoft Excel and Graphpad Prism 5. The results of this example are shown in Table 20 and FIGs. 4A to 4F.

**Table 20. The in vitro internalization experiment of MUC1-C humanized antibodies on T47D cells**

| Antibody | IC₅₀ (nM) | Imax (%) |
|---|---|---|
| M4-H1L1 | 0.2719 | 52.85 |
| M4-H2L1 | 0.2985 | 52.86 |
| M4-H3L1 | 0.2770 | 51.27 |
| M4-H1L2 | 0.1951 | 51.07 |
| M4-H2L2 | 0.1777 | 53.25 |
| M4-H3L2 | 0.1591 | 55.01 |
| M6-H1L1 | 0.3121 | 49.91 |
| M6-H2L1 | 0.2745 | 52.80 |
| M6-H3L1 | 0.2030 | 55.12 |
| M6-H4L1 | 0.2295 | 52.46 |
| M6-H2L2 | 0.1614 | 49.13 |
| M6-H3L2 | 0.1025 | 48.57 |
| M6-H4L2 | 0.1034 | 47.82 |
| M6-H1L3 | 0.3694 | 52.99 |
| M6-H2L3 | 0.3206 | 48.87 |
| M6-H3L3 | 0.1835 | 49.65 |
| M6-H4L3 | 0.2446 | 50.03 |
| F4-1-H1L2 | 0.1688 | 50.11 |
| F4-1-H2L2 | 0.1913 | 47.71 |
| F4-1-H1L4 | 0.1716 | 45.20 |
| F4-18-H1L1 | 0.9109 | 50.02 |
| F4-18-H2L1 | 0.6158 | 50.57 |
| F4-18-H2L2 | 0.5971 | 51.34 |
| F4-18-H2L3 | 0.3549 | 58.62 |
| F4-18-H4L3 | 0.5052 | 53.80 |
| F4-18-H5L3 | 0.3697 | 53.04 |
| F4-18-H1L4 | 0.2607 | 55.16 |
| F4-18-H2L4 | 0.366 | 53.88 |
| F4-18-H4L4 | 0.2829 | 56.13 |

The results show that the humanized antibodies of the present disclosure exhibited relatively strong endocytic activity on the T47D tumor cell strain.

### Test Example 5: Determination of Affinity of Anti-MUC1-C Antibodies Using Biacore

1. Instrument: Biacore 8K, Cytiva
2. Material: Protein A biosensor chip (Cat. # 29127556, Cytiva)
3. Reagents:
   1) 10*HBS-EP+ buffer solution (pH 7.4) (Cat. # BR-1006-69, Cytiva)
   2) 10 mM glycine-HCl (pH 1.5) (Cat. # BR-1003-54, Cytiva)
   3) MUC1-C-L-His (prepared in-house)
   4) Test antibody samples (prepared in-house)

The affinity of the antibodies of the present disclosure for the human MUC1-C-L-His antigen was determined using a Biacore 8K instrument.

Method: An antibody molecule was affinity-captured by a Protein A biosensor chip, and the antigen molecule, at a certain concentration, was then allowed to flow through the surface of the chip continuously for 180 s, followed by 600 s of natural dissociation. The reaction signals were detected in real time by the Biacore 8K instrument to obtain an association and dissociation curve. After the dissociation in each experimental cycle was complete, the biosensor chip was regenerated with 10 mM glycine-HCl (pH 1.5). Data fitting was performed using the Kinetics 1:1 binding model. The experimental results are shown in Table 21.

**Table 21. The affinity of anti-MUC1 antibodies for the human MUC1 antigen**

| Antibody | ka (1/Ms) | kd (1/s) | KD (M) |
|---|---|---|---|
| F4-1-H1L2 | 3.05E+05 | 1.40E-03 | 4.59E-09 |
| F4-1-H2L2 | 2.88E+05 | 1.76E-03 | 6.11E-09 |
| F4-1-H1L4 | 3.02E+05 | 1.75E-03 | 5.78E-09 |
| F4-18-H1L1 | 1.33E+05 | 2.68E-03 | 2.01E-08 |
| F4-18-H2L1 | 1.23E+05 | 2.24E-03 | 1.82E-08 |
| F4-18-H2L2 | 1.31E+05 | 3.43E-03 | 2.61E-08 |
| F4-18-H2L3 | 1.70E+05 | 8.35E-04 | 4.90E-09 |
| F4-18-H4L3 | 2.00E+05 | 1.50E-03 | 7.49E-09 |
| F4-18-H5L3 | 1.50E+05 | 3.51E-03 | 2.35E-08 |
| F4-18-H1L4 | 1.84E+05 | 9.43E-04 | 5.11E-09 |
| F4-18-H2L4 | 1.59E+05 | 9.15E-04 | 5.76E-09 |
| F4-18-H4L4 | 1.88E+05 | 1.65E-03 | 8.77E-09 |
| M4-H1L1 | 9.50E+04 | 5.01E-04 | 5.27E-09 |
| M4-H1L2 | 8.74E+04 | 2.85E-04 | 3.26E-09 |
| M4-H1L3 | 1.20E+05 | 2.19E-04 | 1.83E-09 |
| M4-H2L2 | 8.58E+04 | 4.16E-04 | 4.85E-09 |
| M4-H2L3 | 1.12E+05 | 2.29E-04 | 2.05E-09 |
| M4-H3L2 | 9.05E+04 | 2.73E-04 | 3.02E-09 |
| M4-H3L3 | 1.10E+05 | 2.28E-04 | 2.07E-09 |
| M6-H1L1 | 3.09E+05 | 3.59E-03 | 1.16E-08 |
| M6-H1L3 | 2.47E+05 | 4.32E-03 | 1.74E-08 |
| M6-H2L1 | 2.29E+05 | 8.96E-04 | 3.92E-09 |
| M6-H2L3 | 2.16E+05 | 9.94E-04 | 4.61E-09 |
| M6-H3L1 | 2.06E+05 | 7.95E-04 | 3.85E-09 |
| M6-H3L3 | 1.77E+05 | 9.51E-04 | 5.38E-09 |
| M6-H4L1 | 2.60E+05 | 7.84E-04 | 3.02E-09 |
| M6-H4L3 | 2.46E+05 | 9.62E-04 | 3.91E-09 |

The results show that the anti-MUC1-C antibodies of the present disclosure exhibited high affinity for the human MUC1-C antigen.

### Test Example 6: Determination of Affinity of Anti-EGFR Antibodies Using Biacore

1. Instrument: Biacore 8K, Cytiva
2. Material: Protein A biosensor chip (Cat. # 29127556, Cytiva)
3. Reagents:
   1) 10*HBS-EP+ buffer solution (pH 7.4) (Cat. # BR-1006-69, Cytiva)
   2) 10 mM glycine-HCl (pH 1.5) (Cat. # BR-1003-54, Cytiva)
   3) EGFR-His (prepared in-house)
   4) Test antibody samples (prepared in-house)

The affinity of the antibodies of the present disclosure for the human EGFR-His antigen was determined using a Biacore 8K instrument.

Method: An antibody molecule was affinity-captured by a Protein A biosensor chip, and the antigen molecule, at a certain concentration, was then allowed to flow through the surface of the chip continuously for 180 s, followed by 600 s of natural dissociation. The reaction signals were detected in real time by the Biacore 8K instrument to obtain an association and dissociation curve. After the dissociation in each experimental cycle was complete, the biosensor chip was regenerated with 10 mM glycine-HCl (pH 1.5). Data fitting was performed using the Kinetics 1:1 binding model. The experimental results are shown in Table 22.

**Table 22. The affinity of anti-EGFR antibodies for the human EGFR antigen**

| Antibody | ka (1/Ms) | kd (1/s) | KD (M) |
|---|---|---|---|
| ZalH' | 6.59E+04 | 1.41E-03 | 2.14E-08 |
| ZalH1 | 6.84E+04 | 1.18E-03 | 1.72E-08 |
| ZalH2 | 8.74E+04 | 8.12E-03 | 9.29E-08 |
| ZalH3 | 8.05E+04 | 2.43E-02 | 3.02E-07 |
| ZalH4 | 4.74E+04 | 2.10E-03 | 4.43E-08 |
| ZalH5 | 1.29E+05 | 1.25E-02 | 9.64E-08 |
| ZalH7 | 7.95E+04 | 7.61E-03 | 9.57E-08 |
| ZalH8 | 7.65E+04 | 3.69E-02 | 4.83E-07 |
| ZalH9 | 4.54E+04 | 2.34E-03 | 5.16E-08 |
| ZalH10 | 1.31E+05 | 1.74E-02 | 1.33E-07 |
| ZalH12 | 1.20E+05 | 1.44E-02 | 1.20E-07 |
| ZalH13 | 9.55E+04 | 1.87E-04 | 1.96E-09 |
| ZalH14 | 1.31E+05 | 2.75E-02 | 2.10E-07 |

The results show that the mutants of the anti-EGFR antibodies of the present disclosure exhibited different levels of affinity for the human EGFR antigen.

### Test Example 7: Determination of Affinity of Anti-EGFR-MUC1 Bispecific Antibodies Using Biacore

1. Instrument: Biacore 8K, Cytiva
2. Material: Protein A biosensor chip (Cat. # 29127556, Cytiva)
3. Reagents:
   1) 10*HBS-EP+ buffer solution (pH 7.4) (Cat. # BR-1006-69, Cytiva)
   2) 10 mM glycine-HCl (pH 1.5) (Cat. # BR-1003-54, Cytiva)
   3) EGFR-His (prepared in-house)
   4) Test antibody samples (prepared in-house)

The affinity of the antibodies of the present disclosure for the human EGFR-His antigen was determined using a Biacore 8K instrument.

Method: An antibody molecule was affinity-captured by a Protein A biosensor chip, and the antigen molecule, at a certain concentration, was then allowed to flow through the surface of the chip continuously for 180 s, followed by 600 s of natural dissociation. The reaction signals were detected in real time by the Biacore 8K instrument to obtain an association and dissociation curve. After the dissociation in each experimental cycle was complete, the biosensor chip was regenerated with 10 mM glycine-HCl (pH 1.5). Data fitting was performed using the Kinetics 1:1 binding model. The results are shown in Table 23.

**Table 23. The affinity of anti-EGFR-MUC1 bispecific antibodies for the human EGFR antigen**

| Antibody | ka (1/Ms) | kd (1/s) | KD (M) |
|---|---|---|---|
| M4H1L1-ZalH' | 1.74E+05 | 7.20E-04 | 4.13E-09 |
| M4H1L1-ZalH4 | 1.39E+05 | 1.44E-03 | 1.04E-08 |
| M4H1L1-ZalH8 | 1.71E+05 | 2.75E-02 | 1.62E-07 |
| M4H1L1-ZalH10 | 2.01E+05 | 1.13E-02 | 5.64E-08 |
| M4H1L1-ZalH13 | 2.80E+05 | 4.57E-05 | 1.63E-10 |

The results show that the anti-EGFR-MUC1 bispecific antibodies of the present disclosure exhibited different levels of affinity for the human EGFR antigen.

### Test Example 8: FACS-Based Determination of Affinity of Anti-EGFR-MUC1 Bispecific Antibodies

HCC827 cells were digested and added to a 96-well plate at 1E5 cells/well. After the plate was centrifuged at 300 g for 5 min, the supernatant was discarded, and the cells were washed once with a PBS buffer containing 2% FBS. The antibodies were 5-fold diluted from a starting concentration of 20 µg/mL using a PBS buffer containing 2% FBS to obtain a total of 8 concentration gradients. The antibody samples were added at 100 µL/well, and the cells were resuspended and incubated at 4 °C for 1 h. After the plate was centrifuged at 300 g for 5 min, the supernatant was discarded, and the cells were washed twice with a PBS buffer containing 2% FBS. A secondary antibody (Alexa Fluor^{®} 488 goat anti-human IgG (H+L) 1:1000, Invitrogen, A11013) was added, and the plate was incubated at 4 °C for 40 min. After the plate was centrifuged at 300 g for 5 min, the supernatant was discarded, and the cells were washed twice with a PBS buffer containing 2% FBS. The cells were resuspended in 100 µL of a PBS buffer containing 2% FBS and analyzed using an instrument. The FACS results are shown in Table 24.

**Table 24. The experimental results of the FACS-based determination of the affinity of anti-EGFR-MUC1 bispecific antibodies for cells**

| Antibody | HCC827 | |
|---|---|---|
| | Emax | EC₅₀ |
| M4H1L1-ZalH' | 49519 | 1.239 |
| M4H1L1-ZalH4 | 42094 | 2.735 |
| M4H1L1-ZalH10 | 22354 | 11.40 |
| M4H1L1-ZalH13 | 57083 | 0.7439 |
| IgG1 | 87.1 | / |

| | | |
|---|---|---|
| Note: / means undetectable. | | |

The results show that the anti-EGFR-MUC1 bispecific antibodies of the present disclosure exhibited different levels of affinity for HCC827 tumor cells.

### Test Example 9: Determination of In Vitro Killing Activity of Anti-EGFR-MUC1 Bispecific Antibody ADCs

1. Objective: To determine the inhibitory effects of EGFR-MUC1 bispecific antibody ADCs on HCC827/HCC70 cell proliferation.
2. Materials:
   HCC827 cells: purchased from the Chinese Academy of Sciences, Cat. No. TCHul53 HCC70 cells: purchased from ATCC, Cat. No. CRL-2315
3. Method:
   1) Cell culture: HCC827 and HCC70 cells were cultured in 1640 culture media (MeilunBio, Cat. No. PWL015-L) containing 10% FBS.
   2) Cell preparation: HCC827 and HCC70 cells in the logarithmic growth phase were collected, washed once with PBS (phosphate-buffered saline, Shanghai BasalMedia Technologies Co., Ltd.), and then digested with 2-3 mL of trypsin (0.25% trypsion-EDTA (100 mL), MeilunBio, Cat. No. PWL060) for 2-3 min. After the cells were fully digested, 10-15 mL of the cell culture medium was added to wash off the digested cells. The mixtures were centrifuged at 1000 rpm for 5 min, and the supernatants were discarded. Subsequently, 10-20 mL of the cell culture medium was added to resuspend the cells, resulting in single-cell suspensions.
   3) Cell plating: The HCC827 and HCC70 single-cell suspensions were each well mixed, and their viable cell density was adjusted to 3.7 × 10³ cells/mL using the cell culture medium. The cell suspensions with the adjusted density were each well mixed and added to a 96-well cell culture plate at 135 µL/well. Only 150 µL of the culture medium was added to each peripheral well of the 96-well plate. The plate was incubated in an incubator for 24 h (37 °C, 5% CO₂).
   4) Preparation of ADC drugs. The ADC drugs were diluted with PBS (phosphate-buffered saline, Shanghai BasalMedia Technologies Co., Ltd.). The test ADCs were diluted to 10 µM and 5-fold diluted with PBS to obtain 9 concentration points. Finally, the 10th point was a drug-free well. Thus, the drugs were prepared in different concentration gradients.
   5) Sample addition. The test samples prepared at different concentrations (15 µL) were added to the culture plate, with each sample added in duplicate. The culture plate was incubated in an incubator for 6 days (37 °C, 5% CO₂). The final ADC drug concentration gradients in the cell plate were 1000, 200, 40, 8, 1.6, 0.32, 0.064, 0.0128, 0.00256, and 0 nM.
   6) Color development: The 96-well cell culture plate was taken out, and 75 µL of CTG solution was added to each well. The plate was then incubated at room temperature for 10 min.
   7) Plate reading: The 96-well cell culture plate was taken out and placed in a microplate reader (PE, Envision), and the chemiluminescence was measured using the microplate reader.

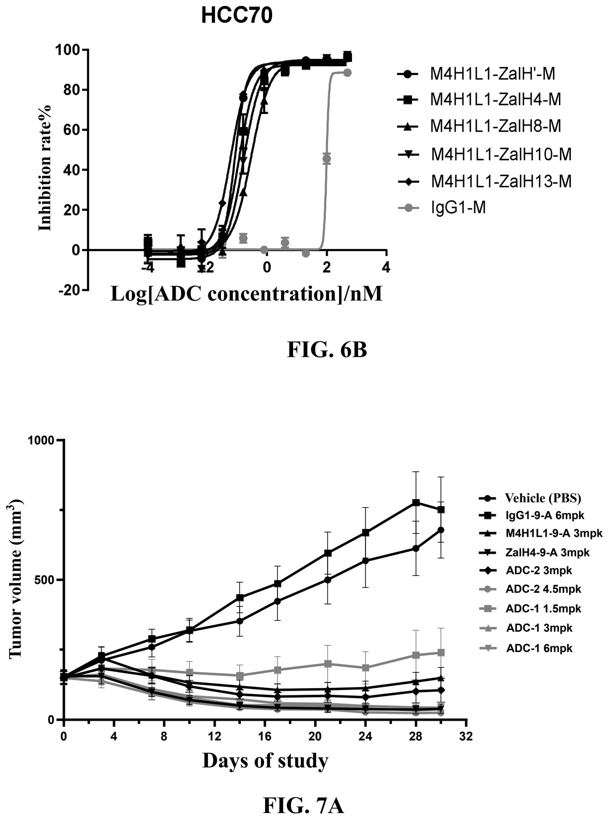

The results are shown in Table 25 and FIGs. 6A and 6B.

**Table 25. The in vitro killing activity of EGFR-MUC1 bispecific antibody ADCs**

| Sample | HCC827 | | HCC70 | |
|---|---|---|---|---|
| | IC₅₀ (nM) | Imax (%) | IC₅₀ (nM) | Imax (%) |
| M4H1L1-ZalH'-M | 0.09591 | 85.70 | 0.08193 | 96.15 |
| M4H1L1-ZalH4-M | 0.1239 | 85.16 | 0.1165 | 96.19 |
| M4H1L1-ZalH8-M | 0.4145 | 82.30 | 0.3075 | 96.02 |
| M4H1L1-ZalH10-M | 0.1644 | 83.78 | 0.1663 | 97.37 |
| M4H1L1-ZalH13-M | 0.08636 | 86.71 | 0.05833 | 95.66 |
| IgG1-M | 147.7 | 75.63 | 208.40 | 88.54 |

The results show that the anti-EGFR-MUC1 bispecific antibodies of the present disclosure exhibited different levels of killing activity against the HCC827 and HCC70 tumor cell lines.

### Test Example 10: Determination of Human-Monkey Cross-Activity of M4H1L1-ZalH4 Using Biacore

1. Instrument: Biacore T200, Cytiva
2. Material: Protein A biosensor chip (Cat. # 29127556, Cytiva)
3. Reagents:
   1) 10*HBS-EP+ buffer solution (pH 7.4) (Cat. # BR-1006-69, Cytiva)
   2) 10 mM glycine-HCl (pH 1.5) (Cat. # BR-1003-54, Cytiva)
   3) hEGFR-His and hMUC1-C-His (prepared in-house)
   4) Cynomolgus MUC1 his (Cat. # MU1-C52H5, Acro) and cynomolgus EGFR-his (Cat. # 90285-C08H, Sino Biological)
   5) Test antibody samples (prepared in-house)

The affinity of the antibodies of the present disclosure for the human and monkey EGFR and MUC1-C antigens was determined using a Biacore T200 instrument.

### Method:

An antibody molecule was affinity-captured by a Protein A biosensor chip, and an antigen molecule at a certain concentration was then allowed to flow through the surface of the chip continuously for 180 s, followed by 600 s of natural dissociation. The reaction signals were detected in real time by the Biacore T200 instrument to obtain an association and dissociation curve. After the dissociation in each experimental cycle was complete, the biosensor chip was regenerated with 10 mM glycine-HCl (pH 1.5). Data fitting was performed using the Kinetics 1:1 binding model. The results are shown in Table 26.

**Table 26. The affinity of antibodies for the human and monkey EGFR and MUC1-C antigens**

| Stationary phase | Mobile phase | KD (M) |
|---|---|---|
| ZalH4 | hEGFR-His | 3.30 E-08 |
| | Cyno EGFR-His | 4.77 E-07 |
| M4-H1L1 | hMUC1-C-His | 3.44 E-09 |
| | Cyno MUC1-His | 1.89 E-08 |
| M4H1L1-ZalH4 | hEGFR-His | 1.48 E-08 |
| | Cyno EGFR-His | 2.43 E-07 |
| | hMUC1-C-His | 3.31 E-09 |
| | Cyno MUC1-His | 1.78 E-08 |

The results show that the anti-EGFR-MUC1 bispecific antibody of the present disclosure, M4H1L1-ZalH4, exhibited good cross-binding activity to the human and monkey EGFR and MUC1-C antigens.

### Test Example 11: Determination of Ability of M4H1L1-ZalH4 to Bind to Two Targets Simultaneously Using Biacore

1. Instrument: Biacore 8K, Cytiva
2. Material: Protein A biosensor chip (Cat. # 29127556, Cytiva)
3. Reagents:
   1) 10*HBS-EP+ buffer solution (pH 7.4) (Cat. # BR-1006-69, Cytiva)
   2) 10 mM glycine-HCl (pH 1.5) (Cat. # BR-1003-54, Cytiva)
   3) hEGFR-His and hMUC1-C-His (prepared in-house)
   4) Test antibody samples (prepared in-house)

The affinity of the antibody of the present disclosure simultaneously binding to the human EGFR and MUC1-C antigens was determined using a Biacore 8K instrument.

Method: The antibody molecule was affinity-captured by a Protein A biosensor chip, and a first antigen molecule (Ag1; the blanks mean that there was no antigen but solvent) at high concentration was then allowed to flow through the surface of the chip continuously for 180 s. Then, a second antigen (dissolved in the first antigen or a blank solvent) was injected, followed by 360 s of dissociation in the high-concentration first antigen (or the blank solvent). The reaction signals were detected in real time by the Biacore 8K instrument to obtain an association and dissociation curve. After the dissociation in each experimental cycle was complete, the biosensor chip was regenerated with 10 mM glycine-HCl (pH 1.5). Data fitting was performed using the Kinetics 1:1 binding model. The results are shown in Table 27.

**Table 27. The affinity of the antibody simultaneously binding to the human EGFR and MUC1-C antigens**

| Stationary phase | Ag1 | Ag2 | KD (M) |
|---|---|---|---|
| M4H1L1-ZalH4 | | hEGFR-His | 1.80 E-08 |
| | | hMUC1-C-His | 2.56 E-09 |
| | hMUC1-C-His | hEGFR-His | 1.96 E-08 |
| | hEGFR-His | hMUC1-C-His | 3.52 E-09 |

| | | | |
|---|---|---|---|
| Note: Ag1 and Ag2 stand for the first antigen and the second antigen, respectively. | | | |

The results show that the affinity of the anti-EGFR-MUC1 bispecific antibody of the present disclosure, M4H1L1-ZalH4, binding to hMUC1-C-His and then to hEGFR-His (1.96E-08 M) was substantially consistent with the affinity of the bispecific antibody only binding to hEGFR-His (1.80E-08 M); similarly, the affinity of M4H1L1-ZalH4 binding to hEGFR-His and then to hMUC1-C-His (3.52E-09 M) was substantially consistent with the affinity of the bispecific antibody only binding to hMUC1-C-His (2.56E-09 M), indicating that EGFR-MUC1 bispecific antibody M4H1L1-ZalH4 has the ability to bind to the human EGFR and MUC1-C antigens simultaneously, and did not affect the binding to Ag2 after binding to Ag1.

### Test Example 12: FACS-Based Determination of Affinity of Monoclonal Antibodies, Bispecific Antibodies, and ADCs for Tumor Cells

The culture medium in the cell culture flask was discarded, and the cells were washed once with PBS and then digested with an appropriate amount of trypsin (Invitrogen, Cat. No. 25200072). HCC827, T47D, and HPAC cells were added to a 96-well plate at 100,000 cells/well. After the plate was centrifuged at 300 g for 5 min, the supernatant was discarded, and the cells were washed once with a PBS buffer containing 2% FBS. The antibodies were 5-fold diluted from a starting concentration of 20 µg/mL using a PBS buffer containing 2% FBS to obtain a total of 8 concentration gradients. The antibody samples were added at 100 µL/well, and the cells were resuspended and incubated at 4 °C for 1 h. After the plate was centrifuged at 300 g for 5 min, the supernatant was discarded, and the cells were washed twice with a PBS buffer containing 2% FBS. A secondary antibody (Alexa Fluor^{®} 488 goat anti-human IgG (H+L) 1:1000, Invitrogen, A11013) was added, and the plate was incubated at 4 °C for 40 min. After the plate was centrifuged at 300 g for 5 min, the supernatant was discarded, and the cells were washed twice with a PBS buffer containing 2% FBS. The cells were resuspended in 100 µL of a PBS buffer containing 2% FBS and analyzed using an instrument. The FACS results are shown in Table 28.

**Table 28. The experimental results of the FACS-based determination of the affinity of monoclonal antibodies, bispecific antibodies, and ADCs for tumor cells**

| Sample | HCC827 | | T47D | | HPAC | |
|---|---|---|---|---|---|---|
| | Emax | EC₅₀ | Emax | EC₅₀ | Emax | EC₅₀ |
| ADC-1 | 42094 | 2.43 | 8236 | 75.01 | 40146 | 13.05 |
| M4H1L1-ZalH4 | 43987 | 2.82 | 8549 | 74.80 | 39472 | 10.81 |
| M4H1L1-9-A | 3693 | 10.98 | 5619 | 8.13 | 29206 | 8.27 |
| M4H1L1 | 3631 | 12.02 | 5251 | 8.52 | 22354 | 6.79 |
| ZalH4-9-A | 31678 | 0.93 | 1968 | 0.69 | 14397 | 0.90 |
| ZalH4 | 37646 | 0.91 | 2163 | 0.82 | 12963 | 0.70 |
| IgG1-9-A | 580 | / | 145 | / | 1315 | / |
| IgG1 | 262 | / | 119 | / | 127 | / |

| | | | | | | |
|---|---|---|---|---|---|---|
| Note: / means undetectable. | | | | | | |

The results show that the ADCs of the present disclosure were comparable to the corresponding naked antibodies in affinity for the tumor cell lines, indicating that the toxin conjugation did not affect the affinity of the antibodies for the tumor cells.

### Test Example 13: Determination of Endocytic Activity of M4H1L1-ZalH4 on Tumor Cells

1. Objective: The objective of this experiment was to assess the killing of cells by activated DT after the DT3C protein enters the cells, which indirectly reflects the endocytosis of EGFR-MUC1 bispecific antibodies. The endocytic activity of antibodies was evaluated based on their IC₅₀ and Emax.
2. Principle: DT3C is a recombinantly expressed fusion protein formed by fusing fragment A of diphtheria toxin (toxin moiety only) and fragment 3C of group G streptococcus (IgG binding moiety). The protein has high affinity for the Fc of an antibody. When the antibody is endocytosed by a cell, the protein enters the cell along with the antibody. Under the action of furin protease in the cell, the protein releases DT, which is toxic and can inhibit the activity of EF2-ADP ribosylation, thereby blocking the protein translation process and finally leading to the death of the cell. DT3C molecules outside the cell have no cell killing activity. The endocytic activity of the antibody can be evaluated based on cell killing.
3. Materials:
   HCC827 cells: purchased from the Chinese Academy of Sciences, Cat. No. TCHul53
   HPAC cells: purchased from Nanjing Cobioer, Cat. No. CBP60539
   T47D cells: purchased from ATCC, Cat. No. HTB-133
4. Method:
   1) A cell suspension was prepared using a fresh cell culture medium containing 20% FBS (low IgG) and added to a 96-well cell culture plate at 2000 cells/well/50 µL, and the plate was incubated at 37 °C with 5% carbon dioxide for 16 h.
   2) A 4× DT3C solution (with a molar concentration that was 6 times the molar concentration of the antibody) was prepared using a serum-free culture medium. A 4× antibody solution was prepared using the serum-free culture medium. 80 µL of the DT3C solution and 80 µL of the antibody solution were well mixed at a 1:1 volume ratio, and the mixture was left to stand at room temperature for 30 min.
   3) The mixture was serially diluted 4-fold using the serum-free culture medium to obtain a total of 9 gradients, and the 10th point was the culture medium alone. The final concentrations were 200, 50, 12.5, 3.125, 0.7812, 0.1953, 0.0488, 0.0122, 0.00305, and 0 nM.
   4) 50 µL of an antibody dilution was added to 50 µL of cells, and the mixture was incubated in an incubator at 37 °C with 5% carbon dioxide for 72 h.
   5) The plate was taken out, and 50 µL of CTG was added to each well. The plate was then incubated in a dark place at room temperature for 10 min, and the chemiluminescence was measured using PHERAstar.
   6) Data processing.

The results are shown in Table 29.

**Table 29. The experimental results of the determination of the endocytic activity of antibodies on tumor cells**

| Antibody | HCC827 | | HPAC | | T47D | |
|---|---|---|---|---|---|---|
| | IC₅₀/nM | Imax% | IC₅₀/nM | Imax% | IC₅₀/nM | Imax% |
| ZalH4 | 1.486 | 83.88 | 0.1035 | 24.44 | 133.1 | 25.34 |
| M4H1L1 | 5.161 | 35.81 | 1.47 | 37.84 | 0.3325 | 45.30 |
| M4H1L1-ZalH4 | 0.3877 | 87.26 | 0.5919 | 54.18 | 0.3698 | 50.70 |
| IgG1 | N.A | 19.37 | N.A | 9.39 | N.A | 8.17 |

The experimental results show that the anti-EGFR-MUC1 bispecific antibody of the present disclosure, M4H1L1-ZalH4, exhibited relatively good endocytic activity on the HCC827, T47D, and HPAC tumor cell lines; in the HCC827 tumor cell line, in which EGFR expression levels dominate, its endocytic activity was comparable to that of the EGFR monoclonal antibody ZalH4; in the T47D tumor cell line, in which MUC1 expression levels dominate, its endocytic activity was comparable to that of the MUC1 monoclonal antibody M4H1L1; in the HPAC tumor cell line, in which both EGFR and MUC1 expression levels are high, its endocytic activity was superior to that of the EGFR monoclonal antibody ZalH4 and the MUC1 monoclonal antibody M4H1L1.

### Test Example 14: Determination of Killing Activity of EGFR-MUC1 Bispecific Antibody ADCs Against Tumor Cells

1. Objective: To determine the inhibitory effects of EGFR-MUC1 bispecific antibody ADCs on HCC827/T47D/HPAC cell proliferation.
2. Materials:
   HCC827 cells: purchased from the Chinese Academy of Sciences, Cat. No. TCHul53
   HPAC cells: purchased from Nanjing Cobioer, Cat. No. CBP60539
   T47D cells: purchased from ATCC, Cat. No. HTB-133
3. Method:
   1) Cell culture: HCC827 cells, HPAC cells, and T47D cells were cultured in a 1640 culture medium (MeilunBio, Cat. No. PWL015-L) containing 10% FBS, a DMEM/F12 (1:1) culture medium (MeilunBio, Cat. No. PWL005) containing 10% FBS, 0.002 mg/mL insulin, 0.005 mg/mL transferrin, and 40 ng/mL hydrocortisone, and a 1640 culture medium (MeilunBio, Cat. No. PWL015-L) containing 10% FBS and 10 µg/mL human insulin, respectively.
   2) Cell preparation: HCC827, HPAC, and T47D cells in the logarithmic growth phase were collected, washed once with PBS (phosphate-buffered saline, Shanghai BasalMedia Technologies Co., Ltd.), and then digested with 2-3 mL of trypsin (0.25% trypsin-disodium edetate (100 mL), MeilunBio) for 2-3 min. After the cells were fully digested, 10-15 mL of the cell culture medium was added to wash off the digested cells. The mixtures were centrifuged at 1000 rpm for 5 min, and the supernatants were discarded. Subsequently, 10-20 mL of the cell culture medium was added to resuspend the cells, resulting in single-cell suspensions.
   3) Cell plating: The HCC827, HPAC, and T47D single-cell suspensions were each well mixed, and their viable cell densities were adjusted to 3.7 × 10³ cells/mL, 3.7 × 10³ cells/mL, and 7.4 × 10³ cells/mL, respectively. The cell suspensions with the adjusted densities were each well mixed and added to a 96-well cell culture plate at 135 µL/well. Only 150 µL of the culture medium was added to each peripheral well of the 96-well plate. The plate was incubated in an incubator for 24 h (37 °C, 5% CO₂).
   4) Preparation of ADC drugs. The ADC drugs were diluted with PBS (phosphate-buffered saline, Shanghai BasalMedia Technologies Co., Ltd.). The test ADCs were diluted to 10 µM and 5-fold diluted with PBS to obtain 9 concentration points. Finally, the 10th point was a drug-free well. Thus, the drugs were prepared in different concentration gradients.
   5) Sample addition. The test samples prepared at different concentrations (15 µL) were added to the culture plate, with each sample added in duplicate. The culture plate was incubated in an incubator for 6 days (37 °C, 5% CO₂). The final ADC drug concentration gradients in the cell plate were 1000, 200, 40, 8, 1.6, 0.32, 0.064, 0.0128, 0.00256, and 0 nM.
   6) Color development: The 96-well cell culture plate was taken out, and 75 µL of CTG solution was added to each well. The plate was then incubated at room temperature for 10 min.
   7) Plate reading: The 96-well cell culture plate was taken out and placed in a microplate reader (PE, Envision), and the chemiluminescence was measured using the microplate reader.

The results are shown in Table 30.

**Table 30. The in vitro killing activity of EGFR-MUC1 bispecific antibody ADCs**

| Sample | HCC827 | | HPAC | | T47D | |
|---|---|---|---|---|---|---|
| | IC₅₀ (nM) | IMax% | IC₅₀ (nM) | IMax% | IC₅₀ (nM) | IMax% |
| ADC-1 | 0.5035 | 87.99 | 13.66 | 69.43 | 25.04 | 89.19 |
| ZalH4-9-A | 0.1585 | 88.04 | 204.4 | 60.81 | 102.2 | 85.53 |
| M4H1L1-9-A | 70.36 | 83.89 | 5.295 | 64.84 | 10.22 | 89.00 |
| IgG1-9-A | 163.1 | 84.25 | >1000 | 50.13 | 273.6 | 80.28 |

The results show that the anti-EGFR-MUC1 bispecific antibody ADC-1 of the present disclosure exhibited relatively good killing activity against the HCC827, T47D, and HPAC tumor cell lines; in the HCC827 tumor cell line, in which EGFR expression levels dominate, the killing activity of anti-EGFR-MUC1 bispecific antibody ADC-1 was comparable to that of the EGFR monoclonal antibody ADC ZalH4-9-A; in the T47D tumor cell line, in which MUC1 expression levels dominate, the killing activity of anti-EGFR-MUC1 bispecific antibody ADC-1 was comparable to that of the MUC1 monoclonal antibody ADC M4H1L1-9-A; in the HPAC tumor cell line, in which both EGFR and MUC1 expression levels are high, the killing activity of anti-EGFR-MUC1 bispecific antibody ADC-1 was comparable to that of the MUC1 monoclonal antibody ADC M4H1L1-9-A.

### Test Example 15: FACS-Based Determination of Affinity of EGFR-MUC1 Bispecific Antibodies for Tumor Cells

The culture medium in the cell culture flask was discarded, and the cells were washed once with PBS and then digested with an appropriate amount of trypsin (Invitrogen, Cat. No. 25200072). HCC827, T47D, and HPAC cells were added to a 96-well plate at 100,000 cells/well. After the plate was centrifuged at 300 g for 5 min, the supernatant was discarded, and the cells were washed once with a PBS buffer containing 2% FBS. The antibodies were 5-fold diluted from a starting concentration of 20 µg/mL using a PBS buffer containing 2% FBS to obtain a total of 8 concentration gradients. The antibody samples were added at 100 µL/well, and the cells were resuspended and incubated at 4 °C for 1 h. After the plate was centrifuged at 300 g for 5 min, the supernatant was discarded, and the cells were washed twice with a PBS buffer containing 2% FBS. A secondary antibody (Alexa Fluor^{®} 488 goat anti-human IgG (H+L) 1:1000, Invitrogen, A11013) was added, and the plate was incubated at 4 °C for 40 min. After the plate was centrifuged at 300 g for 5 min, the supernatant was discarded, and the cells were washed twice with a PBS buffer containing 2% FBS. The cells were resuspended in 100 µL of a PBS buffer containing 2% FBS and analyzed using an instrument. The FACS results are shown in Table 31.

**Table 31. The experimental results of the FACS-based determination of the affinity of anti-EGFR-MUC1 bispecific antibodies for tumor cells**

| Antibody | HCC827 | | T47D | | HPAC | |
|---|---|---|---|---|---|---|
| | Emax | EC₅₀ | Emax | EC₅₀ | Emax | EC₅₀ |
| M4H1L1-ZalH4-L ALA | 52987 | 2.909 | 8964 | 54.74 | 43249 | 10.84 |
| M4H1L1-ZalH4 | 43987 | 2.822 | 8549 | 74.80 | 39472 | 10.81 |

The results show that the anti-EGFR-MUC1 bispecific antibody of the present disclosure, M4H1L1-ZalH4-LALA, exhibited relatively good affinity for the HCC827, T47D, and HPAC tumor cell lines, and that its affinity was comparable to that of M4H1L1-ZalH4.

### Test Example 16: Determination of Endocytic Activity of EGFR-MUC1 Bispecific Antibodies on Tumor Cells

1. Objective: The objective of this experiment was to assess the killing of cells by activated DT after the DT3C protein enters the cells, which indirectly reflects the endocytosis of EGFR-MUC1 bispecific antibodies. The endocytic activity of antibodies was evaluated based on their IC₅₀ and Emax.
2. Principle: DT3C is a recombinantly expressed fusion protein formed by fusing fragment A of diphtheria toxin (toxin moiety only) and fragment 3C of group G streptococcus (IgG binding moiety). The protein has high affinity for the Fc of an antibody. When the antibody is endocytosed by a cell, the protein enters the cell along with the antibody. Under the action of furin protease in the cell, the protein releases DT, which is toxic and can inhibit the activity of EF2-ADP ribosylation, thereby blocking the protein translation process and finally leading to the death of the cell. DT3C molecules outside the cell have no cell killing activity. The endocytic activity of the antibody can be evaluated based on cell killing.
3. Materials:
   HCC827 cells: purchased from the Chinese Academy of Sciences, Cat. No. TCHul53
   HPAC cells: purchased from Nanjing Cobioer, Cat. No. CBP60539
   T47D cells: purchased from ATCC, Cat. No. HTB-133
4. Method:
   1) A cell suspension was prepared using a fresh cell culture medium containing 20% FBS (low IgG) and added to a 96-well cell culture plate at 2000 cells/well/50 µL, and the plate was incubated at 37 °C with 5% carbon dioxide for 16 h.
   2) A 4× DT3C solution (with a molar concentration that was 6 times the molar concentration of the antibody) was prepared using a serum-free culture medium. A 4× antibody solution was prepared using the serum-free culture medium. 80 µL of the DT3C solution and 80 µL of the antibody solution were well mixed at a 1:1 volume ratio, and the mixture was left to stand at room temperature for 30 min.
   3) The mixture was serially diluted 4-fold using the serum-free culture medium to obtain a total of 9 gradients, and the 10th point was the culture medium alone. The final concentrations were 200, 50, 12.5, 3.125, 0.7812, 0.1953, 0.0488, 0.0122, 0.00305, and 0 nM.
   4) 50 µL of an antibody dilution was added to 50 µL of cells, and the mixture was incubated in an incubator at 37 °C with 5% carbon dioxide for 72 h.
   5) The plate was taken out, and 50 µL of CTG was added to each well. The plate was then incubated in a dark place at room temperature for 10 min, and the chemiluminescence was measured using PHERAstar.
   6) Data processing.

The results are shown in Table 32.

**Table 32. The experimental results of the determination of the endocytic activity of antibodies on tumor cells**

| Antibody | HCC827 | | HPAC | | T47D | |
|---|---|---|---|---|---|---|
| | IC₅₀/nM | Imax% | IC₅₀/nM | Imax% | IC₅₀/nM | Imax% |
| M4H1L1-ZalH4-LALA | 0.4473 | 87.25 | 0.6315 | 52.02 | 0.7346 | 50.60 |
| M4H1L1-ZalH4 | 0.3877 | 87.26 | 0.5919 | 54.18 | 0.3698 | 50.70 |

The experimental results show that the anti-EGFR-MUC1 bispecific antibody of the present disclosure, M4H1L1-ZalH4-LALA, exhibited relatively good endocytic activity on the HCC827, T47D, and HPAC tumor cell lines, and that its endocytic activity was comparable to that of M4H1L1-ZalH4.

### Test Example 17: Determination of Killing Activity of EGFR-MUC1 Bispecific Antibody ADCs Against Tumor Cells

1. Objective: To determine the inhibitory effects of EGFR-MUC1 bispecific antibody ADCs on HCC827/T47D/HPAC cell proliferation.
2. Materials:
   HCC827 cells: purchased from the Chinese Academy of Sciences, Cat. No. TCHul53
   HPAC cells: purchased from Nanjing Cobioer, Cat. No. CBP60539
   T47D cells: purchased from ATCC, Cat. No. HTB-133
3. Method:
   1) Cell culture: HCC827 cells, HPAC cells, and T47D cells were cultured in a 1640 culture medium (MeilunBio, Cat. No. PWL015-L) containing 10% FBS, a DMEM/F12 (1:1) culture medium (MeilunBio, Cat. No. PWL005) containing 10% FBS, 0.002 mg/mL insulin, 0.005 mg/mL transferrin, and 40 ng/mL hydrocortisone, and a 1640 culture medium (MeilunBio, Cat. No. PWL015-L) containing 10% FBS and 10 µg/mL human insulin, respectively.
   2) Cell preparation: HCC827, HPAC, and T47D cells in the logarithmic growth phase were collected, washed once with PBS (phosphate-buffered saline, Shanghai BasalMedia Technologies Co., Ltd.), and then digested with 2-3 mL of trypsin (0.25% trypsin-disodium edetate (100 mL), MeilunBio) for 2-3 min. After the cells were fully digested, 10-15 mL of the cell culture medium was added to wash off the digested cells. The mixtures were centrifuged at 1000 rpm for 5 min, and the supernatants were discarded. Subsequently, 10-20 mL of the cell culture medium was added to resuspend the cells, resulting in single-cell suspensions.
   3) Cell plating: The HCC827, HPAC, and T47D single-cell suspensions were each well mixed, and their viable cell densities were adjusted to 3.7 × 10³ cells/mL, 3.7 × 10³ cells/mL, and 7.4 × 10³ cells/mL, respectively. The cell suspensions with the adjusted densities were each well mixed and added to a 96-well cell culture plate at 135 µL/well. Only 150 µL of the culture medium was added to each peripheral well of the 96-well plate. The plate was incubated in an incubator for 24 h (37 °C, 5% CO₂).
   4) Preparation of ADC drugs. The ADC drugs were diluted with PBS (phosphate-buffered saline, Shanghai BasalMedia Technologies Co., Ltd.). The test ADCs were diluted to 10 µM and 5-fold diluted with PBS to obtain 9 concentration points. Finally, the 10th point was a drug-free well. Thus, the drugs were prepared in different concentration gradients.
   5) Sample addition. The test samples prepared at different concentrations (15 µL) were added to the culture plate, with each sample added in duplicate. The culture plate was incubated in an incubator for 6 days (37 °C, 5% CO₂). The final ADC drug concentration gradients in the cell plate were 1000, 200, 40, 8, 1.6, 0.32, 0.064, 0.0128, 0.00256, and 0 nM.
   6) Color development: The 96-well cell culture plate was taken out, and 75 µL of CTG solution was added to each well. The plate was then incubated at room temperature for 10 min.
   7) Plate reading: The 96-well cell culture plate was taken out and placed in a microplate reader (PE, Envision), and the chemiluminescence was measured using the microplate reader.

The results are shown in Table 33.

**Table 33. The in vitro killing activity of EGFR-MUC1 bispecific antibody ADCs**

| Sample | HCC827 | | HPAC | | T47D | |
|---|---|---|---|---|---|---|
| | IC₅₀ (nM) | IMax% | IC₅₀ (nM) | IMax% | IC₅₀ (nM) | IMax% |
| ADC-1 | 1.03 | 86.05 | 15.41 | 69.70 | 21.24 | 89.41 |
| ADC-3 | 0.90 | 92.47 | 9.26 | 68.08 | 19.91 | 90.92 |

The results show that the anti-EGFR-MUC1 bispecific antibody ADC of the present disclosure, ADC-3, exhibited relatively good killing activity against the HCC827, T47D, and HPAC tumor cell lines, and that its killing activity was comparable to that of ADC-1.

### Test Example 18: Efficacy of ADCs in In Vivo HCC827 CDX Model

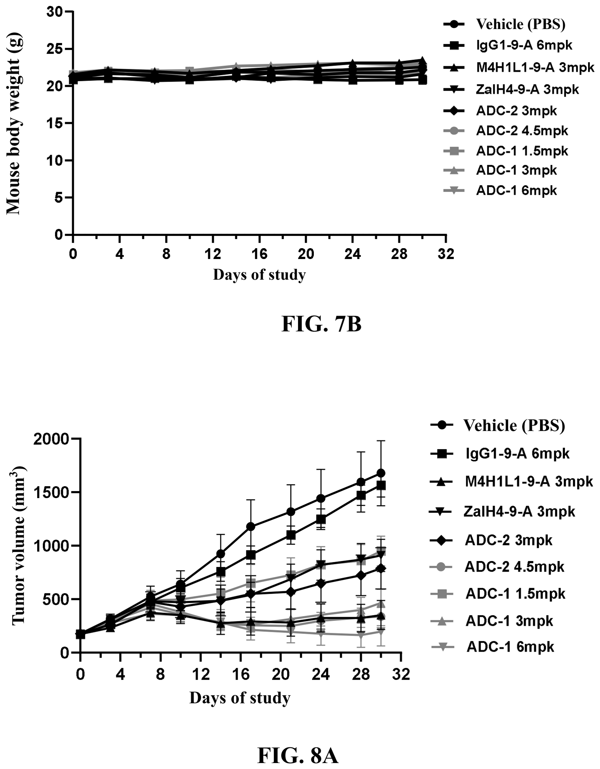

1. Objective: The objective of this experiment was to evaluate, after intraperitoneal administration, the *in vivo* efficacy and toxic and side effects of different doses of bispecific antibody ADCs and their corresponding monoclonal antibody ADCs in human non-small cell lung cancer HCC827 tumor-bearing mice.
2. Principle: In this experiment, an HCC827 tumor-bearing mouse model was constructed using Balb/c nude mice and intraperitoneally given the test molecules, pharmacodynamic evaluations of different molecules were conducted, and the bispecific antibody ADCs and monoclonal antibody ADCs were compared in terms of efficacy.
3. Animals: Balb/c nude mice, purchased from Beijing Vital River Laboratory Animal Technology Co., Ltd.
4. Instruments:
   CO2 incubator: HERACELL 150i GP INCUBATOR, Thermo Fisher Scientific, USA
   Biosafety cabinet: HFsafe-1800LC, Heal Force
   Inverted microscope: DMi1, LEICA
   Digital vernier caliper: 1195-200C, INSIZE
   Electronic balance: MP6001, Shanghai Sunny Hengping Instrument Co., Ltd.
   Centrifuge: L500-A, Hunan Xiangyi Laboratory Instrument Development Co., Ltd.
   Electric constant-temperature water bath: DK-S22, Shanghai Jinghong
   Electronic balance: ML204T-02, Mettler
   Microplate reader: Spark, TECAN
   Biochemical incubator: BSP-150, Shanghai Boxun Medical Biological Instrument Corp.
5. Steps: 100 µL of HCC827 human non-small cell lung cancer cells (5 × 10⁶ cells) was subcutaneously inoculated into the right flank of each of 120 female nude mice (purchased from Beijing Vital River Laboratory Animal Technology Co., Ltd.). After 25 days, their tumors grew to about 150.48 ± 21.32 mm3. Then, the mice that were too large in body weight or tumor size and those that were too small in body weight or tumor size were excluded, and the remaining mice were randomly divided into 9 groups of 10 according to their tumor volume (D0): a vehicle (PBS) control group, a negative control group (IgG1-9-A), an MUC1 monoclonal antibody ADC group (M4H1L1-9-A), an EGFR monoclonal antibody ADC group (ZalH4-9-A), EGFR-MUC1 bispecific antibody ADC-2 groups, and ADC-1 groups. The ADCs were intraperitoneally injected once on D1. The tumor volume and body weight of the animals were measured twice a week, and the measurements were recorded.
6. Data processing: The tumor volume of the animals in each group was expressed as mean ± standard error of the mean (Mean ± SEM) and plotted using Graphpad Prism 10 software. Statistical analysis was performed using two/one-way ANOVA, and the tumor growth inhibition rate was calculated using the formula: tumor proliferation rate (T/C %) = (T - T0 / C - C0) × 100%.
7. Experimental results: Thirty days after the administration, the tumor growth inhibition rate of the negative control group (6 mpk) was -13.71% (ns vs. blank control (vehicle)); the tumor growth inhibition rate of the MUC1 monoclonal antibody ADC (3 mpk) was 100.08% (P < 0.0001 vs. blank control); the tumor growth inhibition rate of the EGFR monoclonal antibody ADC (3 mpk) was 122.10% (P < 0.0001 vs. blank control); the tumor growth inhibition rates of EGFR-MUC1 bispecific antibody ADC-2 (3 mpk/4.5 mpk) were 108.35% (P < 0.0001 vs. blank control) and 123.58% (P < 0.0001 vs. blank control), respectively; the tumor growth inhibition rates of EGFR-MUC1 bispecific antibody ADC-1 (1.5 mpk/3 mpk/6 mpk) were 82.71% (P < 0.0001 vs. blank control), 120.82% (P < 0.0001 vs. blank control), and 121.83% (P < 0.0001 vs. blank control), respectively. The results are shown in Table 34 and FIGs. 7A to 7B.

**Table 34. The efficacy of ADCs in the in vivo HCC827 CDX model (analysis using day-30 data)**

| Sample | Frequency of administration | Route of administration | Mean tumor volume (mm³) | | Tumor growth inhibition rate (%) D30 | P (2-way ANOVA) (vs. blank control group) | Number of animals per group (number of deaths) |
|---|---|---|---|---|---|---|---|
| | | | D0 | D30 | | | |
| Blank control (PBS) | One dose | Intraperitoneal injection | 151.82 | 678.60 | / | / | 10(0) |
| IgG1-9-A 6mpk | One dose | Intraperitoneal injection | 152.47 | 751.50 | -13.71 | ns | 10(0) |
| M4H1L1-9-A 3mpk | One dose | Intraperitoneal injection | 149.97 | 149.60 | 100.08 | <0.0001 | 10(0) |
| ZalH4-9-A 3mpk | One dose | Intraperitoneal injection | 154.70 | 38.30 | 122.10 | <0.0001 | 10(0) |
| ADC-2 3mpk | One dose | Intraperitoneal injection | 149.65 | 105.70 | 108.35 | <0.0001 | 10(0) |
| ADC-2 4.5mpk | One dose | Intraperitoneal injection | 148.79 | 24.60 | 123.58 | <0.0001 | 10(0) |
| ADC-1 1.5mpk | One dose | Intraperitoneal injection | 148.95 | 240.00 | 82.71 | <0.0001 | 10(0) |
| ADC-1 3mpk | One dose | Intraperitoneal injection | 147.73 | 42.30 | 120.82 | <0.0001 | 10(0) |
| ADC-1 6mpk | One dose | Intraperitoneal injection | 152.67 | 37.70 | 121.83 | <0.0001 | 10(0) |

| | | | | | | | |
|---|---|---|---|---|---|---|---|
| Note: D0 represents the day of grouping, D1 represents the first day, and D9 represents the ninth day; / means that it cannot be calculated; ns means that there is no statistical significance, and the same applies hereinafter. | | | | | | | |

The results show that the anti-EGFR-MUC1 bispecific antibody **ADC-1** of the present disclosure exhibited a relatively good tumor growth inhibition rate in the HCC827 CDX model, and that its tumor growth inhibition rate was comparable to that of the same dose of the EGFR monoclonal antibody ADC and superior to that of the same dose of the MUC1 monoclonal antibody ADC.

### Test Example 19: Efficacy of ADCs in In Vivo HPAC CDX Model

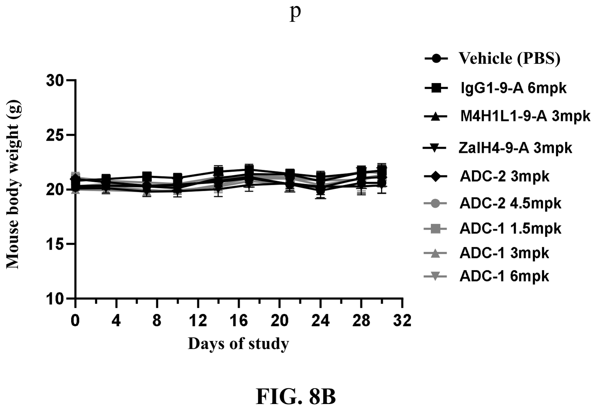

1. Objective: The objective of this experiment was to evaluate, after intraperitoneal administration, the *in vivo* efficacy and toxic and side effects of different doses of bispecific antibody ADCs and their corresponding monoclonal antibody ADCs in human pancreatic acinar epithelial carcinoma cell HPAC tumor-bearing mice.
2. Principle: In this experiment, an HPAC tumor-bearing mouse model was constructed using Balb/c nude mice and intraperitoneally given the test molecules, pharmacodynamic evaluations of different molecules were conducted, and the bispecific antibody ADCs and monoclonal antibody ADCs were compared in terms of efficacy.
3. Animals: Balb/c nude mice, purchased from Beijing Vital River Laboratory Animal Technology Co., Ltd.
4. Instruments:
   Benchtop centrifuge: BECKMAN COULTER
   Inverted microscope: NIKON NIKON TS100
   Carbon dioxide incubator: Thermo
   Refrigeration freezer: BCD-610WKM, Hefei Midea Refrigerator Co., Ltd.
   Electronic balance: PL2001-L Mettler-Toledo Instruments (Shanghai) Co., Ltd. Biosafety cabinet: ESCO AC2-4S1
5. Steps: 200 µL of human pancreatic acinar epithelial carcinoma (HPAC) cells (1 × 10⁶ cells) (containing 50% matrigel) was subcutaneously inoculated into the right flank of each of 100 female nude mice (purchased from Beijing Vital River Laboratory Animal Technology Co., Ltd.). After 7 days, their tumors grew to about 175.48 ± 17.18 mm³. Then, the mice that were too large in body weight or tumor size and those that were too small in body weight or tumor size were excluded, and the remaining mice were randomly divided into 9 groups of 8 according to their tumor volume (D0): a blank control (PBS) group, a negative control group (IgG1-9-A), an MUC1 monoclonal antibody ADC group (M4H1L1-9-A), an EGFR monoclonal antibody ADC group (ZalH4-9-A), EGFR-MUC1 bispecific antibody ADC-1 groups, and ADC-2 groups. The ADCs were intraperitoneally injected on D1 and D9, two times in total. The tumor volume and body weight of the animals were measured twice a week, and the measurements were recorded.
6. Data processing: The tumor volume of the animals in each group was expressed as mean + standard error of the mean (Mean ± SEM) and plotted using Graphpad Prism 10 software. Statistical analysis was performed using two/one-way ANOVA, and the tumor growth inhibition rate was calculated using the formula: tumor proliferation rate (T/C %) = (T - T0 / C - C0) × 100%.
7. Experimental results: Thirty days after the administration, the tumor growth inhibition rate of the negative control group (6 mpk) was 7.22% (ns vs. blank control); the tumor growth inhibition rate of the MUC1 monoclonal antibody ADC (3 mpk) was 88.17% (P < 0.0001 vs. blank control); the tumor growth inhibition rate of the EGFR monoclonal antibody ADC (3 mpk) was 50.90% (P < 0.05 vs. blank control); the tumor growth inhibition rates of EGFR-MUC1 bispecific antibody ADC-2 (3 mpk/4.5 mpk) were 59.23% (P < 0.001 vs. blank control) and 89.18% (P < 0.0001 vs. blank control), respectively; the tumor growth inhibition rates of EGFR-MUC1 bispecific antibody ADC-1 (1.5 mpk/3 mpk/6 mpk) were 48.44% (P < 0.05 vs. vehicle), 80.57% (P < 0.0001 vs. blank control), and 98.52% (P < 0.0001 vs. blank control), respectively. The results are shown in Table 35 and FIGs. 8A to 8B.

**Table 35. The efficacy of ADCs in the in vivo HPAC CDX model (analysis using day-30 data)**

| Sample | Frequency of administration | Route of administration | Mean tumor volume (mm³) | | Tumor growth inhibition rate (%) D30 | P (2-way ANOVA) (vs. blank control group) | Number of animals per group (number of deaths) |
|---|---|---|---|---|---|---|---|
| | | | D0 | D30 | | | |
| Blank control (PBS) | D1 D9 | Intraperitoneal injection | 176.87 | 1678.68 | / | / | 8(0) |
| IgG1-9-A 6mpk | D1 D9 | Intraperitoneal injection | 173.00 | 1566.45 | 7.22 | ns | 8(0) |
| M4H1L1-9-A 3mpk | D1 D9 | Intraperitoneal injection | 174.47 | 352.07 | 88.17 | <0.0001 | 8(0) |
| ZalH4-9-A 3mpk | D1 D9 | Intraperitoneal injection | 174.14 | 911.47 | 50.90 | <0.05 | 8(0) |
| ADC-2 3mpk | D1 D9 | Intraperitoneal injection | 176.77 | 789.02 | 59.23 | <0.001 | 8(0) |
| ADC-2 4.5mpk | D1 D9 | Intraperitoneal injection | 176.89 | 339.34 | 89.18 | <0.0001 | 8(0) |
| ADC-1 1.5mpk | D1 D9 | Intraperitoneal injection | 176.36 | 950.72 | 48.44 | <0.05 | 8(0) |
| ADC-1 3mpk | D1 D9 | Intraperitoneal injection | 173.97 | 465.73 | 80.57 | <0.0001 | 8(0) |
| ADC-1 6mpk | D1 D9 | Intraperitoneal injection | 176.83 | 198.98 | 98.52 | <0.0001 | 8(0) |

| | | | | | | | |
|---|---|---|---|---|---|---|---|
| Note: D0 represents the day of grouping, D1 represents the first day, and D9 represents the ninth day; / means that it cannot be calculated. | | | | | | | |

The results show that the anti-EGFR-MUC1 bispecific antibody ADC-1 of the present disclosure exhibited a relatively good tumor growth inhibition rate in the HPAC CDX model and demonstrated dose dependence. Its tumor growth inhibition rate was comparable to that of the same dose of the MUC1 monoclonal antibody ADC and superior to that of the same dose of the EGFR monoclonal antibody ADC.

## Claims

1. An anti-MUC1 antibody or an antigen-binding fragment thereof, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. the HCDR1, HCDR2, and HCDR3 of the heavy chain variable region comprise the amino acid sequences of a HCDR1, a HCDR2, and a HCDR3 in SEQ ID NO: 4, respectively, and the LCDR1, LCDR2, and LCDR3 of the light chain variable region comprise the amino acid sequences of a LCDR1, a LCDR2, and a LCDR3 in SEQ ID NO: 5, respectively; or
b. the HCDR1, HCDR2, and HCDR3 of the heavy chain variable region comprise the amino acid sequences of a HCDR1, a HCDR2, and a HCDR3 of any one of SEQ ID NO: 6 or 47, respectively, and the LCDR1, LCDR2, and LCDR3 of the light chain variable region comprise the amino acid sequences of a LCDR1, a LCDR2, and a LCDR3 in SEQ ID NO: 7, respectively; or
c. the HCDR1, HCDR2, and HCDR3 of the heavy chain variable region comprise the amino acid sequences of a HCDR1, a HCDR2, and a HCDR3 in SEQ ID NO: 8, respectively, and the LCDR1, LCDR2, and LCDR3 of the light chain variable region comprise the amino acid sequences of a LCDR1, a LCDR2, and a LCDR3 in SEQ ID NO: 9, respectively; or
d. the HCDR1, HCDR2, and HCDR3 of the heavy chain variable region comprise the amino acid sequences of a HCDR1, a HCDR2, and a HCDR3 of any one of SEQ ID NO: 63, 10, or 64, respectively, and the LCDR1, LCDR2, and LCDR3 of the light chain variable region comprise the amino acid sequences of a LCDR1, a LCDR2, and a LCDR3 in SEQ ID NO: 11, respectively;
preferably,
a. the HCDR1, HCDR2, and HCDR3 of the heavy chain variable region comprise the amino acid sequences of a HCDR1, a HCDR2, and a HCDR3 in SEQ ID NO: 4, respectively, and the LCDR1, LCDR2, and LCDR3 of the light chain variable region comprise the amino acid sequences of a LCDR1, a LCDR2, and a LCDR3 in SEQ ID NO: 5, respectively; or
b. the HCDR1, HCDR2, and HCDR3 of the heavy chain variable region comprise the amino acid sequences of a HCDR1, a HCDR2, and a HCDR3 in SEQ ID NO: 6, respectively, and the LCDR1, LCDR2, and LCDR3 of the light chain variable region comprise the amino acid sequences of a LCDR1, a LCDR2, and a LCDR3 in SEQ ID NO: 7, respectively; or
c. the HCDR1, HCDR2, and HCDR3 of the heavy chain variable region comprise the amino acid sequences of a HCDR1, a HCDR2, and a HCDR3 in SEQ ID NO: 8, respectively, and the LCDR1, LCDR2, and LCDR3 of the light chain variable region comprise the amino acid sequences of a LCDR1, a LCDR2, and a LCDR3 in SEQ ID NO: 9, respectively; or
d. the HCDR1, HCDR2, and HCDR3 of the heavy chain variable region comprise the amino acid sequences of a HCDR1, a HCDR2, and a HCDR3 in SEQ ID NO: 63, respectively, and the LCDR1, LCDR2, and LCDR3 of the light chain variable region comprise the amino acid sequences of a LCDR1, a LCDR2, and a LCDR3 in SEQ ID NO: 11, respectively;
more preferably, the HCDR1, HCDR2, and HCDR3 of the heavy chain variable region comprise the amino acid sequences of a HCDR1, a HCDR2, and a HCDR3 in SEQ ID NO: 4, respectively, and the LCDR1, LCDR2, and LCDR3 of the light chain variable region comprise the amino acid sequences of a LCDR1, a LCDR2, and a LCDR3 in SEQ ID NO: 5, respectively.

2. The anti-MUC1 antibody or the antigen-binding fragment thereof according to claim 1, wherein:
a. in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17; or
b. in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of any one of SEQ ID NO: 20 or 113; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23; or
c. in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29; or
d. in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of any one of SEQ ID NO: 114, 32, or 115; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35;
preferably,
a. in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17;
b. in the heavy chain variable region, the HCDR1 comprises the amino acid sequence of SEQ ID NO: 18, the HCDR2 comprises the amino acid sequence of SEQ ID NO: 19, and the HCDR3 comprises the amino acid sequence of SEQ ID NO: 20; and in the light chain variable region, the LCDR1 comprises the amino acid sequence of SEQ ID NO: 21, the LCDR2 comprises the amino acid sequence of SEQ ID NO: 22, and the LCDR3 comprises the amino acid sequence of SEQ ID NO: 23; or
c. in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29; or
d. in the heavy chain variable region, the HCDR1 comprises the amino acid sequence of SEQ ID NO: 30, the HCDR2 comprises the amino acid sequence of SEQ ID NO: 31, and the HCDR3 comprises the amino acid sequence of SEQ ID NO: 114; and in the light chain variable region, the LCDR1 comprises the amino acid sequence of SEQ ID NO: 33, the LCDR2 comprises the amino acid sequence of SEQ ID NO: 34, and the LCDR3 comprises the amino acid sequence of SEQ ID NO: 35;
more preferably, in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, the LCDR2 comprises the amino acid sequence of SEQ ID NO: 16, and the LCDR3 comprises the amino acid sequence of SEQ ID NO: 17.

3. The anti-MUC1 antibody or the antigen-binding fragment thereof according to claim 1 or 2, being a murine antibody, a chimeric antibody, a humanized antibody, or a fully human-derived antibody, preferably a chimeric antibody or a humanized antibody, and more preferably a humanized antibody.

4. The anti-MUC1 antibody or the antigen-binding fragment thereof according to any one of claims 1 to 3, wherein:
a. the heavy chain variable region comprises an amino acid sequence having at least 70% sequence identity to SEQ ID NO: 36, 37, or 38, and the light chain variable region comprises an amino acid sequence having at least 70% sequence identity to SEQ ID NO: 39, 40, 41, or 42; or the heavy chain variable region comprises an amino acid sequence having at least 70% sequence identity to SEQ ID NO: 4, and the light chain variable region comprises an amino acid sequence having at least 70% sequence identity to SEQ ID NO: 5; or
b. the heavy chain variable region comprises an amino acid sequence having at least 70% sequence identity to SEQ ID NO: 43, 44, 45, 46, 47, 48, or 49, and the light chain variable region comprises an amino acid sequence having at least 70% sequence identity to SEQ ID NO: 50, 51, or 52; or the heavy chain variable region comprises an amino acid sequence having at least 70% sequence identity to SEQ ID NO: 6, and the light chain variable region comprises an amino acid sequence having at least 70% sequence identity to SEQ ID NO: 7; or
c. the heavy chain variable region comprises an amino acid sequence having at least 70% sequence identity to SEQ ID NO: 53, 54, or 55, and the light chain variable region comprises an amino acid sequence having at least 70% sequence identity to SEQ ID NO: 56, 57, 58, or 59; or the heavy chain variable region comprises an amino acid sequence having at least 70% sequence identity to SEQ ID NO: 8, and the light chain variable region comprises an amino acid sequence having at least 70% sequence identity to SEQ ID NO: 9; or
d. the heavy chain variable region comprises an amino acid sequence having at least 70% sequence identity to SEQ ID NO: 63, 60, 61, 62, or 64, and the light chain variable region comprises an amino acid sequence having at least 70% sequence identity to SEQ ID NO: 67, 68, 65, or 66; or the heavy chain variable region comprises an amino acid sequence having at least 70% sequence identity to SEQ ID NO: 10, and the light chain variable region comprises an amino acid sequence having at least 70% sequence identity to SEQ ID NO: 11;
preferably,
a. the heavy chain variable region comprises the amino acid sequence of SEQ ID NO: 36, and the light chain variable region comprises the amino acid sequence of SEQ ID NO: 39 or 40; or
the heavy chain variable region comprises the amino acid sequence of SEQ ID NO: 37, and the light chain variable region comprises the amino acid sequence of SEQ ID NO: 39 or 40; or
the heavy chain variable region comprises the amino acid sequence of SEQ ID NO: 4, and the light chain variable region comprises the amino acid sequence of SEQ ID NO: 5;
b. the heavy chain variable region comprises the amino acid sequence of SEQ ID NO: 43, and the light chain variable region comprises the amino acid sequence of SEQ ID NO: 50; or
the heavy chain variable region comprises the amino acid sequence of SEQ ID NO: 44, and the light chain variable region comprises the amino acid sequence of SEQ ID NO: 51; or
the heavy chain variable region comprises the amino acid sequence of SEQ ID NO: 45, and the light chain variable region comprises the amino acid sequence of SEQ ID NO: 50 or 51; or
the heavy chain variable region comprises the amino acid sequence of SEQ ID NO: 46, and the light chain variable region comprises the amino acid sequence of SEQ ID NO: 51; or
the heavy chain variable region comprises the amino acid sequence of SEQ ID NO: 6, and the light chain variable region comprises the amino acid sequence of SEQ ID NO: 7;
c. the heavy chain variable region comprises the amino acid sequence of SEQ ID NO: 53, and the light chain variable region comprises the amino acid sequence of SEQ ID NO: 56, 57, or 59; or
the heavy chain variable region comprises the amino acid sequence of SEQ ID NO: 54, and the light chain variable region comprises the amino acid sequence of SEQ ID NO: 57; or
the heavy chain variable region comprises the amino acid sequence of SEQ ID NO: 8, and the light chain variable region comprises the amino acid sequence of SEQ ID NO: 9;
d. the heavy chain variable region comprises the amino acid sequence of SEQ ID NO: 60, and the light chain variable region comprises the amino acid sequence of SEQ ID NO: 65 or 68; or
the heavy chain variable region comprises the amino acid sequence of SEQ ID NO: 61, and the light chain variable region comprises the amino acid sequence of SEQ ID NO: 65, 66, 67, or 68; or
the heavy chain variable region comprises the amino acid sequence of SEQ ID NO: 63, and the light chain variable region comprises the amino acid sequence of SEQ ID NO: 67 or 68; or
the heavy chain variable region comprises the amino acid sequence of SEQ ID NO: 10, and the light chain variable region comprises the amino acid sequence of SEQ ID NO: 11;
more preferably, the heavy chain variable region comprises the amino acid sequence of SEQ ID NO: 36, and the light chain variable region comprises the amino acid sequence of SEQ ID NO: 39.

5. The anti-MUC1 antibody or the antigen-binding fragment thereof according to any one of claims 1 to 4, being an antibody fragment, wherein preferably, the antibody fragment is selected from the group consisting of Fab, Fab', F(ab')2, Fd, Fv, scFv, dsFv, and dAb.

6. The anti-MUC1 antibody or the antigen-binding fragment thereof according to any one of claims 1 to 4, wherein the anti-MUC1 antibody comprises a heavy chain constant region and a light chain constant region;
preferably, the heavy chain constant region is a human IgG1, IgG2, IgG3, or IgG4 heavy chain constant region, and
the light chain constant region is a human κ or λ light chain constant region;
more preferably, the heavy chain constant region comprises the amino acid sequence of SEQ ID NO: 69 or 186, and
the light chain constant region comprises the amino acid sequence of SEQ ID NO: 70.

7. The anti-MUC1 antibody or the antigen-binding fragment thereof according to claim 6, wherein the anti-MUC1 antibody or the antigen-binding fragment thereof comprises a heavy chain and a light chain, wherein:
a. the heavy chain comprises an amino acid sequence having at least 70% sequence identity to SEQ ID NO: 71, 73, 75, or 77, and the light chain comprises an amino acid sequence having at least 70% sequence identity to SEQ ID NO: 72, 74, 76, or 78; or
b. the heavy chain comprises an amino acid sequence having at least 70% sequence identity to SEQ ID NO: 79, 81, 83, 85, or 87, and the light chain comprises an amino acid sequence having at least 70% sequence identity to SEQ ID NO: 80, 82, 84, 86, or 88; or
c. the heavy chain comprises an amino acid sequence having at least 70% sequence identity to SEQ ID NO: 89, 91, 93, or 95, and the light chain comprises an amino acid sequence having at least 70% sequence identity to SEQ ID NO: 90, 92, 94, or 96; or
d. the heavy chain comprises an amino acid sequence having at least 70% sequence identity to SEQ ID NO: 97, 99, 101, 103, 105, 107, 109, or 111, and the light chain comprises an amino acid sequence having at least 70% sequence identity to SEQ ID NO: 98, 100, 102, 104, 106, 108, 110, or 112;
preferably,
a. the amino acid sequence of the heavy chain is set forth in SEQ ID NO: 71, and the amino acid sequence of the light chain is set forth in SEQ ID NO: 72; or
the amino acid sequence of the heavy chain is set forth in SEQ ID NO: 73, and the amino acid sequence of the light chain is set forth in SEQ ID NO: 74; or
the amino acid sequence of the heavy chain is set forth in SEQ ID NO: 75, and the amino acid sequence of the light chain is set forth in SEQ ID NO: 76; or
the amino acid sequence of the heavy chain is set forth in SEQ ID NO: 77, and the amino acid sequence of the light chain is set forth in SEQ ID NO: 78;
b. the amino acid sequence of the heavy chain is set forth in SEQ ID NO: 79, and the amino acid sequence of the light chain is set forth in SEQ ID NO: 80; or
the amino acid sequence of the heavy chain is set forth in SEQ ID NO: 81, and the amino acid sequence of the light chain is set forth in SEQ ID NO: 82; or
the amino acid sequence of the heavy chain is set forth in SEQ ID NO: 83, and the amino acid sequence of the light chain is set forth in SEQ ID NO: 84; or
the amino acid sequence of the heavy chain is set forth in SEQ ID NO: 85, and the amino acid sequence of the light chain is set forth in SEQ ID NO: 86; or
the amino acid sequence of the heavy chain is set forth in SEQ ID NO: 87, and the amino acid sequence of the light chain is set forth in SEQ ID NO: 88;
c. the amino acid sequence of the heavy chain is set forth in SEQ ID NO: 89, and the amino acid sequence of the light chain is set forth in SEQ ID NO: 90; or
the amino acid sequence of the heavy chain is set forth in SEQ ID NO: 91, and the amino acid sequence of the light chain is set forth in SEQ ID NO: 92; or
the amino acid sequence of the heavy chain is set forth in SEQ ID NO: 93, and the amino acid sequence of the light chain is set forth in SEQ ID NO: 94; or
the amino acid sequence of the heavy chain is set forth in SEQ ID NO: 95, and the amino acid sequence of the light chain is set forth in SEQ ID NO: 96;
d. the amino acid sequence of the heavy chain is set forth in SEQ ID NO: 97, and the amino acid sequence of the light chain is set forth in SEQ ID NO: 98; or
the amino acid sequence of the heavy chain is set forth in SEQ ID NO: 99, and the amino acid sequence of the light chain is set forth in SEQ ID NO: 100; or
the amino acid sequence of the heavy chain is set forth in SEQ ID NO: 101, and the amino acid sequence of the light chain is set forth in SEQ ID NO: 102; or
the amino acid sequence of the heavy chain is set forth in SEQ ID NO: 103, and the amino acid sequence of the light chain is set forth in SEQ ID NO: 104; or
the amino acid sequence of the heavy chain is set forth in SEQ ID NO: 105, and the amino acid sequence of the light chain is set forth in SEQ ID NO: 106; or
the amino acid sequence of the heavy chain is set forth in SEQ ID NO: 107, and the amino acid sequence of the light chain is set forth in SEQ ID NO: 108; or
the amino acid sequence of the heavy chain is set forth in SEQ ID NO: 109, and the amino acid sequence of the light chain is set forth in SEQ ID NO: 110; or
the amino acid sequence of the heavy chain is set forth in SEQ ID NO: 111, and the amino acid sequence of the light chain is set forth in SEQ ID NO: 112;
more preferably, the amino acid sequence of the heavy chain is set forth in SEQ ID NO: 71, and the amino acid sequence of the light chain is set forth in SEQ ID NO: 72.

8. An anti-EGFR antibody or an antigen-binding fragment thereof, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein:
a. in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129, 128, 130, or 131; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
b. in the heavy chain variable region, the HCDR1 comprises the amino acid sequence of SEQ ID NO: 126 or 133, the HCDR2 comprises the amino acid sequence of SEQ ID NO: 117, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
c. in the heavy chain variable region, the HCDR1 comprises the amino acid sequence of SEQ ID NO: 116, the HCDR2 comprises the amino acid sequence of SEQ ID NO: 127, 132, or 134, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
d. in the heavy chain variable region, the HCDR1 comprises the amino acid sequence of SEQ ID NO: 126, the HCDR2 comprises the amino acid sequence of SEQ ID NO: 127, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121;
e. in the heavy chain variable region, the HCDR1 comprises the amino acid sequence of SEQ ID NO: 126, the HCDR2 comprises the amino acid sequence of SEQ ID NO: 117, and the HCDR3 comprises the amino acid sequence of SEQ ID NO: 128, 129, 130, or 131; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121;
preferably,
a. in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
b. in the heavy chain variable region, the HCDR1 comprises the amino acid sequence of SEQ ID NO: 126, the HCDR2 comprises the amino acid sequence of SEQ ID NO: 117, and the HCDR3 comprises the amino acid sequence of SEQ ID NO: 128 or 130; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121; or
c. in the heavy chain variable region, the HCDR1 comprises the amino acid sequence of SEQ ID NO: 133, the HCDR2 comprises the amino acid sequence of SEQ ID NO: 117, and the HCDR3 comprises the amino acid sequence of SEQ ID NO: 118; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121;
more preferably,
in the heavy chain variable region, the HCDR1 comprises the amino acid sequence of SEQ ID NO: 116, the HCDR2 comprises the amino acid sequence of SEQ ID NO: 117, and the HCDR3 comprises the amino acid sequence of SEQ ID NO: 129; and in the light chain variable region, the LCDR1 comprises the amino acid sequence of SEQ ID NO: 119, the LCDR2 comprises the amino acid sequence of SEQ ID NO: 120, and the LCDR3 comprises the amino acid sequence of SEQ ID NO: 121.

9. The anti-EGFR antibody or the antigen-binding fragment thereof according to claim 8, wherein:
the heavy chain variable region comprises the amino acid sequence of SEQ ID NO: 138, 135, 136, 137, 139, 140, 141, 142, 143, 144, 145, 146, 147, or 148, and the light chain variable region comprises the amino acid sequence of SEQ ID NO: 149;
preferably, the heavy chain variable region comprises the amino acid sequence of SEQ ID NO: 138, 142, 144, or 147, and the light chain variable region comprises the amino acid sequence of SEQ ID NO: 149;
more preferably, the heavy chain variable region comprises the amino acid sequence of SEQ ID NO: 138, and the light chain variable region comprises the amino acid sequence of SEQ ID NO: 149.

10. The anti-EGFR antibody or the antigen-binding fragment thereof according to claim 9, being an antibody fragment, wherein preferably, the antibody fragment is selected from the group consisting of Fab, Fab', F(ab')2, Fd, Fv, scFv, dsFv, and dAb.

11. The anti-EGFR antibody or the antigen-binding fragment thereof according to claim 9, wherein the anti-EGFR antibody comprises a heavy chain constant region and a light chain constant region;
preferably, the heavy chain constant region is a human IgG1, IgG2, IgG3, or IgG4 heavy chain constant region, and
the light chain constant region is a human κ or λ light chain constant region;
more preferably, the heavy chain constant region comprises the amino acid sequence of SEQ ID NO: 69 or 186, and
the light chain constant region comprises the amino acid sequence of SEQ ID NO: 70.

12. The anti-EGFR antibody or the antigen-binding fragment thereof according to claim 11, comprising a heavy chain and a light chain, wherein:
the heavy chain comprises the amino acid sequence of SEQ ID NO: 153, 150, 151, 152, 154, 155, 156, 157, 158, 159, 160, 161, 162, or 163, and the light chain comprises the amino acid sequence of SEQ ID NO: 164;
preferably, the heavy chain comprises the amino acid sequence of SEQ ID NO: 153, 157, 159, or 162, and the light chain comprises the amino acid sequence of SEQ ID NO: 164;
more preferably, the heavy chain comprises the amino acid sequence of SEQ ID NO: 153, and the light chain comprises the amino acid sequence of SEQ ID NO: 164.

13. An antigen-binding molecule that specifically binds to EGFR and MUC1, comprising:
at least one antigen-binding moiety that specifically binds to EGFR, and
at least one antigen-binding moiety that specifically binds to MUC1, wherein:
the antigen-binding moiety that specifically binds to EGFR comprises a heavy chain variable region EGFR-VH and a light chain variable region EGFR-VL, and
the antigen-binding moiety that specifically binds to MUC1 comprises a heavy chain variable region MUC1-VH and a light chain variable region MUC1-VL, wherein:
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, 128, 130, or 131, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126 or 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 116, a HCDR2 comprising the amino acid sequence of SEQ ID NO: 127, 132, or 134, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
d. the EGFR-VH comprises a HCDR1 comprising the amino acid sequence of SEQ ID NO: 126, a HCDR2 comprising the amino acid sequence of SEQ ID NO: 127, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
e. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128, 129, 130, or 131, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
preferably,
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128 or 130, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
more preferably,
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121.

14. The antigen-binding molecule that specifically binds to EGFR and MUC1 according to claim 13, wherein:
the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and
the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17.

15. The antigen-binding molecule that specifically binds to EGFR and MUC1 according to claim 14, wherein:
the EGFR-VH comprises the amino acid sequence of SEQ ID NO: 138, 135, 136, 137, 139, 140, 141, 142, 143, 144, 145, 146, 147, or 148, and the EGFR-VL comprises the amino acid sequence of SEQ ID NO: 149;
preferably, the EGFR-VH comprises the amino acid sequence of SEQ ID NO: 138, 142, 144, or 147, and the EGFR-VL comprises the amino acid sequence of SEQ ID NO: 149;
more preferably, the EGFR-VH comprises the amino acid sequence of SEQ ID NO: 138, and the EGFR-VL comprises the amino acid sequence of SEQ ID NO: 149.

16. The antigen-binding molecule that specifically binds to EGFR and MUC1 according to claim 15, wherein the MUC1-VH comprises the amino acid sequence of SEQ ID NO: 36, and the MUC1-VL comprises the amino acid sequence of SEQ ID NO: 39.

17. The antigen-binding molecule that specifically binds to EGFR and MUC1 according to any one of claims 13 to 16, wherein the antigen-binding moiety that specifically binds to EGFR or the antigen-binding moiety that specifically binds to MUC1 independently comprises a Titin chain and an Obscurin chain that are capable of forming a dimer;
preferably, the Titin chain comprises the amino acid sequence of SEQ ID NO: 165, and the Obscurin chain comprises the amino acid sequence of SEQ ID NO: 166.

18. The antigen-binding molecule that specifically binds to EGFR and MUC1 according to any one of claims 13 to 17, further comprising an Fc region, wherein:
preferably, the Fc region is an IgG Fc region;
further preferably, the Fc region is an IgG1 Fc region;
more preferably, the Fc region comprises one or more amino acid substitutions capable of reducing the binding of the Fc region to an Fcγ receptor.

19. The antigen-binding molecule that specifically binds to EGFR and MUC1 according to claim 18, comprising an Fc region, wherein the Fc region comprises a first subunit Fc1 and a second subunit Fc2 that are capable of associating with each other; the Fc1 and Fc2 each independently comprise one or more amino acid substitutions that reduce the homodimerization of the Fc region;
preferably, the Fc1 has a knob structure according to the knob-into-hole technique, and the Fc2 has a hole structure according to the knob-into-hole technique; or, the Fc2 has a knob structure according to the knob-into-hole technique, and the Fc1 has a hole structure according to the knob-into-hole technique;
more preferably, in the Fc1, the amino acid at position 358 is C, and the amino acid at position 370 is W; and in the Fc2, the amino acid at position 357 is C, the amino acid at position 374 is S, the amino acid at position 376 is A, and the amino acid at position 415 is V, as numbered according to the EU index; or, in the Fc2, the amino acid at position 358 is C, and the amino acid at position 370 is W; and in the Fc1, the amino acid at position 357 is C, the amino acid at position 374 is S, the amino acid at position 376 is A, and the amino acid at position 415 is V, as numbered according to the EU index;
most preferably, the Fc1 comprises the amino acid sequence of SEQ ID NO: 169, and the Fc2 comprises the amino acid sequence of SEQ ID NO: 170; or the Fc1 comprises the amino acid sequence of SEQ ID NO: 182, and the Fc2 comprises the amino acid sequence of SEQ ID NO: 183; or, the Fc2 comprises the amino acid sequence of SEQ ID NO: 169, and the Fc1 comprises the amino acid sequence of SEQ ID NO: 170; or the Fc2 comprises the amino acid sequence of SEQ ID NO: 182, and the Fc1 comprises the amino acid sequence of SEQ ID NO: 183.

20. The antigen-binding molecule that specifically binds to EGFR and MUC1 according to any one of claims 13 to 19, comprising:
one antigen-binding moiety that specifically binds to EGFR, and
one antigen-binding moiety that specifically binds to MUC1, wherein:
the antigen-binding moiety that specifically binds to MUC1 is a Fab;
the antigen-binding moiety that specifically binds to EGFR is a replaced Fab comprising a Titin chain and an Obscurin chain that are capable of forming a dimer;
preferably,
the antigen-binding molecule comprises one first chain having a structure represented by formula (a), one second chain having a structure represented by formula (b), one third chain having a structure represented by formula (c), and one fourth chain having a structure represented by formula (d):
formula (a) [MUC1-VH]-[CH1]-[Fc1],
formula (b) [MUC1-VL]-[CL],
formula (c) [EGFR-VH]-[linker 1]-[Titin]-[Fc2], and
formula (d) [EGFR-VL]-[linker 2]-[Obscurin],
wherein:
linker 1 and linker 2 are identical or different and are peptide linkers; or linker 1 or
linker 2 is absent;
the structures represented by formulas (a), (b), (c), and (d) are arranged from the N-terminus to the C-terminus;
further preferably, the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and
a. the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
b. the EGFR-VH comprises a HCDR1 comprising the amino acid sequence of SEQ ID NO: 126, a HCDR2 comprising the amino acid sequence of SEQ ID NO: 117, and a HCDR3 comprising the amino acid sequence of SEQ ID NO: 128 or 130, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121; or
c. the EGFR-VH comprises a HCDR1 comprising the amino acid sequence of SEQ ID NO: 133, a HCDR2 comprising the amino acid sequence of SEQ ID NO: 117, and a HCDR3 comprising the amino acid sequence of SEQ ID NO: 118, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
more preferably,
the MUC1-VH comprises the amino acid sequence of SEQ ID NO: 36, and the MUC1-VL comprises the amino acid sequence of SEQ ID NO: 39; and
the EGFR-VH comprises the amino acid sequence of SEQ ID NO: 138, 142, 144, or 147, and the EGFR-VL comprises the amino acid sequence of SEQ ID NO: 149;
most preferably, the antigen-binding molecule comprises:
one first chain comprising the amino acid sequence of SEQ ID NO: 171,
one second chain comprising the amino acid sequence of SEQ ID NO: 74,
one third chain comprising the amino acid sequence of SEQ ID NO: 174, 172, 175, 176, or 177, and
one fourth chain comprising the amino acid sequence of SEQ ID NO: 173;
or, the antigen-binding molecule comprises:
one first chain comprising the amino acid sequence of SEQ ID NO: 178,
one second chain comprising the amino acid sequence of SEQ ID NO: 74,
one third chain comprising the amino acid sequence of SEQ ID NO: 179, and
one fourth chain comprising the amino acid sequence of SEQ ID NO: 173.

21. The antigen-binding molecule that specifically binds to EGFR and MUC1 according to claim 20, wherein the antigen-binding molecule comprises:
one antigen-binding moiety that specifically binds to EGFR, and
one antigen-binding moiety that specifically binds to MUC1, wherein:
the antigen-binding moiety that specifically binds to MUC1 is a Fab;
the antigen-binding moiety that specifically binds to EGFR is a replaced Fab comprising a Titin chain and an Obscurin chain that are capable of forming a dimer;
preferably,
the antigen-binding molecule comprises one first chain having a structure represented by formula (a), one second chain having a structure represented by formula (b), one third chain having a structure represented by formula (c), and one fourth chain having a structure represented by formula (d):
formula (a) [MUC1-VH]-[CH1]-[Fc1],
formula (b) [MUC1-VL]-[CL],
formula (c) [EGFR-VH]-[linker 1]-[Titin]-[Fc2], and
formula (d) [EGFR-VL]-[linker 2]-[Obscurin],
wherein:
linker 1 and linker 2 are identical or different and are peptide linkers; or linker 1 or
linker 2 is absent;
the structures represented by formulas (a), (b), (c), and (d) are arranged from the N-terminus to the C-terminus;
further preferably, the MUC1-VH comprises a HCDR1 comprising the amino acid sequence of SEQ ID NO: 12, a HCDR2 comprising the amino acid sequence of SEQ ID NO: 13, and a HCDR3 comprising the amino acid sequence of SEQ ID NO: 14, and the MUC1-VL comprises a LCDR1 comprising the amino acid sequence of SEQ ID NO: 15, a LCDR2 comprising the amino acid sequence of SEQ ID NO: 16, and a LCDR3 comprising the amino acid sequence of SEQ ID NO: 17; and
the EGFR-VH comprises a HCDR1 comprising the amino acid sequence of SEQ ID NO: 116, a HCDR2 comprising the amino acid sequence of SEQ ID NO: 117, and a HCDR3 comprising the amino acid sequence of SEQ ID NO: 129, and the EGFR-VL comprises a LCDR1 comprising the amino acid sequence of SEQ ID NO: 119, a LCDR2 comprising the amino acid sequence of SEQ ID NO: 120, and a LCDR3 comprising the amino acid sequence of SEQ ID NO: 121;
more preferably, the MUC1-VH comprises the amino acid sequence of SEQ ID NO: 36, and the MUC1-VL comprises the amino acid sequence of SEQ ID NO: 39; and the EGFR-VH comprises the amino acid sequence of SEQ ID NO: 138, and the EGFR-VL comprises the amino acid sequence of SEQ ID NO: 149;
most preferably, the antigen-binding molecule comprises:
one first chain comprising the amino acid sequence of SEQ ID NO: 171,
one second chain comprising the amino acid sequence of SEQ ID NO: 74,
one third chain comprising the amino acid sequence of SEQ ID NO: 174, and
one fourth chain comprising the amino acid sequence of SEQ ID NO: 173;
or the antigen-binding molecule comprises:
one first chain comprising the amino acid sequence of SEQ ID NO: 178,
one second chain comprising the amino acid sequence of SEQ ID NO: 74,
one third chain comprising the amino acid sequence of SEQ ID NO: 179, and
one fourth chain comprising the amino acid sequence of SEQ ID NO: 173.

22. An immunoconjugate, comprising:
the anti-MUC1 antibody or the antigen-binding fragment thereof according to any one of claims 1 to 7 and an effector, or
the anti-EGFR antibody or the antigen-binding fragment thereof according to any one of claims 8 to 12 and an effector, or
the antigen-binding molecule that specifically binds to EGFR and MUC1 according to any one of claims 13 to 21 and an effector,
wherein the effector is coupled to the anti-MUC1 antibody or the antigen-binding fragment thereof, the anti-EGFR antibody or the antigen-binding fragment thereof, or
the antigen-binding molecule that specifically binds to EGFR and MUC1;
preferably, the effector is selected from the group consisting of an anti-tumor agent, an immunomodulator, a biological response modifier, a lectin, a cytotoxic drug, a chromophore, a fluorophore, a chemiluminescent compound, an enzyme, and a metal ion, and any combination thereof.

23. An antibody-drug conjugate represented by general formula (Pc-L-Y-D) or a pharmaceutically acceptable salt thereof, wherein: 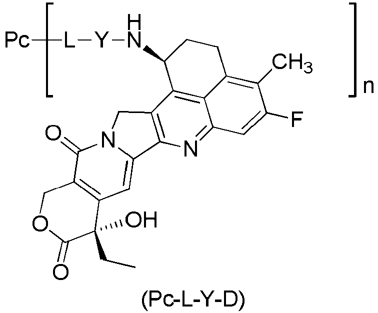
Y is selected from the group consisting of -O-(CR^{a}R^{b})ₘ-CR¹R²-C(O)-, -O-CR¹R²-(CR^{a}R^{b})ₘ-, -O-CR¹R²-, -NH-(CR^{a}R^{b})ₘ-CR¹R²-C(O)-, and -S-(CR^{a}R^{b})ₘ-CR¹R²-C(O)-;
R^{a} and R^{b} are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, heterocyclyl, aryl, and heteroaryl;
or, R^{a} and R^{b}, together with the carbon atom to which they are attached, form cycloalkyl, heterocyclyl, aryl, or heteroaryl;
R¹ is selected from the group consisting of halogen, alkyl, haloalkyl, deuterated alkyl, hydroxy, alkoxy, cyano, amino, cycloalkyl, cycloalkylalkyl, alkoxyalkyl, heterocyclyl, aryl, and heteroaryl;
R² is selected from the group consisting of hydrogen, deuterium, halogen, alkyl, haloalkyl, deuterated alkyl, hydroxy, alkoxy, cyano, amino, cycloalkyl, cycloalkylalkyl, alkoxyalkyl, heterocyclyl, aryl, and heteroaryl;
or, R¹ and R², together with the carbon atom to which they are attached, form cycloalkyl, heterocyclyl, aryl, or heteroaryl;
or, R^{a} and R², together with the carbon atoms to which they are attached, form cycloalkyl, heterocyclyl, aryl, or heteroaryl;
m is 0, 1, 2, 3, or 4;
n is 1 to 10;
L is a linker unit;
Pc is the anti-MUC1 antibody or the antigen-binding fragment thereof according to any one of claims 1 to 7, or the anti-EGFR antibody or the antigen-binding fragment thereof according to any one of claims 8 to 12, or the antigen-binding molecule that specifically binds to EGFR and MUC1 according to any one of claims 13 to 21;
preferably, Pc is the antigen-binding molecule that specifically binds to EGFR and MUC1 according to any one of claims 13 to 21.

24. The antibody-drug conjugate represented by general formula (Pc-L-Y-D) or the pharmaceutically acceptable salt thereof according to claim 23, wherein:
Y is
-O-(CR^{a}R^{b})ₘ-CR¹R²-C(O)-,
wherein R^{a} and R^{b} are identical or different and are each independently selected from the group consisting of hydrogen, deuterium, halogen, and alkyl;
R¹ is cycloalkyl-alkyl or cycloalkyl;
R² is selected from the group consisting of hydrogen, haloalkyl, and cycloalkyl;
or, R¹ and R², together with the carbon atom to which they are attached, form cycloalkyl;
m is 0, 1, 2, 3, or 4;
n is 1 to 10;
L is a linker unit;
Pc is the anti-MUC1 antibody or the antigen-binding fragment thereof according to any one of claims 1 to 7, or the anti-EGFR antibody or the antigen-binding fragment thereof according to any one of claims 8 to 12, or the antigen-binding molecule that specifically binds to EGFR and MUC1 according to any one of claims 13 to 21;
preferably, Pc is the antigen-binding molecule that specifically binds to EGFR and MUC1 according to any one of claims 13 to 21.

25. The antibody-drug conjugate represented by general formula (Pc-L-Y-D) or the pharmaceutically acceptable salt thereof according to claim 23 or 24, wherein the linker unit -L- is -L¹-L²-L³-L⁴-,
wherein:
L¹ is selected from the group consisting of -(succinimid-3-yl-*N*)-W-C(O)-, -CH₂-C(O)-NR³-W-C(O)-, and -C(O)-W-C(O)-, and W is selected from the group consisting of alkylene and alkylene-cycloalkyl, wherein the alkylene or alkylene-cycloalkyl is independently and optionally substituted with one or more substituents selected from the group consisting of halogen, hydroxy, cyano, amino, alkyl, haloalkyl, deuterated alkyl, alkoxy, and cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine, glutamic acid, and aspartic acid, and are optionally substituted with one or more substituents selected from the group consisting of halogen, hydroxy, cyano, amino, alkyl, haloalkyl, deuterated alkyl, alkoxy, and cycloalkyl;
L⁴ is selected from the group consisting of -NR⁵(CR⁶R⁷)ₜ-, -C(O)NR⁵-, -C(O)NR⁵(CH₂)ₜ-, and a chemical bond, wherein t is 1, 2, 3, 4, 5, or 6;
R³, R⁴, and R⁵ are identical or different and are each independently selected from the group consisting of hydrogen, alkyl, haloalkyl, deuterated alkyl, and hydroxyalkyl;
R⁶ and R⁷ are identical or different and are each independently selected from the group consisting of hydrogen, halogen, alkyl, haloalkyl, deuterated alkyl, and hydroxyalkyl;
preferably, the linker unit -L¹-L²-L³-L⁴- is as follows:
L¹ is 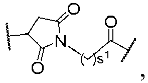 wherein s¹ is 2, 3, 4, 5, 6, 7, or 8;
L² is a chemical bond;
L³ is a tetrapeptide residue; preferably, L³ is a tetrapeptide residue set forth in SEQ ID NO: 180;
L⁴ is -NR⁵(CR⁶R⁷)t-, wherein R⁵, R⁶, or R⁷ is identical or different and is independently hydrogen or alkyl, and t is 1 or 2;
the L¹ terminus of -L- is attached to Pc, and the L⁴ terminus is attached to Y.

26. The antibody-drug conjugate represented by general formula (Pc-L-Y-D) or the pharmaceutically acceptable salt thereof according to any one of claims 23 to 25, being an antibody-drug conjugate represented by general formula (Pc-Lₐ-Y-D) or a pharmaceutically acceptable salt thereof: 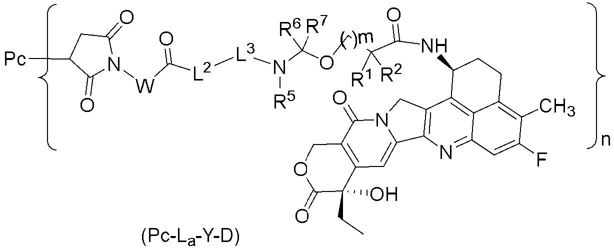 wherein:
Pc is the anti-MUC1 antibody or the antigen-binding fragment thereof according to any one of claims 1 to 7, or the anti-EGFR antibody or the antigen-binding fragment thereof according to any one of claims 8 to 12, or the antigen-binding molecule that specifically binds to EGFR and MUC1 according to any one of claims 13 to 21;
preferably, Pc is the antigen-binding molecule that specifically binds to EGFR and MUC1 according to any one of claims 13 to 21;
m is 0, 1, 2, 3, or 4;
n is 1 to 10;
R¹ is cycloalkyl-alkyl or cycloalkyl;
R² is selected from the group consisting of hydrogen, haloalkyl, and cycloalkyl;
or, R¹ and R², together with the carbon atom to which they are attached, form cycloalkyl;
W is selected from the group consisting of alkylene and alkylene-cycloalkyl, wherein the alkylene and alkylene-cycloalkyl are each independently and optionally substituted with one or more substituents selected from the group consisting of halogen, hydroxy, cyano, amino, alkyl, haloalkyl, deuterated alkyl, alkoxy, and cycloalkyl;
L² is selected from the group consisting of -NR⁴(CH₂CH₂O)p¹CH₂CH₂C(O)-, -NR⁴(CH₂CH₂O)p¹CH₂C(O)-, -S(CH₂)p¹C(O)-, and a chemical bond, wherein p¹ is an integer from 1 to 20;
L³ is a peptide residue consisting of 2 to 7 amino acid residues, wherein the amino acid residues are selected from the group consisting of amino acid residues formed from amino acids from phenylalanine, alanine, glycine, valine, lysine, citrulline, serine,
glutamic acid, and aspartic acid, and are optionally substituted with one or more substituents selected from the group consisting of halogen, hydroxy, cyano, amino, alkyl, haloalkyl, deuterated alkyl, alkoxy, and cycloalkyl;
R⁴ and R⁵ are selected from the group consisting of hydrogen, alkyl, haloalkyl, deuterated alkyl, and hydroxyalkyl;
R⁶ and R⁷ are identical or different and are each independently selected from the group consisting of hydrogen, halogen, alkyl, haloalkyl, deuterated alkyl, and hydroxyalkyl.

27. A method for preparing an antibody-drug conjugate represented by general formula (Pc-Lₐ-Y-D) or a pharmaceutically acceptable salt thereof, comprising the following step: 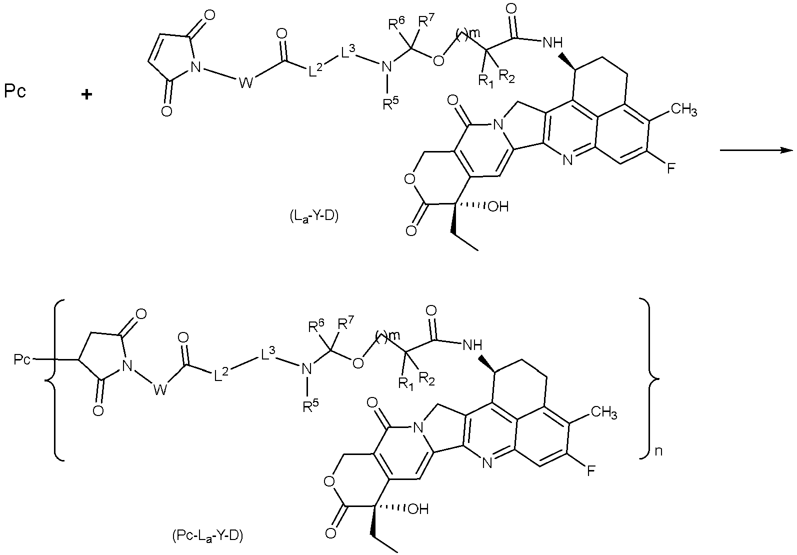 subjecting reduced Pc to a coupling reaction with a compound represented by general formula (Lₐ-Y-D) or a salt thereof to give the antibody-drug conjugate represented by general formula (Pc-Lₐ-Y-D) or the pharmaceutically acceptable salt thereof,
wherein:
Pc is the anti-MUC1 antibody or the antigen-binding fragment thereof according to any one of claims 1 to 7, or the anti-EGFR antibody or the antigen-binding fragment thereof according to any one of claims 8 to 12, or the antigen-binding molecule that specifically binds to EGFR and MUC1 according to any one of claims 13 to 21; preferably, Pc is the antigen-binding molecule that specifically binds to EGFR and MUC1 according to any one of claims 13 to 21;
W, L², L³, R¹, R², R⁵ to R⁷, m, and n are as defined in claim 26.

28. A pharmaceutical composition, comprising:
the anti-MUC1 antibody or the antigen-binding fragment thereof according to any one of claims 1 to 7, the anti-EGFR antibody or the antigen-binding fragment thereof according to any one of claims 8 to 12, the antigen-binding molecule that specifically binds to EGFR and MUC1 according to any one of claims 13 to 21, or the antibody-drug conjugate or the pharmaceutically acceptable salt thereof according to any one of claims 23 to 26, and
one or more pharmaceutically acceptable carriers, diluents, or excipients.

29. An isolated nucleic acid, encoding the anti-MUC1 antibody or the antigen-binding fragment thereof according to any one of claims 1 to 7 or the anti-EGFR antibody or the antigen-binding fragment thereof according to any one of claims 8 to 12.

30. A host cell, comprising the isolated nucleic acid according to claim 29.

31. A method for preventing or treating a disease, comprising a step of:
administering to a subject a prophylactically or therapeutically effective amount of the anti-MUC1 antibody or the antigen-binding fragment thereof according to any one of claims 1 to 7, the anti-EGFR antibody or the antigen-binding fragment thereof according to any one of claims 8 to 12, the antigen-binding molecule that specifically binds to EGFR and MUC1 according to any one of claims 13 to 21, the antibody-drug conjugate or the pharmaceutically acceptable salt thereof according to any one of claims 23 to 26, or the pharmaceutical composition according to claim 28, wherein:
preferably, the disease is a tumor;
more preferably, the disease is selected from the group consisting of astrocytoma, glioblastoma, bladder cancer, bone cancer, brain cancer, breast cancer, cervical cancer, colorectal cancer, fallopian tube cancer, gallbladder cancer, gastric cancer, head and neck cancer, idiopathic myelofibrosis, renal cancer, leukemia, liver cancer, esophageal cancer, lung cancer, medulloblastoma, melanoma, Merkel cell carcinoma, mesothelioma, multiple myeloma, neuroblastoma, oligodendroglioma, ovarian cancer, peritoneal tumor, pancreatic cancer, polycythemia vera, primary neuroectodermal tumor, prostate cancer, retinoblastoma, sarcoma, squamous cell carcinoma, thyroid cancer, endometrial cancer, vestibular schwannoma, blastoma, vulvar cancer, thymoma, testicular cancer, cholangiocarcinoma, pheochromocytoma, paraganglioma, and adenoid cystic carcinoma;
most preferably, the disease is selected from the group consisting of lung cancer, head and neck cancer, esophageal cancer, breast cancer, pancreatic cancer, prostate cancer, thyroid cancer, gastric cancer, ovarian cancer, colorectal cancer, liver cancer, gallbladder cancer, renal cancer, cervical cancer, and bladder cancer.
